# EUROPEAN PATENT SPECIFICATION

(11) **EP 4 680 608 B1**
(45) Date of publication and mention of the grant of the patent: **03.06.2026**
(21) Application number: 25728145.1
(22) Date of filing: 28.05.2025
(51) Int. Cl.: C07D 471/04, C07D 487/04, A61K 31/437, A61P 31/00, A61K 31/5025, A61K 31/519

(54) **2,6-DIHYDRO-7H-PYRAZOLO[3,4-C]PYRIDIN-7-ONE DERIVATIVES AS ANTI-MALARIAL AGENTS**
2,6-DIHYDRO-7H-PYRAZOLO[3,4-C]PYRIDIN-7-ONDERIVATE ALS ANTIMALARIAMITTEL
DÉRIVÉS DE 2,6-DIHYDRO-7H-PYRAZOLO[3,4-C]PYRIDIN-7-ONE COMME AGENTS ANTIPALUDIQUES

(30) Priority: 30.05.2024 US 202463653364 P; 25.06.2024 EP 24184378
(43) Date of publication of application: 21.01.2026
(73) Proprietor: MMV Medicines for Malaria Venture, 1215 Geneva (CH); The Board of Regents of the University of Texas System, Austin, TX 78701 (US)
(72) Inventor: BONNERT, Roger Victor, Loughborough Leicestershire LE127LY (GB); NIE, Zhe, San Diego, California 92127 (US); PHILLIPS, Margaret, DALLAS, Texas 75214 (US); CHARMAN, Susan A., South Melbourne, Victoria, Victoria 3205 (AU); FEHER, Victoria, Richmond, California 94804 (US); HIGGS, Christopher, Oakland, California 94619 (US); PAULSEN, Janet, Camas, Washington 98607 (US); QIN, Tian, Dallas, Texas 75225 (US); PALMER, Michael, Broadstairs Kent CT10 2XA (GB); LALEU, Benoît, 1213 Petit-Lancy (CH)
(74) Representative: reuteler & cie SA
(86) International application number: PCT/EP2025/064743
(87) International publication number: WO 2025/247949

(56) References cited:
- SHARMA MANMOHAN ET AL: "A comprehensive review of synthetic strategies and SAR studies for the discovery of PfDHODH inhibitors as antimalarial agents. Part 1: triazolopyrimidine, isoxazolopyrimidine and pyrrole-based (DSM) compounds", BIOORGANIC CHEMISTRY, ACADEMIC PRESS INC., NEW YORK, NY, US, vol. 146, 3 March 2024 (2024-03-03), XP087504636, ISSN: 0045-2068, [retrieved on 20240303], DOI: 10.1016/J.BIOORG.2024.107249
- GEHLOT PINKY ET AL: "Recent advances on patents of Plasmodium falciparum dihydroorotate dehydrogenase ( Pf DHODH) inhibitors as antimalarial agents", EXPERT OPINION ON THERAPEUTIC PATENTS, vol. 33, no. 9, 2 September 2023 (2023-09-02), GB, pages 579 - 596, XP093223604, ISSN: 1354-3776, DOI: 10.1080/13543776.2023.2280596
- MADHAV HARI ET AL: "An insight into the recent development of the clinical candidates for the treatment of malaria and their target proteins", EUROPEAN JOURNAL OF MEDICINAL CHEMISTRY, ELSEVIER MASSON, AMSTERDAM, NL, vol. 210, 22 October 2020 (2020-10-22), XP086432060, ISSN: 0223-5234, [retrieved on 20201022], DOI: 10.1016/J.EJMECH.2020.112955
- DANIELSON MICHAEL E. ET AL: "Deprotonation and Regioselective Addition of 2 H -Pyrazolo[3,4- c ]quinolines to Electrophiles", vol. 72, no. 12, 1 June 2007 (2007-06-01), United States, pages 4570 - 4573, XP093223368, ISSN: 0022-3263, Retrieved from the Internet <URL:https://pubs.acs.org/doi/pdf/10.1021/jo070392t> DOI: 10.1021/jo070392t

## Description

### Field of the Invention

The present invention relates to anti-malarial agents. Specifically, the present invention is related to agents useful for the preparation of a pharmaceutical formulation for preventing or treating malaria and methods of their use and manufacture.

### Background of the Invention

Malaria remains one of the most widespread and deadly global infectious diseases (WHO. World Malaria Report. 2023). It is caused by single-celled protozoal parasites from the *Plasmodium* genus, and is transmitted to the human host by mosquito vectors *(*Poespoprodjo et al., 2023, Lancet, 402 (10419), 2328-2345. DOI: 10.1016/S0140-6736(23)01249-7*;* Phillips, et al., 2017, Nat Rev Dis Primers, 3, 17050. DOI: 10.1038/nrdp.2017.50*).*

In 2022 the WHO estimated that there were 249 million clinical infections across 85 endemic countries, leading to ∼0.6 million deaths mostly amongst young children living in sub-Saharan Africa. Five species of *Plasmodium* cause malaria, but *Plasmodium falciparum* and *P. vivax* are the most consequential. Most deaths are caused by *P. falciparum,* which is the main species circulating in sub-Saharan Africa, while *P. vivax,* is the dominant parasite in most other endemic countries, and has dormant liver stage that can reactivate the infection if not fully treated.

Malaria treatment and control has relied on chemotherapy for treatment and prevention of malaria as well as vector control, such as the use of insecticide treated bed nets *(Poespoprodjo et al*., *2023, supra; Phillips e al*., *2017, supra).*

Current frontline therapies for treatment of malaria, artemisinin combination therapies (ACTs), provide fast parasite clearance and until recently high efficacy. However, the spread of artemisinin resistance alleles first identified in Southeast Asia is threatening malaria treatment programs. Artemisinin resistance is associated with mutations in the kelch protein (PfK13), has been linked to reduced parasite clearance times, and was followed by the emergence of resistance to partner drugs and treatment failures *(*Dondorp et al., 2009, N Engl J Med, 361 (5), 455-467. DOI: 10.1056/NEJMoa0808859*;* Phyo et al., 2016, Clin Infect Dis, 63 (6), 784-791. DOI: 10.1093/cid/ciw388*).*

Recent studies have found PfK13 resistance alleles have independently arisen in Uganda, leading to partial resistance to artemisinins *(*Conrad et al., 2023 N Engl J Med., 389 (8), 722-732. DOI: 10.1056/NEJMoa2211803*;* Mihreteab et al., 2023, N Engl J Med, 389 (13), 1191-1202. DOI: 10.1056/NEJMoa2210956). While artemisinin combinations with lumefantrine remain effective, the identification of PfK13 mutations in Africa threatens current treatment programs at a time when no new drug candidates are yet approved for clinical use.

Vaccines have been robustly pursued but have been hampered by limited efficacy. Despite these limitations, the WHO has recently recommended two pre-erythrocytic vaccines for prevention of malaria in young children (RTS,S approved 2021 and R21/Matrix M vaccine approved in 2023), and while their efficacy is partial (reduced clinical malaria episodes by 36% over 3-4 years) they have been shown to reduce the frequency and severity of infection in children *(Poespoprodjo et al*., *2023, supra; Phillips e al*., *2017, supra).* Recent studies suggest that their efficacy is improved by co-administration of seasonal malaria chemopreventative agents (sulfadoxine-pyrimethamine (SP) plus amodiaquine) and vice versa *(*Chandramohan etval., 2021, N Engl J Med, 385 (11), 1005-1017. DOI: 10.1056/NEJMoa2026330). This positive outcome was noted despite the presence of resistance alleles to SP *(*Plowe et al., 2022, Malar J 2022, 21 (1), 104. DOI: 10.1186/s12936-022-04115-8*)* suggesting co-administration of new classes of chemopreventative agents that don't yet have resistance in the field would be even more effective.

Thus, given the continued threat of drug resistance to current malaria treatment programs and the lack of sterilizing vaccines, new drugs that can overcome these limitations are badly needed *(Phillips e al*., *2017, supra;* Ashton et al., 2019, J Med Chem, 62 (23), 10526-10562. DOI: 10.1021/acs.jmedchem.9b00761*;* Siqueira-Neto et al., 2023, Nat Rev Drug Discov, 22 (10), 807-826. DOI: 10.1038/s41573-023-00772-9*).*

Significant progress has been made in recent years to identify new classes of compounds with efficacy against malaria that target new parasite proteins and pathways that are not yet compromised by resistance *(*Abraham, et al., 2020, ACS Infect Dis, 6 (4), 613-628. DOI: 10.1021/acsinfecdis.9b00482*;* Hovlid et al., 2016, Trends Parasitol, 32 (9), 697-707. DOI: 10.1016/j.pt.2016.04.014*;* Carolino et al., 2020, Curr Opin Microbiol, 57, 49-55. DOI: 10.1016/j.mib.2020.06.004*).* Both phenotypic and target based high throughput screens have been employed to identify chemical starting points, and new targets have been identified based on phenotypic hits followed up by identification of resistance alleles and genomic approaches to identify targets. A number of compounds have reached clinical development *(Ashton et al*., *2019, supra; Siqueira-Neto et al*., *2023, supra)* and the most advanced of these KAF156 has completed Phase II clinical development where it has shown good safety and efficacy *(*Ogutu, et al., 2023, Lancet Infect Dis., 23 (9), 1051-1061. DOI: 10.1016/S1473-3099(23)00209-8*).*

A target-based approach allowed to identify inhibitors of *P. falcparium* dihydroorotate dehydrogenase (DHODH) *(*Baldwin et al., 2005, J Biol Chem, 280 (23), 21847-21853*)* identifying a potent triazolopyrimidine-based series that reached Phase II clinical development for treatment of malaria *(*Coteron et al., 2011, J Med Chem, 54 (15), 5540-5561. DOI: 10.1021/jm200592f*;* Llanos-Cuentas et al., 2018, Lancet Infect Dis, 18 (8), 874-883. DOI: 10.1016/S1473-3099(18)30309-8*;* Phillips et al., 2015, Sci Transl Med, 7 (296), 296ra111. DOI: 10.1126/scitranslmed.aaa6645*).*

DHODH is an essential enzyme in the biosynthesis of pyrimidine nucleotides needed for the synthesis of DNA, RNA and nucleotide sugars *(*Phillips et al., 2010, Infect Disord Drug Target, 10 (3), 226-239. DOI: BSP/ ID DT/E-Pub/-0025-10-3 [pii*]). Plasmodium* species lack salvage pathways for the formation of pyrimidines and thus rely on the *de novo* pathway for synthesis of these essential building blocks. DHODH is a flavin-dependent enzyme and both the mammalian and *Plasmodium* enzymes are localized to the mitochondria where they use ubiquinone as the terminal electron acceptor. **DSM265** showed high safety and efficacy in Phase I and II clinical studies, providing a single dose cure for *P. falciparum* malaria for patients in Peru *(Llanos-Cuentas et al*., *2018, supra;* McCarthy et al., 2017, Lancet Infect Dis, 17 (6), 626-635. DOI: 10.1016/s1473-3099(17)30171-8*)* and was also shown to be effective for prevention of malaria in human challenge models (Murphy et al., 2018, J Infect Dis, 217 (5), 693-702. DOI: 10.1093/infdis/jix613 and Sulyok et al., 2017, Lancet Infect Dis, 17 (6), 636-644. DOI: 10.1016/S1473-3099(17)30139-1), consistent with its demonstrated antischizontal blood and liver stage activity *(*Phillips et al., 2015, Sci TranslMed, 7 (296), 296ra111. DOI: 10.1126/scitranslmed.aaa6645*;* Flannery et al., 2018, JCI Insight, 3 (1), e92587. DOI: 10.1172/jci.insight.92587). These studies validated DHODH as a clinically useful target for the treatment and prevention of malaria.

Subsequent structure-based lead optimization of a pyrrole-based series led to the identification of **DSM705** which had several improved properties over **DSM265,** including better solubility and equivalent activity on *P. falciparum* and *P. vivax (*Kokkonda et al., 2020, J Med Chem 63 (9), 4929-4956. DOI: 10.1021/acs.jmedchem.0c00311*;* Palmer et al., 2021, J Med Chem, 64 (9), 6085-6136. DOI: 10.1021/acs.jmedchem.1c00173).

However, widespread emergence of drug resistance of malaria parasites in endemic countries has compromised many of the current chemotherapies and there is a continued need for new chemotherapeutic approaches.

### Summary of the Invention

The present invention is defined by the appended claims only.

In addition, in the subsequent paragraphs of this description, any reference to methods of treatment is to be interpreted as a reference to the compounds or pharmaceutical compositions of the present invention for use in a method for said treatment of the human or animal body by therapy.

The present invention is directed towards compounds which are useful in the treatment and/or prophylaxis of malaria, pharmaceutical formulation, use and manufacture thereof. It has been found that those compounds unexpectedly present several advantages over earlier found DHODH inhibitors, namely high potency and excellent ADME/PK properties with long predicted human half-life.

A first aspect of the invention provides a compound according to the invention or a pharmaceutically acceptable salt thereof.

Another aspect of the invention relates to a compound or a pharmaceutically acceptable salt thereof according to the invention for use as a medicament.

Another aspect of the invention relates to a compound according to the invention or a pharmaceutically acceptable salt thereof for use in the prevention and/or treatment of malaria.

Another aspect of the invention relates to the use of a compound according to the invention or a pharmaceutically acceptable salt thereof for the preparation of a pharmaceutical composition for the prevention and/or treatment of malaria. Another aspect of the invention resides in a pharmaceutical formulation comprising at least one compound according to the invention or a pharmaceutically acceptable salt thereof and a pharmaceutically acceptable carrier, diluent or excipient thereof.

Also disclosed herein is a method for preventing and/or treating malaria in a subject. The method comprises administering a compound according to the invention or a pharmaceutically acceptable salt thereof or a pharmaceutically active derivative thereof in a subject in need thereof.

Another aspect of the invention provides a method for the preparation of a compound according to the invention or a pharmaceutically acceptable salt thereof according to the invention and intermediates thereof.

Another aspect of the invention provides a method for the preparation of a compound of Formula (I).

Herein is disclosed an intermediate of a formula selected from Formulae GS-1.3, GS-1.4, GS-1.9, GS-1.13, GS-1.14, GS-1.17 and GS-1.18 useful in a method according to the invention. The intermediates of a formula selected from Formulae GS-1.3 and GS-1.17 are compounds of the invention.

Other features and advantages of the invention will be apparent from the following detailed description.

### Detailed Description of the invention

The following paragraphs provide definitions of the various chemical moieties that make up the compounds according to the invention and are intended to apply uniformly through-out the specification and claims, unless an otherwise expressly set out definition provides a broader definition.

The term "C₁-C₆ alkyl" when used alone or in combination with other terms, comprises a straight chain or branched C₁-C₆ alkyl which refers to monovalent alkyl groups having 1 to 6 carbon atoms. This term is exemplified by groups such as methyl, ethyl, n-propyl, i-propyl, n-butyl, s-butyl, i-butyl, t-butyl, n-pentyl, 1-ethylpropyl, 2-methylbutyl, 3-methylbutyl, 2,2-dimethylpropyl, n-hexyl, 2-methylpentyl, 3-methylpentyl, 4-methylpentyl, and the like.

The term "C₃-C₈-cycloalkyl" refers to a saturated carbocyclic group of from 3 to 8 carbon atoms having a single ring (*e.g.* cyclohexyl) or multiple condensed rings *(e.g.* norbornyl). C₃-C₈-cycloalkyl includes cyclopropyl, cyclopentyl, cyclohexyl, norbornyl and the like.

The term "halogen" refers to fluoro, chloro, bromo and iodo atoms.

The term "acyl" refers to the group -C(O)R where R includes H, "alkyl," preferably "C₁-C₆ alkyl," "aryl," "heteroaryl," "C₃-C₈-cycloalkyl," "heterocycloalkyl," "aryl C₁-C₆ alkyl," "heteroaryl C₁-C₆ alkyl," "C₃-C₈-cycloalkyl C₁-C₆ alkyl" or "heterocycloalkyl C₁-C₆ alkyl", including acetyl and the like.

Unless otherwise constrained by the definition of the individual substituent, the term "substituted" refers to groups substituted with from 1 to 5 substituents selected from the group consisting of "C₁-C₆ alkyl," "C₂-C₆ alkenyl," "C₂-C₆ alkynyl," "C₃-C₈-cycloalkyl," "heterocycloalkyl," "C₁-C₆ alkyl aryl," "C₁-C₆ alkyl heteroaryl," "C₁-C₆ alkyl C₃-C₈-cycloalkyl," "C₁-C₆ alkyl heterocycloalkyl," "acyl", "amino," "amide", "aminosulfonyl," "ammonium," "acyl amino," "aminocarbonyl," "aryl," "heteroaryl," "sulfinyl," "sulfonyl," "sulphonamide", "alkoxy," "alkoxy carbonyl," "carbamate," "sulfanyl," "halogen," trihalomethyl, cyano, hydroxy, mercapto, nitro, and the like.

The term "pharmaceutically acceptable salts or complexes" refers to salts or complexes of the compounds according to the invention. Examples of such salts are formed from acid addition salts formed with inorganic acids (e.g. hydrochloric acid, hydrobromic acid, sulfuric acid, phosphoric acid, nitric acid, and the like), as well as salts formed with organic acids such as acetic acid, oxalic acid, tartaric acid, succinic acid, malic acid, fumaric acid, maleic acid, ascorbic acid, benzoic acid, tannic acid, palmoic acid, alginic acid, polyglutamic acid, naphthalene sulfonic acid, naphthalene disulfonic acid, methane sulfonic acid, p-toluene sulfonic acid and poly-galacturonic acid.

"Pharmaceutically active derivative" refers to any compound that upon administration to the recipient, is capable of providing directly or indirectly, the activity disclosed herein. The term "indirectly" also encompasses prodrugs which may be converted to the active form of the drug via endogenous enzymes or metabolism. The prodrug is a derivative of the compounds according to the invention and presenting anti-malarial activity that has a chemically or metabolically decomposable group, and a compound that may be converted into a pharmaceutically active compound according to the invention *in vivo* by solvolysis under physiological conditions. The prodrug is converted into a compound according to the present invention by a reaction with an enzyme, gastric acid or the like under a physiological condition in the living body, e.g. by oxidation, reduction, hydrolysis or the like, each of which is carried out enzymatically. These compounds can be produced from compounds of the present invention according to well-known methods.

The term "indirectly" also encompasses metabolites of compounds according to the invention. The term "metabolite" refers to all molecules derived from any of the compounds according to the present invention in a cell or organism, preferably mammal.

In the context of the present invention are encompassed pharmaceutically acceptable salts, hydrates, solvates, or polymorphs of compounds of the invention.

The term "malaria" includes disease and conditions related to an infection by *Plasmodium.*

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it; (b) inhibiting the disease, i.e., arresting its development; or relieving the disease, i.e., causing regression of the disease and/or its symptoms or conditions.

The term "effective amount" includes "prophylaxis-effective amount" as well as "treatment-effective amount".

The term "prophylaxis-effective amount" refers to a concentration of compound of this invention that is effective in inhibiting, decreasing the likelihood of the disease by malarial parasites, or preventing malarial infection or preventing the delayed onset of the disease by malarial parasites, when administered before infection, i.e. before, during and/or slightly after the exposure period to malarial parasites.

The term "prophylaxis" includes causal prophylaxis, i.e. antimalarial activity comprising preventing the pre-erythrocytic development of the parasite, suppressive prophylaxis, i.e. antimalarial activity comprising suppressing the development of the blood stage infection and terminal prophylaxis, i.e. antimalarial activity comprising suppressing the development of intra-hepatic stage infection. This term includes primary prophylaxis (i.e. preventing initial infection) where the antimalarial compound is administered before, during and/or after the exposure period to malarial parasites and terminal prophylaxis (i.e. to prevent relapses or delayed onset of clinical symptoms of malaria) when the antimalarial compound is administered towards the end of and/or slightly after the exposure period to malarial parasites but before the clinical symptoms. Typically, against *P. falciparum* infections, suppressive phophylaxis is used whereas against *P. vivax* or a combination of *P. falciparum* and *P. vivax,* terminal prophylaxis is used.

Likewise, the term "treatment-effective amount" refers to a concentration of compound that is effective in treating malaria infection, e.g. leads to a reduction in parasite numbers in blood following microscopic examination when administered after infection has occurred.

The term "subject" as used herein refers to mammals. For examples, mammals contemplated by the present invention include humans and the like.

### Compounds

According to one embodiment, is provided a compound according to Formula (I): 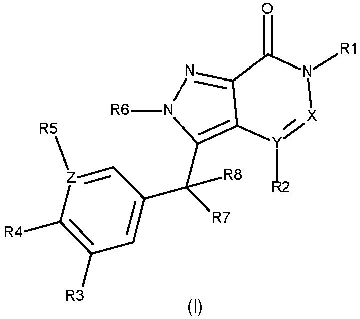
wherein **R1** is selected from an optionally substituted C₁-C₄ alkyl (e.g. optionally substituted methyl, optionally substituted ethyl, optionally substituted isopropyl) and optionally substituted C₃-C₆ cycloalkyl (e.g. optionally substituted cyclopropyl);
**X** is selected from N and CR9; **Y-R2** is CR2 or N;
**R2** is selected from H, halogen (e.g. F, Cl, Br), an optionally substituted C₁-C₄ alkyl (e.g. optionally substituted methyl, optionally substituted ethyl, optionally substituted isopropyl) and optionally substituted C₃-C₆ cycloalkyl (e.g. optionally substituted cyclopropyl);
**R3, R4** and **R5** are independently selected from Cl, F, CF₃, CN and H;
**Z-R5** is selected from nitrogen and **CR5;**
**R6** is selected from an optionally substituted C₁-C₄ alkyl (e.g. optionally substituted methyl, optionally substituted ethyl, optionally substituted propyl) and optionally substituted C₃-C₆ cycloalkyl (e.g. optionally substituted cyclopropyl);
**R8** is selected from OH and NH₂;
**R7** is selected from H, an optionally substituted C₁-C₄ alkyl (e.g. optionally substituted methyl such as CF₃, optionally substituted ethyl, optionally substituted isopropyl) and an optionally substituted C₃-C₆ cycloalkyl (e.g. optionally substituted cyclopropyl);
**R9** is selected from H and optionally substituted C₁-C₄ alkyl (e.g. optionally substituted methyl); as well as pharmaceutically acceptable salts, tautomers, polymorphs, racemic mixtures, optically active forms thereof

In a particular embodiment, the invention provides a compound according to the invention wherein R1 is optionally substituted C₃-C₆ cycloalkyl.

In another particular embodiment, the invention provides a compound according to the invention wherein R1 is optionally substituted C₁-C₄ alkyl (e.g. optionally substituted ethyl such as trifluoroethyl, optionally substituted isopropyl).

In another particular embodiment, the invention provides a compound according to the invention wherein R1 is cyclopropyl.

In another particular embodiment, the invention provides a compound according to the invention wherein Y-R2 is N and X is CR9.

In another particular embodiment, the invention provides a compound according to the invention wherein X is N and Y-R2 is CR2.

In another particular embodiment, the invention provides a compound according to the invention wherein ZR5 is N.

In another particular embodiment, the invention provides a compound according to the invention wherein ZR5 is CR5.

In another particular embodiment, the invention provides a compound according to the invention wherein R2 is H.

In another particular embodiment, the invention provides a compound according to the invention wherein R3 is halogen (e.g. Cl).

In another particular embodiment, the invention provides a compound according to the invention wherein R3 is CN.

In another particular embodiment, the invention provides a compound according to the invention wherein R3 is H.

In another particular embodiment, the invention provides a compound according to the invention wherein R3 is Cl.

In another particular embodiment, the invention provides a compound according to the invention wherein R4 is CF₃.

In another particular embodiment, the invention provides a compound according to the invention wherein R5 is H.

In another particular embodiment, the invention provides a compound according to the invention wherein R5 is CN.

In another particular embodiment, the invention provides a compound according to the invention wherein R5 is Cl.

In another particular embodiment, the invention provides a compound according to the invention wherein R6 is optionally substituted C₁-C₄ alkyl (e.g. optionally substituted methyl or optionally substituted ethyl).

In another particular embodiment, the invention provides a compound according to the invention wherein R6 is an optionally substituted C₃-C₆ cycloalkyl (e.g. optionally substituted cyclopropyl).

In another particular embodiment, the invention provides a compound according to the invention wherein R7 is H.

In another particular embodiment, the invention provides a compound according to the invention wherein R7 is an optionally substituted C₁-C₄ alkyl (e.g. optionally substituted methyl such as CF₃ or CHF₂ or optionally substituted ethyl).

In another particular embodiment, the invention provides a compound according to the invention wherein R7 is an optionally substituted C₃-C₆ cycloalkyl (e.g. optionally substituted cyclopropyl).

In another particular embodiment, the invention provides a compound according to the invention wherein R8 is OH.

In another particular embodiment, the invention provides a compound according to the invention wherein R8 is NH₂.

In another particular embodiment, the invention provides a compound according to the invention wherein R9 is H.

In another particular embodiment, the invention provides a compound according to the invention wherein R9 is optionally substituted C₁-C₄ alkyl (e.g. optionally substituted methyl).

In a particular embodiment is provided a compound selected from the following group:
(R)-3-(1-(5-chloro-6-(trifluoromethyl)pyridin-3-yl)-1-hydroxy-2-methylpropyl)-6-cyclopropyl-2-methyl-2,6-dihydro-7H-pyrazolo[3,4-c]pyridin-7-one;
(R)-5-(cyclopropyl(6-cyclopropyl-2-methyl-7-oxo-6,7-dihydro-2H-pyrazolo[3,4-c]pyridin-3-yl)(hydroxy)methyl)-2-(trifluoromethyl)benzonitrile;
(R)-5-(1-(6-cyclopropyl-2-methyl-7-oxo-6,7-dihydro-2H-pyrazolo[3,4-c]pyridin-3-yl)-1-hydroxypropyl)-2-(trifluoromethyl)benzonitrile;
3-(1-(5-chloro-6-(trifluoromethyl)pyridin-3-yl)-1-hydroxypropyl)-6-cyclopropyl-2,5-dimethyl-2,6-dihydro-7H-pyrazolo[3,4-c]pyridin-7-one;
(R)-3-(1-(3-chloro-4-fluorophenyl)-1-hydroxyethyl)-6-cyclopropyl-2-methyl-2,6-dihydro-7H-pyrazolo[3,4-c]pyridin-7-one;
(R)-3-(1-(5-chloro-6-(trifluoromethyl)pyridin-3-yl)-1-hydroxyethyl)-6-cyclopropyl-2-methyl-2,6-dihydro-7H-pyrazolo[3,4-c]pyridin-7-one;
(R)-3-(1-(3-chloro-4-fluorophenyl)-1-hydroxypropyl)-6-cyclopropyl-2-methyl-2,6-dihydro-7H-pyrazolo[3,4-c]pyridin-7-one;
(R)-3-(1-(5-chloro-6-(trifluoromethyl)pyridin-3-yl)-1-hydroxypropyl)-6-cyclopropyl-2-methyl-2,6-dihydro-7H-pyrazolo[3,4-c]pyridin-7-one;
(R)-3-((5-chloro-6-(trifluoromethyl)pyridin-3-yl)(cyclopropyl)(hydroxy)methyl)-6-cyclopropyl-2-methyl-2,6-dihydro-7H-pyrazolo[3,4-c]pyridin-7-one;
(R)-3-((3-chloro-4-fluorophenyl)(cyclopropyl)(hydroxy)methyl)-6-cyclopropyl-2-methyl-2,6-dihydro-7H-pyrazolo[3,4-c]pyridin-7-one;
(R)-3-(1-(4-chloro-3,5-difluorophenyl)-1-hydroxypropyl)-6-cyclopropyl-2-methyl-2,6-dihydro-7H-pyrazolo[3,4-c]pyridin-7-one;
(R)-6-cyclopropyl-3-(1-hydroxy-1-(6-(trifluoromethyl)pyridin-3-yl)propyl)-2-methyl-2,6-dihydro-7H-pyrazolo[3,4-c]pyridin-7-one;
(R)-6-ethyl-4-fluoro-3-(1-hydroxy-1-(6-(trifluoromethyl)pyridin-3-yl)propyl)-2-methyl-2,6-dihydro-7H-pyrazolo[3,4-c]pyridin-7-one;
(R)-6-cyclopropyl-3-(cyclopropyl(hydroxy)(6-(trifluoromethyl)pyridin-3-yl)methyl)-2-methyl-2,6-dihydro-7H-pyrazolo[3,4-c]pyridin-7-one;
(R)-3-((5-chloro-6-(trifluoromethyl)pyridin-3-yl)(hydroxy)methyl)-4-cyclopropyl-6-ethyl-2-methyl-2,6-dihydro-7H-pyrazolo[3,4-c]pyridin-7-one;
(R)-3-((4-chloro-3-fluorophenyl)(hydroxy)methyl)-6-ethyl-2,4-dimethyl-2,6-dihydro-7H-pyrazolo[3,4-c]pyridin-7-one;
(R)-3-((5-chloro-6-(trifluoromethyl)pyridin-3-yl)(cyclopropyl)(hydroxy)methyl)-6-ethyl-2-methyl-2,6-dihydro-7H-pyrazolo[3,4-c]pyridin-7-one;
(R)-3-(1-(3-chloro-4-fluorophenyl)-1-hydroxypropyl)-6-ethyl-2-methyl-2,6-dihydro-7H-pyrazolo[3,4-c]pyridin-7-one;
(R)-3-(1-(5-chloro-6-(trifluoromethyl)pyridin-3-yl)-1-hydroxyethyl)-6-ethyl-2-methyl-2,6-dihydro-7H-pyrazolo[3,4-c]pyridin-7-one;
(R)-3-((3-chloro-4-fluorophenyl)(cyclopropyl)(hydroxy)methyl)-6-ethyl-2-methyl-2,6-dihydro-7H-pyrazolo[3,4-c]pyridin-7-one;
(R)-3-(1-(3-chloro-4-fluorophenyl)-1-hydroxyethyl)-6-ethyl-2-methyl-2,6-dihydro-7H-pyrazolo[3,4-c]pyridin-7-one;
(R)-3-(1-(5-chloro-6-(trifluoromethyl)pyridin-3-yl)-1-hydroxypropyl)-6-ethyl-2-methyl-2,6-dihydro-7H-pyrazolo[3,4-c]pyridin-7-one;
(R)-3-(cyclopropyl(hydroxy)(6-(trifluoromethyl)pyridin-3-yl)methyl)-6-ethyl-2-methyl-2,6-dihydro-7H-pyrazolo[3,4-c]pyridin-7-one;
(R)-3-(1-(3-chloro-4-(trifluoromethyl)phenyl)-1-hydroxyethyl)-6-ethyl-2-methyl-2,6-dihydro-7H-pyrazolo[3,4-c]pyridin-7-one;
(R)-6-ethyl-3-(1-hydroxy-2-methyl-1-(6-(trifluoromethyl)pyridin-3-yl)propyl)-2-methyl-2,6-dihydro-7H-pyrazolo[3,4-c]pyridin-7-one;
(R)-6-ethyl-3-(1-hydroxy-1-(6-(trifluoromethyl)pyridin-3-yl)propyl)-2-methyl-2,6-dihydro-7H-pyrazolo[3,4-c]pyridin-7-one;
(R)-3-(1-(5-chloro-6-(trifluoromethyl)pyridin-3-yl)-1-hydroxypropyl)-2,6-dicyclopropyl-2,6-dihydro-7H-pyrazolo[3,4-d]pyridazin-7-one;
(R)-3-(1-(5-chloro-6-(trifluoromethyl)pyridin-3-yl)-1-hydroxypropyl)-6-cyclopropyl-2-ethyl-2,6-dihydro-7H-pyrazolo[3,4-d]pyridazin-7-one;
(R)-5-(1-(6-cyclopropyl-2-methyl-7-oxo-6,7-dihydro-2H-pyrazolo[3,4-d]pyridazin-3-yl)-1-hydroxypropyl)-2-(trifluoromethyl)benzonitrile;
(R)-5-(cyclopropyl(6-cyclopropyl-2-methyl-7-oxo-6,7-dihydro-2H-pyrazolo[3,4-d]pyridazin-3-yl)(hydroxy)methyl)-2-(trifluoromethyl)benzonitrile;
(R)-3-((5-chloro-6-(trifluoromethyl)pyridin-3-yl)(cyclopropyl)(hydroxy)methyl)-6-cyclopropyl-2-methyl-2,6-dihydro-7H-pyrazolo[3,4-d]pyridazin-7-one;
(R)-3-((3-chloro-4-(trifluoromethyl)phenyl)(cyclopropyl)(hydroxy)methyl)-6-cyclopropyl-2-methyl-2,6-dihydro-7H-pyrazolo[3,4-d]pyridazin-7-one;
(R)-3-(1-(5-chloro-6-(trifluoromethyl)pyridin-3-yl)-1-hydroxypropyl)-6-cyclopropyl-2-methyl-2,6-dihydro-7H-pyrazolo[3,4-d]pyridazin-7-one;
(R)-3-(1-(3-chloro-4-(trifluoromethyl)phenyl)-1-hydroxypropyl)-6-cyclopropyl-2-methyl-2,6-dihydro-7H-pyrazolo[3,4-d]pyridazin-7-one;
(R)-3-(1-(3-chloro-4-fluorophenyl)-1-hydroxypropyl)-6-cyclopropyl-2-methyl-2,6-dihydro-7H-pyrazolo[3,4-d]pyridazin-7-one;
(R)-3-(1-(3-chloro-4-(trifluoromethyl)phenyl)-1-hydroxypropyl)-6-ethyl-2-methyl-2,6-dihydro-7H-pyrazolo[3,4-d]pyridazin-7-one;
(R)-3-(1-(5-chloro-6-(trifluoromethyl)pyridin-3-yl)-1-hydroxyethyl)-6-cyclopropyl-2-methyl-2,6-dihydro-7H-pyrazolo[4,3-d]pyrimidin-7-one;
(R)-3-((5-chloro-6-(trifluoromethyl)pyridin-3-yl)(cyclopropyl)(hydroxy)methyl)-6-cyclopropyl-2-methyl-2,6-dihydro-7H-pyrazolo[4,3-d]pyrimidin-7-one;
(R)-3-(1-(5-chloro-6-(trifluoromethyl)pyridin-3-yl)-1-hydroxypropyl)-6-cyclopropyl-2-methyl-2,6-dihydro-7H-pyrazolo[4,3-d]pyrimidin-7-one;
(R)-6-cyclopropyl-3-(1-hydroxy-1-(6-(trifluoromethyl)pyridin-3-yl)propyl)-2-methyl-2,6-dihydro-7H-pyrazolo[4,3-d]pyrimidin-7-one;
(R)-6-cyclopropyl-3-(cyclopropyl(hydroxy)(6-(trifluoromethyl)pyridin-3-yl)methyl)-2-methyl-2,6-dihydro-7H-pyrazolo[4,3-d]pyrimidin-7-one;
(R)-5-(1-(6-ethyl-2-methyl-7-oxo-6,7-dihydro-2H-pyrazolo[4,3-d]pyrimidin-3-yl)-1-hydroxyethyl)-2-(trifluoromethyl)benzonitrile;
(R)-3-(1-amino-1-(5-chloro-6-(trifluoromethyl)pyridin-3-yl)-2,2,2-trifluoroethyl)-6-cyclopropyl-2-methyl-2,6-dihydro-7H-pyrazolo[3,4-c]pyridin-7-one;
(R)-3-(1-amino-1-(3-chloro-4-(trifluoromethyl)phenyl)ethyl)-6-cyclopropyl-2-methyl-2,6-dihydro-7H-pyrazolo[3,4-c]pyridin-7-one;
(R)-3-(1-amino-1-(3-fluoro-4-(trifluoromethyl)phenyl)propyl)-6-cyclopropyl-2-methyl-2,6-dihydro-7H-pyrazolo[3,4-c]pyridin-7-one;
(R)-3-(1-amino-1-(3-chloro-4-(trifluoromethyl)phenyl)propyl)-6-cyclopropyl-2-methyl-2,6-dihydro-7H-pyrazolo[3,4-c]pyridin-7-one;
(R)-3-(amino(5-chloro-6-(trifluoromethyl)pyridin-3-yl)(cyclopropyl)methyl)-6-cyclopropyl-2-methyl-2,6-dihydro-7H-pyrazolo[3,4-c]pyridin-7-one;
(R)-3-(1-amino-1-(5-chloro-6-(trifluoromethyl)pyridin-3-yl)propyl)-6-cyclopropyl-2-methyl-2,6-dihydro-7H-pyrazolo[3,4-c]pyridin-7-one;
(R)-5-(amino(6-ethyl-2,4-dimethyl-7-oxo-6,7-dihydro-2H-pyrazolo[3,4-c]pyridin-3-yl) methyl)-2-(trifluoromethyl)benzonitrile;
(R)-3-(amino(3-chloro-4-(trifluoromethyl)phenyl)(cyclopropyl)methyl)-6-cyclopropyl-2-methyl-2,6-dihydro-7H-pyrazolo[3,4-d]pyridazin-7-one;
(R)-3-(1-amino-1-(3-chloro-4-(trifluoromethyl)phenyl)propyl)-6-cyclopropyl-2-methyl-2,6-dihydro-7H-pyrazolo[3,4-d]pyridazin-7-one;
(R)-3-(1-amino-1-(3-chloro-4-(trifluoromethyl)phenyl)ethyl)-6-cyclopropyl-2-methyl-2,6-dihydro-7H-pyrazolo[4,3-d]pyrimidin-7-one;
(R)-3-(1-amino-1-(3-chloro-4-fluorophenyl)propyl)-6-cyclopropyl-2-methyl-2,6-dihydro-7H-pyrazolo[4,3-d]pyrimidin-7-one;
(R)-3-(amino(3-chloro-4-(trifluoromethyl)phenyl)(cyclopropyl)methyl)-6-cyclopropyl-2-methyl-2,6-dihydro-7H-pyrazolo[4,3-d]pyrimidin-7-one;
(R)-3-(1-amino-1-(3-chloro-4-(trifluoromethyl)phenyl)propyl)-6-cyclopropyl-2-methyl-2,6-dihydro-7H-pyrazolo[4,3-d]pyrimidin-7-one;
(R)-3-(1-amino-1-(5-chloro-6-(trifluoromethyl)pyridin-3-yl)propyl)-6-cyclopropyl-2-methyl-2,6-dihydro-7H-pyrazolo[4,3-d]pyrimidin-7-one;
(R)-3-(1-(5-chloro-6-(trifluoromethyl)pyridin-3-yl)-2,2,2-trifluoro-1-hydroxyethyl)-6-cyclopropyl-2-methyl-2,6-dihydro-7H-pyrazolo[3,4-d]pyridazin-7-one;
(R)-3-(1-(5-chloro-6-(trifluoromethyl)pyridin-3-yl)-2,2,2-trifluoro-1-hydroxyethyl)-6-cyclopropyl-2-methyl-2,6-dihydro-7H-pyrazolo[4,3-d]pyrimidin-7-one;
(R)-3-(1-(5-chloro-6-(trifluoromethyl)pyridin-3-yl)-2,2,2-trifluoro-1-hydroxyethyl)-6-cyclopropyl-2-methyl-2,6-dihydro-7H-pyrazolo[3,4-c]pyridin-7-one;
(R)-3-(1-(3-chloro-4-(trifluoromethyl)phenyl)-2,2,2-trifluoro-1-hydroxyethyl)-6-cyclopropyl-2-methyl-2,6-dihydro-7H-pyrazolo[3,4-c]pyridin-7-one;
(R)-3-(1-(3-chloro-4-fluorophenyl)-2,2,2-trifluoro-1-hydroxyethyl)-6-cyclopropyl-2-methyl-2,6-dihydro-7H-pyrazolo[3,4-c]pyridin-7-one;
(R)-6-cyclopropyl-2-methyl-3-(2,2,2-trifluoro-1-hydroxy-1-(6-(trifluoromethyl)pyridin-3-yl) ethyl)-2,6-dihydro-7H-pyrazolo[3,4-c]pyridin-7-one,
(R)-5-(1-(6-cyclopropyl-2-methyl-7-oxo-6,7-dihydro-2H-pyrazolo[3,4-d]pyridazin-3-yl)-2,2,2-trifluoro-1-hydroxyethyl)-2-(trifluoromethyl)benzonitrile
(R)-3-(1-(3-chloro-4-(trifluoromethyl)phenyl)-2,2,2-trifluoro-1-hydroxyethyl)-6-cyclopropyl-2-methyl-2,6-dihydro-7H-pyrazolo[3,4-d]pyridazin-7-one
(R)-3-(1-(3-chloro-4-(trifluoromethyl)phenyl)-2,2,2-trifluoro-1-hydroxyethyl)-6-cyclopropyl-2-methyl-2,6-dihydro-7H-pyrazolo[4,3-d]pyrimidin-7-one
(R)-5-(1-(6-cyclopropyl-2-methyl-7-oxo-6,7-dihydro-2H-pyrazolo[4,3-d]pyrimidin-3-yl)-2,2,2-trifluoro-1-hydroxyethyl)-2-(trifluoromethyl)benzonitrile; and
(R)-6-cyclopropyl-2-methyl-3-(2,2,2-trifluoro-1-hydroxy-1-(6-(trifluoromethyl)pyridin-3-yl)ethyl)-2,6-dihydro-7H-pyrazolo[4,3-d]pyrimidin-7-one, as well as pharmaceutically acceptable salts, hydrates, solvates, tautomers, polymorphs, racemic mixtures, optically active forms thereof.

In another particular embodiment is provided a compound selected from the following group:
(R)-3-(1-(5-chloro-6-(trifluoromethyl)pyridin-3-yl)-2,2-difluoro-1-hydroxyethyl)-6-cyclopropyl-2-methyl-2,6-dihydro-7H-pyrazolo[4,3-d]pyrimidin-7-one;
(R)-3-(1-(3-chloro-4-(trifluoromethyl)phenyl)-2,2,2-trifluoro-1-hydroxyethyl)-2-methyl-6-(2,2,2-trifluoroethyl)-2,6-dihydro-7H-pyrazolo[4,3-d]pyrimidin-7-one;
(R)-3-(1-(3-chloro-4-(trifluoromethyl)phenyl)-2,2,2-trifluoro-1-hydroxyethyl)-6-isopropyl-2-methyl-2,6-dihydro-7H-pyrazolo[4,3-d]pyrimidin-7-one;
(R)-5-(1-amino-1-(6-cyclopropyl-2-methyl-7-oxo-6,7-dihydro-2H-pyrazolo[4,3-d]pyrimidin-3-yl)-2,2,2-trifluoroethyl)-2-(trifluoromethyl)benzonitrile;
(R)-5-(1-amino-1-(6-cyclopropyl-2-methyl-7-oxo-6,7-dihydro-2H-pyrazolo[3,4-c]pyridin-3-yl)-2,2,2-trifluoroethyl)-2-(trifluoromethyl)benzonitrile;
(R)-3-(1-amino-1-(3-chloro-4-(trifluoromethyl)phenyl)-2,2,2-trifluoroethyl)-6-cyclopropyl-2-methyl-2,6-dihydro-7H-pyrazolo[3,4-c]pyridin-7-one;
(R)-3-(1-amino-1-(3-chloro-4-(trifluoromethyl)phenyl)-2,2,2-trifluoroethyl)-6-cyclopropyl-2-methyl-2,6-dihydro-7H-pyrazolo[4,3-d]pyrimidin-7-one;
(R)-5-(1-(6-cyclopropyl-2-methyl-7-oxo-6,7-dihydro-2H-pyrazolo[4,3-d]pyrimidin-3-yl)-2,2,2-trifluoro-1-hydroxyethyl)-2-(trifluoromethyl)nicotinonitrile;
(R)-5-(1-(6-cyclopropyl-2-methyl-7-oxo-6,7-dihydro-2H-pyrazolo[4,3-d]pyrimidin-3-yl)-2,2,2-trifluoro-1-hydroxyethyl)-2-(difluoromethyl)benzonitrile; and (R)-5-(1-(6-cyclopropyl-2-methyl-7-oxo-6,7-dihydro-2H-pyrazolo[4,3-d]pyrimidin-3-yl)-2,2,2-trifluoro-1-hydroxyethyl)-2-(trifluoromethyl)benzonitrile.
as well as pharmaceutically acceptable salts, hydrates, solvates, tautomers, polymorphs, racemic mixtures, optically active forms thereof

In a further particular embodiment is provided an enantiomer of a compound according to the invention which is in configuration R.

In a further particular embodiment is provided an enantiomer of a compound according to the invention which is in configuration S.

The compounds of the invention are useful in the manufacture of a medicament for the prevention or treatment of malaria, are capable of killing and/or inhibiting malaria parasite replication.

### Preparation of compounds of the invention

The compounds of the invention can be prepared according to the schemes below. Preparation of Weinreb **(GS-1.2)** or the corresponding aldehyde **(GS-1.7)** are described in the experimental section, are commercially available or can be prepared by someone skilled in the art. The Weinreb amide **(GS-1.2)** is typically prepared from the corresponding acid via standard literature methods. 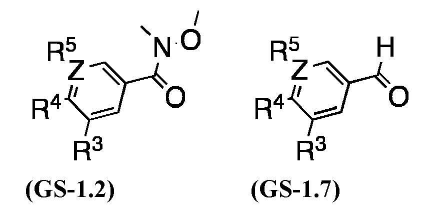 wherein R3-R5 and Z are as described herein.

The preparation of the intermediate bromides **(GS-1.1)** is described in the experimental section 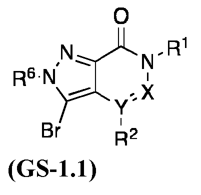 wherein R1, R2, R6, X and Y are as described herein.

Compounds of Formula (I) where R8 = OH can be prepared according to Scheme 1 below.

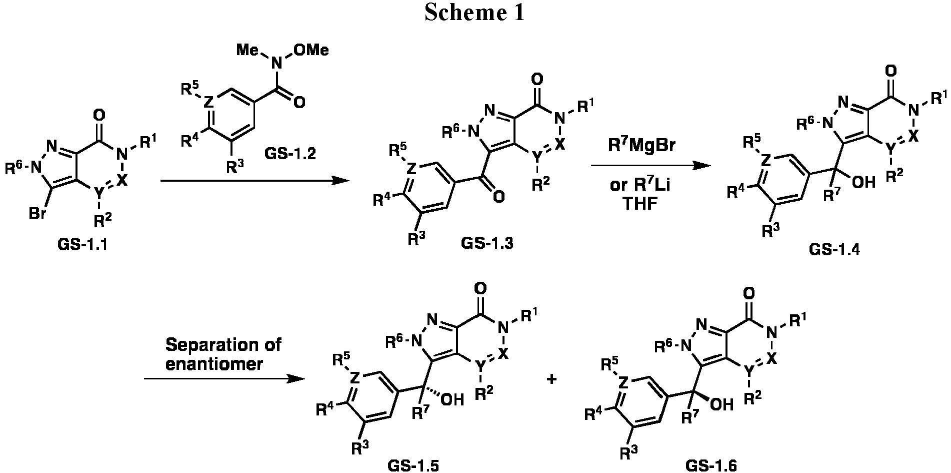

The bromide intermediate (GS-1.1) can be reacted with a Grignard such as PrⁱMgCl or an alkyl lithium and the intermediate anion further reacted with an intermediate of formula (GS-1.2) to give a ketone (GS-1.3) which is then further reacted with an alkyl Grignard or alkyl lithium, in particular MeMgBr, EtMgBr, PrⁱMgBr or cPrMgBr to give a racemic alcohol (GS-1.4). The racemic alcohol is then separated by chiral HPLC to give a compound of Formula (GS-1.5, GS-1.6).

Ketones of formula (GS-1.3) can also be prepared, as shown in Scheme 2, by reacting a bromide (GS-1.1)) with a Grignard such as PrⁱMgCl or an alkyl lithium and the intermediate anion is further reacted with an aldehyde of formula (GS-1.7) to give racemic alcohol (GS-1.8) which can be oxidized with an oxidizing agent such as MnO₂ to give a ketone **(GS-1.3).** This ketone can be further reacted as described above to give compounds of Formula (I):

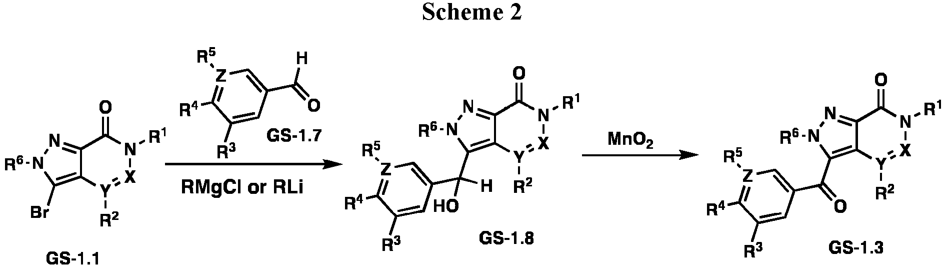

Compounds of Formula (I) where R8 = OH and R7 is CF₃ can be prepared as shown under Scheme 3 below.

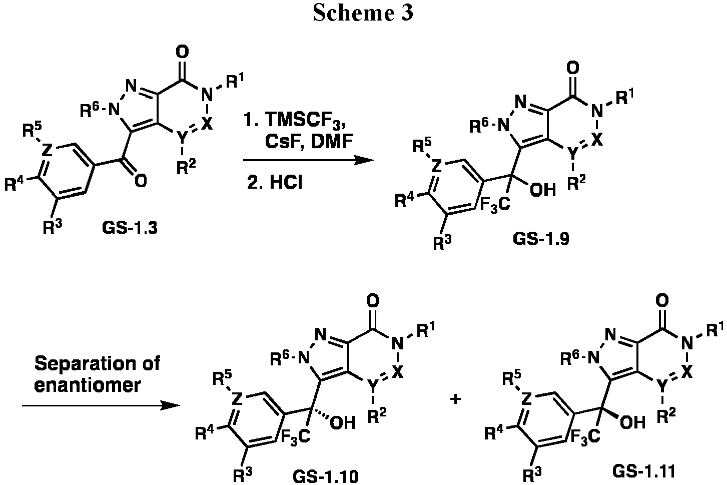

A ketone **(GS-1.3)** is reacted with trifluoromethyltrimethylsilane and the resultant intermediate is hydrolyzed with an acid to give a racemic alcohol **(GS-1.9).** The racemic alcohol is then separated by chiral HPLC to give a compound of Formula **(GS-1.10 and GS-1.11).**

Compounds of Formula (I) where R8 = NH₂ can be prepared as described under Scheme 4.

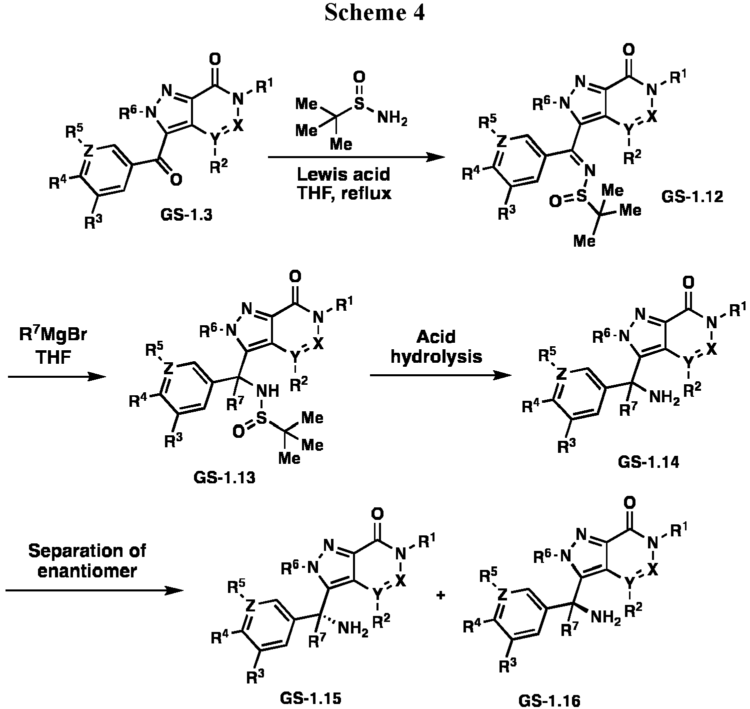

A ketone **(GS-1.3)** is reacted with a sulfinamide, 2-methylpropane-2-sulphinamide in the presence of a Lewis acid, such as titanium tetraisopropoxide to form the sulfinamide (GS-1.12) which can then be reacted with a Grignard reagent in particular MeMgBr, EtMgBr, PrⁱMgBr or cPrMgBr to give an intermediate sulfinamide **(GS-1.13)** which is hydrolyzed with acid such as hydrochloric acid in methanol to give the racemic amine **(GS-1.14)** which is then separated to the two isomer by chiral HPLC to give a compound of Formula **(GS1.15 and GS1.16).**

Compounds of Formula (I) where R8 = NH2 and R7 is CF₃ can be prepared as shown under Scheme 5.

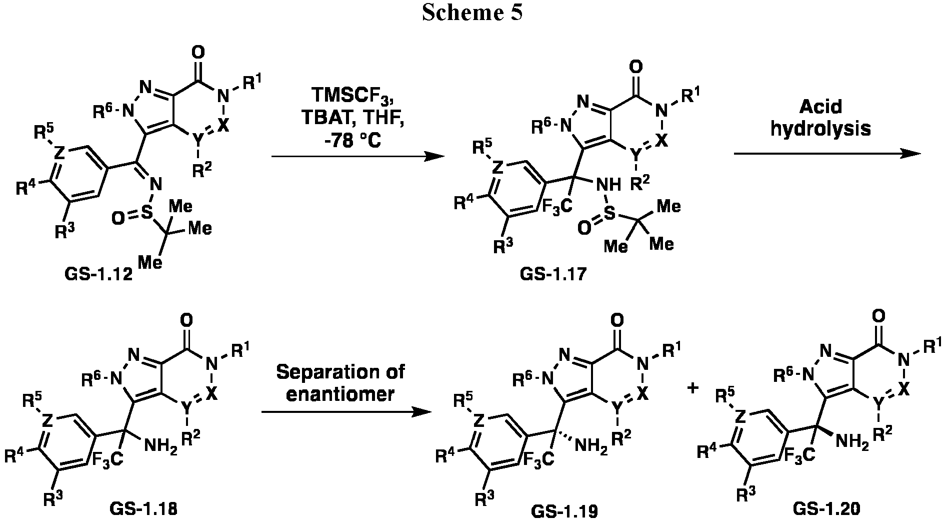

A sulfinamide **(GS-1.12)** is reacted with trifluoromethyl-trimethylsilane to give the intermediate sulfinamide **(GS-1.17)** which is then hydrolyzed with acid to give racemic amine **(GS-1.18).** The racemic amine is then separated by chiral HPLC to give a compound of Formula **(GS-1.19 and GS1.20)**

Compounds of Formula (I) exist as two isomeric forms and are separated by chiral HPLC. In general, the absolute assignment is arbitrary but where the absolute stereo chemistry is known, the R isomer is the more potent.

### Compositions

The invention provides pharmaceutical compositions useful for the prophylaxis or treatment of malaria. The invention further provides methods for treating a mammalian patient, and most preferably a human patient, who is suffering from malaria.

In another particular embodiment, is provided a pharmaceutical formulation containing at least one derivative according to the invention and a pharmaceutically acceptable carrier, diluent or excipient thereof.

In another particular embodiment, is provided a pharmaceutical formulation comprising a compound according to Formula (I) and a further antimalarial agent as defined in the detailed description.

In another particular embodiment, is provided a pharmaceutical formulation comprising a compound according to Formula (I) and at least one further antimalarial agent selected from artemisinin and its derivatives such as artemether, artesunate, dihydroartemisinin, chloroquine, hydroxychloroquine, quinine, mefloquine, amodiaquine, atovaquone/proguanil, doxycycline, clindamycin, halofantrine, lumefantrine, pyronaridine, pyrimethamine-sulfadoxine, ferroquine, tafenoquine, piperaquine and primaquine, spiro[3H-indole-3,1'-[1H]pyrido[3,4-b]indol]-2(1H)-one, 5,7'-dichloro-6'-fluoro-2',3',4',9'-tetrahydro-3'-methyl-, (1'R,3'S)- (CAS Registry Number: 1193314-23-6), 2-(1,1-difluoroethyl)-5-methyl-N-[4-(pentafluoro-λ⁶-sulfanyl)phenyl]-[1,2,4]triazolo[1,5-a]pyrimidin-7-amine (CAS Registry Number: 1282041-94-4), [3,3'-Bipyridin]-2-amine, 5-[4-(methylsulfonyl)phenyl]-6'-(trifluoromethyl)- (CAS Registry Number: 1314883-11-8) and Ethanone, 2-amino-1-[2-(4-fluorophenyl)-3-[(4-fluorophenyl)amino]-5,6-dihydroimidazo [1,2-a]pyrazin-7(8H)-yl]- (CAS Registry Number 1261109-90-3).

Pharmaceutical compositions of the invention can contain one or more compound(s) of the invention in any form described herein. Compositions of this invention may further comprise one or more pharmaceutically acceptable additional ingredient(s), such as alum, stabilizers, antimicrobial agents, buffers, coloring agents, flavoring agents, adjuvants, and the like.

The compounds of the invention, together with a conventionally employed adjuvant, carrier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous) use. Such pharmaceutical compositions and unit dosage forms thereof may comprise ingredients in conventional proportions, with or without additional active compounds or principles, and such unit dosage forms may contain any suitable effective amount of the active ingredient commensurate with the intended dosage range to be employed. Compositions according to the invention are preferably oral.

Compositions of this invention may be liquid formulations, including, but not limited to, aqueous or oily suspensions, solutions, emulsions, syrups, and elixirs. Liquid forms suitable for oral administration may include a suitable aqueous or non-aqueous vehicle with buffers, suspending and dispensing agents, colorants, flavors and the like. The compositions may also be formulated as a dry product for reconstitution with water or other suitable vehicle before use. Such liquid preparations may contain additives, including, but not limited to, suspending agents, emulsifying agents, non-aqueous vehicles and preservatives. Suspending agents include, but are not limited to, sorbitol syrup, methyl cellulose, glucose/sugar syrup, gelatin, hydroxyethyl cellulose, carboxymethyl cellulose, aluminum stearate gel, and hydrogenated edible fats. Emulsifying agents include, but are not limited to, lecithin, sorbitan monooleate, and acacia. Non-aqueous vehicles include, but are not limited to, edible oils, almond oil, fractionated coconut oil, oily esters, propylene glycol, and ethyl alcohol. Preservatives include, but are not limited to, methyl or propyl p-hydroxybenzoate and sorbic acid. Further materials as well as processing techniques and the like are set out in out in Part 5 of Remington's "The Science and Practice of Pharmacy", 23rd Edition, 2020, Editor: Adeboye Adejare*.* Solid compositions of this invention may be in the form of tablets or lozenges formulated in a conventional manner. For example, tablets and capsules for oral administration may contain conventional excipients including, but not limited to, binding agents, fillers, lubricants, disintegrants and wetting agents. Binding agents include, but are not limited to, syrup, acacia, gelatin, sorbitol, tragacanth, mucilage of starch and polyvinylpyrrolidone. Fillers include, but are not limited to, lactose, sugar, microcrystalline cellulose, maizestarch, calcium phosphate, and sorbitol. Lubricants include, but are not limited to, magnesium stearate, stearic acid, talc, polyethylene glycol, and silica. Disintegrants include, but are not limited to, potato starch and sodium starch glycollate. Wetting agents include, but are not limited to, sodium lauryl sulfate. Tablets may be coated according to methods well known in the art.

Injectable compositions are typically based upon injectable sterile saline or phosphate-buffered saline or other injectable carriers known in the art.

Compositions of this invention may also be formulated as suppositories, which may contain suppository bases including, but not limited to, cocoa butter or glycerides. Compositions of this invention may also be formulated for inhalation, which may be in a form including, but not limited to, a solution, suspension, or emulsion that may be administered as a dry powder or in the form of an aerosol using a propellant, such as dichlorodifluoromethane or trichlorofluoromethane. Compositions of this invention may also be formulated transdermal formulations comprising aqueous or non-aqueous vehicles including, but not limited to, creams, ointments, lotions, pastes, medicated plaster, patch, or membrane.

Compositions of this invention may also be formulated for parenteral administration, including, but not limited to, by injection or continuous infusion. Formulations for injection may be in the form of suspensions, solutions, or emulsions in oily or aqueous vehicles, and may contain formulation agents including, but not limited to, suspending, stabilizing, and dispersing agents. The composition may also be provided in a powder form for reconstitution with a suitable vehicle including, but not limited to, sterile, pyrogen-free water.

Compositions of this invention may also be formulated as a depot preparation, which may be administered by implantation or by intramuscular injection. The compositions may be formulated with suitable polymeric or hydrophobic materials (as an emulsion in an acceptable oil, for example), ion exchange resins, or as sparingly soluble derivatives (as a sparingly soluble salt, for example).

Compositions of this invention may also be formulated as a liposome preparation. The liposome preparation can comprise liposomes which penetrate the cells of interest or the *stratum corneum,* and fuse with the cell membrane, resulting in delivery of the contents of the liposome into the cell. Other suitable formulations can employ niosomes. Niosomes are lipid vesicles similar to liposomes, with membranes consisting largely of non-ionic lipids, some forms of which are effective for transporting compounds across the *stratum corneum.*

The compounds of this invention can also be administered in sustained release forms or from sustained release drug delivery systems. A description of representative sustained release materials can also be found in *Remington's Pharmaceutical*

*Sciences.*

### Mode of administration

Compositions of this invention may be administered in any manner, including, but not limited to, orally, parenterally, sublingually, transdermally, vaginally, rectally, transmucosally, topically, via inhalation, via buccal or intranasal administration, or combinations thereof. Parenteral administration includes, but is not limited to, intravenous, intra-arterial, intraperitoneal, subcutaneous, intramuscular, intra-thecal, and intra-articular. The compositions of this invention may also be administered in the form of an implant, which allows slow release of the compositions as well as a slow controlled i.v. infusion. In a preferred embodiment, compounds according to the invention are administered orally.

This invention is further illustrated by the following examples that are not intended to limit the scope of the invention in any way.

The dosage administered, as single or multiple doses, to an individual will vary depending upon a variety of factors, including pharmacokinetic properties, patient conditions and characteristics (sex, age, body weight, health, size), extent of symptoms, concurrent treatments, frequency of treatment and the effect desired.

### Combination

According to the invention, the compounds of the invention and pharmaceutical formulations thereof can be administered alone or in combination with a co-agent useful in the treatment of malaria, such as substances useful in the treatment and/or prevention of malaria e.g. for example a co-agent selected from artemisinin and its derivatives such as artemether, artesunate, dihydroartemisinin, chloroquine, hydroxychloroquine, quinine, mefloquine, amodiaquine, atovaquone/proguanil, doxycycline, clindamycin, halofantrine, lumefantrine, pyronaridine, pyrimethamine-sulfadoxine, ferroquine, tafenoquine, piperaquine and primaquine.

Further co-agents useful in combination with the compounds of the invention are selected from Spiro[3H-indole-3,1'-[1H]pyrido[3,4-b]indol]-2(1H)-one, 5,7'-dichloro-6'-fluoro-2',3',4',9'-tetrahydro-3'-methyl-,(1'R,3'S)- (CAS Registry Number: 1193314-23-6), 2-(1,1-difluoroethyl)-5-methyl-N-[4-(pentafluoro-λ⁶-sulfanyl)phenyl]-[1,2,4]triazolo[1,5-a] pyrimidin-7-amine (CAS Registry Number: 1282041-94-4), [3,3'-Bipyridin]-2-amine, 5-[4-(methylsulfonyl)phenyl]-6'-(trifluoromethyl)- (CAS Registry Number: 1314883-11-8), Ethanone, 2-amino-1-[2-(4-fluorophenyl)-3-[(4-fluorophenyl)amino]-5,6-dihydroimidazo [1,2-a]pyrazin-7(8H)-yl]- (CAS Registry Number 1261109-90-3).

The invention encompasses the administration of a compound according to the invention or of a pharmaceutical formulation thereof, wherein the compounds of the invention or the pharmaceutical formulation thereof are administered to an individual prior to, simultaneously or sequentially with other therapeutic regimens or co-agents useful in the treatment of malaria (e.g. multiple drug regimens), in an effective amount. Compounds of the invention or the pharmaceutical formulations thereof that are administered simultaneously with said co-agents can be administered in the same or different composition(s) and by the same or different route(s) of administration.

### Patients

In an embodiment, patients according to the invention are patients suffering from malaria.

In another embodiment, patients according to the invention are patients with a high risk of being infected by *Plasmodium.*

In another embodiment, patients according to the invention are patients with a high risk of being infected by *Plasmodium falciparum.*

In another embodiment, patients according to the invention are patients with a high risk of being infected by *Plasmodium vivax.*

In another embodiment, patients according to the invention are patients with a high risk of being infected by *Plasmodium ovale.*

In another embodiment, patients according to the invention are patients with a high risk of being infected by *Plasmodium malariae.*

In another embodiment, patients according to the invention are patients with a high risk of being infected by *Plasmodium knowlesi.*

### Use according to the invention

In one embodiment, the invention provides a compound according to Formula (I) as well as pharmaceutically acceptable salts, hydrates, solvates, or polymorphs, and pharmaceutically active derivative thereof for the treatment or prophylaxis of malaria.

In another embodiment, the invention provides a method for preventing or treating malaria in a subject. The method comprises administering an effective amount of a compound according to the invention, or a pharmaceutically acceptable salt or a pharmaceutically active derivative thereof or a pharmaceutical formulation thereof in a subject in need thereof.

In another embodiment, the invention provides a use of a compound or a method according to the invention wherein the compound is to be administered in combination with a co-agent useful in the treatment of malaria.

In another embodiment, the invention provides a pharmaceutical composition comprising a compound according to the invention in combination with a co-agent useful in the treatment of malaria.

In another embodiment, the invention provides a method for the preparation of a compound according to the invention comprising the step of transforming a compound according to Formula GS-1.3 into a compound of G-S1.4 as described under Scheme 6 in presence of an alkyl Grignard or alkyl lithium.

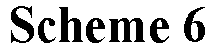

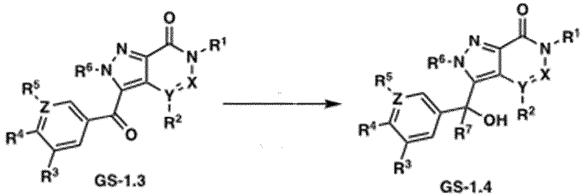

In another embodiment, the invention provides a method for the preparation of a compound according to the invention comprising a step of transforming a compound according to Formula GS-1.3 into a compound of GS-1.9 after acidic hydrolyzation as described under Scheme 7 in presence of trifluoromethyltrimethylsilane:

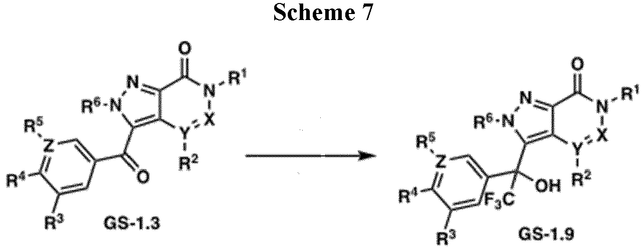

In another embodiment, the invention provides a method for the preparation of a compound according to the invention comprising a step of transforming a compound according to Formula GS-1.13 into a compound of GS-1.14 after acidic hydrolyzation as described under Scheme 8:

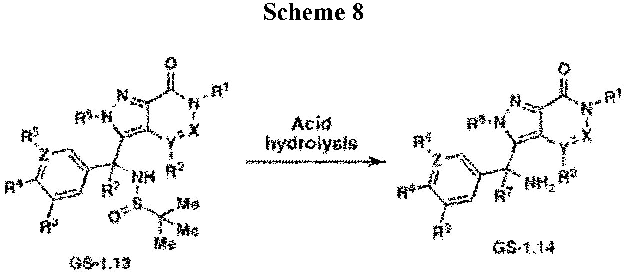

In another embodiment, the invention provides a method for the preparation of a compound according to the invention comprising a step of transforming a compound according to Formula GS-1.17 into a compound of GS-1.18 after acidic hydrolyzation as described under Scheme 9:

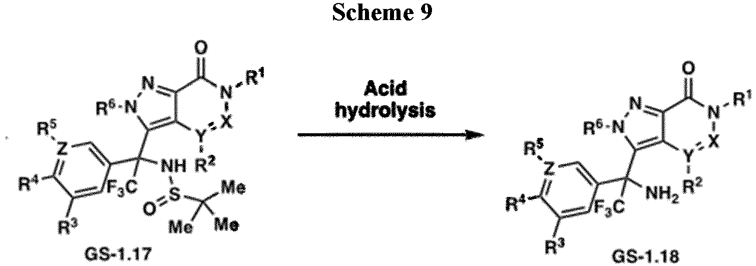

In another embodiment, the invention provides intermediates of Formulae GS-1.3, GS-1.4, GS-1.9, GS-1.13, GS-1.14, GS-1.17 and GS-1.18 wherein R1-R7, X, Y and Z are as defined above. In another embodiment, the invention provides methods of preparation of intermediates according to the invention.

The present invention is not to be limited in scope by the specific embodiments described herein, which are intended as single illustrations of individual aspects of the invention, and functionally equivalent methods and components are within the scope of the invention. In the following the present invention shall be illustrated by means of some examples, which are not to be viewed as limiting the scope of the invention. The scope of the invention is defined by the appended claims.

### EXAMPLES

### The following abbreviations refer respectively to the definitions below:

CsF (Cesium Fluoride), DCM (Dichloromethane), DCE (1,2-Dichloroethane), DME (1,2-dimethoxyethane), DMF (Dimethylformamide), EtOAc (Ethyl Acetate), **iPrMgCl.LiCl** (Isopropyl magnesium chloride. Lithium chloride complex), MeMgBr (Methyl magnesium bromide), **MnO₂** (Manganese dioxide), MS (Mass Spectrometry), **NaHCO₃** (Sodium Bicarbonate), **Na₂SO₄** (Sodium Sulphate), **Na₂S₂O₃** (Sodium Thiosulfate), NMR (Nuclear magnetic resonance), **NH₄Cl** (Ammonium chloride), **Pd₂dba₃** [Tris(dibenzylideneacetone)dipalladium(0)], **POBr₃** (Phosphorus (V) oxy bromide), RT (room temperature), **THF** (Tetrahydrofuran), **TLC** (Thin layer chromatography), **TMSCF₃** (Trimethyl(trifluoromethyl)silane), **Xantphos** (9,9-Dimethyl-9H-xanthene-4,5-diyl)bis(diphenylphosphane), **Zn(CN)₂** (Zinc cyanide).

The compounds of invention have been named according to the IUPAC standards used in the program Chem Bio Draw Ultra (Version 14.0).

All reagents and starting materials were obtained from commercial suppliers and used without further purification. For all compounds, reaction progress was monitored by thin layer chromatography (TLC) on preloaded silica gel 60 F254 plates. Visualization was achieved with UV light and iodine vapor. Flash chromatography was carried out using prepacked Teledyne Isco Redisep^{™} Rf silica-gel columns as the stationary phase and analytical grade solvents as the eluent unless otherwise stated. ¹H nuclear magnetic resonance (NMR) spectra were recorded on ¹H NMR was recorded at 400.20 & 400.10, 400.17 respectively, on a Bruker Avance II & III spectrometer, using solvents from Merck Laboratories. Chemical shifts (δ, ppm) are reported relative to the solvent peak (CDCl₃: 7.25 [1H], DMSO-d₆: 2.50 [1H]. Proton resonances are annotated as: chemical shift (δ), multiplicity (s, singlet; d, doublet; t, triplet; q, quartet; m, multiplet; br, broad), coupling constant (*J*, Hz), and number of protons. Total ion current traces were obtained for electrospray positive and negative ionization (ES+/ES-) on a Waters Acquity H-class UPLC attached with Waters SQD₂ mass spectrometer. Purity of all final compounds were reported >95% pure and judged by high-performance liquid chromatography (HPLC) using ACQUITY BEH C18 (50x2.1mm)1.7u, Luna Omega Polar (100*4.6) mm, ACQUITY UPLC HSS T3 (2.1X100m m) 1.8 and method (MOBILE PHASE A: ACN MOBILE PHASE B:0.05% HCOOH in H₂O). (MOBILE PHASE A: 100% ACN, MOBILE PHASE B:0.05% TFA in H₂O). Analytical grade solvents as the eluent were used unless otherwise stated. Chiral purification was carried out by supercritical fluid chromatography (SFC) using prepacked (R,R)Whel1k-01, Chiralpak-1C, Chiralpak-1G, C-Amylose-A, 1-cellulose-A/Z/J columns and analytical grade solvents were used as the eluent unless otherwise stated.

### Example 1: Synthesis of compounds according to the invention

The compounds of the invention can be prepared from readily available starting materials using methods and procedures known from the skilled person. It will be appreciated that where typical or preferred experimental conditions (i.e. reaction temperatures, time, moles of reagents, solvents etc.) are given, other experimental conditions can also be used unless otherwise stated. Optimum reaction conditions may vary with the particular reactants or solvents used, but such conditions can be determined by the person skilled in the art, using routine optimisation procedures.

The following of the invention are synthesized as described in the general synthetic routes described herein.

### Preparation of the intermediates

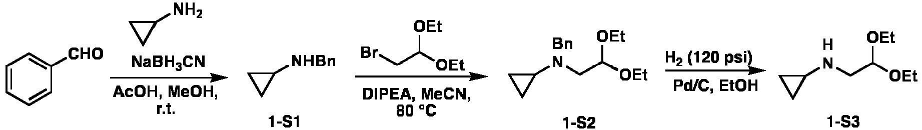

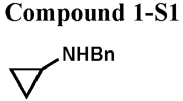

### N-benzylcyclopropanamine (1-S1)

To a stirred solution of benzaldehyde (25 g, 235.58 mmol) and cyclopropylamine (32.6 mL, 471.16 mmol) in methanol (200 mL), acetic acid (13.5 mL, 235.58 mmol) was added and stirred at room temperature for 16 h. After that NaBH₃CN (22 g, 353.3 mmol) was added portion wise at 0 °C and stirred for 3 h. After completion, the reaction mixture was quenched with ice cold water, neutralized with aqueous NaHCO₃ solution, extracted with dichloromethane, washed with brine, dried over anhydrous Na₂SO₄ and concentrated. The crude N-benzylcyclopropanamine (1-S1) (30 g, 86%) was taken directly for the next step. **¹H NMR (400 MHz, CDCl₃): *δ*** 7.39-7.24 (m, 5 H), 3.84 (s, 2 H), 2.18-2.14 (m, 1 H), 0.47-0.40 (m, 4 H) ppm. **LC-MS (ESI, m/z):** calcd for [M+H]⁺ 148.1; found: 148.1.

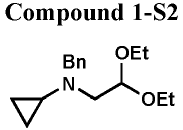

### N-benzyl-N-(2,2-diethoxyethyl) cyclopropanamine (1-S2)

To a stirred solution of *N*-benzyl cyclopropanamine (1-S1) (60 g, 407.60 mmol) and bromo acetaldehyde diethyl acetal (85 mL, 570.6 mmol) in dry acetonitrile (300 mL), DIPEA (173 mL, 1013.0 mmol) was added and stirred at 80 °C for overnight. After completion, the reaction mixture was diluted with water, extracted with ethyl acetate, washed with brine, dried over anhydrous Na₂SO₄ and concentrated. The crude product was purified by CombiFlash chromatography (5% ethyl acetate in hexane) to afford N-benzyl-N-(2,2-diethoxyethyl)cyclopropanamine **(1-S2)** as a pale yellow oil (57 g, 53%). **Physical state:** yellow oil. **¹H NMR (400 MHz, CDCl₃):** *δ* 7.39-7.24 (m, 5 H), 4.67-4.62 (m, 1 H), 3.82 (s, 2 H), 3.61-3.53 (m, 2 H), 3.47-3.42 (m, 2 H), 2.71 (d, *J* = 5.4 Hz, 2 H), 1.93-1.90 (m, 1 H), 1.18-1.15 (m, 6 H), 0.45-0.31 (m, 4 H) ppm. **LC-MS (ESI, m/z):** calcd for [M+H]⁺ 264.2; found: 263.9.

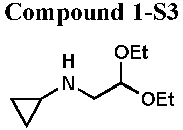

### N-(2,2-diethoxyethyl)cyclopropanamine (1-S3)

In an autoclave vessel, a solution of *N*-benzyl-*N*-(2,2-diethoxyethyl)cyclopropanamine **(1-S2)** (57 g, 216.42 mmol) in ethanol (300 ml) was degassed with argon for 15 minutes and then Pd/C (15 g) was added. The vessel was sealed and the reaction mixture was stirred under 120 psi hydrogen gas pressure for 16 h. After completion, the reaction mixture was filtered through celite bed and the filtrate was concentrated. The crude product was purified by column chromatography (SiO₂ 100-200, 25% ethyl acetate in hexane) to afford N-(2,2-diethoxyethyl)cyclopropanamine (1-S3) (20 g, 53%) as a colorless oil. **Physical state:** colorless oil. **¹H NMR (400 MHz, CDCl₃):** *δ* 4.57 (t, *J* = 5.64 Hz, 1 H), 3.73-3.65 (m, 2 H), 3.56-3.49 (m, 2 H), 2.79 (d, *J =* 5.6 Hz, 2 H), 2.15-2.12 (m, 1 H), 1.20 (t, *J =* 7.04 Hz, 6 H), 0.42-0.37 (m, 2 H), 0.34-0.30 (m, 2 H) ppm. LC-MS (ESI, m/z): calcd for [M+H]⁺ 174.1; found: 174.1

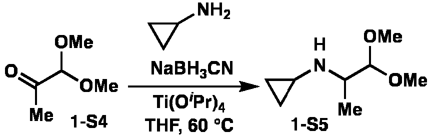

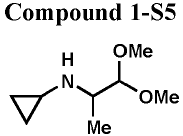

### N-(1,1-dimethoxypropan-2-yl)cyclopropanamine (1-S5)

To a stirred solution of 1,1-dimethoxypropan-2-one (1-S4, **7** g, 59.32 mmol) and cyclopropylamine (20.5 mL, 296 mmol) in anhydrous THF (100 mL), Ti(OPr)₄ (26.32 mL, 88.98 mmol) was added dropwise at 0 °C and the reaction mixture was stirred at 60 °C for 16 h. After that the reaction mixture was cooled to 0 °C and NaBH₃CN (5.58 g, 88.98 mmol) was added portion wise and stirred at room temperature for 3 h. After completion, the reaction mixture was quenched with ice cold water, extracted with ethyl acetate, washed with brine, dried over anhydrous Na₂SO₄ and concentrated. The crude product was purified by Combiflash chromatography (30-45% ethyl acetate in hexane) to afford *N*-(1,1-dimethoxypropan-2-yl)cyclopropanamine (1-S5) as a colourless oil (4.9 g, 52%). **Physical state:** colorless oil. **¹H NMR (400 MHz, DMSO-d*₆*)**: *δ* 4.09 (d, *J =* 6 Hz, 1H), 3.55 (d, *J =* 8.1 Hz, 1H), 3.38 (s, 6H), 2.93-2.87 (m, 1H), 2.08-2.02 (m, 1H), 1.08 (d, *J =* 6.2 Hz, 3H), 0.45-0.39 (m, 4H) ppm.

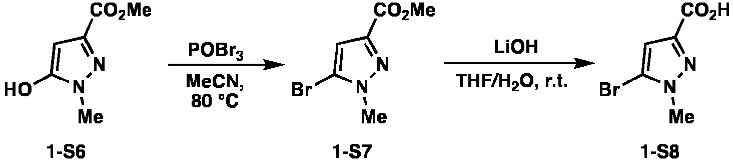

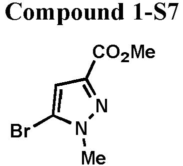

### Methyl 5-bromo-1-methyl-1H-pyrazole-3-carboxylate (1-S7)

In a reaction tube, to a stirred solution of methyl 5-hydroxy-1-methyl-1*H*-pyrazole-3-carboxylate (1-S6) (5 g, 32.02 mmol) in acetonitrile (50 mL), POBr₃ (27.54 g, 96.07 mmol) was added. The tube was sealed and the mixture was stirred at 80 °C for overnight. After completion, the reaction mixture was cooled and quenched by slowly adding the reaction mixture into ice cooled saturated NaHCO₃ solution, extracted with ethyl acetate, washed with brine, dried over anhydrous Na₂SO₄ and concentrated. The crude methyl 5-bromo-1-methyl-1H-pyrazole-3-carboxylate **(1-S7)** was taken directly for the next step (3.7 g, 53%). **¹H NMR (400 MHz, CDCl₃):** *δ* 6.83 (s, 1 H), 3.95 (s, 3 H), 3.91 (s, 3 H) ppm. **LC-MS (ESI, m/z):** calcd for [M+H]⁺ 219.1; found: 219.2, 221[M+2+H]⁺.

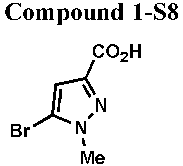

### 5-bromo-1-methyl-1H-pyrazole-3-carboxylic acid (1-S8)

To a stirred solution of 5-bromo-1-methyl-1*H*-pyrazole-3-carboxylate (1-S7) in THF: H₂O (3:1) (20 mL), LiOH•H₂O (3.07 g, 73.05 mmol) was added and the reaction mixture was stirred at room temperature for 3 h. After completion, the reaction mixture was concentrated, acidified with aqueous 6N HCl, extracted with ethyl acetate, washed with brine, dried over anhydrous Na₂SO₄ and concentrated. The crude 5-bromo-1-methyl-1H-pyrazole-3-carboxylic acid (1-S8) was taken directly for the next step (5.2 g, 69%). **¹H NMR (400 MHz, DMSO-d*₆*):** *δ* 12.86 (br s, 1 H), 6.86 (s, 1 H), 3.88 (s, 3 H) ppm. **LC-MS (ESI, m/z):** calcd for [M+H]⁺ 203.1; found: 203.0, 205.0 [M+2+H]⁺.

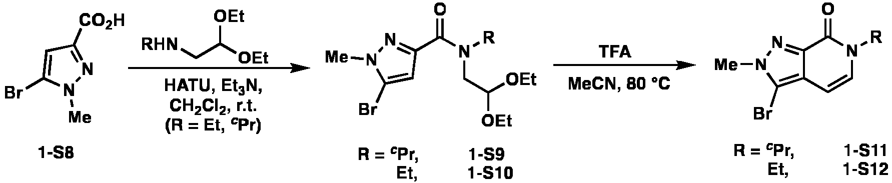

### General procedure A1 of amide coupling of pyrazole carboxylic acid 1-S8 and alkylamines

To a stirred solution of 5-bromo-1-methyl-1*H*-pyrazole-3-carboxylic acid (1-S8) (1.0 equiv.) in dichloromethane (0.3 M), HATU (2.0 equiv.) followed by Et₃N (3.0 equiv.) was added and stirred for 10 minutes. After that *N*-(2,2-diethoxyethyl) alkyl amine or *N*-(2,2-dimethoxyethyl) alkyl amine (0.9-1.5 equiv.) was added and stirred at room temperature for 3-12 h. After completion, the reaction mixture was extracted with dichloromethane, washed with water, brine, dried over anhydrous Na₂SO₄ and concentrated. The crude amide **(1-S9, 1-S10)** (crude) was taken directly for the next step.

### General procedure B1 of pyrazolo[3,4-c]pyridin-7-one derivatives (1-S11 & 1-S12) formation

To a stirred solution of 5-bromo-*N*-alkyl-*N*-(2,2-diethoxyethyl)-1-methyl-1H-pyrazole-3-carboxamide **(1-S9, 1-S10)** (1.0 equiv.) in anhydrous acetonitrile (0.4-0.75 M), trifluroacetic acid (4.0-5.0 equiv.) was added and the reaction mixture was stirred at 80 °C for 16 h. After completion, the reaction mixture was concentrated, quenched with aqueous NaHCO₃ solution, extracted with ethyl acetate, wash with brine, dried over anhydrous Na₂SO₄ and concentrated. The crude product was purified by CombiFlash chromatography (50%-70% ethyl acetate in hexane) to afford the 3-bromo-6-alkyl-2-methyl-2,6-dihydro-7*H*-pyrazolo[3,4-c] pyridin-7-one (2.5 g, 12%) **(1-S11, 1-S12).**

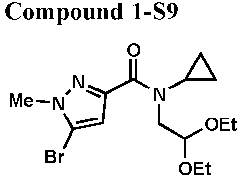

### 5-bromo-N-cyclopropyl-N-(2,2-diethoxyethyl)-1-methyl-1H-pyrazole-3-carboxamide (1-S9)

Following **General Procedure A1** on 75.61 mmol scale with 5-bromo-1-methyl-1*H*-pyrazole-3-carboxylic acid **(1-S7)** and N-(2,2-diethoxyethyl)cyclopropanamine **(1-S3).** The crude 5-bromo-N-cyclopropyl-*N*-(2,2-diethoxyethyl)-1-methyl-1*H*-pyrazole-3-carboxamide **(1-S9)** (27 g, crude) was taken directly for the next step. **LC-MS (ESI, m/z):** calcd for [M+H]⁺ 360.1; found: 360.29.

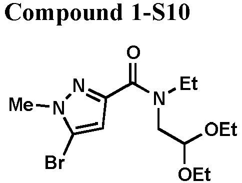

### 5-bromo-N-(2,2-diethoxyethyl)-N-ethyl-1-methyl-1H-pyrazole-3-carboxamide (1-S10)

Following **General Procedure A1** on 13.17 mmol scale with 5-bromo-1-methyl-1H-pyrazole-3-carboxylic acid **(1-S7)** and 2,2-diethoxy-*N*-ethylethan-1-amine. The reaction crude afforded 5-bromo-N-(2,2-diethoxyethyl)-N-ethyl-1-methyl-1H-pyrazole-3-carboxamide (1-S10) as a brown gum (4.5 g, 98%). **LC-MS (ESI, m/z):** calcd for [M+H]⁺ 348.1; found: 348, 350 [M+2+H]⁺.

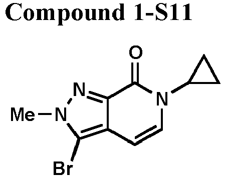

### 3-bromo-6-cyclopropyl-2-methyl-2,6-dihydro-7H-pyrazolo[3,4-c]pyridin-7-one (1-S11)

Following **General Procedure B1** on 75 mmol scale with 5-bromo-N-cyclopropyl-N-(2,2-diethoxyethyl)-1-methyl-1H-pyrazole-3-carboxamide (1-S9). Purification by CombiFlash chromatography (70% ethyl acetate in hexane) afforded 2.5 **g** (12%) of3-bromo-6-cyclopropyl-2-methyl-2,6-dihydro-7H-pyrazolo[3,4-c] pyridin-7-one **(1-S11)** as a brown solid. **Physical state:** brown solid. **¹H NMR (400 MHz, DMSO-d*₆*):** *δ* 7.08 (d, *J* = 7.4 Hz, 1H), 6.22 (d, *J* = 7.4 Hz, 1H), 4.05 (s, 3 H), 3.24-3.22 (m, 1H), 1.02-0.97 (m, 2H), 0.82-0.75 (m, 2H) ppm. **LC-MS (ESI, m/z):** calcd for [M+H]⁺268.0; found: 268.0.

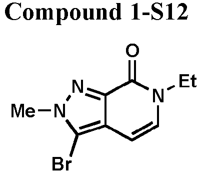

### 3-bromo-6-ethyl-2-methyl-2,6-dihydro-7H-pyrazolo[3,4-c]pyridin-7-one(1-S12)

Following **General Procedure B1** on 12.92 mmol scale with 5-bromo-N-(2,2-diethoxyethyl)-*N*-ethyl-1-methyl-1*H*-pyrazole-3 -carboxamide (1-S10). Purification by column chromatography (SiO₂ 100-200, 50% ethyl acetate in hexane) afforded 1.5 g (45%) of 3-bromo-6-cyclopropyl-2-methyl-2,6-dihydro-7H-pyrazolo[3,4-c] pyridin-7-one (1-S12) as an off-white solid. **Physical state:** off-white solid. **¹H NMR (400 MHz, DMSO-d*₆*)**: *δ* 7.22 (d, *J* = 7.32 Hz, 1 H), 6.29 (d, *J* = 7.28 Hz, 1 H), 4.06 (s, 3 H), 3.93 (q, *J* = 7.12 Hz, 2 H), 1.20 (t, *J* = 7.04 Hz, 3 H) ppm; **LC-MS (ESI,** m/z): calcd for [M+H]⁺256.1; found: 256.12, 258.14 [M+2+H]⁺.

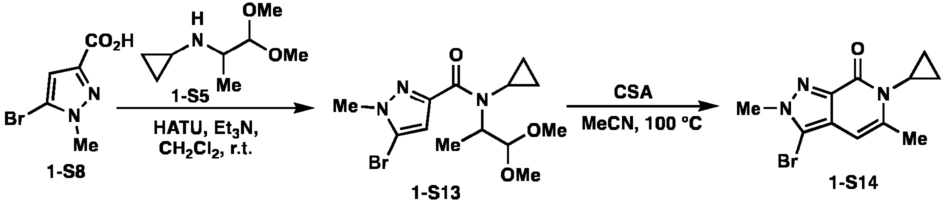

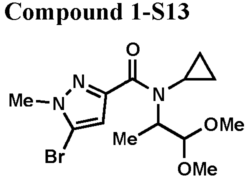

### 5-bromo-N-cyclopropyl-N-(1,1-dimethoxypropan-2-yl)-1-methyl-1H-pyrazole-3-carboxamide (1-S13)

Following **General Procedure A1** on 13.79 mmol scale with 5-bromo-1-methyl-1H-pyrazole-3-carboxylic acid **(1-S8)** and *N*-(1,1-dimethoxypropan-2-yl)cyclopropanamine **(1-S5).** After completion, the reaction mixture was quenched with aqueous NaHCO₃ solution, extracted with dichloromethane, washed with brine, dried over anhydrous Na₂SO₄ and concentrated. The crude 5-bromo-N-cyclopropyl-N-(1,1-dimethoxypropan-2-yl)-1-methyl-1H-pyrazole-3-carboxamide **(1-S13)** was taken directly for the next step (2.5 g crude). **LC-MS (ESI, m/z):** calcd for [M+H]⁺346.1; found: 346.0.

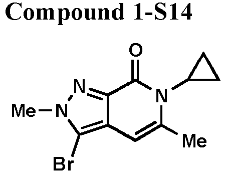

### 3-bromo-6-cyclopropyl-2,5-dimethyl-2,6-dihydro-7H-pyrazolo[3,4-c]pyridin-7-one (1-S14)

To a stirred solution of 5-bromo-*N*-cyclopropyl-*N*-(1,1-dimethoxypropan-2-yl)-1-methyl-1*H-*pyrazole-3-carboxamide **(1-S13)** (1.5 g, 4.33 mmol) in acetonitrile (10 mL), camphorsulfonic acid (0.50 g, 2.16 mmol) was added and stirred at 100 °C for 16 h. After completion, the reaction mixture was quenched with aqueous NaHCO₃ solution, extracted with ethyl acetate, washed with brine, dried over anhydrous Na₂SO₄ and concentrated. The crude product was purified by Combiflash chromatography (70 % ethyl acetate in hexane) to afford 3-bromo-6-cyclopropyl-2,5-dimethyl-2,6-dihydro-7H-pyrazolo[3,4-c]pyridin-7-one **(1-S14)** as a brown solid (0.1 g, 8%). **¹H NMR (400 MHz, DMSO-d*₆*)**: *δ* 6.13 (s, 1H), 4.01 (s, 1H), 2.90-2.80 (m, 1H), 2.43 (s, 3H), 1.15-1.10 (m, 2H), 0.80-0.70 (m, 2H) ppm. **LC-MS (ESI, m/z): calcd** for [M+H]⁺282.1; found: 282.0.

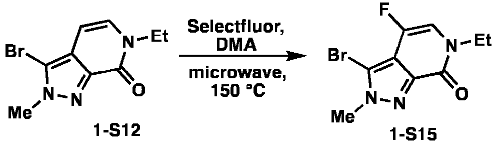

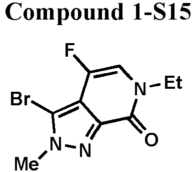

### 3-bromo-6-ethyl-4-fluoro-2-methyl-2,6-dihydro-7H-pyrazolo[3,4-c]pyridin-7-one (1-S15)

In a reaction tube, to a stirred solution of 3-bromo-6-ethyl-2-methyl-2,6-dihydro-7*H-*pyrazolo[3,4-c]pyridin-7-one **(1-S12)** (1 g, 3.92 mmol) in anhydrous dimethylacetamide (DMA) (20 mL), Selectflour (1.67 g, 4.71 mmol) was added. The tube was sealed and the reaction mixture was stirred at 150 °C for 20 minutes under microwave irradiation. After completion, the reaction mixture was cooled, quenched with aqueous NaHCO₃ solution, extracted with ethyl acetate, washed with brine, dried over anhydrous Na₂SO₄ and concentrated. The crude product was purified by CombiFlash chromatography (40% ethyl acetate in hexane) to afford 3-bromo-6-ethyl-4-fluoro-2-methyl-2,6-dihydro-7*H*-pyrazolo[3,4-c]pyridin-7-one (250 mg, 23%) **(1-S15)** as an off white solid. **¹H NMR (400 MHz, CDCl₃):** *δ* 6.77 (d, *J* = 5.8 Hz, 1 H), 4.11 (s, 3 H), 3.99 (q, *J* = 7.16 Hz, 2 H), 1.32 (t, *J* = 7.24 Hz, 3 H) ppm. LC-MS **(ESI, m/z):** calcd for [M+H]⁺274.0; found: 273.8.

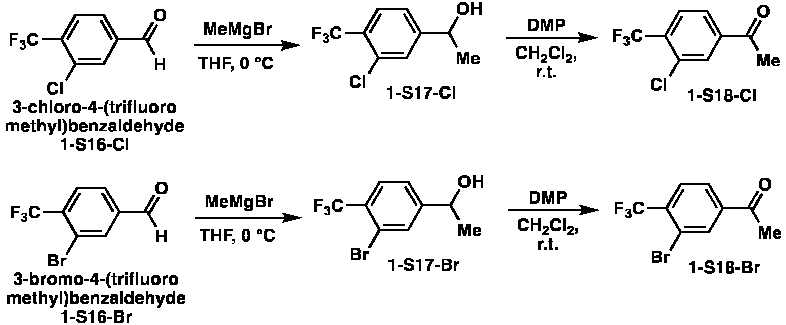

Compound **1-S18-Cl** was prepared via the same procedure as that of **1-S18-Br.**

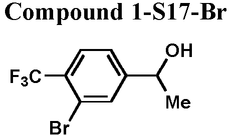

### 1-(3-bromo-4-(trifluoromethyl)phenyl)ethan-1-ol (1-S17-Br)

To a stirred solution of 3-bromo-4-(trifluoromethyl)benzaldehyde **(1-S16-Br)** (300 mg, 1.19 mmol) in anhydrous THF (5 mL), MeMgBr (3 M in THF) (0.4 mL, 1.19 mmol) was added dropwise at 0 °C and stirred for 30 minutes. After completion, the reaction mixture was quenched by the addition of aqueous saturated NH₄Cl solution, extracted with ethyl acetate, washed with brine, dried over anhydrous Na₂SO₄ and concentrated. The crude 1-(3-bromo-4-(trifluoromethyl)phenyl)ethan-1-ol **(1-S17-Br)** obtained as a light green liquid (300 g, 75%) was taken directly for the next step. **Notes:** The reaction was performed in 5 g scale and 4 g of 1-(3-bromo-4-(trifluoromethyl)phenyl)ethan-1-ol **(1-S17-Br)** was obtained (75%). **Physical State:** light green oil. **¹H NMR (400 MHz, CDCl₃):** *δ* 7.73 (s, 1H), 7.65 (d, *J =* 8.08 Hz, 1H), 7.39 (d, *J =* 8.08 Hz, 1H), 4.92 (q, *J =* 6.4 Hz, 1H), 1.88 (s, 1H), 1.49 (d, *J =* 6.4 Hz, 3H) ppm.

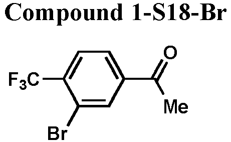

### 1-(3-bromo-4-(trifluoromethyl)phenyl)ethan-1-one (1-S18-Br)

To a stirred solution of 1-(3-bromo-4-(trifluoromethyl)phenyl)ethan-1-ol **(1-S17-Br)** (4 g, 14.87 mmol) in dichloromethane (30 mL), Dess-Martin periodinane (6.31 g, 14.87 mmol) was added at 0 °C and the reaction mixture was stirred at room temperature for 1 h. After completion, the reaction mixture was quenched with aqueous saturated NaHCO₃ solution, extracted with dichloromethane, washed with brine, dried over anhydrous Na₂SO₄ and concentrated. The crude product was purified by CombiFlash chromatography (5% ethyl acetate in hexane) to afford 1-(3-bromo-4-(trifluoromethyl)phenyl)ethan-1-one **(1-S18-Br) (2** g, 50%) as a pale yellow liquid. **Physical State:** pale yellow oil. **¹H NMR (400 MHz, CDCl₃):** *δ* 8.25 (s, 1H), 7.95 (d, *J=* 8.16 Hz, 1H), 7.79 (d, *J =* 8.16 Hz, 1H), 2.63 (s, 3H) ppm.

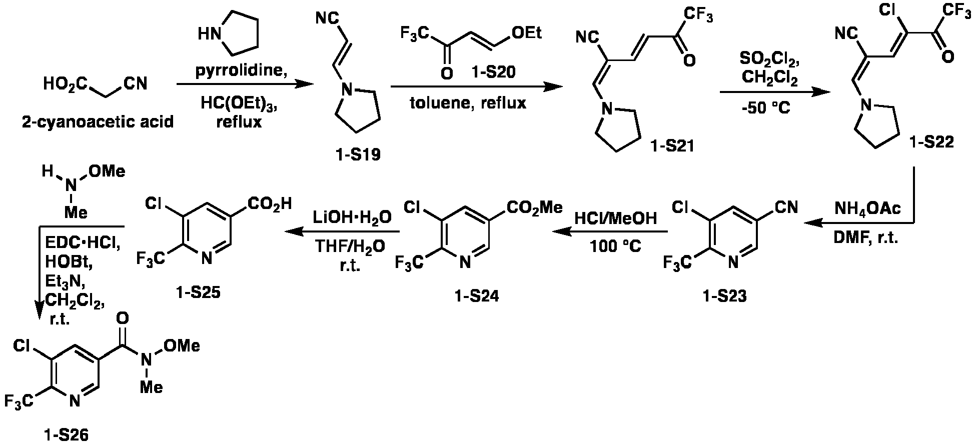

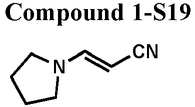

### 3-(pyrrolidin-1-yl)acrylonitrile (1-S19)

To a stirred mixture of 2-cyanoacetic acid (15 g, 176.47 mmol) and pyrrolidine (14.48 mL, 176.47 mmol), triethyl orthoformate (26.15 g, 176.47 mmol) was added and the resulting mixture was refluxed for 2 h. After completion, the mixture was evaporated under vacuo and extracted with dichloromethane, washed with aqueous NaHCO₃ solution, brine, dried over anhydrous Na₂SO₄ and concentrated. The crude mixture was recrystallized in diethyl ether to afford 3-(pyrrolidin-1-yl) acrylonitrile **(1-S19)** as a yellow solid (12 g, 56%). **Physical state:** yellow solid. **¹H NMR (400 MHz, DMSO-d*₆*)**: *δ* 7.37 (d, *J* = 13.4 Hz, 1 H), 3.72 (d, *J* = 13.4 Hz, 1 H), 3.55-3.25 (m, 2 H), 2.98 (br s, 2 H), 1.90-1.70 (m, 4 H) ppm. **LC-MS (ESI, m/z):** calcd for [M+H]⁺ 123.1; found: 123.0.

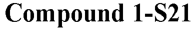

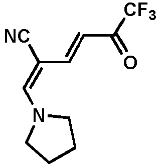

### 6,6,6-trifluoro-5-oxo-2-(pyrrolidin-1-ylmethylene)hex-3-enenitrile (1-S21)

To a stirred solution of 3-(pyrrolidin-1-yl)acrylonitrile (1-S19) (5 g, 40.98 mmol) in toluene (25 mL), 4-ethoxy-1,1,1-trifluorobut-3-en-2-one (1-S20) (40.8 mL, 286.86 mmol) was added and the reaction mixture was stirred at 110 °C for 3 h. After completion, the reaction mixture was cooled, extracted with ethyl acetate, washed with brine, dried over anhydrous Na₂SO₄ and concentrated. The crude product was purified by CombiFlash chromatography (5% ethyl acetate in hexane) to afford 6,6,6-trifluoro-5-oxo-2-(pyrrolidin-1-yl-methylene)-hex-3-enenitrile (1-S21) as a yellow solid (6.5g, 65%). Physical state: yellow solid. LC-MS (ESI, m/z): calcd for [M+H]⁺245.1; found: 244.9.

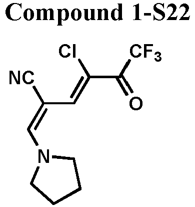

### 4-chloro-6,6,6-trifluoro-5-oxo-2-(pyrrolidin-1-ylmethylene)hex-3-enenitrile (1-S22)

To a stirred solution of 6,6,6-trifluoro-5-oxo-2-(pyrrolidin-1-yl-methylene)-hex-3-enenitrile (1-**S21)** (6.5 g, 26.64 mmol) in dichloromethane (30 mL), sulfuryl chloride (2.5 mL, 31.97 mmol) was added dropwise at -50 °C and the reaction mixture was stirred at that temperature for 30 minutes. After that Et₃N (4.5 mL, 31.97 mmol) was added dropwise at -50 °C and the reaction mixture was stirred at that temperature for 60 minutes. After completion, the mixture was diluted with dichloromethane, washed with aqueous 10% NaHCO₃ solution, brine, dried over anhydrous Na₂SO₄ and concentrated. The crude product was purified by CombiFlash chromatography (5% ethyl acetate in hexane) to afford 4-chloro-6,6,6-trifluoro-5-oxo-2-(pyrrolidin-1-yl-methylene)hex-3-enenitrile **(1-S22)** as a yellow solid (4 g, 54%). **Physical state:** yellow solid. **¹H NMR (400 MHz, DMSO-d*₆*):** *δ* 8.34 (s, 1 H), 7.58 (s, 1 H), 3.92 (t, *J =* 7 Hz, 2 H), 3.74 (t, *J=* 7 Hz, 2 H), 2.04-1.97 (m, 2 H), 1.87-1.81 (m, 2 H) ppm. **LC-MS (ESI,** m/z): calcd for [M+H]⁺279.1; found: 279.1.

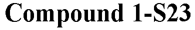

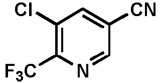

### 5-chloro-6-(trifluoromethyl)nicotinonitrile (1-S23)

To a stirred solution of 4-chloro-6,6,6-trifluoro-5-oxo-2-(pyrrolidin-1-ylmethylene)hex-3-enenitrile **(1-S22)** (15 g, 53.95 mmol) in anhydrous DMF (120 mL), ammonium acetate (19.1, 248.16 mmol) was added and the reaction mixture was stirred at room temperature for 16 h. After completion, the resulting mixture was diluted with cold water, extracted with ethyl acetate, washed with brine, dried over anhydrous Na₂SO₄ and concentrated. The crude product was purified by CombiFlash chromatography (5% ethyl acetate in hexane) to afford 5-chloro-6-(trifluoromethyl)nicotinonitrile **(1-S23)** as an off white solid (5 g, 45%). **Physical state: off-**white solid. **¹H NMR (400 MHz, CDCl₃):** *δ* 8.83 (s, 1 H), 8.16 (s, 1 H) ppm. **LC-MS (ESI, m/z):** calcd for [M-H]⁻ 205.0; found: 205.2.

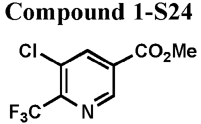

### methyl 5-chloro-6-(trifluoromethyl)nicotinate (1-S24)

In a reaction tube, a solution of 5-chloro-6-(trifluoromethyl)nicotinonitrile **(1-S23)** (2.5 g, 12.13 mmol) in methanol solution of HCl (4 M) (20 mL) was stirred at 100 °C for 3 h. After completion, the reaction mixture was concentrated, quenched with aqueous NaHCO₃ solution, extracted with ethyl acetate, washed with brine, dried over anhydrous Na₂SO₄ and concentrated. The crude product was purified by CombiFlash chromatography (5% ethyl acetate in hexane) to afford methyl 5-chloro-6-(trifluoromethyl)nicotinate **(1-S24)** as an off white solid (1.7 g, 58%). **Physical state:** off-white solid. **LC-MS (ESI, m/z):** calcd for [M+H]⁺ 240.1; found: 240.1.

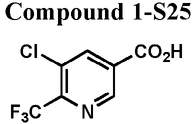

### 5-chloro-6-(trifluoromethyl)nicotinic acid (1-S25)

To a stirred solution of methyl 5-chloro-6-(trifluoromethyl)nicotinate **(1-S24)** (1.6 g, 6.69 mmol) in THF-water (3:1) (20 mL), LiOH•H₂O (0.77 g, 33.48 mmol) was added and the reaction mixture was stirred at room temperature for 24 h. After completion, the reaction mixture was concentrated, quenched with aqueous 5% H₃PO₄ solution, extracted with ethyl acetate, washed with brine, dried over anhydrous Na₂SO₄ and concentrated to afford 5-chloro-6-(trifluoromethyl) nicotinic acid **(1-S25)** as an off white solid (1.38 g, 92%). **Physical state:** off-white solid. **¹H NMR (400 MHz, DMSO-d*₆*)**: *δ* 14.2 (br s, 1H), 9.09 (s, 1H), 8.57 (s, 1H) ppm. **LC-MS (ESI, m/z):** calcd for [M-H]⁻ 224.0; found: 224.0.

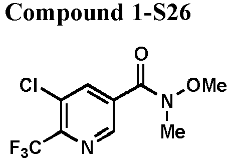

### 5-fluoro-N-methoxy-N-methyl-6-(trifluoromethyl)nicotinamide (1-S26)

To a stirred solution of 5-fluoro-6-(trifluoromethyl)nicotinic acid **(1-S25)** (1.5 g, 7.85 mmol) in dichloromethane (20 mL), Et₃N (3.23 mL, 23.56 mmol) followed by HOBT (2.13 g, 15.71 mmol) and EDC•HCl (3.0 g, 15.70 mmol) were added. After few minutes of stirring Weinrab salt (0.92 g, 97.54 mmol) was added and the reaction mixture was stirred at room temperature for 4 h. After completion, the reaction mixture was diluted with water, extracted with dichloromethane, washed with aqueous NaHCO₃ solution, brine, dried over anhydrous Na₂SO₄ and concentrated. The crude product was purified by CombiFlash chromatography (10% ethyl acetate in hexane) to afford 5-fluoro-*N*-methoxy-*N*-methyl-6-(trifluoromethyl)nicotinamide **(1-S26)** as a colourless oil (1.2 g, 61%). **Physical state:** colorless oil. **¹H NMR (400 MHz, CDCl₃):** *δ* 8.87 (s, 1H), 8.19 (s, 1H), 3.57 (s, 3H), 3.40 (s, 3H) ppm.

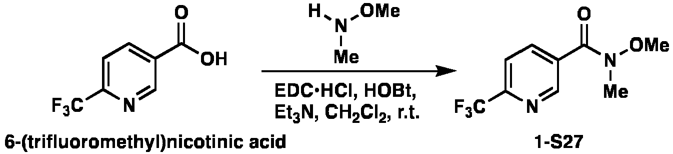

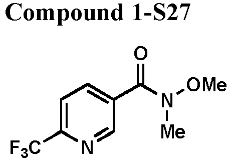

### N-methoxy-N-methyl-6- (trifluoromethyl) nicotinamide (1-S27)

To a stirred solution of 6-(trifluoromethyl)nicotinic acid (5 g, 26.16 mmol) and triethylamine (10.9 mL, 78.49 mmol) in dichloromethane (25 mL), were added EDC•HCl (10.03 g, 52.33 mmol) and HOBT (7.07 g, 52.33 mmol) and stirred for 5 minutes. After that Weinreb salt (3.06 g, 31.4 mmol) was added and stirred at room temperature for 12 h. After completion, the reaction mixture was extracted with dichloromethane, washed with aqueous saturated NaHCO₃ solution, brine, dried over anhydrous Na₂SO₄ and concentrated. The crude product was purified by column chromatography (SiO₂ 100-200, 20% ethyl acetate in hexane) to afford N-methoxy-*N*-methyl-6-(trifluoromethyl)nicotinamide (1-S27) as a light yellow oil (5.7 g, 93%). **Physical state:** light yellow **oil. ¹H NMR (400 MHz, CDCl₃):** *δ* 9.04 (s, 1H), 8.20 (d, *J =* 8 Hz, 1H), 7.74 (d, *J= 8.4* Hz, 1H), 3.56 (s, 3H), 3.41 (s, 3H) ppm. **LC-MS (ESI, m/z):** calcd for [M+H]⁺ 235.1; found: 235.14.

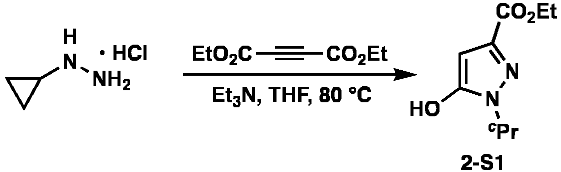

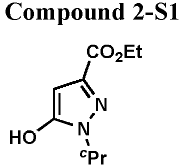

### ethyl 1-cyclopropyl-5-hydroxy-1H-pyrazole-3-carboxylate (2-S1)

To a stirred solution of cyclopropylhydrazine hydrochloride (14 g, 128.95 mmol) in anhydrous THF (100 mL), Et₃N (44.9 mL, 322.37 mmol) was added and the reaction mixture was stirred at room temperature for 10 minutes. After that diethyl but-2-ynedioate (20.64 mL, 128.95 mmol) was added dropwise and the reaction mixture was stirred at 80 °C for 16 h. After completion, the reaction mixture was filtered, and the filtrate was concentrated. The crude product was purified by CombiFlash chromatography (70% ethyl acetate in hexane) to afford ethyl 1-cyclopropyl-5-hydroxy-1*H*-pyrazole-3-carboxylate **(2-S1)** as an off-white solid (3.8 g, 15%). **Physical state:** off-white solid. **¹H NMR (400 MHz, DMSO-d*₆*):** *δ* 11.42 (s, 1H), 5.75 (s, 1H), 4.20 (q, *J =* 7.1 Hz, 2H), 3.49-3.43 (m, 1H), 1.25 (t, *J =* 7.1 Hz, 3H), 1.01-0.91 (m, 4H) ppm. **LC-MS (ESI, m/z):** calcd for [M+H]⁺ 197.1; found: 197.05.

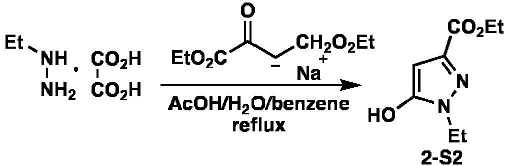

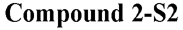

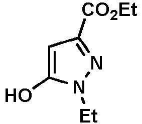

### ethyl 1-ethyl-5-hydroxy-1H-pyrazole-3-carboxylate (2-S2)

To a stirred solution of sodium diethyloxalacetate (12.8 g, 60.90 mmol) in benzene (175 mL), acetic acid (100 mL) was added dropwise and stirred at room temperature for 1 h. After that ethyl hydrazine oxalate (9.1g, 60.90 mmol) in warm water (40 mL) was added dropwise and the reaction mixture was refluxed for 36 h. After completion, the reaction mixture was cooled to room temperature, quenched with cold water, extracted with ethyl acetate, washed with brine, dried over anhydrous Na₂SO₄ and concentrated. The crude product was purified by Combiflash chromatography (65-75% ethyl acetate in hexane) to afford ethyl 1-ethyl-5-hydroxy-1*H-*pyrazole-3-carboxylate **(2-S2)** as an off-white solid (7.5 g, 67%). **Physical state:** off-white solid. **¹H NMR (400 MHz, DMSO-d*₆*)**: *δ* 11.36 (s, 1H), 4.20 (q, *J* = 7.1 Hz, 2H), 3.94 (q, *J* = 7.1 Hz, 2H), 1.27-1.15 (m, 6H), ppm. **LC-MS (ESI, m/z):** calcd for [M+H]⁺ 185.1; found: 185.0.

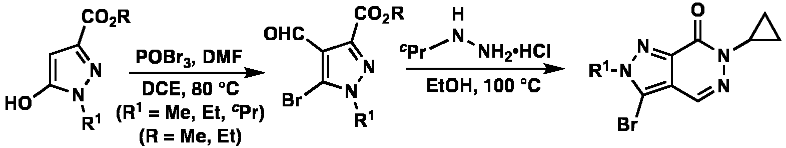

### General procedure A2 of preparation of 3-ester-5-hydroxy-1-alkyl-1H-pyrazole

To a stirred solution of alkyl (Me, Et) 5-hydroxy-1-alkyl-1*H*-pyrazole-3-carboxylate (1-cyclopropyl-1*H*-pyrazole, **2-S1;** 1-ethyl-1*H*-pyrazole, **2-S2)** (1.0 equiv. 200 mg, 1.01 mmol) in 1,2-dichloroethane (0.15-1.55 M 7 mL), POBr₃ (5.0-6.0 equiv. 1736 mg, 6.05 mmol) followed by DMF (2.0 equiv. 0.16 mL, 2.02 mmol) was added and the mixture was stirred at 90 °C for 16 h. After completion the mixture was cooled, poured into crushed ice slowly, extracted with methylene chloride, washed with brine, dried over anhydrous Na₂SO₄ and concentrated. The crude product was purified by CombiFlash chromatography (20% ethyl acetate in hexane) to afford ethyl 5-bromo-4-formyl-1-alkyl-1H-pyrazole-3-carboxylate (2-S3, 2-S4, 2-S5).

### General procedure B2 of pyrazolo[3,4-d]pyridazin-7-one derivatives (2-S6-2-S8) formation

In a reaction tube, to a stirred solution of alkyl (Me, Et) 5-bromo-4-formyl-1-alkyl(R¹)-1*H-*pyrazole-3-carboxylate **(2-S3, 2-S4, 2-S5)** (1.0 equiv. 1.8 g, 6.23 mmol) in ethanol (0.4 M 15 mL), cyclopropyl hydrazine•HCl (1.0 equiv. 0.68 g, 6.23 mmol) was added at 25 °C. The tube was sealed, and the mixture was stirred at 100-110 °C for 3 h. After completion, the reaction mixture was concentrated, quenched with aqueous NaHCO₃ solution, extracted with ethyl acetate, washed with brine, dried over anhydrous Na₂SO₄ and concentrated. The crude product was purified by CombiFlash chromatography to afford 3-bromo-6-R²-2-R¹-2,6-dihydro-7*H-*pyrazolo[3,4-d]pyridazin-7-one **(2-S6, 2-S7, 2-S8)** as an off-white solid (0.3 g, 16%).

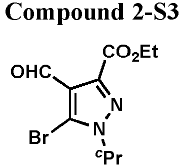

### ethyl 5-bromo-1-cyclopropyl-4-formyl-1H-pyrazole-3-carboxylate (2-S3)

Following **General Procedure A2** on 4.08 mmol scale with ethyl 1-cyclopropyl-5-hydroxy-1H-pyrazole-3-carboxylate **(2-S1)** in DCE (15 mL) at 90 °C. Purification by CombiFlash chromatography (30% ethyl acetate in hexane) to afford ethyl 5-bromo-1-cyclopropyl-4-formyl-1H-pyrazole-3-carboxylate **(2-S3)** as an off-white solid (0.25 g, 21%). **Physical state:** off-white solid. **¹H NMR (400 MHz, CDCl₃):** *δ* 10.38 (s, 1H), 4.45 (q, *J* = 7.0 Hz, 2H), 3.64-3.58 (m, 1H), 1.40 (t, *J =* 7.1 Hz, 3H), 1.35-1.31 (m, 2H), 1.21-1.16 (m, 2H) ppm. **LC-MS (ESI, m/z):** calcd for [M+H]⁺287.1; found: 287.0, 287.2 [M+H+2]⁺.

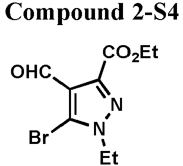

### ethyl 5-bromo-1-ethyl-4-formyl-1H-pyrazole-3-carboxylate (2-S4)

Following **General Procedure A2** on 54.29 mmol scale with ethyl 1-ethyl-5-hydroxy-1H-pyrazole-3-carboxylate **(2-S2)** in DCE (35 mL) at 90 °C. Purification by Combiflash chromatography (70% ethyl acetate in hexane) to afford ethyl 5-bromo-1-ethyl-4-formyl-1H-pyrazole-3-carboxylate **(2-S4)** as an off-white solid (1.5 g, 15%). **Physical state:** off-white solid. **¹H NMR (400 MHz, DMSO-d*₆*): *δ* 10.23 (s,** 1H), 4.39-4.28 (m, 4H), 1.38-1.13 (m, 6H) ppm. **LC-MS (ESI, m/z):** calcd for [M+H]⁺275.1; found: 275.0, 276.9 [M+H+2]⁺.

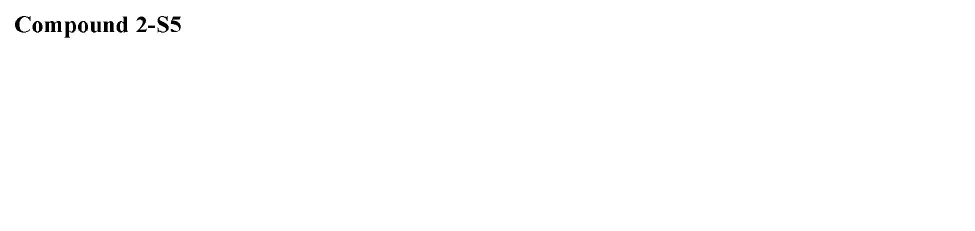

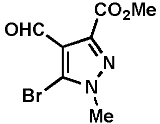

### methyl 5-bromo-4-formyl-1-methyl-1H-pyrazole-3-carboxylate (2-S5)

Following **General Procedure A2** on 38.43 mmol scale with methyl 5-hydroxy-1-methyl-1H-pyrazole-3-carboxylate in DCE (70 mL) at 90 °C. Purification by CombiFlash Chromatography (30% ethyl acetate in hexane) to afford methyl 5-bromo-4-formyl-1-methyl-1*H*-pyrazole-3-carboxylate **(2-S5)** as an off-white solid (1 g, 11%). **Physical state:** off-white solid. **¹H NMR (400 MHz, DMSO-d*₆*)**: *δ* 10.22 (s, 1H), 3.94 (s, 3H), 3.88 (s, 3H) ppm. **LC-MS (ESI, m/z):** calcd for [M+H]⁺ 247.1; found: 247.0, 248.79 [M+2+H]⁺.

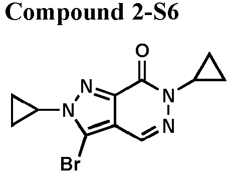

### 3-bromo-2,6-dicyclopropyl-2,6-dihydro-7H-pyrazolo[3,4-d]pyridazin-7-one (5-S6)

Following **General Procedure B2** on 4.18 mmol scale with ethyl 5-bromo-1-cyclopropyl-4-formyl-1*H*-pyrazole-3-carboxylate **(2-S3)** and cyclopropylhydrazine•HCl. Purification by CombiFlash chromatography (50% ethyl acetate in hexane) to afford 3-bromo-2,6-dicyclopropyl-2,6-dihydro-7H-pyrazolo[3,4-d]pyridazin-7-one **(2-S6)** as an off-white solid (0.58 g, 47%). **Physical state:** off-white solid. **¹H NMR (400 MHz, DMSO-d*₆*):** *δ* 8.17 (s, 1H), 4.02-3.98 (m, 1H), 3.94-3.90 (m, 1H), 1.29-1.19 (m, 4H), 0.95-0.92 (m, 4H) ppm. **LC-MS (ESI, m/z):** calcd for [M+H]⁺295.1; found: 295.29, 297.27 [M+H+2]⁺.

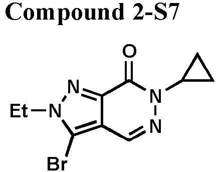

### 3-bromo-6-cyclopropyl-2-ethyl-2,6-dihydro-7H-pyrazolo[3,4-d]pyridazin-7-one (2-S7)

Following **General Procedure B2** on 5.45 mmol scale with ethyl 5-bromo-1-ethyl-4-formyl-1H-pyrazole-3-carboxylate **(2-S4)** and cyclopropylhydrazine • HCl. Purification by Combiflash chromatography (75% ethyl acetate in hexane) to afford 3-bromo-6-cyclopropyl-2-ethyl-2,6-dihydro-7H-pyrazolo[3,4-d]pyridazin-7-one (2-S7) as off-white solid (600 mg, 39%). **Physical state:** off-white solid. **¹H NMR (400 MHz, DMSO-d*₆*):** *δ* 8.18 (s, 1H), 4.45 (q, *J =* 7.2 Hz, 2H), 3.93-3.90 (m, 1H), 1.44 (t, *J* = 7.2 Hz, 3H), 0.96-0.92 (m, 4H) ppm. **LC-MS (ESI, m/z):** calcd for [M+H]⁺283.1; found: 282.7.

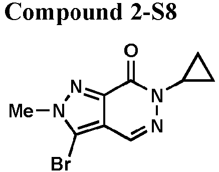

### 3-bromo-6-cyclopropyl-2-methyl-2,6-dihydro- 7H-pyrazolo[3,4-d]pyridazin-7-one (2-S8)

Following **General Procedure B2** on 5.45 mmol scale with ethyl 5-bromo-1-ethyl-4-formyl-1*H*-pyrazole-3-carboxylate **(2-S5)** and cyclopropylhydrazine • HCl (5.0 equiv.). Purification by Combiflash chromatography (60% ethyl acetate in hexane) to afford 3-bromo-6-cyclopropyl-2-methyl-2,6-dihydro-7*H*-pyrazolo[3,4-d]pyridazin-7-one **(2-S8)** as an off-white solid (0.7 g, 54%). **Physical state:** off-white solid. **¹H NMR (400 MHz, DMSO-d*₆*)**: *δ* 8.17 (s, 1H), 4.11 (s, 3H), 3.96-3.92 (m, 1H), 0.98-0.90 (m, 4H) ppm. **LC-MS (ESI, m/z):** calcd for [M+H]⁺269.1; found: 269.1, 271.12 [M+H+2]⁺.

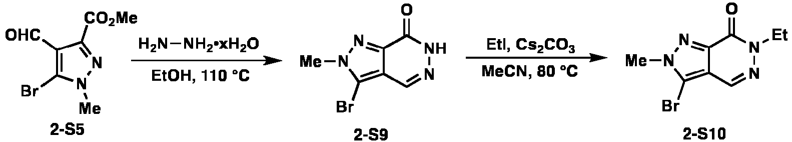

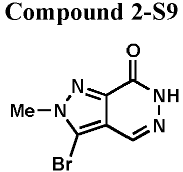

### 3-bromo-2-methyl-2,6-dihydro-7H-pyrazolo[3,4-d]pyridazin-7-one (2-S9)

To a stirred solution of methyl 5-bromo-4-formyl-1-methyl-1*H*-pyrazole-3-carboxylate **(2-S5)** (2 g, 8.10 mmol) in ethanol (20 mL), hydrazine monohydrate (2 mL, 40.48 mmol) was added and stirred at 110 °C for 3 h. After completion, the reaction mixture was concentrated, quenched with water, extracted with ethyl acetate, washed with brine, dried over anhydrous Na₂SO₄ and concentrated. The crude 3-bromo-2-methyl-2,6-dihydro-7H-pyrazolo[3,4-d]pyridazin-7-one **(2-S9)** was directly taken for the next step (1 g, 54%). **¹H NMR (400 MHz, DMSO-d*₆*):** *δ* 12.43 (s, 1H), 8.15 (s, 1H), 4.11 (s, 3H) ppm. **LC-MS (ESI, m/z):** calcd for [M+H]⁺ 229.1; found: 229.0, 231.0 [M+H+2]⁺.

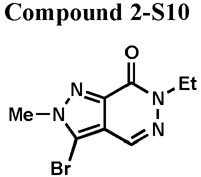

### 3-bromo-6-ethyl-2-methyl-2,6-dihydro-7H-pyrazolo[3,4-d]pyridazin-7-one (2-S10)

To a stirred solution of 3-bromo-2-methyl-2,6-dihydro-7*H*-pyrazolo[3,4-*d*]pyridazin-7-one (2-**S9)** (1 g, 4.37 mmol) in acetonitrile (10 mL), Cs₂CO₃ (2.85 g, 8.73 mmol) followed by iodoethane (0.6 mL, 7.42 mmol) was added and the reaction mixture was stirred at 80 °C for 2 h. After completion, the reaction mixture was filtered, washed with 10% methanol in dichloromethane and the filtrated was concentrated. The crude product was purified by CombiFlash Chromatography (50% ethyl acetate in hexane) to afford 3-bromo-6-ethyl-2-methyl-2,6-dihydro-7H-pyrazolo[3,4-d]pyridazin-7-one (2-S10) as an off-white solid (0.7 g, 62%). **Physical state:** off-white solid. **¹H NMR (400 MHz, CDCl₃): *δ* 7.98** (s, 1H), 4.26 (q, *J* = 7.12 Hz, 2H), 4.15 (s, 3H), 1.37 (t, *J* = 7.12 Hz, 3H) ppm. **LC-MS (ESI, m/z):** calcd for [M+H]⁺257.1; found: 257.0, 259.0 [M+H+2]⁺.

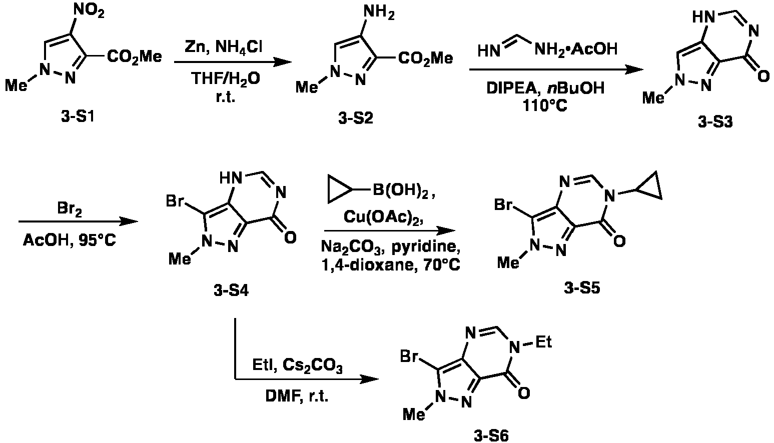

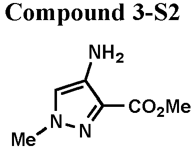

### methyl 4-amino-1-methyl-1H-pyrazole-3-carboxylate (3-S2)

To a stirred solution of methyl 1-methyl-4-nitro-1H-pyrazole-3-carboxylate (3-S1) (8 g, 43.21 mmol) and NH₄Cl (23.11 g, 432.11 mmol) in THF-H₂O (5:1) (120 mL), Zn-dust (28.26 g, 432.11 mmol) was added portion wise and the reaction mixture was stirred at room temperature for 4 h. After completion, the reaction mixture was filtered, concentrated and the crude was extracted with ethyl acetate, washed with water, brine, dried over anhydrous Na₂SO₄ and concentrated. The crude methyl 4-amino-1-methyl-1H-pyrazole-3-carboxylate (3-S2) was directly taken for the next step (6.7 g, 99%). **¹H NMR (400 MHz, CDCl₃):** *δ* 6.95 (s, 1H), 3.90 (s, 3H), 3.84 (s, 3H), 3.05 (*br.,* 2H) ppm. **LC-MS (ESI, m/z):** calcd for [M+H]⁺ 156.1; found: 156.2.

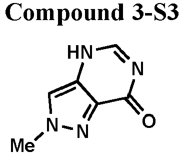

### 2-methyl-2,4-dihydro-7H-pyrazolo[4,3-d]pyrimidin-7-one (3-S3)

To a stirred solution of methyl 4-amino-1-methyl-1*H*-pyrazole-3-carboxylate **(3-S2)** (6.7 g, 43.18 mmol) in DIPEA (35 mL) and n-butanol (35 mL), was added formamidine acetate (5.4 g, 51.82 mmol) and the resulting mixture was stirred at 110 °C for 1 hour. After completion, the reaction mixture was cooled to room temperature, a white solid was appeared. The white precipitate was filtered, washed with diethyl ether twice and dried to afford 2-methyl-2,4-dihydro-7*H*-pyrazolo[4,3-*d*]pyrimidin-7-one (3-S3) as an off-white solid (6.4 g, 99%). **Physical state:** off-white solid. **¹H NMR (400 MHz, DMSO-d*₆*)**: *δ* 11.79 *(br.,* 1H), 8.29 (s, 1H), 7.75 (s, 1H), 4.07 (s, 3H) ppm. **LC-MS (ESI, m/z):** calcd for [M+H]⁺ 151.1; found: 151.2.

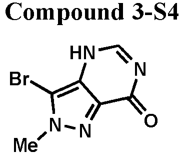

### 3-bromo-2-methyl-2,4-dihydro-7H-pyrazolo[4,3-d]pyrimidin-7-one (3-S4)

To a stirred solution of 2-methyl-2,4-dihydro-7*H*-pyrazolo[4,3-*d*]pyrimidin-7-one (3-S3) (10 g, 66.61 mmol) in acetic acid (70 mL), bromine (10.24 mL, 199.81 mmol) was added and the mixture was stirred at 95 °C for 16 h. After completion, the reaction mixture was cooled, methanol (100 mL) and diethyl ether (100 mL) were added. The resulting precipitate was filtered, washed with aqueous NaHCO₃ solution, water and dried to afford 3-bromo-2-methyl-2,4-dihydro-7*H*-pyrazolo[4,3-*d*]pyrimidin-7-one **(3-S4)** as an off-white solid (14 g, 92%). **Physical state:** off-white solid. **¹H NMR (400 MHz, DMSO-d*₆*)**: *δ* 12.03 *(br.,* 1H), 7.84 (d, *J* = 3.44 Hz, 1H), 4.07 (s, 3H) ppm. **LC-MS (ESI,** m/z): calcd for [M+H]⁺229.1; found: 228.8.

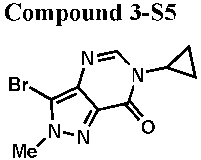

### 3-bromo-6-cyclopropyl-2-methyl-2,6-dihydro- 7H-pyrazolo[4,3-d]pyrimidin-7-one (3-S5)

To a stirred solution of 3-bromo-2-methyl-2,4-dihydro-7*H*-pyrazolo[4,3-d]pyrimidin-7-one (1 g, 4.37 mmol) **(3-S4)** and cyclopropylboronic acid (0.64 g, 7.42 mmol) in 1,4-dioxane (10 mL), pyridine (1.76 mL, 21.83 mmol), Na₂CO₃ (0.93 g, 8.73 mmol) and Cu(OAc)₂ (0.79 g, 4.37 mmol) were added. The reaction mixture was then stirred under oxygen balloon pressure at 70 °C for 12 h. After completion, the reaction mixture was extracted with ethyl acetate, washed with aqueous saturated NH₄Cl solution, brine, dried over anhydrous Na₂SO₄ and concentrated. The crude product was purified by Combiflash chromatography (70% ethyl acetate in hexane) to afford 3-bromo-6-cyclopropyl-2-methyl-2,6-dihydro-7*H*-pyrazolo[4,3-*d*]pyrimidin-7-one **(3-S5)** as an off-white solid (0.25 g, 21%). **Physical state:** off-white solid. **¹H NMR (400 MHz, DMSO-d*₆*)**: *δ* 8.06 (s, 1H), 4.07 (s, 3H), 3.18-3.14 (m, 1H), 1.05-1.00 (m, 2H), 0.91-0.86 (m, 2H) ppm. **LC-MS (ESI, m/z):** calcd for [M+H]⁺269.1; found: 269.0, 271.2 [M+H+2]⁺.

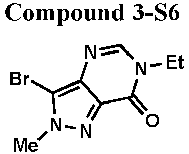

### 3-bromo-6-ethyl-2-methyl-2,6-dihydro-7H-pyrazolo[4,3-d]pyrimidin-7-one (3-S6)

To a stirred solution of 3-bromo-2-methyl-2,4-dihydro-7*H*-pyrazolo[4,3-*d*]pyrimidin-7-one **(3-S4)** (10 g, 43.66 mmol) in DMF (30 mL), Cs₂CO₃ (28.45 g, 87.325 mmol) followed by iodoethane (5.3 mL, 65.49 mmol) was added and the reaction mixture was stirred at room temperature for 24 h. After completion, the reaction mixture was filtered, washed with ethyl acetate and the filtrate was concentrated. The crude product was purified by Combiflash chromatography (50% ethyl acetate in hexane) to afford 3-bromo-6-ethyl-2-methyl-2,6-dihydro-7*H*-pyrazolo[4,3-d]pyrimidin-7-one **(3-S6)** as an off-white solid (8 g, 71%). **Physical state:** off-white solid. **¹H NMR (400 MHz, DMSO-d*₆*):** *δ* 8.19 (s, 1H), 4.07 (s, 3H), 3.98 (q, *J* = 7.04 Hz, 2H), 1.24 (t, *J* = 7.08 Hz, 3H) ppm. **LC-MS (ESI, m/z):** calcd for [M+H]⁺ 259.1; found: 258.86.

### General procedure C of Grignard addition to Weinreb amides

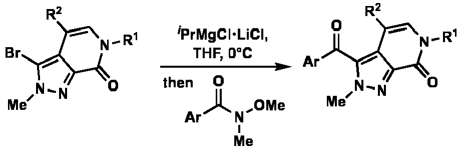

**General procedure C1:** To a stirred solution of 3-bromo-6-alkyl-2-methyl-2,6-dihydro-7H-pyrazolo[3,4-c]pyridin-7-one **(1-S11, 1-S12, 1-S14, 1-S15)** (1.0 equiv.) in anhydrous THF (0.1-0.6 M, 5-20 mL), *ⁱ*PrMgCl•LiCl (1.3M in THF) (1.3 equiv.) was added dropwise at 0 °C and stirred for 15 minutes. After that a solution of N-methoxy-N-methyl-aryl amide **(1-S26 or 1-S27)** (1.0-1.2 equiv.) in anhydrous THF (1 M) was added at 0 °C and stirred at that temperature for 30 minutes. After completion, the reaction mixture was quenched with aqueous saturated NH₄Cl solution, extracted with ethyl acetate, washed with brine, dried over anhydrous Na₂SO₄ and concentrated. The crude product was purified by CombiFlash chromatography to afford the ketone.

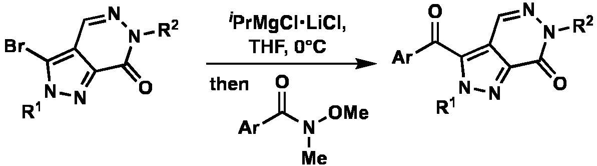

**General procedure C2:** To a stirred solution of 3-bromo-6-alkyl-2-alkyl-2,6-dihydro-7*H-*pyrazolo[3,4-*d*]pyridazin-7-one **(2-S6-2-S8)** (1.0 equiv.) in anhydrous THF (0.07-0.46 M), iPrMgCl.LiCl (1.3M in THF) (1.3-1.5 equiv.) was added dropwise at 0 °C and stirred for 30 minutes. After that a solution of N-methoxy-N-methyl-aryl amide **(1-S26)** (1.0-1.2 equiv.) in anhydrous THF (0.5-1.0 M, 2-3 mL) was added and stirred at 0 °C for 30 minutes. After completion, the reaction mixture was quenched with aqueous saturated NH₄Cl solution, extracted with ethyl acetate, washed with brine, dried over anhydrous Na₂SO₄ and concentrated. The crude product was purified by CombiFlash chromatography to afford the desired ketone **(2-S11, 2-S12, 2-S16).**

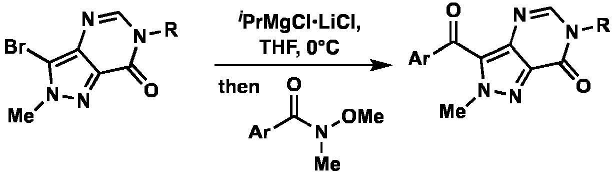

**General procedure C3:** To a stirred solution of 3-bromo-6-cyclopropyl-2-methyl-2,6-dihydro-7*H*-pyrazolo[4,3-*d*]pyrimidin-7-one **(3-S5)** (1.0 equiv.) in anhydrous THF (0.3-0.45 M), iPrMgCl•LiCl (1.3M in THF) (1.3-1.5 equiv.) was added dropwise at 0 °C and stirred for 30 minutes. After that a solution of N-methoxy-N-methyl-aryl amide **(1-S26** or **1-S27)** (1.0-1.2 equiv.) in anhydrous THF (0.8-1.1 M, 5 mL) was added and stirred at 0 °C for 30 minutes. After completion, the reaction mixture was quenched with aqueous saturated NH₄Cl solution, extracted with ethyl acetate, washed with brine, dried over anhydrous Na₂SO₄ and concentrated. The crude product was purified by CombiFlash chromatography to afford the desired ketone **(3-S7, 3-S8).**

### General procedure D of Grignard addition to aldehydes

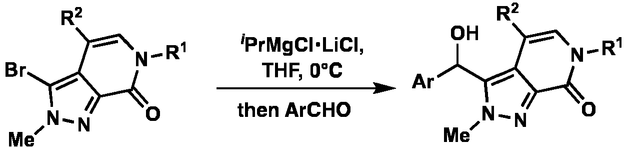

**General procedure D1:** To a stirred solution of 3-bromo-6-alkyl-2-methyl-2,6-dihydro-7*H-*pyrazolo[3,4-c]pyridin-7-one **(1-S11 & 1-S12)** (1.0 equiv.) in anhydrous THF (0.1-0.55 M, 5-10 mL), *ⁱ*PrMgCl•LiCl (1.3M in THF) (1.3 equiv.) was added at 0 °C and stirred at that temperature for 10 minutes. After that a solution of aryl aldehydes or aryl methyl ketone **(1-S18)** (1.0-1.1 equiv.) in anhydrous THF (0.3-1.1 M) was added and the reaction mixture was stirred at that temperature for another 30 minutes. After completion, the reaction mixture was quenched with aqueous saturated NH₄Cl solution, extracted with ethyl acetate, washed with brine, dried over anhydrous Na₂SO₄ and concentrated. The crude product was purified by CombiFlash chromatography (90% ethyl acetate in hexane) to afford the alcohols **(1-S29, 1-S33, 1-S35, 1-S42, 1-S44, 1-P23, 4-S3, 4-S8,** 4-S14).

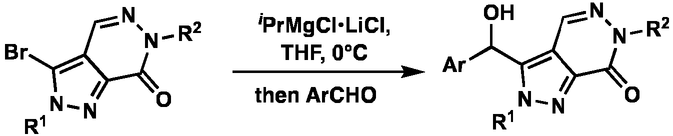

**General procedure D2:** To a stirred solution of 3-bromo-6-alkyl-2-methyl-2,6-dihydro-7*H-*pyrazolo[3,4-*d*]pyridazin-7-one (alkyl: cPr or Et, **2-S8 or 2-S10)** (1.0 equiv.) in anhydrous THF (0.23-0.57 M, 5-10 mL), iPrMgCl.LiCl (1.3M in THF) (1.3 equiv.) was added dropwise at 0 °C and stirred for 30 minutes. After that the aldehyde (1.0 equiv.) in anhydrous THF (0.55-1.3 M, 2-5 mL) was added and stirred at 0 °C for 30 minutes. After completion, the reaction mixture was quenched with aqueous saturated NH₄Cl solution, extracted with ethyl acetate, washed with brine, dried over anhydrous Na₂SO₄ and concentrated. The crude product was purified by CombiFlash chromatography to afford desired diarylmethanol product **(2-S13, 2-S17, 2-S19, 2-S21).**

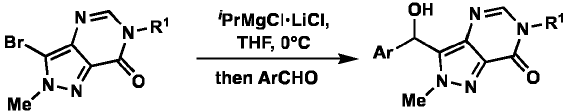

**General procedure D3:** To a stirred solution of 3-bromo-6-alkyl-2-methyl-2,6-dihydro-7H-pyrazolo[3,4-*d*]pyridazin-7-one **(3-S6, 3-S5)** (1.0 equiv.) in anhydrous THF (0.27-0.5 M, 7 mL), iPrMgCl•LiCl (1.3M in THF) (1.3 equiv.) was added dropwise at 0 °C and stirred for 30 minutes. After that 3-bromo-4-(trifluoromethyl)benzaldehyde (1.2 equiv.) in anhydrous THF (0.6-1.4 M, 3-5 mL) was added and stirred at 0 °C for 30 minutes. After completion, the reaction mixture was quenched with aqueous saturated NH₄Cl solution, extracted with ethyl acetate, washed with brine, dried over anhydrous Na₂SO₄ and concentrated. The crude product was purified by CombiFlash chromatography to afford desired diarylmethanol product **(3-S9, 4-S23, 4-S29).**

### General procedure E of MnO₂ oxidation of diarylmethanols

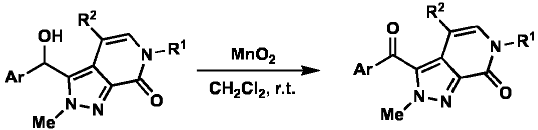

**General procedure E1:** To a stirred solution of diarylmethanol **(1-S30, 1-S33, 1-S35, 1-S44, 4-S3, 4-S8) (1.0** equiv.) in dichloromethane (0.03-0.2 M), MnO₂ (10-15 equiv.) was added and the reaction mixture was stirred at room temperature for 12-16 h. After completion, the reaction mixture was filtered through Celite bed, washed with dichloromethane and the filtrate was concentrated. The crude product was purified by CombiFlash chromatography to afford diaryl ketone **(1-S31, 1-S34, 1-S36, 1-S45, 4-S4, 4-S9).**

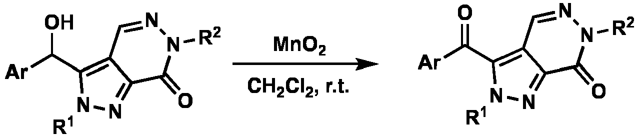

**General procedure E2**: To a stirred solution of diarylmethanol **(2-S14, 2-S17, 2-S19, 2-S21,** 1.0 equiv.) in dichloromethane (0.1-0.18 M), MnO₂ (10.0 equiv.) was added and the reaction mixture was stirred at room temperature for 12 h. After completion, the reaction mixture was filtered and concentrated. The crude product was purified by CombiFlash chromatography (40% ethyl acetate in hexane) to afford desired diaryl ketone **(2-S15, 2-S18, 2-S20, 2-S22).**

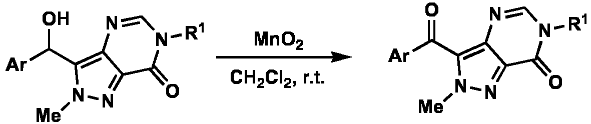

**General procedure E3**: To a stirred solution of diarylmethanol **(3-S10, 4-S23, 4-S29)** (1.0 equiv.) in dichloromethane (0.08-0.18 M), MnO₂ (10.0 equiv.) was added and the reaction mixture was stirred at room temperature for 2-12 h. After completion, the reaction mixture was filtered and concentrated. The crude product was purified by CombiFlash chromatography (40% ethyl acetate in hexane) to afford desired diaryl ketone **(3-S11, 4-S24, 4-S30).**

### General procedure F of Grignard addition to diaryl ketones

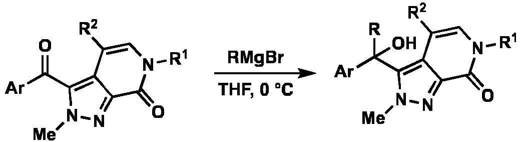

**General procedure F1:** To a stirred solution of diaryl ketone (1.0 equiv.) in anhydrous THF (0.05-0.16 M), RMgBr (R = Me or Et, 3 M in diethyl ether or THF; *ⁱ*Pr, 1.3 M in THF; *^{c}*Pr, 0.7 M in THF) (1.2-3.0 equiv.) was added dropwise at -5°C-0 °C and stirred at that temperature for 30 minutes. After completion, the reaction mixture was quenched with aqueous saturated NH₄Cl solution, extracted with ethyl acetate, washed with brine, dried over anhydrous Na₂SO₄ and concentrated. The crude product was purified by CombiFlash chromatography to afford the final drug analogue.

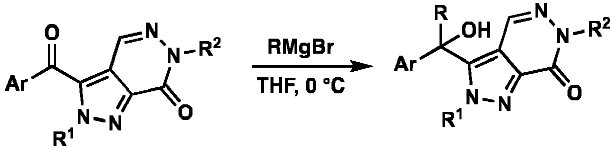

**General procedure F2:** To a stirred solution of diaryl ketone **(2-S11, 2-S12, 2-S15, 2-S16, 2-S18, 2-S20, 2-S22,** 1.0 equiv.) in anhydrous THF (0.06-0.22 M, 3-5 mL), RMgBr (R = Et, 3M in diethyl ether; R = *^{c}*Pr, 1.0 M in 2-Me-THF) (1.0-2.5 equiv.) was added at -5-0 °C and stirred for 30 minutes. After completion, the reaction mixture was quenched with aqueous saturated NH₄Cl solution, extracted with ethyl acetate, washed with brine, dried over anhydrous Na₂SO₄ and concentrated. The crude product was purified by CombiFlash chromatography to afford the final drug analogue **(2-1** to **2-20).**

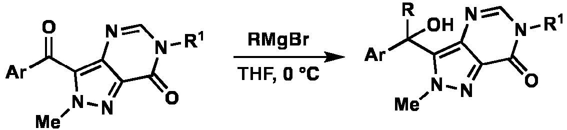

**General procedure F3**: To a stirred solution of diaryl ketone **(3-S7, 3-S8, 3-S11)** (1.0 equiv.) in anhydrous THF (0.06-0.12 M, 3-5 mL), RMgBr (R = Me, Et, 3M in diethyl ether; R =*^{c}*Pr, 1.0 M in 2-Me-THF) (1.0-2.5 equiv.) was added at -5-0 °C and stirred for 30 minutes. After completion, the reaction mixture was quenched with aqueous saturated NH₄Cl solution, extracted with ethyl acetate, washed with brine, dried over anhydrous Na₂SO₄ and concentrated. The crude product was purified by CombiFlash chromatography to afford the final drug analogue **(3-P1** to **3-P6).**

### General procedure G1 of NaBH₄ reduction of ketones

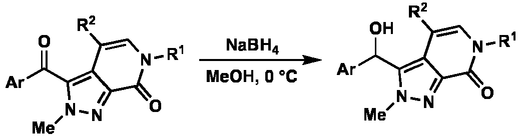

To a stirred solution of diaryl ketone (1.0 equiv.) in methanol (0.045-0.4 M, 3-15 mL), NaBH₄ (3.0-5.0 equiv.) was added at 0 °C and stirred at room temperature for 2 h. After completion, the reaction mixture was diluted with cold water, extracted with ethyl acetate, washed with brine, dried over anhydrous Na₂SO₄ and concentrated. The crude product was purified by CombiFlash chromatography to afford the alcohol.

### General procedure H1 of bromination of diaryl substituted methanol

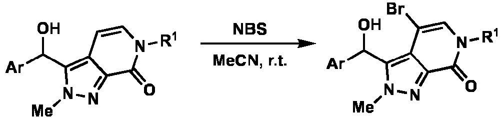

To a stirred solution of diaryl substituted methanol **(1-S40, 1-S42)** (1.0 equiv.) in acetonitrile (0.1-0.4 M), NBS (1.1-1.2 equiv.) was added and the mixture was stirred at room temperature for 2 h. After completion, the reaction mixture was quenched by the addition of aqueous Na₂S₂O₃ solution, extracted with ethyl acetate, washed with brine and dried over anhydrous Na₂SO₄ and concentrated. The crude product was purified by CombiFlash chromatography (70% ethyl acetate in hexane) to afford brominated product **(1-S41, 1-S43).**

### General procedure I of Suzuki coupling of alkylation

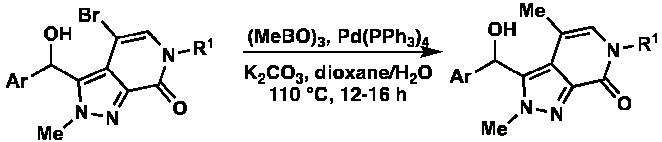

**General procedure I1 of Methylation:** In a reaction tube, to a stirred solution of aryl bromide (**1-S43**) (1.0 equiv.) in 1,4-dioxane-water (0.1-0.2 M, 3:1 for methylation) (12 mL), K₂CO₃ (3.0 equiv.) followed by trimethylboraxine (4.0 equiv.) was added. The mixture was degassed with argon for 15 minutes and then Pd(PPh₃)₄ (5 mol%) was added. The tube was sealed and the reaction mixture was stirred at 110 °C for 3-16 h. After completion, the reaction mixture was extracted with ethyl acetate, washed with water, brine, dried over anhydrous Na₂SO₄ and concentrated. The crude product was purified by CombiFlash chromatography to afford the methylated product **(1-P15).**

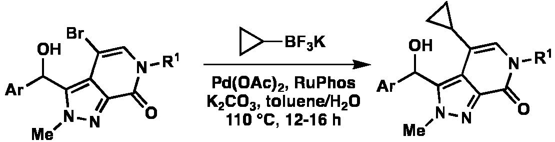

**General procedure I2 of Cyclopropanation:** In a reaction tube, to a stirred solution of aryl bromide **(1-S41,1.0** equiv.) in toluene-water (4:1) (0.16 M), K₂CO₃ (3.0 equiv.) followed by potassium cyclopropyltrifluroborate (1.7-2.0 equiv.) was added. The reaction mixture was degassed with argon for 10 minutes and then Pd(OAc)₂ (10 mol%) followed by Ruphos (20 mol%) was added. The tube was sealed and the reaction mixture was stirred at 110 °C for 12-16 h. After completion, the reaction mixture was cooled, diluted with water, extracted with ethyl acetate, washed with brine, dried over anhydrous Na₂SO₄ and concentrated. The crude product was purified by CombiFlash chromatography to afford cyclopropyl coupling product **(1-P14).**

### General procedure F4 of condensation of diaryl ketone and tert-butylsulfinamide

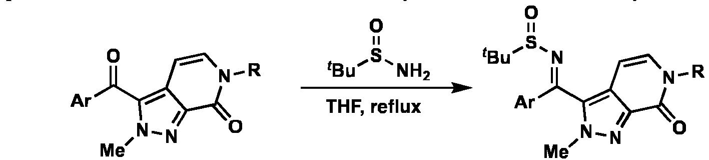

**General procedure F4a:** To a stirred solution of diaryl ketone **(1-S36, 4-S4)** (1.0 equiv.) and 2-methylpropane-2-sulfinamide (2.0-3.0 equiv.) in anhydrous THF (0.1-0.25 M), Ti(OiPr)₄ (5.0-10.0 equiv.) was added and the reaction mixture was stirred at 80 °C for 16 h. After completion, the reaction mixture was quenched with aqueous saturated NH₄Cl solution, filtered through Celite bed and washed with ethyl acetate. The combined organic layer was washed with brine, dried over anhydrous Na₂SO₄ and concentrated. The crude product was purified by CombiFlash chromatography to afford sulfinamide **(4-S1, 4-S5).**

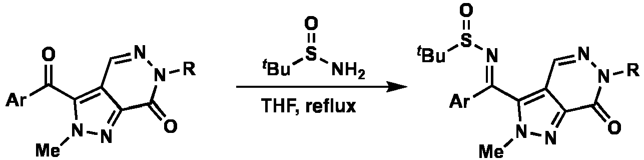

**General procedure F4b:** To a stirred solution of diaryl ketone **(2-S18)** (1.0 equiv.) and *tert-*butanesulfinamide (2.0-3.0 equiv.) in anhydrous THF (0.1-0.21 M), Ti(OiPr)₄ (5.0-7.0 equiv.) was added and the reaction mixture was stirred under refluxing condition for overnight. After completion, the reaction mixture was cooled, quenched with aqueous saturated NH₄Cl solution, extracted with ethyl acetate, washed with water, brine, dried over anhydrous Na₂SO₄ and concentrated. The crude product was purified by CombiFlash chromatography to afford sulfinamide **(4-S20)** as a yellow solid (E and Z mixture), which was taken directly for the next step.

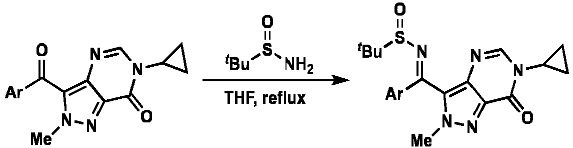

**General procedure F4c:** To a stirred solution of diaryl ketone **(4-S24, 4-S30, 3-S7)** (1.0 equiv.) and tert-butanesulfinamide (2.0-3.0 equiv.) in anhydrous THF (0.1-0.21 M), Ti(OiPr)₄ (5.0-7.0 equiv.) was added and the reaction mixture was stirred under refluxing condition for overnight. After completion, the reaction mixture was cooled, quenched with aqueous saturated NH₄Cl solution, extracted with ethyl acetate, washed with water, brine, dried over anhydrous Na₂SO₄ and concentrated. The crude product was purified by CombiFlash chromatography to afford sulfinamide **(4-S25, 4-S31, 4-S33)** as a yellow solid (E and Z mixture), which was taken directly for the next step.

### General procedure G4 of Grignard addition to tert-butyl sulfinamide

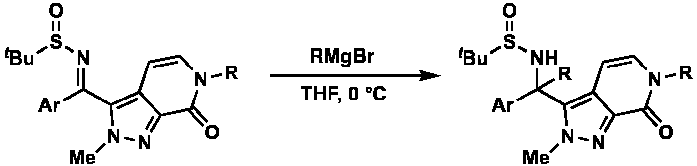

**General procedure G4a:** To a stirred solution of tert-butyl sulfinamide **(4-S1, 4-S5, 4-S7, 4-S9, 4-S10)** (1.0 equiv.) in anhydrous THF (0.1-0.14 M, 5-10 mL), alkylmagnesium bromide (R = Me or Et, 3 M in THF, R = *ⁱ*Pr, 1.3 M in THF; R = *^{c}*Pr, 0.7 M in THF) (1.2-4.0 equiv.) was added dropwise at 0 °C and stirred at that temperature for 30 minutes. After completion, the reaction mixture was quenched with aqueous saturated NH₄Cl solution, extracted with ethyl acetate, washed with brine, dried over anhydrous Na₂SO₄ and concentrated. The reaction crude was taken directly for the next step.

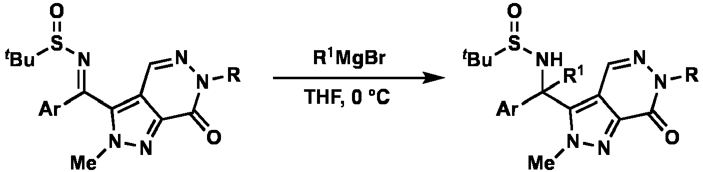

**General procedure G4b:** To a stirred solution of tert-butyl sulfinamide **(4-S20)** (1.0 equiv.) in anhydrous THF (0.10-0.15 M, 5 mL), alkylmagnesium bromide (R = Et, 3 M in Et₂O, R = cPr, 0.7 M in THF) (1.2-1.5 equiv.) was added dropwise at -5 - 0 °C and stirred at that temperature for 30 minutes. After completion, the reaction mixture was quenched with aqueous saturated NH₄Cl solution, extracted with ethyl acetate, washed with brine, dried over anhydrous Na₂SO₄ and concentrated. The reaction crude was taken directly for the next step **(4-S21, 4-S22).**

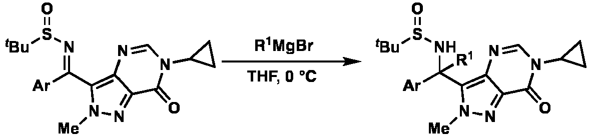

**General procedure G4c:** To a stirred solution of tert-butyl sulfinamide **(4-S25, 4-S31, 4-S33)** (1.0 equiv.) in anhydrous THF (0.10-0.15 M, 5 mL), alkylmagnesium bromide (R = Me or Et, 3 M in Et₂O, R = cPr, 0.7 M in THF) (1.2-1.5 equiv.) was added dropwise at -5 - 0 °C and stirred at that temperature for 30 minutes. After completion, the reaction mixture was quenched with aqueous saturated NH₄Cl solution, extracted with ethyl acetate, washed with brine, dried over anhydrous Na₂SO₄ and concentrated. The reaction crude was taken directly for the next step **(4-S26, 4-S27, 4-S28, 4-S32, 4-S34).**

### General procedure H4 of hydrolysis of sulfinamide

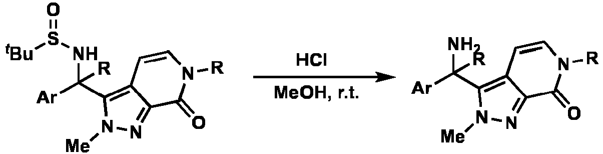

**General procedure H4a:** A solution of sulfinamide **(4-S2, 4-S6, 4-S11, 4-S12, 4-S13)** (1.0 equiv.) in methanolic HCl (4M) (0.035-0.08 M, 7 mL) was stirred at room temperature for 1-2 h. After completion, the reaction mixture was concentrated, quenched with aqueous saturated NaHCO₃ solution, extracted with ethyl acetate, washed with brine, dried over anhydrous Na₂SO₄ and concentrated. The crude product was purified by CombiFlash chromatography to afford tertiary amine.

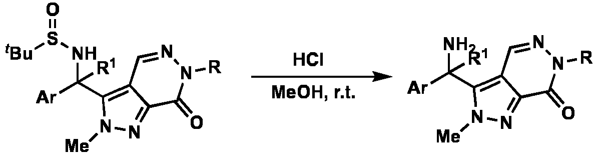

**General procedure H4b:** A solution of sulfinamide **(4-S21, 4-S22)** (1.0 equiv.) in methanolic HCl (4M) (0.035-0.09 M, 7 mL) was stirred at room temperature for 1-2 h. After completion, the reaction mixture was concentrated, quenched with aqueous saturated NaHCO₃ solution, extracted with ethyl acetate, washed with brine, dried over anhydrous Na₂SO₄ and concentrated. The crude product was purified by CombiFlash chromatography to afford tertiary amine **(4-P8, 4-P9).**

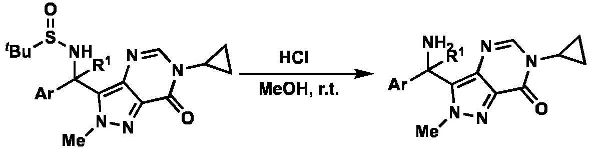

**General procedure H4c:** A solution of sulfinamide **(4-S26, 4-S27, 4-S28, 4-S32, 4-S34)** (1.0 equiv.) in methanolic HCl (4M) (0.035-0.09 M, 7 mL) was stirred at room temperature for 1-2 h. After completion, the reaction mixture was concentrated, quenched with aqueous saturated NaHCO₃ solution, extracted with ethyl acetate, washed with brine, dried over anhydrous Na₂SO₄ and concentrated. The crude product was purified by CombiFlash chromatography to afford tertiary amine **(4-P10** to **4-P14).**

### General procedure F5 of trifluoromethylation of diaryl ketone

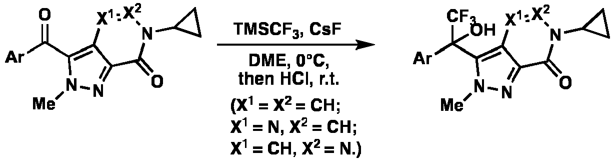

To a stirred solution of diaryl ketone **(2-S16, 3-S7, 1-S28, 5-S2, 1-S34, 1-S37)** (1.0 equiv.) in 1,2-dimethoxyethane (0.06-0.25 M, 5 mL), TMSCF₃ (2.0-3.0 equiv.) followed by CsF (0.1-0.5 equiv.) was added at 0 °C and the reaction mixture was stirred at room temperature for 2-16 h. After that 5 M HCl (1.2-3.0 equiv., 0.2-0.3 mL) or aqueous saturated NH₄Cl solution was added and stirred at room temperature for 2 h. After completion, the reaction mixture was concentrated, neutralized with aqueous NaHCO₃ solution, extracted with ethyl acetate, washed with brine, dried over anhydrous Na₂SO₄ and concentrated. The crude product was purified by CombiFlash chromatography (50-70% ethyl acetate in hexane) to afford the tertiary alcohol **(5-P1** to **5-P5)** as desired product.

### Synthesis of compounds of the invention 1-1, 1-2, 1-10, 1-11, 1-14, 1-15, 1-16, 1-17

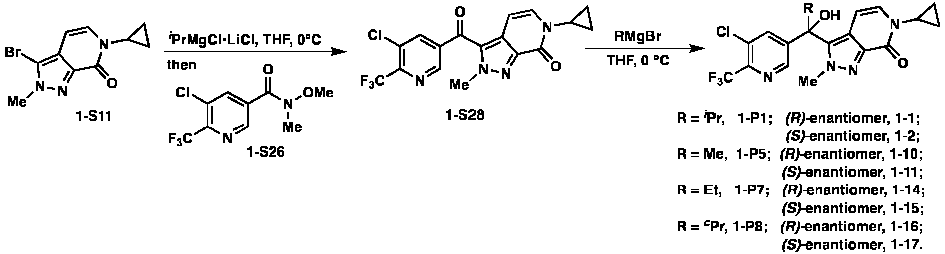

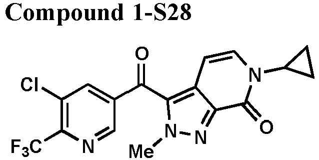

### 3-(5-chloro-6-(trifluoromethyl)nicotinoyl)-6-cyclopropyl-2-methyl-2,6-dihydro-7H-pyrazolo[3,4-c]pyridin-7-one (1-S28)

Following **General Procedure C1** on 0.89 mmol scale with 3-bromo-6-cyclopropyl-2-methyl-2,6-dihydro-7*H*-pyrazolo[3,4-*c*]pyridin-7-one **(1-S11)** and 5-chloro-*N*-methoxy-*N*-methyl-6-(tri-fluoromethyl)nicotinamide **(1-S26).** Purification by CombiFlash chromatography (75% ethyl acetate in hexane) to afford 3-(5-chloro-6 (trifluoromethyl)nicotinoyl)-6-cyclopropyl-2-methyl-2,6-dihydro-7*H*-pyrazolo[3,4-*c*]pyridin-7-one **(1-S28)** as a yellow solid (85 mg, 24%). **Physical state:** yellow solid. **¹H NMR (400 MHz, DMSO-*d₆*):** *δ* 8.96 (s, 1H), 8.56 (s, 1H), 7.15 (d, *J* = 7.6 Hz, 1H), 5.63 (d, *J =* 7.6 Hz, 1H), 4.32 (s, 3H), 3.27-3.25 (m, 1H), 1.05-1.00 (m, 2H), 0.85-0.80 (m, 2H) ppm. **LC-MS (ESI, m/z):** calcd for [M+H]⁺397.1; found: 397.0.

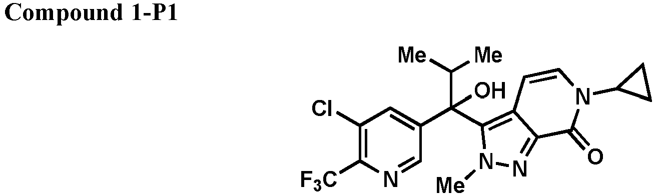

**3-(1-(5-chloro-6-(trifluoromethyl)pyridin-3-yl)-1-hydroxy-2-methylpropyl)-6-cyclopropyl-2-methyl-2,6-dihydro-7H-pyrazolo[3,4-c]pyridin-7-one (1-P1)** Following **General Procedure F1** on 0.25 mmol scale with 3-(5-chloro-6-(trifluoromethyl)nicotinoyl)-6-cyclopropyl-2-methyl-2,6-dihydro-7*H*-pyrazolo[3,4-*c*]pyridin-7-one **(1-S28)** and *ⁱ*PrMgCl•LiCl (1.3M in THF). Purification by C Combiflash chromatography (70% ethyl acetate in hexane) to afford 3-(1-(5-chloro-6-(trifluoromethyl)pyridin-3-yl)-1-hydroxy-2-methylpropyl)-6-cyclopropyl-2-methyl-2,6-dihydro-7H-pyrazolo[3,4-c]pyridin-7-one **(1-P1)** as off white solid (40 mg, 36%). **Physical state:** off-white solid.

The racemic product was purified from PREP-HPLC-SFC to afford Enantiomer-1 **(1-2)** (9 mg) and Enantiomer-2 **(1-1)** (10 mg).

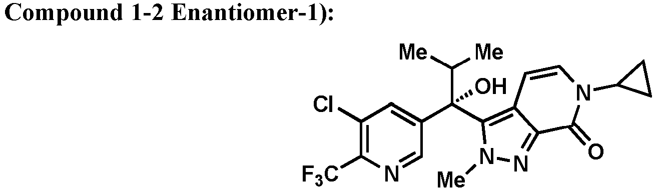

### (S)-3-(1-(5-chloro-6-(trifluoromethyl)pyridin-3-yl)-1-hydroxy-2-methylpropyl)-6-cyclopropyl-2-methyl-2,6-dihydro-7H-pyrazolo[3,4-c]pyridin-7-one (1-2)

**¹H NMR (400 MHz, DMSO-*d₆*):** *δ* 8.53 (s, 1H), 8.00 (s, 1H), 7.03 (d, *J* =7.6 Hz, 1H), 6.55 (d, *J* =7.7 Hz, 1H), 6.48 (s, 1H), 3.86 (s, 3H), 3.27-3.24 (m, 1H), 3.01-2.98 (m, 1H), 1.05-1.02 (m, 3H), 1.00- 0.97 (m, 2H), 0.85-0.81 (m, 2H), 0.70-0.69 (m, 3H) ppm.

**LC-MS (ESI, m/z):** calcd for [M+H]⁺ 441.1; found: 441.2. **Retention time:** 3.46 min.

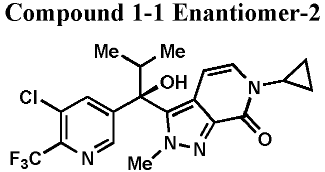

### (R)-3-(1-(5-chloro-6-(trifluoromethyl)pyridin-3-yl)-1-hydroxy-2-methylpropyl)-6-cyclopropyl-2-methyl-2,6-dihydro-7H-pyrazolo[3,4-c]pyridin-7-one (1-1)

**¹H NMR (400 MHz, DMSO-*d₆*):** *δ* 8.53 (s, 1H), 8.00 (s, 1H), 7.03 (d, *J* =7.6 Hz, 1H), 6.55 (d, *J* =7.7 Hz, 1H), 6.48 (s, 1H), 3.86 (s, 3H), 3.27-3.24 (m, 1H), 3.01-2.98 (m, 1H), 1.05-1.02 (m, 3H), 1.00- 0.97 (m, 2H), 0.85-0.81 (m, 2H), 0.70-0.69 (m, 3H) ppm.

**LC-MS (ESI, m/z):** calcd for [M+H]⁺441.1; found: 441.2. **Retention time:** 4.85 min.

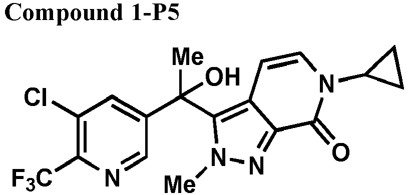

### 3-(1-(5-chloro-6-(trifluoromethyl)pyridin-3-yl)-1-hydroxyethyl)-6-cyclopropyl-2-methyl-2,6-dihydro-7H-pyrazolo[3,4-c]pyridin-7-one (1-P5)

Following **General Procedure F1** on 0.20 mmol scale with 3-(5-chloro-6-(trifluoromethyl)nicotinoyl)-6-cyclopropyl-2-methyl-2,6-dihydro-7*H*-pyrazolo[3,4-*c*]pyridin-7-one **(1-S28)** and MeMgBr (3M in diethyl ether). Purification by CombiFlash chromatography (80% ethyl acetate in hexane) to afford 3-(1-(5-chloro-6-(trifluoromethyl)pyridin-3-yl)-1-hydroxyethyl)-6-cyclopropyl-2-methyl-2,6-dihydro-7*H*-pyra-zolo[3,4-*c*]pyridin-7-one **(1-P5)** as an off white solid (50 mg, 60%). **Physical state:** off white solid.

The racemic compound **1-P5** was purified from preparative-HPLC-Chiral (SFC) to afford Enantiomer-1 **(1-11)** (12 mg) and Enantiomer-2 **(1-10)** (13 mg).

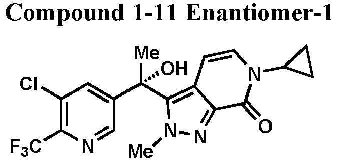

### (S)-3-(1-(5-chloro-6-(trifluoromethyl)pyridin-3-yl)-1-hydroxyethyl)-6-ethyl-2-methyl-2,6-dihydro-7H-pyrazolo[3,4-c]pyridin-7-one (1-11)

**¹H NMR (400 MHz, DMSO-*d₆*):** *δ* 8.56 (s, 1H), 8.04 (s, 1H), 6.99 (d, *J =* 7.6 Hz, 1H), 6.92 (s, 1H), 6.41 (d, *J =* 7.6 Hz, 1H), 3.78 (s, 3H), 3.27-3.24 (m, 1H), 2.07 (s, 3H), 1.02-0.97 (m, 2H), 0.82-0.78 (m, 2H) ppm. **LC-MS (ESI, m/z):** calcd for [M+H]⁺ 413.1; found: 413.1. **Retention time:** 1.52 min.

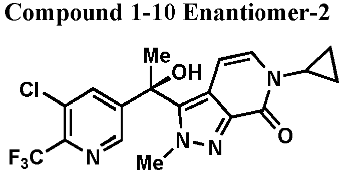

### (R)-3-(1-(5-chloro-6-(trifluoromethyl)pyridin-3-yl)-1-hydroxyethyl)-6-ethyl-2-methyl-2,6-dihydro-7H-pyrazolo[3,4-c]pyridin-7-one (1-10)

**¹H NMR (400 MHz, DMSO-*d₆*):** *δ* 8.56 (s, 1H), 8.04 (s, 1H), 6.99 (d, *J =* 7.6 Hz, 1H), 6.93 (s, 1H), 6.41 (d, *J* = 7.6 Hz, 1H), 3.78 (s, 3H), 3.27-3.24 (m, 1H), 2.07 (s, 3H) 1.01-0.97 (m, 2H), 0.83-0.80 (m, 2H) ppm. **LC-MS (ESI, m/z):** calcd for [M+H]⁺ 413.1; found: 413.0. **Retention time:** 1.97 min.

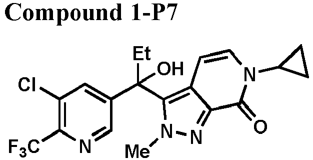

### 3-(1-(5-chloro-6-(trifluoromethyl)pyridin-3-yl)-1-hydroxypropyl)-6-cyclopropyl-2-methyl-2,6-dihydro-7H-pyrazolo[3,4-c]pyridin-7-one (1-P7)

Following **General Procedure F1** on 0.40 mmol scale with3-(5-chloro-6-(trifluoromethyl)nicotinoyl)-6-cyclopropyl-2-methyl-2,6-dihydro-7*H*-pyrazolo[3,4-*c*]pyridin-7-one **(1-S28)** and EtMgBr (3M in THF). Purification by CombiFlash chromatography (50% ethyl acetate in hexane) to afford 3-(1-(5-chloro-6-(trifluoromethyl)pyridin-3-yl)-1-hydroxypropyl)-6-cyclopropyl-2-methyl-2,6-dihydro-7*H*-pyrazolo[3,4-*c*]pyridin-7-one **(1-P7)** as an off white solid (55 mg, 32%). **Physical state:** off-white solid.

The racemic compound **1-P7** was purified from Preparative-HPLC-Chiral (NP) to afford Enantiomer-1 **(1-15)** (10 mg) and Enantiomer-2 **(1-14)** (10 mg).

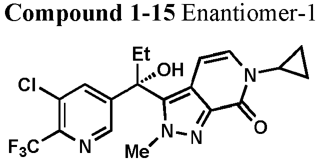

### (S)-3-(1-(5-chloro-6-(trifluoromethyl)pyridin-3-yl)-1-hydroxypropyl)-6-cyclopropyl-2-methyl-2, 6-dihydro-7H-pyrazolo[3,4-c]pyridin-7-one (1-15)

**¹H NMR (400 MHz, DMSO-*d₆*):** *δ* 8.53 (s, 1 H), 7.99 (s, 1 H), 7.01 (d, *J =* 7.7 Hz, 1 H), 6.63 (s, 1 H), 6.51 (d, *J =* 7.6 Hz, 1 H), 3.78 (s, 3 H), 3.26-3.24 (m, 1 H), 2.56-2.33 (m, 2 H), 1.00-0.97 (m, 2 H), 0.82-0.78 (m, 5 H) ppm. **LC-MS (ESI, m/z):** calcd for [M+H]⁺ 427.1; found: 427.1. **Retention time:** 4.61 min.

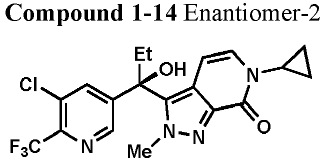

### (R)-3-(1-(5-chloro-6-(trifluoromethyl)pyridin-3-yl)-1-hydroxypropyl)-6-cyclopropyl-2-methyl-2, 6-dihydro-7H-pyrazolo[3,4-c]pyridin-7-one (1-14)

**¹H NMR (400 MHz, DMSO-*d₆*):** *δ* 8.53 (s, 1 H), 7.99 (s, 1 H), 7.01 (d, *J =* 7.7 Hz, 1 H), 6.63 (s, 1 H), 6.51 (d, *J =* 7.6 Hz, 1 H), 3.78 (s, 3 H), 3.26-3.24 (m, 1 H), 2.56-2.33 (m, 2 H), 1.00-0.97 (m, 2 H), 0.82-0.78 (m, 5 H) ppm. **LC-MS (ESI, m/z):** calcd for [M+H]⁺ 427.1; found: 427.1. **Retention time:** 5.31 min.

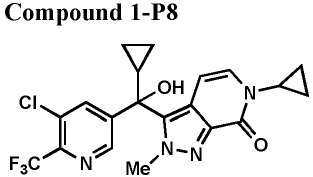

### 3-((5-chloro-6-(trifluoromethyl)pyridin-3-yl)(cyclopropyl)(hydroxy) methyl)-6-cyclopropyl-2-methyl-2,6-dihydro-7H-pyrazolo[3,4-c]pyridin-7-one (1-P8)

Following **General Procedure F1** on 0.46 mmol scale with 3-(5-chloro-6-(trifluoromethyl)nicotinoyl)-6-cyclopropyl-2-methyl-2,6-dihydro-7*H*-pyrazolo[3,4-*c*]pyridin-7-one **(1-S28)** and cyclopropyl magnesium bromide (1M in THF). Purification by CombiFlash chromatography (50% ethyl acetate in hexane) to afford 3-((5-chloro-6-(trifluoromethyl)pyridin-3-yl)(cyclopropyl)(hydroxy)methyl)-6-cyclopropyl-2-methyl-2,6-dihydro-7*H*-pyrazolo[3,4-*c*]pyridin-7-one **(1-P8)** as an off white solid (48 mg, 24%). **Physical state:** off white solid.

The racemic compound **1-P8** was submitted for Chiral separation (Prep-HPLC-Chiral-NP) to afford Enantiomer-1 **(1-17)** (8 mg) and Enantiomer-2 **(1-16)** (8 mg).

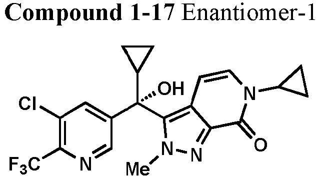

### (S)-3-((5-chloro-6-(trifluoromethyl)pyridin-3-yl)(cyclopropyl) (hydroxy) methyl)-6-cyclopropyl-2-methyl-2,6-dihydro-7H-pyrazolo[3,4-c]pyridin-7-one (1-17)

¹H NMR (400 MHz, DMSO-d*₆*) *δ* 8.51 (s, 1 H), 7.96 (s, 1 H), 6.99 (d, *J =* 7.6 Hz, 1 H), 6.63 (d, *J =* 7.6 Hz, 1 H), 6.39 (s, 1 H), 3.70 (s, 3 H), 3.29-3.24 (m, 1 H), 1.90-1.84 (m, 1 H), 1.01-0.99 (m, 2 H), 0.85-0.77 (m, 3 H), 0.76-0.71 (m, 1 H), 0.58-0.54 (m, 1 H), 0.50-0.44 (m, 1 H) ppm. **LC-MS (ESI, m/z):** calcd for [M+H]⁺ 439.1; found: 437.1. **Retention time:** 5.50 min.

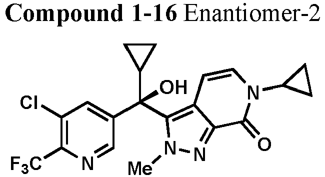

### (R)-3-((5-chloro-6-(trifluoromethyl)pyridin-3-yl)(cyclopropyl)(hydroxy)methyl)-6-cyclopropyl-2-methyl-2,6-dihydro-7H-pyrazolo[3,4-c]pyridin-7-one (1-16)

**¹H NMR (400 MHz, DMSO-*d₆*):** *δ* 8.51 (s, 1 H), 7.96 (s, 1 H), 6.99 (d, *J =* 7 .6 Hz, 1 H), 6.63 (d, *J =* 7.6 Hz, 1 H), 6.39 (s, 1 H), 3.70 (s, 3 H), 3.29-3.24 (m, 1 H), 1.90-1.84 (m, 1 H), 1.01-0.99 (m, 2 H), 0.85-0.77 (m, 3 H), 0.76-0.71 (m, 1 H), 0.58-0.54 (m, 1 H), 0.50-0.44 (m, 1 H) ppm; **LC-MS (ESI, m/z):** calcd for [M+H]⁺ 439.1; found: 437.1. **Retention time:** 6.86 min.

### Synthesis of compounds of the invention 1-3, 1-4, 1-5, 1-6

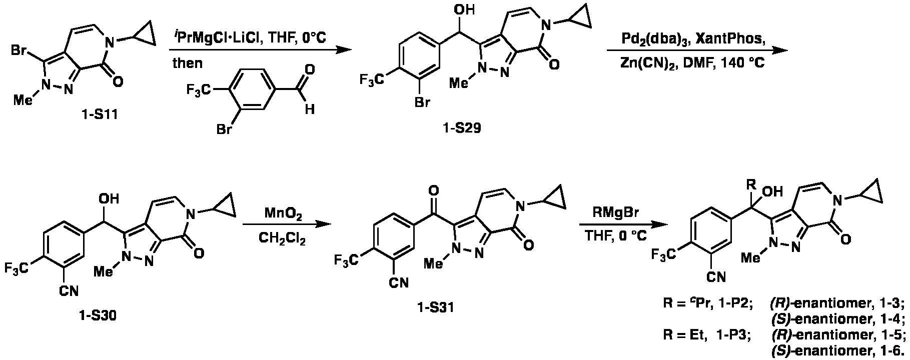

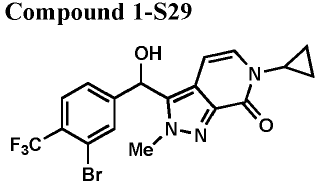

### 3-((3-bromo-4-(trijluoromethyl)phenyl)(hydroxy)methyl)-6-cyclopropyl-2-methyl-2,6-dihydro-7H-pyrazolo[3,4-c]pyridin-7-one (1-S29)

Following **General Procedure D1** on 2.61 mmol scale with 3-bromo-6-cyclopropyl-2-methyl-2,6-dihydro-7*H*-pyrazolo[3,4-c]pyridin-7-one **(1-S11)** and 3-bromo-4-(trifluoromethyl)benzaldehyde. Purification by Combiflash chromatography (90% ethyl acetate in hexane) to afford 3-((3-bromo-4-(trifluoromethyl)phenyl)(hydroxy)methyl)-6-cyclopropyl-2-methyl-2,6-dihydro-7*H*-pyrazolo[3,4-*c*]pyridin-7-one **(1-S29)** as an off-white solid (500 mg, 40%). **Physical State:** off-white solid. **¹H NMR (400 MHz, DMSO-*d₆*):** *δ* 7.89-7.86 (m, 2H), 7.55 (d, *J =* 8.2 Hz, 1H), 6.89 (d, *J =* 7.5 Hz, 1H), 6.67 (d, *J =* 4.8 Hz, 1H), 6.33 (d, *J* = 4.3 Hz, 1H), 5.84 (d, *J* = 7.4 Hz, 1H), 4.03 (s, 3H), 3.23-3.19 (m, 1H), 0.97-0.92 (m, 2H), 0.78-0.76 (m, 2H) ppm. **LC-MS (ESI, m/z):** calcd for [M+H]⁺ 442.1; found: 441.8.

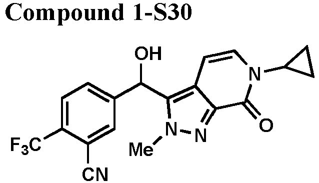

### 5-((6-cyclopropyl-2-methyl-7-oxo-6,7-dihydro-2H-pyrazolo[3,4-c]pyridin-3-yl)(hydroxy)methyl)-2-(trifluoromethyl)benzonitrile (1-S30)

In a reaction tube, a stirred solution of 3-((3-bromo-4-(trifluoromethyl)phenyl)(hydroxy)methyl)-6-cyclopropyl-2-methyl-2,6-dihydro-7*H*-pyrazolo [3,4-*c*]pyridin-7-one **(1-S29)** (500 mg, 1.13 mmol) in anhydrous DMF (10 mL) was degassed with argon for 15 minutes. After that Zn(CN)₂ (332 mg, 2.83 mmol) followed by Pd₂dba₃ (104 mg, 0.11 mmol) and Xantphos (131 mg, 0.23 mmol) were added, the tube was sealed and the reaction mixture was stirred at 140 °C for 16 h. After completion, the reaction mixture was diluted with cold water, extracted with ethyl acetate, washed with brine, dried over anhydrous Na₂SO₄ and concentrated. The crude product was purified by Combiflash chromatography (2-5% MeOH in dichloromethane) to afford 5-((6-cyclopropyl-2-methyl-7-oxo-6,7-dihydro-2*H-*pyrazolo[3,4-*c*]pyridin-3-yl)(hydroxy)methyl)-2-(trifluoromethyl) benzonitrile **(1-S30)** as an off-white solid (410 mg, 93%). **Physical State:** off-white solid.

**¹H NMR (400 MHz, DMSO-*d₆*):** *δ* 8.15 (s, 1H), 8.05 (d, *J =* 8.2 Hz, 1H), 7.92 (d, *J =* 8.3 Hz, 1H), 6.89 (d, *J =* 7.5 Hz, 1H), 6.79 (d, *J =* 4.7 Hz, 1H), 6.39 (d, *J =* 4.6 Hz, 1H), 5.86 (d, *J =* 7.5 Hz, 1H), 4.04 (s, 3H), 3.22-3.20 (m, 1H), 0.96-0.93 (m, 2H), 0.77-0.74 (m, 2H) ppm. **LC-MS (ESI, m/z):** calcd for [M+H]⁺ 389.1; found: 389.1.

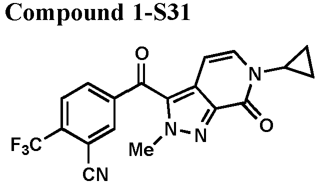

### 5-(6-cyclopropyl-2-methyl-7-oxo-6,7-dihydro-2H-pyrazolo[3,4-c]pyridine-3-carbonyl)-2-(trifluoromethyl)benzonitrile (1-S31)

Following **General Procedure E1** on 1.05 mmol scale with 3-((4-chloro-3,5-difluorophenyl)(hydroxy)methyl)-6-cyclopropyl-2-methyl-2,6-dihydro-7*H*-pyrazolo[3,4-*c*]pyridin-7-one **(1-S30).** After completion, the reaction mixture was filtered through celite bed, washed with dichloromethane and the filtrate was concentrated to afford 5-(6-cyclopropyl-2-methyl-7-oxo-6,7-dihydro-2*H*-pyrazolo[3,4-*c*]pyridine-3-carbonyl)-2-(trifluoromethyl)benzonitrile **(1-S31)** as an off-white solid (400 mg, 98%). **Physical State:** off-white solid. **¹H NMR (400 MHz, DMSO-*d₆*):** *δ* 8.51 (s, 1H), 8.25 (s, 2H), 7.12 (d, *J =* 7.5 Hz, 1H), 5.50 (d, *J* = 7.5 Hz, 1H), 4.32 (s, 3H), 3.27-3.24 (m, 1H), 1.02-0.97 (m, 2H), 0.81-0.77 (m, 2H) ppm. **LC-MS (ESI, m/z):**

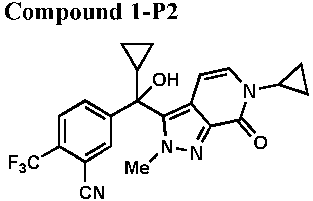

### 5-(cyclopropyl(6-cyclopropyl-2-methyl-7-oxo-6,7-dihydro-2H-pyrazolo[3,4-c]pyridin-3-yl)(hydroxy)methyl)-2-(trifluoromethyl)benzonitrile (1-P2)

Following **General Procedure F1** on 0.25 mmol scale with 5-(6-cyclopropyl-2-methyl-7-oxo-6,7-dihydro-2*H*-pyrazolo[3,4-*c*]pyridine-3-carbonyl)-2-(trifluoromethyl)benzonitrile **(1-S31)** and cyclopropyl magnesium bromide (0.7 M in THF). Purification by Combiflash chromatography (70% ethyl acetate in hexane) to afford 5-(cyclopropyl(6-cyclopropyl-2-methyl-7-oxo-6,7-dihydro-2*H*-pyrazolo[3,4-*c*]pyridin-3-yl)(hydroxy)methyl)-2-(trifluoromethyl) benzonitrile as an off-white solid **(1-P2)** (60 mg, 54%). **Physical state:** off-white solid.

The racemic compound **1-P2** was purified from PREP-HPLC-SFC to afford Enantiomer-1 **(1-4)** (15 mg) and Enantiomer-2 **(1-3)** (15 mg).

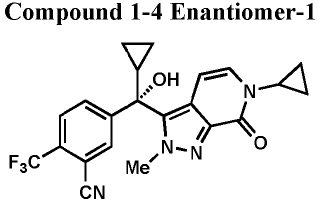

### (S)-5-(cyclopropyl(6-cyclopropyl-2-methyl-7-oxo-6,7-dihydro-2H-pyrazolo[3,4-c]pyridin-3-yl)(hydroxy)methyl)-2-(trifluoromethyl)benzonitrile (1-4)

**¹H NMR (400 MHz, DMSO-d*₆*):** *δ* 7.99-7.96 (m, 2H), 7.66 (d, *J =* 8.2 Hz, 1H), 7.00 (d, *J =* 7.6 Hz, 1H), 6.68 (d, *J =* 7.6 Hz, 1H), 6.32 (s, 1H), 3.62 (s, 3H), 3.28-3.25 (m, 1H), 1.80-1.70 (m, 1H), 1.10-1.00 (m, 2H), 0.85-0.80 (m, 2H), 0.76-0.69 (m, 2H), 0.60-0.50 (m, 1H), 0.40-0.30 (m, 1H) ppm. **LC-MS (ESI, m/z):** calcd for [M+H]⁺ 429.1; found: 429.2. **Retention time:** 1.03 min.

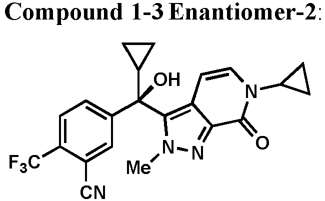

### (R)-5-(cyclopropyl(6-cyclopropyl-2-methyl-7-oxo-6,7-dihydro-2H-pyrazolo[3,4-c]pyridin-3-yl)(hydroxy)methyl)-2-(trifluoromethyl)benzonitrile (1-3)

**¹H NMR (400 MHz, DMSO-d*₆*):** *δ* 7.99-7.96 (m, 2H), 7.66 (d, *J =* 8.2 Hz, 1H), 7.00 (d, *J =* 7.6 Hz, 1H), 6.68 (d, *J =* 7.6 Hz, 1H), 6.32 (s, 1H), 3.62 (s, 3H), 3.28-3.25 (m, 1H), 1.80-1.70 (m, 1H), 1.10-1.00 (m, 2H), 0.85-0.80 (m, 2H), 0.76-0.69 (m, 2H), 0.60-0.50 (m, 1H), 0.40-0.30 (m, 1H) ppm. **LC-MS (ESI, m/z):** calcd for [M+H]⁺ 429.1; found: 429.2. **Retention time:** 1.30 min.

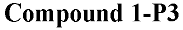

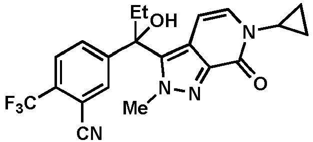

### 5-(1-(6-cyclopropyl-2-methyl-7-oxo-6,7-dihydro-2H-pyrazolo[3,4-c]pyridin-3-yl)-1-hydroxypropyl)-2-(trifluoromethyl)benzonitrile (1-P3)

Following **General Procedure F1** on 0.77 mmol scale with 5-(6-cyclopropyl-2-methyl-7-oxo-6,7-dihydro-2*H*-pyrazolo[3,4-c]pyridine-3-carbonyl)-2-(trifluoromethyl)benzonitrile **(1-S31)** and EtMgBr (3M in diethyl ether). Purification by Combiflash chromatography (85% ethyl acetate in hexane) to afford 5-(1-(6-cyclopropyl-2-methyl-7-oxo-6,7-dihydro-2H-pyrazolo[3,4-c]pyridin-3-yl)-1-hydroxypropyl)-2-(trifluoromethyl)-benzonitrile as an off-white solid **(1-P3)** (120 mg, 37%). **Physical state:** off-white solid.

The racemic compound **1-P3** was purified from PREP-HPLC-SFC to afford Enanatiomer-1 **(1-6)** (12 mg) and Enantiomer-2 **(1-5)** (10 mg).

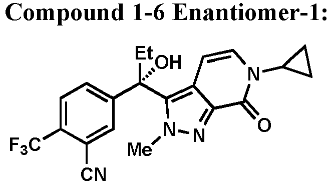

### (S)-5-(1-(6-cyclopropyl-2-methyl-7-oxo-6,7-dihydro-2H-pyrazolo[3,4-c]pyridin-3-yl)-1-hydroxypropyl)-2-(trifluoromethyl)benzonitrile (1-6)

**¹H NMR (400 MHz, DMSO-*d*₆):** *δ* 7.97 (t, *J =* 4.0 Hz, 2H), 7.71 (d, *J =* 8.4 Hz, 1H), 7.02 (d, *J =* 7.6 Hz, 1H), 6.55 (d, *J* = 9.2 Hz, 2H), 3.69 (s, 3H), 3.27-3.24 (m, 1H), 2.40-2.33 (m, 2H), 1.02-0.98 (m, 2H), 0.83-0.75 (m, 5H) ppm. **LC-MS (ESI, m/z):** calcd for [M+H]⁺ 417.1; found: 417.2. **Retention time:** 1.5 min.

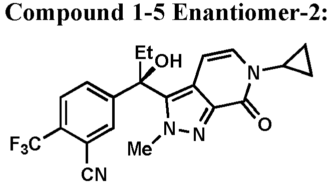

### (R)-5-(1-(6-cyclopropyl-2-methyl-7-oxo-6,7-dihydro-2H-pyrazolo[3,4-c]pyridin-3-yl)-1-hydroxypropyl)-2-(trifluoromethyl)benzonitrile (1-5)

**¹H NMR (400 MHz, DMSO-*d*₆):** *δ* 7.97 (t, *J =* 4.0 Hz, 2H), 7.71 (d, *J =* 8.4 Hz, 1H), 7.02 (d, *J =* 7.6 Hz, 1H), 6.55 (d, *J* = 9.2 Hz, 2H), 3.69 (s, 3H), 3.27-3.24 (m, 1H), 2.40-2.33 (m, 2H), 1.02-0.98 ( m, 2H), 0.83-0.75 (m, 5H) ppm. **LC-MS (ESI, m/z):** calcd for [M+H]⁺ 417.1; found: 417.2. **Retention time:** 1.73 min.

### Synthesis of compound of the invention 1-7

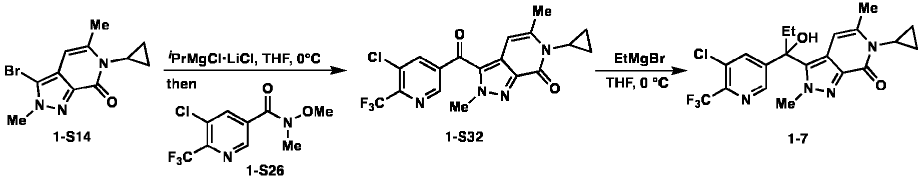

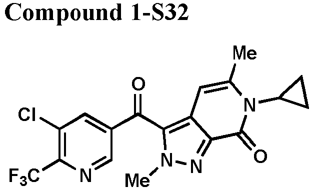

### 3-(5-chloro-6-(trifluoromethyl)nicotinoyl)-6-cyclopropyl-2,5-dimethyl-2,6-dihydro-7H-pyrazolo[3,4-c]pyridin-7-one (1-S32)

Following **General Procedure C1** on 0.71 mmol scale with 3-bromo-6-cyclopropyl-2,5-dimethyl-2,6-dihydro-7*H*-pyrazolo[3,4-*c*]pyridin-7-one **(1-S14)** and 5-chloro-*N*-methoxy-*N-*methyl-6-(tri-fluoromethyl)nicotinamide **(1-S26).** Purification by Combiflash chromatography (80 % ethyl acetate in hexane) to afford 3-(5-chloro-6-(trifluoromethyl)nicotinoyl)-6-cyclopropyl-2,5-dimethyl-2,6-dihydro-7*H*-pyrazolo[3,4-*c*]pyridin-7-one **(1-S32)** as a desired product (65 mg, 22%). **LC-MS (ESI, m/z):** calcd for [M+H]⁺ 411.1; found: 411.2.

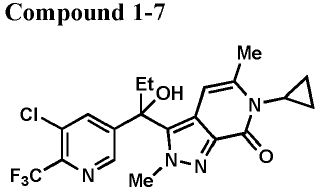

### 3-(1-(5-chloro-6-(trifluoromethyl)pyridin-3-yl)-1-hydroxypropyl)-6-cyclopropyl-2,5-dimethyl-2,6-dihydro-7H-pyrazolo[3,4-c]pyridin-7-one (1-7)

Following **General Procedure F1** on 0.14 mmol scale with 3-(5-chloro-6-(trifluoromethyl)nicotinoyl)-6-cyclopropyl-2,5-dimethyl-2,6-dihydro-7*H*-pyrazolo[3,4-c]pyridin-7-one **(1-S32)** and EtMgBr (3M in diethyl ether). Purification by Combiflash chromatography (65% ethyl acetate in hexane) to afford 3-(1-(5-chloro-6-(trifluoromethyl)pyridin-3-yl)-1-hydroxypropyl)-6-cyclopropyl-2,5-dimethyl-2,6-dihydro-7*H-*pyrazolo[3,4-*c*]pyridin-7-one **(1-7)** as an off white solid (7 mg, 11%). **Physical state:** off white solid. **¹H NMR (400 MHz, DMSO-*d₆*):** *δ* 8.52 (d, *J =* 1.5 Hz, 1H), 7.97 (d, *J =* 1.1 Hz, 1H), 6.58 (s, 1H), 6.48 (s, 1H), 3.73 (s, 3H), 2.87-2.85 (m, 1H), 2.46 (s, 3H), 1.25-1.22 (m, 3H), 1.12-1.11 (m, 2H), 0.85-0.76 (m, 4 H) ppm. **LC-MS (ESI, m/z):** calcd for [M+H]⁺ 441.1; found: 441.2.

### Synthesis of compounds 1-8, 1-9, 1-12, 1-13, 1-18, 1-19

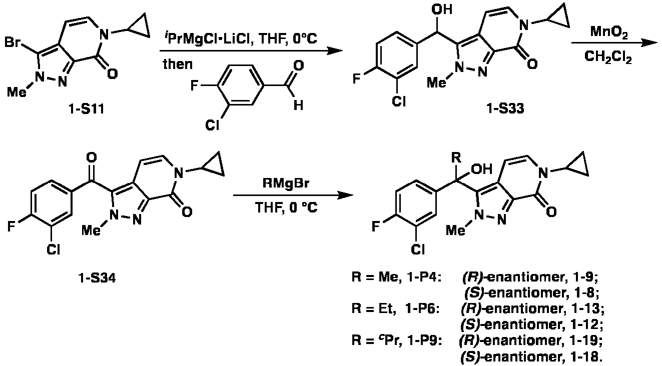

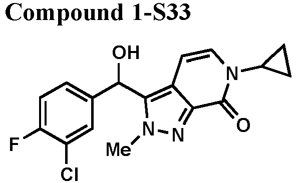

### 3-((3-chloro-4-fluorophenyl)(hydroxy)methyl)-6-cyclopropyl-2-methyl-2,6-dihydro-7H-pyrazolo[3,4-c]pyridin-7-one (1-S33)

Following **General Procedure D1** on 2.62 mmol scale with 3-bromo-6-cyclopropyl-2-methyl-2,6-dihydro-7*H*-pyrazolo[3,4-*c*]pyridin-7-one **(1-S11)** and 3-chloro-4-fluorobenzaldehyde. Purification by CombiFlash chromatography (90% ethyl acetate in hexane) to afford 3-((3-chloro-4-fluorophenyl)(hydroxy)methyl)-6-cyclopropyl-2-methyl-2,6-dihydro-7*H-*pyrazolo[3,4-*c*]pyridin-7-one **(1-S33)** as an off-white solid (550 mg, 60%). **Physical State:** off-white solid. **LC-MS (ESI, m/z):** calcd for [M+H]⁺ 348.1; found: 348.0.

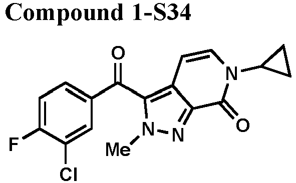

### 3-(3-chloro-4-fluorobenzoyl)-6-cyclopropyl-2-methyl-2,6-dihydro-7H-pyrazolo[3,4-c]pyridin-7-one (1-S34)

Following **General Procedure E1** on 0.35 mmol scale with 3-((3-chloro-4-fluorophenyl)(hydroxy)methyl)-6-cyclopropyl-2-methyl-2,6-dihydro-7*H*-pyrazolo[3,4-*c*]pyridin-7-one **(1-S33).** Purification by CombiFlash chromatography (70% ethyl acetate in hexane) to afford 3-(3-chloro-4-fluorobenzoyl)-6-cyclopropyl-2-methyl-2,6-dihydro-7*H-*pyrazolo[3,4-*c*] pyridine-7-one **(1-S34)** as a yellow solid (100 mg, 83%). **Physical State:** yellow solid. **¹H NMR (400 MHz, DMSO-d*₆*):** *δ* 7.99 (d, *J* = 6.9 Hz, 1H), 7.80 (br s, 1H), 7.65 (t, *J =* 9.0 Hz, 1H), 7.12 (d, *J =* 7.4 Hz, 1H), 5.61 (d, *J =* 7.5 Hz, 1H), 4.27 (s, 3H), 3.21-3.19 (m, 1H), 1.00-0.95 (m, 2H), 0.80-0.75 (m, 2H) ppm. **LC-MS (ESI, m/z):** calcd for [M+H]⁺ 348.1; found: 348.0.

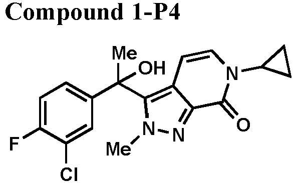

### 3-(1-(3-chloro-4-fluorophenyl)-1-hydroxyethyl)-6-cyclopropyl-2-methyl-2,6-dihydro-7H-pyrazolo[3,4-c]pyridin-7-one (1-P4)

Following **General Procedure F1** on 0.29 mmol scale with 3-(3-chloro-4-fluorobenzoyl)-6-cyclopropyl-2-methyl-2,6-dihydro-7*H*-pyrazolo[3,4-*c*]pyridin-7-one **(1-S34)** and MeMgBr (3M in diethyl ether). Purification by CombiFlash chromatography (80% ethyl acetate in hexane) to afford 3-(1-(3-chloro-4-fluorophenyl)-1-hydroxyethyl)-6-cyclopropyl-2-methyl-2,6-dihydro-7*H*-pyra-zolo[3,4-*c*]pyridin-7-one **(1-P4)** as an off white solid (50 mg, 47%). **Physical State:** off white solid.

The racemic compound **1-P4** was purified from preparative-HPLC-Chiral (SFC) to afford Enantiomer-1 **(1-9)** (15 mg) and Enantiomer-2 **(1-8)** (15 mg).

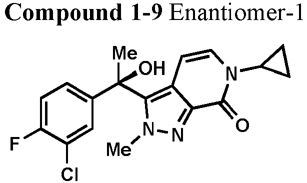

***(R)-3-(1-(3-chloro-4-fluorophenyl)-1-hydroxyethyl)-6-cyclopropyl-2-methyl-2,6-dihydro-7H-pyrazolo[3,4-c]pyridin-7-one* (1-9) ¹H NMR (400 MHz, DMSO-*d₆*):** *δ* 7.48 (d, *J =* 5.6 Hz, 1H), 7.36 (t, *J* = 8.9 Hz, 1H), 7.16 (br s, 1H), 6.96 (d, *J =* 7.6 Hz, 1H), 6.60 (s, 1H), 6.36 (d, *J* = 7.6 Hz, 1H), 3.73 (s, 3H), 3.26-3.24 (m, 1H), 1.97 (s, 3H), 1.02-0.98 (m, 2H), 0.85-0.80 (m, 2H) ppm. **LC-MS (ESI, m/z):** calcd for [M+H]⁺ 362.1; found: 362.1. **Retention time:** 1.72 min.

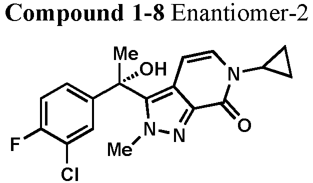

### (S)-3-(1-(3-chloro-4-fluorophenyl)-1-hydroxyethyl)-6-cyclopropyl-2-methyl-2,6-dihydro-7H-pyrazolo[3,4-c]pyridin-7-one (1-8)

**¹H NMR (400 MHz, DMSO-d*₆*):** *δ* 7.48 (d, *J =* 5.6 Hz, 1H), 7.36 (t, *J =* 8.9 Hz, 1H), 7.16 (br s, 1H), 6.96 (d, *J= 7.6* Hz, 1H), 6.60 (s, 1H), 6.36 (d, *J =* 7.6 Hz, 1H), 3.73 (s, 3H), 3.26-3.24 (m, 1H), 1.97 (s, 3H), 1.02-0.98 (m, 2H), 0.85-0.80 (m, 2H) ppm. **LC-MS (ESI, m/z):** calcd for [M+H]⁺ 362.1; found: 362.1. **Retention time:** 2.08 min.

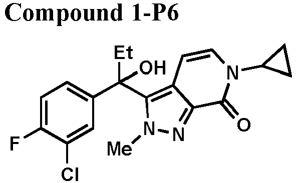

### 3-(1-(3-chloro-4-fluorophenyl)-1-hydroxypropyl)-6-cyclopropyl-2-methyl-2,6-dihydro-7H-pyrazolo[3,4-c]pyridin-7-one (1-P6)

Following **General Procedure F1** on 0.29 mmol scale with 3-(3-chloro-4-fluorobenzoyl)-6-cyclopropyl-2-methyl-2,6-dihydro-7*H*-pyrazolo[3,4-*c*]pyridin-7-one **(1-S34)** and EtMgBr (3M in THF). Purification by CombiFlash chromatography (70% ethyl acetate in hexane) to afford 3-(1-(3-chloro-4-fluorophenyl)-1-hydroxypropyl)-6-cyclopropyl-2-methyl-2,6-dihydro-7*H-*pyrazolo-[3,4-*c*]pyridin-7-one **(1-P6)** as an off-white solid (55 mg, 25%). **Physical State:** off-white solid.

The racemic compound **1-P6** was purified from Preparative-HPLC-Chiral (NP) to afford enantiomer-1 **(1-13)** (10 mg) and enantiomer-2 **(1-12)** (10 mg).

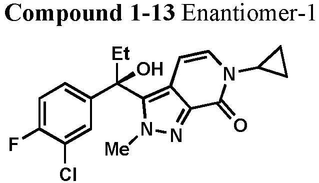

### (R)-3-(1-(3-chloro-4-fluorophenyl)-1-hydroxypropyl)-6-cyclopropyl-2-methyl-2,6-dihydro-7H-pyrazolo[3,4-c]pyridin-7-one (1-13)

**¹H NMR (400 MHz, DMSO-*d₆*):** *δ* 7.43-7.41 (m, 1 H), 7.36 (t, *J =* 8.9 Hz, 1 H), 7.15-7.12 (m, 1 H), 6.98 (d, *J =* 7.6 Hz, 1 H), 6.50 (d, *J =* 7.7 Hz, 1 H), 6.30 (s, 1 H), 3.72 (s, 3 H), 3.27-3.22 (m, 1 H), 2.46-2.41 (m, 1 H), 2.33-2.26 (m, 1 H), 1.02-0.97 (m, 2 H), 0.83-0.76 (m, 5 H) ppm. **LC-MS (ESI, m/z):** calcd for [M+H]⁺ 376.1; found: 376.2. **Retention time:** 4.37 min.

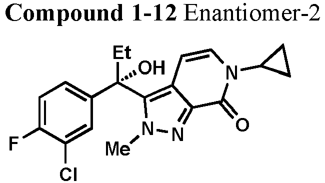

### (S)-3-(1-(3-chloro-4-fluorophenyl)-1-hydroxypropyl)-6-cyclopropyl-2-methyl-2,6-dihydro-7H-pyrazolo[3,4-c]pyridin-7-one (1-12)

**¹H NMR (400 MHz, DMSO-*d₆*):** *δ* 7.43 (dd, *J* = 7.2*,* 2 Hz, 1 H), 7.36 (t, *J =* 8.9 Hz, 1 H), 7.15-7.12 (m, 1 H), 6.98 (d, *J* = 7.6 Hz, 1 H), 6.50 (d, *J =* 7.7 Hz, 1 H), 6.30 (s, 1 H), 3.72 (s, 3 H), 3.27-3.22 (m, 1 H), 2.46-2.41 (m, 1 H), 2.33-2.26 (m, 1 H), 1.02-0.97 (m, 2 H), 0.83-0.76 (m, 5 H) ppm. **LC-MS (ESI, m/z):** calcd for [M+H]⁺ 376.1; found: 376.2. **Retention time:** 4.82 min.

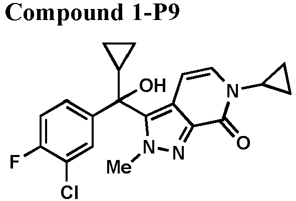

### 3-((3-chloro-4-fluorophenyl)(cyclopropyl)(hydroxy)methyl)-6-cyclopropyl-2-methyl-2,6-di-hydro-7H-pyrazolo[3,4-c]pyridin-7-one (1-P9)

Following **General Procedure F1** on 0.58 mmol scale with 3-(3-chloro-4-fluorobenzoyl)-6-cyclopropyl-2-methyl-2,6-dihydro-7*H*-pyrazolo[3,4-*c*]pyridin-7-one **(1-S34)** and cyclopropyl magnesium bromide (0.7 M in THF). Purification by CombiFlash chromatography (70% ethyl acetate in hexane) to afford CombiFlash chromatography (70% ethyl acetate in hexane) to afford 3-((3-chloro-4-fluorophenyl)(cyclopropyl)(hydroxy)methyl)-6-cyclopropyl-2-methyl-2,6-di-hydro-7*H*-pyrazolo[3,4-*c*]pyridin-7-one **(1-P9)** as an off-white solid (45 mg, 20%).

**Physical State:** off-white solid.

The racemic compound **1-P9** was purified from Preparative-HPLC-Chiral (NP) to afford enantiomer-1 **(1-19)** (8 mg) and enantiomer-2 **(1-18)** (8 mg).

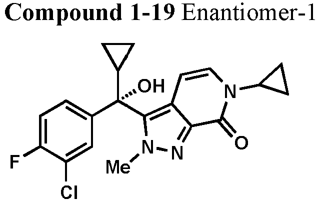

### (S)-3-((3-chloro-4-fluorophenyl)(cyclopropyl)(hydroxy)methyl)-6-cyclopropyl-2-methyl-2,6-dihydro-7H-pyrazolo[3,4-c]pyridin-7-one (1-19)

**¹H NMR (400 MHz, DMSO-*d₆*):** *δ* 7.40-7.34 (m, 2H), 7.09 (d, *J =* 2.4 Hz, 1H), 6.96 (d, *J =* 7.6 Hz, 1H), 6.63 (d, *J =* 7.6 Hz, 1H), 6.09 (s, 1H), 3.65 (s, 3H), 3.29-3.25 (m, 1H), 1.75-1.70 (m, 1H), 1.00-0.98 (m, 2H), 0.85-0.81 (m, 2H), 0.63-0.62 (m, 2H), 0.51-0.45 (m, 2H) ppm. **LC-MS (ESI, m/z):** calcd for [M+H]⁺ 388.1; found: 388.1. **Retention time:** 5.93 min.

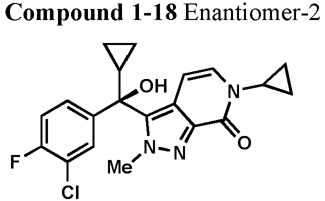

### (R)-3-((3-chloro-4-fluorophenyl)(cyclopropyl)(hydroxy)methyl)-6-cyclopropyl-2-methyl-2,6-dihydro-7H-pyrazolo[3,4-c]pyridin-7-one (1-18)

**¹H NMR (400 MHz, DMSO-*d₆*):** *δ* 7.40-7.34 (m, 2H), 7.10-7.05 (m, 1H), 6.96 (d, *J =* 7.6 Hz, 1H), 6.63 (d, *J =* 7.6 Hz, 1H), 6.09 (s, 1H), 3.65 (s, 3H), 3.29-3.25 (m, 1H), 1.75-1.70 (m, 1H), 1.00-0.98 (m, 2H), 0.85-0.81 (m, 2H), 0.63-0.62 (m, 2H), 0.51-0.45 (m, 2H) ppm. **LC-MS (ESI, m/z):** calcd for [M+H]⁺ 388.1; found: 388.1. **Retention time:** 7.97 min.

### Synthesis of compounds 1-20, 1-21

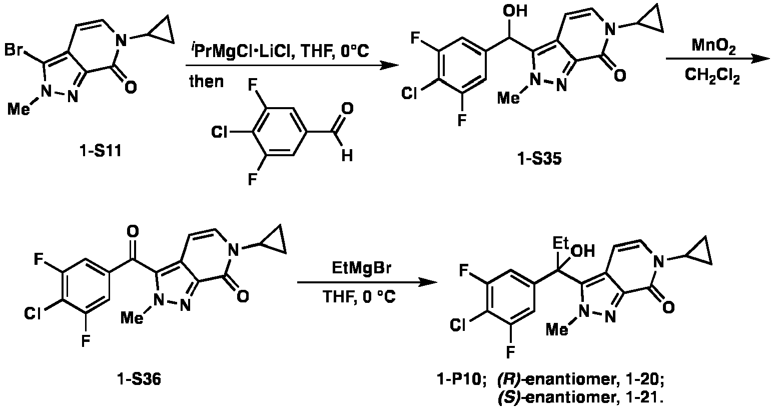

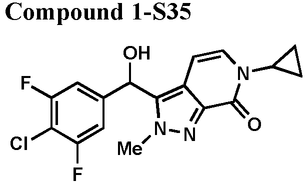

### 3-((4-chloro-3,5-dijluorophenyl)(hydroxy)methyl)-6-cyclopropyl-2-methyl-2,6-dihydro-7H-pyrazolo[3,4-c]pyridin-7-one (1-S35)

Following **General Procedure D1** on 0.74 mmol scale with 3-bromo-6-cyclopropyl-2-methyl-2,6-dihydro-7*H*-pyrazolo[3,4-*c*]pyridin-7-one **(1-S11)** and 4-chloro-3,5-difluorobenzaldehyde. Purification by CombiFlash chromatography (90% ethyl acetate in hexane) to afford 3-((4-chloro-3,5-difluorophenyl)(hydroxy)methyl)-6-cyclopropyl-2-methyl-2,6-dihydro-7*H-*pyrazolo[3,4-*c*]pyridin-7-one **(1-S35)** as an off-white solid (150 mg, 55%). **Physical State:** off-white solid. **¹H NMR (400 MHz, DMSO-*d₆*):** *δ* 7.31 (d, *J =* 8.4 Hz, 2H), 6.88 (d, *J =* 7.4 Hz, 1H), 6.67 (d, *J =* 4.8 Hz, 1H), 6.24-6.23 (m, 1H), 5.82 (d, *J =* 7.4 Hz, 1H), 4.05 (s, 3H), 3.21-3.20 (m, 1H), 0.95-0.93 (m, 2H), 0.76-0.75 (m, 2H) ppm. **LC-MS (ESI, m/z):** calcd for [M+H]⁺ 366.1; found: 366.1.

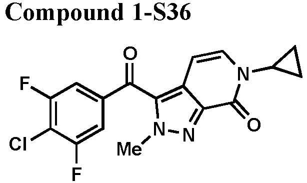

### 3-(4-chloro-3,5-difluorobenzoyl)-6-cyclopropyl-2-methyl-2,6-dihydro-7H-pyrazolo[3,4-c]pyri-din-7-one (1-S36)

Following **General Procedure E1** on 0.33 mmol scale with 3-((4-chloro-3,5-difluorophenyl)(hydroxy)methyl)-6-cyclopropyl-2-methyl-2,6-dihydro-7*H*-pyrazolo[3,4-*c*]pyridin-7-one **(1-S35).** Purification by CombiFlash chromatography (75% ethyl acetate in hexane) to afford 3-(4-chloro-3,5-difluorobenzoyl)-6-cyclopropyl-2-methyl-2,6-dihydro-7*H-*pyrazolo[3,4-*c*]pyridin-7-one **(1-S36)** as a yellow solid (100 mg, 83%). **Physical State:** yellow solid. **¹H NMR (400 MHz, DMSO-*d₆*):** *δ* 7.72 (d, *J =* 7.5 Hz, 2H), 7.12 (d, *J =* 7.1 Hz, 1H), 5.68 (d, *J =* 7 Hz, 1H), 4.28 (s, 3H), 3.30-3.25 (m, 1H), 1.00-0.95 (m, 2H), 0.80-0.70 (m, 2H) ppm. **LC-MS (ESI, m/z):** calcd for [M+H]⁺ 364.1; found: 364.2.

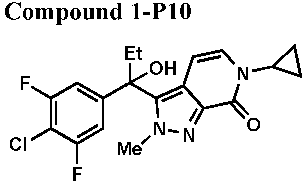

### 3-(1-(4-chloro-3,5-difluorophenyl)-1-hydroxypropyl)-6-cyclopropyl-2-methyl-2,6-dihydro-7H-pyrazolo[3,4-c]pyridin-7-one (1-P10)

Following **General Procedure F1** on 0.27 mmol scale with 3-(4-chloro-3,5-difluorobenzoyl)-6-cyclopropyl-2-methyl-2,6-dihydro-7*H*-pyrazolo[3,4-*c*]pyridin-7-one **(1-S36)** and EtMgBr (3M in THF). Purification by CombiFlash chromatography (80% ethyl acetate in hexane) to afford 3-(1-(4-chloro-3,5-difluorophenyl)-1-hydroxypropyl)-6-cyclopropyl-2-methyl-2,6-dihydro-7*H*-pyrazolo[3,4-*c*]pyridin-7-one **(1-P10)** (35 mg, 32%) as an-off white solid. **Physical State:** off-white solid.

The racemic compound was purified from Preparative-HPLC-Chiral (NP) to afford Enantiomer-1 **(1-21)** (8 mg) and Enantiomer-2 **(1-20)** (8 mg).

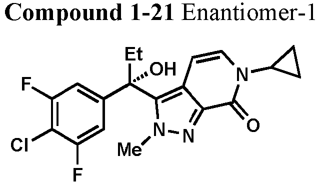

***(S)-3-(1-(4-chloro-3,5-difluorophenyl)-1-hydroxypropyl)-6-cyclopropyl-2-methyl-2,6-dihydro-7H-pyrazolo[3,4-c]pyridin-7-one* (1-21) ¹H NMR (400 MHz, DMSO-*d₆*):** *δ* 7.10 (d, *J =* 8.4 Hz, 2H), 6.99 (d, *J* = 7.6 Hz, 1H), 6.51 (d, *J* = 7.6 Hz, 1H), 6.43 (s, 1H), 3.75 (s, 3H), 3.27-3.23 (m, 1H), 2.47-2.43 (m, 1H), 2.35-2.32 (m, 1H), 1.00-0.97 (m, 2H), 0.83-0.76 (m, 5H) ppm. **LC-MS (ESI, m/z):** calcd for [M+H]⁺ 394.1; found: 394.1. **Retention time:** 4.57 min.

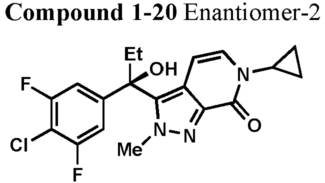

***(R)-3-(1-(4-chloro-3,5-difluorophenyl)-1-hydroxypropyl)-6-cyclopropyl-2-methyl-2,6-dihydro-7H-pyrazolo[3,4-c]pyridin-7-one* (1-20) ¹H NMR (400 MHz, DMSO-*d₆*):** *δ* 7.10 (d, *J =* 8.4 Hz, 2H), 6.99 (d, *J* = 7.6 Hz, 1H), 6.51 (d, *J =* 7.6 Hz, 1H), 6.43 (s, 1H), 3.75 (s, 3H), 3.27-3.23 (m, 1H), 2.47-2.43 (m, 1H), 2.35-2.32 (m, 1H), 1.00-0.97 (m, 2H), 0.83-0.76 (m, 5H) ppm. **LC-MS (ESI, m/z):** calcd for [M+H]⁺ 394.1; found: 394.1. **Retention time:** 5.38 min.

### Synthesis of compounds 1-22, 1-23, 1-26, 1-27

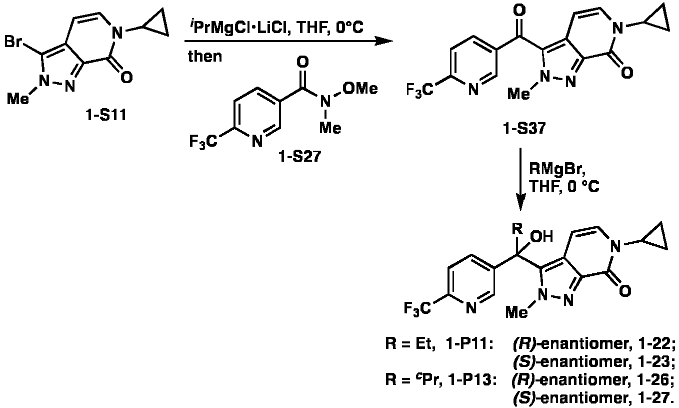

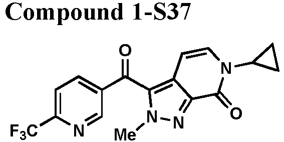

### 6-cyclopropyl-2-methyl-3-(6-(trifluoromethyl)nicotinoyl)-2,6-dihydro-7H-pyrazolo[3,4-c]pyridin-7-one (1-S37)

Following **General Procedure C1** on 4.48 mmol scale with 3-bromo-6-cyclopropyl-2-methyl-2,6-dihydro-7*H*-pyrazolo[3,4-*c*]pyridin-7-one **(1-S11)** and *N*-methoxy-*N*-methyl-6-(trifluoromethyl)nicotinamide **(1-S27).** Purification by CombiFlash chromatography (70% ethyl acetate in hexane) to afford 6-cyclopropyl-2-methyl-3-(6-(trifluoromethyl)nicotinoyl)-2,6-dihydro-7*H*-pyrazolo[3,4-*c*]pyridin-7-one **(1-S37)** as a yellow solid (0.6 g, 37%). **Physical State:** yellow solid. **¹H NMR (400 MHz, DMSO-*d₆*):** *δ* 9.07 (s, 1 H), 8.43-8.41 (m, 1 H), 8.16 (d, *J =* 8.08 Hz, 1 H), 7.14 (d, *J =* 7.56 Hz, 1 H), 5.51 (d, *J =* 7.56 Hz, 1 H), 4.33 (s, 3 H), 3.27-3.25 (m, 1 H), 1.01-0.96 (m, 2 H), 0.81-0.77 (m, 2 H) ppm. **LC-MS (ESI, m/z):** calcd for [M+H]⁺ 363.1; found: 363.1.

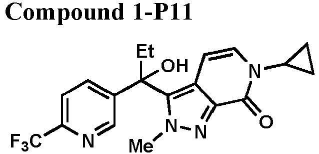

### 6-cyclopropyl-3-(1-hydroxy-1-(6-(trifluoromethyl)pyridin-3-yl)propyl)-2-methyl-2,6-dihydro-7H-pyrazolo[3,4-c]pyridin-7-one (1-P11)

Following **General Procedure F1** on 0.41 mmol scale with 6-cyclopropyl-2-methyl-3-(6-(trifluoromethyl)nicotinoyl)-2,6-dihydro-7*H*-pyrazolo[3,4-*c*]pyridin-7-one **(1-S37)** and EtMgBr (3 M in THF). Purification CombiFlash chromatography (70% ethyl acetate in hexane) to afford 6-cyclopropyl-3-(1-hydroxy-1-(6-(trifluoromethyl)pyridin-3-yl)propyl)-2-methyl-2,6-dihydro-7*H*-pyrazolo[3,4-*c*]pyridin-7-one **(1-P11)** as an off-white solid (70 mg, 43%).

**Physical State:** off-white solid.

The racemic compound **1-P11** was purified by preparative-HPLC-NP to afford enantiomer-1 (**1-23**) (15 mg) and enantiomer-2 **(1-22)** (15 mg).

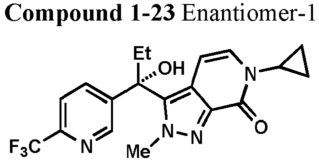

***(S)-6-cyclopropyl-3-(1-hydroxy-1-(6-(trifluoromethyl)pyridin-3-yl)propyl)-2-methyl-2,6-dihydro-7H-pyrazolo[3,4-c]pyridin-7-one* (1-23) ¹H NMR (400 MHz, DMSO-*d₆*):** *δ* 8.68 (s, 1H), 7.87-7.85 (m, 2H), 7.01 (d, *J =* 7.76 Hz, 1H), 6.54-6.52 (m, 2H), 3.73 (s, 3H), 3.25-3.24 (m, 1H), 2.49-2.39 (m, 2H), 1.01-0.98 (m, 2H), 0.82-0.78 (m, 5H) ppm; **LC-MS (ESI, m/z):** calcd for [M+H]⁺ 393.2; found: 393.2. **Retention time:** 7.27 min.

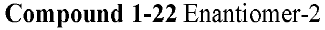

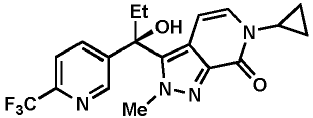

***(R)-6-cyclopropyl-3-(1-hydroxy-1-(6-(trifluoromethyl)pyridin-3-yl)propyl)-2-methyl-2,6-dihydro-7H-pyrazolo[3,4-c]pyridin-7-one* (1-22) ¹H NMR (400 MHz, DMSO-d*₆*)** *δ* 8.68 (s, 1H), 7.87-7.85 (m, 2H), 7.01 (d, *J =* 7.76 Hz, 1H), 6.54-6.52 (m, 2H), 3.73 (s, 3H), 3.25-3.24 (m, 1H), 2.49-2.39 (m, 2H), 1.01-0.98 (m, 2H), 0.82-0.78 (m, 5H) ppm. **LC-MS (ESI, m/z):** calcd for [M+H]⁺ 393.2; found: 393.2. **Retention time:** 9.77 min.

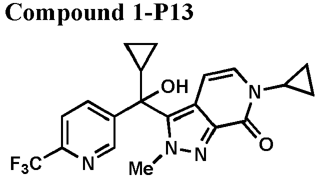

### 6-cyclopropyl-3-(cyclopropyl(hydroxy)(6-(trifluoromethyl)pyridin-3-yl)methyl)-2-methyl-2,6-dihydro-7H-pyrazolo[3,4-c]pyridin-7-one (1-P13)

Following **General Procedure F1** on 0.33 mmol scale with 6-cyclopropyl-2-methyl-3-(6-(trifluoromethyl)nicotinoyl)-2,6-dihydro-7*H*-pyrazolo[3,4-*c*]pyridin-7-one **(1-S37)** and cyclopropyl magnesium bromide (0.7 M in THF). Purification CombiFlash chromatography (70% ethyl acetate in hexane) to afford 6-cyclopropyl-3-(cyclopropyl(hydroxy)(6-(trifluoromethyl)pyridin-3-yl)methyl)-2-methyl-2,6-dihydro-7*H*-pyrazolo[3,4-*c*]pyridin-7-one **(1-P13)** as an off-white solid (80 mg, 60%). **Physical State:** off-white solid.

The racemic compound **1-P13** was submitted for chiral separation (Prep-HPLC-NP) to afford enantiomer-1 **(1-27)** (12 mg) and enantiomer-2 **(1-26)** (12 mg) as a white solid.

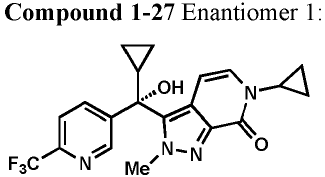

### (S)-6-cyclopropyl-3-(cyclopropyl(hydroxy)(6-(trifluoromethyl)pyridin-3-yl)methyl)-2-methyl-2, 6-dihydro-7H-pyrazolo[3,4-c]pyridin-7-one (1-27)

**¹H NMR (400 MHz, DMSO-*d₆*):** *δ* 8.65 (s, 1H), 7.87 (d, *J =* 8.2 Hz, 1H), 7.79 (d, *J =* 8.2 Hz, 1H), 6.99 (d, *J* = 7.7 Hz, 1H), 6.65 (d, *J =* 7.6 Hz, 1H), 6.30 (s, 1H), 3.65 (s, 3H), 3.27-3.24 (m, 1H), 1.81-1.78 (m, 1H), 1.01-0.99 (m, 2H), 0.82-0.79 (m, 2H), 0.76-0.72 (m, 2H), 0.55-0.53 (m, 1H), 0.50-0.45 (m, 1H) ppm. **LC-MS (ESI, m/z):** calcd for [M+H]⁺ 405.2; found: 405.1. **Retention time:** 5.24 min.

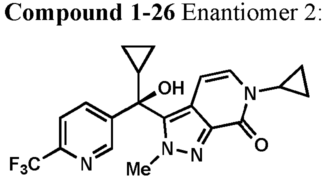

### (R)-6-cyclopropyl-3-(cyclopropyl(hydroxy)(6-(trifluoromethyl)pyridin-3-yl)methyl)-2-methyl-2, 6-dihydro-7H-pyrazolo[3,4-c]pyridin-7-one (1-26)

¹H NMR (400 MHz, DMSO-d*₆*) *δ* 8.65 (s, 1H), 7.87 (d, *J =* 8.2 Hz, 1H), 7.79 (d, *J =* 8.2 Hz, 1H), 6.99 (d, *J* = 7.7 Hz, 1H), 6.65 (d, *J =* 7.6 Hz, 1H), 6.30 (s, 1H), 3.65 (s, 3H), 3.27-3.24 (m, 1H), 1.81-1.78 (m, 1H), 1.01-0.99 (m, 2H), 0.82-0.79 (m, 2H), 0.76-0.72 (m, 2H), 0.55-0.53 (m, 1H), 0.50-0.45 (m, 1H) ppm. **LC-MS (ESI, m/z):** calcd for [M+H]⁺ 405.2; found: 405.1. **Retention time:** 6.92 min.

### Synthesis of compounds 1-24, 1-25

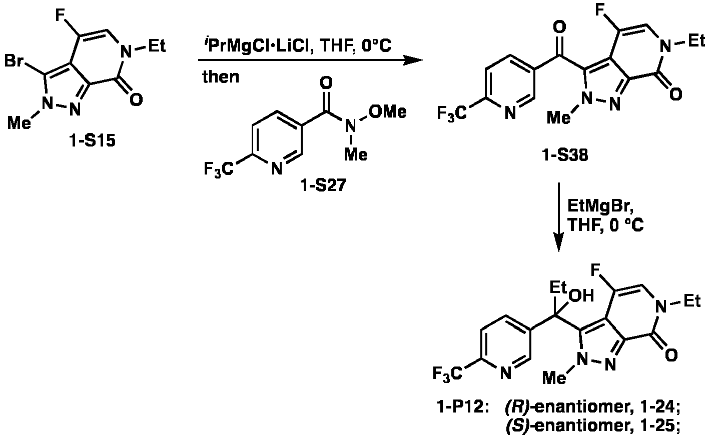

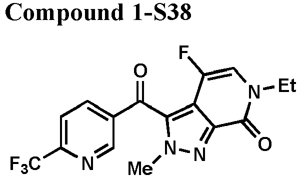

### 6-ethyl-4-fluoro-2-methyl-3-(6-(trifluoromethyl)nicotinoyl)-2,6-dihydro-7H-pyrazolo[3,4-c]pyridin-7-one (1-S38)

Following **General Procedure C1** on 1.46 mmol scale with 3-bromo-6-ethyl-4-fluoro-2-methyl-2,6-dihydro-7*H*-pyrazolo[3,4-c]pyridin-7-one **(1-S15)** and *N*-methoxy-*N*-methyl-6-(trifluoromethyl)nicotinamide **(1-S27).** Purification by CombiFlash chromatography (70% ethyl acetate in hexane) to afford 6-ethyl-4-fluoro-2-methyl-3-(6-(trifluoromethyl)nicotinoyl)-2,6-dihydro-7*H*-pyrazolo[3,4-*c*]pyridin-7-one **(1-S38)** (180 mg, 33%) as a pale yellow solid. **Physical State:** pale yellow solid. **¹H NMR (400 MHz, DMSO-*d₆*):** *δ* 9.12 (s, 1H), 8.47 (d, *J* = 7.8 Hz, 1H), 8.12 (d, *J* =7.5 Hz, 1H), 7.57 (d, *J =* 8 Hz, 1H), 4.30 (s, 3H), 3.92-3.89 (m, 2H), 1.23-1.19 (m, 3H) ppm. **LC-MS (ESI, m/z):** calcd for [M+H]⁺ 369.1; found: 369.1.

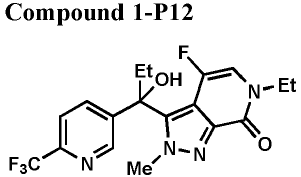

### 6-ethyl-4-fluoro-3-(1-hydroxy-1-(6-(trifluoromethyl)pyridin-3-yl)propyl)-2-methyl-2,6-dihydro-7H-pyrazolo[3,4-c]pyridin-7-one (1-P12)

Following **General Procedure F1** on 0.68 mmol scale with 6-ethyl-4-fluoro-2-methyl-3-(6-(trifluoromethyl)nicotinoyl)-2,6-dihydro-7*H*-pyrazolo[3,4-*c*]pyridin-7-one **(1-S38)** and EtMgBr (3 M in THF). Purification CombiFlash chromatography (70% ethyl acetate in hexane) to afford 6-ethyl-4-fluoro-3-(1-hydroxy-1-(6-(trifluoromethyl)pyridin-3-yl)propyl)-2-methyl-2,6-dihydro-7*H*-pyrazolo[3,4-*c*]pyridin-7-one **(1-P12)** as an off-white solid (100 mg, 37%). **Physical State:** off-white solid. **¹H NMR (400 MHz, DMSO-*d₆*):** *δ* 8.75 (s, 1H), 7.95 (d, *J =* 8.2 Hz, 1H), 7.87 (d, *J* = 8.2 Hz, 1H), 7.34 (d, *J* = 8.2 Hz, 1H), 6.56 (s, 1H), 4.02 (s, 3H), 3.88-3.84 (m, 2H), 2.50-2.40 (m, 2H), 1.23-1.17 (m, 3H), 0.80 (t, *J =* 7.1 Hz, 3H) ppm. **LC-MS (ESI, m/z):** calcd for [M+H]⁺ 399.1; found: 399.2.

The racemic compound **1-P12** was submitted for chiral separation (PREP-HPLC-NP) to afford enantiomer-1 **(1-25)** (10 mg) and enantiomer-2 **(1-24)** (10 mg).

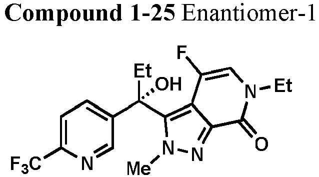

***(S)-6-ethyl-4-fluoro-3-(1-hydroxy-1-(6-(trifluoromethyl)pyridin-3-yl)propyl)-2-methyl-2,6-dihydro-7H-pyrazolo[3,4-c]pyridin-7-one* (1-25) ¹H NMR (400 MHz, DMSO-*d₆*):** *δ* 8.75 (s, 1H), 7.95 (d, *J* = 8.2 Hz, 1H), 7.87 (d, *J =* 8.2 Hz, 1H), 7.34 (d, *J =* 8.2 Hz, 1H), 6.56 (s, 1H), 4.02 (s, 3H), 3.88-3.84 (m, 2H), 2.50-2.40 (m, 2H), 1.23-1.17 (m, 3H), 0.80 (t, *J =* 7.1 Hz, 3H) ppm. **LC-MS (ESI, m/z):** calcd for [M+H]⁺ 399.2; found: 399.2. **Retention time:** 3.33 min.

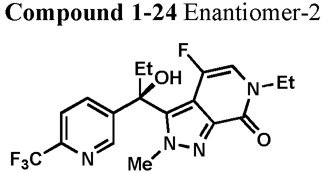

***(R)-6-ethyl-4-fluoro-3-(1-hydroxy-1-(6-(trifluoromethyl)pyridin-3-yl)propyl)-2-methyl-2,6-dihydro-7H-pyrazolo[3,4-c]pyridin-7-one* (1-24) ¹H NMR (400 MHz, DMSO-*d₆*):** *δ* 8.75 (s, 1H), 7.95 (d, *J* = 8.2 Hz, 1H), 7.87 (d, *J =* 8.2 Hz, 1H), 7.34 (d, *J =* 8.2 Hz, 1H), 6.56 (s, 1H), 4.02 (s, 3H), 3.88-3.84 (m, 2H), 2.50-2.40 (m, 2H), 1.23-1.17 (m, 3H), 0.80 (t, *J =* 7.1 Hz, 3H) ppm. **LC-MS (ESI, m/z):** calcd for [M+H]⁺ 399.2; found: 399.2. **Retention time:** 5.21 min.

### Synthesis of compounds 1-28, 1-29.

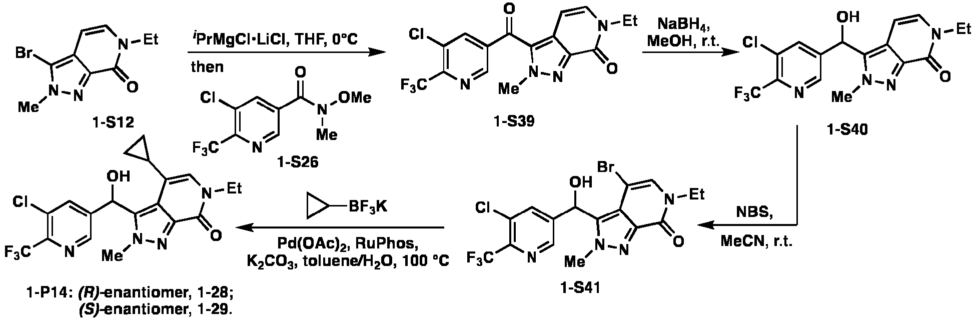

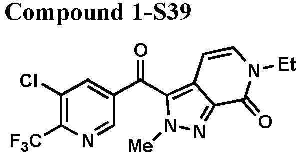

### 3-(5-chloro-6-(trifluoromethyl)nicotinoyl)-6-ethyl-2-methyl-2,6-dihydro-7H-pyrazolo[3,4-c]pyridin-7-one (1-S39)

Following **General Procedure C1** on 3.92 mmol scale with 3-bromo-6-ethyl-2-methyl-2,6-dihydro-7*H*-pyrazolo[3,4-*c*]pyridin-7-one **(1-S12)** and 5-chloro-*N*-methoxy-*N*-methyl-6-(trifluoromethyl)nicotinamide **(1-S26).** Purification by CombiFlash chromatography (30% ethyl acetate in hexane) to afford 3-(5-chloro-6-(trifluoromethyl)nicotinoyl)-6-ethyl-2-methyl-2,6-dihydro-7H-pyrazolo[3,4-c]pyridin-7-one **(1-S39)** as an off-white solid (0.9 g, 60%). **Physical State:** off-white solid. **¹H NMR (400 MHz, DMSO-*d₆*):** *δ* 8.97 (d, *J =* 1.3 Hz, 1H), 8.56 (s, 1H), 7.28 (d, *J =* 7.4 Hz, 1H), 5.73 (d, *J =* 7.4 Hz, 1H), 4.32 (s, 3H), 3.94 (q, *J =* 7.1 Hz, 2H), 1.21 (t, *J =* 7.1 Hz, 3H) ppm. **LC-MS (ESI, m/z):** calcd for [M+H]⁺ 385.1; found: 385.2.

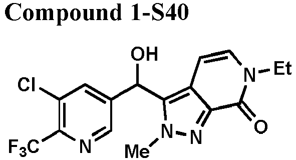

### 3-((5-chloro-6-(trijluoromethyl)pyridin-3-yl)(hydroxy)methyl)-6-ethyl-2-methyl-2,6-dihydro-7H-pyrazolo[3,4-c]pyridin-7-one (1-S40)

Following **General Procedure G1** on 3.90 mmol scale with 3-(5-chloro-6-(trifluoromethyl)nicotinoyl)-6-ethyl-2-methyl-2,6-dihydro-7*H*-pyrazolo[3,4-*c*]pyridin-7-one **(1-S39).** Purification by CombiFlash chromatography (60% ethyl acetate in hexane) to afford 3-((5-chloro-6-(trifluoromethyl)pyridin-3-yl)(hydroxy)methyl)-6-ethyl-2-methyl-2,6-dihydro-7H-pyrazolo[3,4-c]pyridin-7-one **(1-S40)** as an off-white solid (1.3 g, 86%). **Physical State:** off-white solid. **¹H NMR (400 MHz, DMSO-*d₆*):** *δ* 8.70 (d, *J =* 1.2 Hz, 1 H), 8.20 (s, 1 H), 7.03 (d, *J =* 7.4 Hz, 1 H), 6.84 (d, *J =* 4.7 Hz, 1 H), 6.45 (d, *J =* 4.3 Hz, 1 H), 5.97 (d, *J =* 7.4 Hz, 1 H), 4.08 (s, 3 H), 3.88 (q, *J =* 7.2 Hz, 2H), 1.17 (t, *J =* 7.2 Hz, 3H) ppm. **LC-MS (ESI, m/z):** calcd for [M+H]⁺ 387.1; found: 387.0.

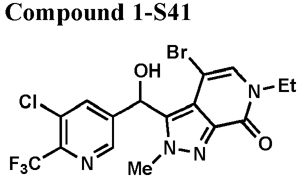

### 4-bromo-3-((5-chloro-6-(trifluoromethyl)pyridin-3-yl)(hydroxy)methyl)-6-ethyl-2-methyl-2,6-dihydro-7H-pyrazolo[3,4-c]pyridin-7-one (1-S41)

Following **General Procedure H1** on 1.09 mmol scale 3-((5-chloro-6-(trifluoromethyl)pyridin-3-yl)(hydroxy)methyl)-6-ethyl-2-methyl-2,6-dihydro-7*H-*pyrazolo[3,4-*c*]pyridin-7-one **(1-S40).** Purification by CombiFlash chromatography (50% ethyl acetate in hexane) to afford 4-bromo-3-((5-chloro-6-(trifluoromethyl)pyridin-3-yl)(hydroxy)methyl)-6-ethyl-2-methyl-2,6-dihydro-7*H*-pyrazolo[3,4-*c*]pyridin-7-one **(1-S41)** as an off-white solid (370 mg, 73%). **Physical State:** off-white solid. **¹H NMR (400 MHz, DMSO-*d₆*):** *δ* 8.54 (s, 1H), 8.03 (s, 1H), 7.55 (s, 1H), 7.15 (d, *J =* 4.1 Hz, 1H), 6.95 (s, 1H), 4.10-3.90 (m, 2H), 3.86 (s, 3H), 1.30-1.10 (m, 3H) ppm. **LC-MS (ESI, m/z):** calcd for [M+H]⁺ 465.0; found: 464.9, 466.9 [M+H+2]⁺.

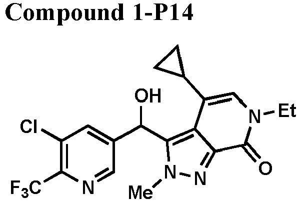

### 3-((5-chloro-6-(trifluoromethyl)pyridin-3-yl)(hydroxy)methyl)-4-cyclopropyl-6-ethyl-2-methyl-2,6-dihydro-7H-pyrazolo[3,4-c]pyridin-7-one (1-P14)

Following **General Procedure I2** on 1.61 mmol scale with 4-bromo-3-((5-chloro-6-(trifluoromethyl)pyridin-3-yl)(hydroxy)methyl)-6-ethyl-2-methyl-2,6-dihydro-7*H-*pyrazolo[3,4-c]pyridin-7-one **(1-S41)** and potassium cyclopropyltrifluroborate. Purification by CombiFlash chromatography (90% ethyl acetate in hexane) to afford 33-((5-chloro-6-(trifluoromethyl)pyridin-3-yl)(hydroxy)methyl)-4-cyclopropyl-6-ethyl-2-methyl-2,6-dihydro-7*H*-pyrazolo[3,4-*c*]pyridin-7-one **(1-P14)** as an off-white solid (100 mg, 15%). **Physical State:** off-white solid.

The racemic compound **1-P14** was submitted for chiral separation (Prep-HPLC-Chiral-NP) to afford enantiomer-1 **(1-29)** (20 mg) and enantiomer-2 **(1-28)** (20 mg).

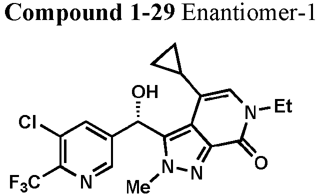

### (S)-3-((5-chloro-6-(trifluoromethyl)pyridin-3-yl)(hydroxy)methyl)-4-cyclopropyl-6-ethyl-2-methyl-2, 6-dihydro-7H-pyrazolo[3,4-c]pyridin-7-one (1-29)

**¹H NMR (400 MHz, DMSO-*d₆*):** *δ* 8.54 (s, 1H), 8.04 (s, 1H), 7.03 (d, *J =* 4.0 Hz, 1H), 6.90 (s, 2H), 3.90 (q, *J =* 7.0 Hz, 2H), 3.82 (s, 3H), 1.95-1.90 (m, 1H), 1.19 (t, *J =* 7.0 Hz, 3H), 0.72-0.58 (m, 4H) ppm. **LC-MS (ESI, m/z):** calcd for [M+H]⁺ 427.1; found: 427.1. **Retention time:** 3.65 min.

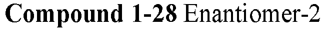

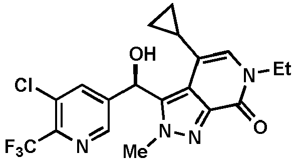

### (R)-3-((5-chloro-6-(trifluoromethyl)pyridin-3-yl)(hydroxy)methyl)-4-cyclopropyl-6-ethyl-2-methyl-2, 6-dihydro-7H-pyrazolo[3,4-c]pyridin-7-one (1-28)

**¹H NMR (400 MHz, DMSO-*d₆*):** *δ* 8.54 (s, 1H), 8.04 (s, 1H), 7.03 (d, *J =* 4.0 Hz, 1H), 6.90 (s, 2H), 3.90 (q, *J =* 7.0 Hz, 2H), 3.82 (s, 3H), 1.95-1.90 (m, 1H), 1.19 (t, *J =* 7.0 Hz, 3H), 0.72-0.58 (m, 4H) ppm. **LC-MS (ESI, m/z):** calcd for [M+H]⁺ 427.1; found: 427.1. **Retention time:** 5.53 min.

### Synthesis of compounds 1-30, 1-31

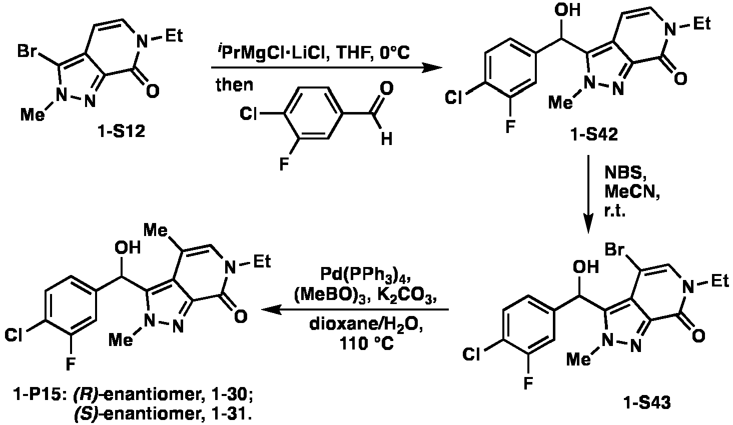

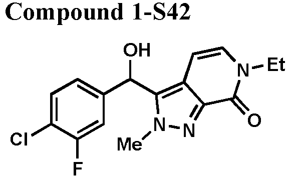

### 3-((4-chloro-3-fluorophenyl)(hydroxy)methyl)-6-ethyl-2-methyl-2,6-dihydro-7H-pyrazolo[3,4-c]pyridin-7-one (1-S42)

Following **General Procedure D1** on 3.90 mmol scale with 3-bromo-6-ethyl-2-methyl-2,6-dihydro-7H-pyrazolo[3,4-c]pyridin-7-one **(1-S12)** and 4-chloro-3-fluorobenzaldehyde. Purification by column chromatography (SiO₂ 100-200, 80% ethyl acetate in hexane) to afford 3-((4-chloro-3-fluorophenyl)(hydroxy)methyl)-6-ethyl-2-methyl-2,6-dihydro-7*H-*pyrazolo[3,4-c]pyridin-7-one **(1-S42)** as an off-white solid (800 mg, 61%). **Physical State:** off-white solid.

**¹H NMR (400 MHz, DMSO-*d₆*):** *δ* 7.60 (t, *J =* 8.0 Hz, 1H), 7.45 (d, *J =* 10.36 Hz, 1H), 7.22 (d, *J =* 8.32 Hz, 1H), 7.00 (d, *J =* 7.36 Hz, 1H), 6.57 (d, *J =* 4.8 Hz, 1H), 6.24 (d, *J =* 4.76 Hz, 1H), 5.90 (d, *J =* 7.4 Hz, 1H), 4.02 (s, 3H), 3.87 (q, *J =* 7.12 Hz, 2H), 1.17 (t, *J =* 7.12 Hz, 3H) ppm. **LC-MS (ESI, m/z):** calcd for [M+H]⁺ 336.1; found: 336.15.

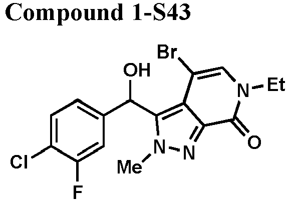

### 4-bromo-3-((4-chloro-3-fluorophenyl)(hydroxy)methyl)-6-ethyl-2-methyl-2,6-dihydro-7H-pyrazolo[3,4-c]pyridin-7-one (1-S43

Following **General Procedure H1** on 0.57 mmol scale with 3-((4-chloro-3-fluorophenyl)(hydroxy)methyl)-6-ethyl-2-methyl-2,6-dihydro-7*H*-pyrazolo[3,4-*c*]pyridin-7-one **(1-S42).** After completion, the reaction mixture was quenched with ice cold water. An off-white solid was appeared, which was filtered, washed with cold water and dried to afford 4-bromo-3-((4-chloro-3-fluorophenyl)(hydroxy)methyl)-6-ethyl-2-methyl-2,6-dihydro-7*H-*pyrazolo[3,4-*c*]pyridin-7-one **(1-S43)** as an off-white solid (200 mg, 85%). **Physical State:** off-white solid. **¹H NMR (400 MHz, DMSO-*d₆*):** *δ* 7.59-7.55 (m, 2H), 7.36 (d, *J =* 10.32 Hz, 1H), 7.02 (d, *J =* 8.28 Hz, 1H), 6.94 (d, *J =* 4.44 Hz, 1H), 6.85 (d, *J =* 4.4 Hz, 1H), 4.00-3.87 (m, 2H), 3.81 (s, 3H), 1.22 (t, *J =* 7 Hz, 3H) ppm. **LC-MS (ESI, m/z):** calcd for [M+H]⁺ 414.1; found: 414.06, 416.07 [M+2+H]⁺.

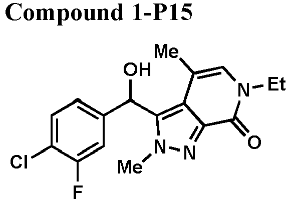

### 3-((4-chloro-3-fluorophenyl)(hydroxy)methyl)-6-ethyl-2,4-dimethyl-2,6-dihydro-7H-pyrazolo[3,4-c]pyridin-7-one (1-P15)

Following **General Procedure I1** on 2.17 mmol scale with 4-bromo-3-((4-chloro-3-fluorophenyl)(hydroxy)methyl)-6-ethyl-2-methyl-2,6-dihydro-7*H*-pyrazolo[3,4-*c*]pyridin-7-one **(1-S43)** and trimethylboraxine. Purification by CombiFlash chromatography (70% ethyl acetate in hexane) to afford 3-((4-chloro-3-fluorophenyl)(hydroxy)methyl)-6-ethyl-2,4-dimethyl-2,6-dihydro-7H-pyrazolo[3,4-c]pyridin-7-one **(1-P15)** as an off-white solid (300 mg, 40%). **Physical State:** off-white solid. **¹H NMR (400 MHz, DMSO-*d₆*):** *δ* 7.55 (t, *J =* 8.0 Hz, 1H), 7.32 (d, *J =* 10.44 Hz, 1H), 6.98 (d, *J =* 8.28 Hz, 1H), 6.89 (s, 1H), 6.75 (d, *J* = 4.16 Hz, 1H), 6.36 (d, *J* = 4.24 Hz, 1H), 3.93-3.84 (m, 2H), 3.82 (s, 3H), 2.12 (s, 3H), 1.20 (t, *J=* 7.16 Hz, 3H) ppm. **LC-MS (ESI, m/z):** calcd for [M+H]⁺ 350.1; found: 350.17.

The racemic compound **1-P15** (100 mg) was submitted to prep-HPLC (SFC) to afford 35 mg of Enantiomer-1 **(1-31)** and 35 mg of Enantiomer-2 **(1-30).**

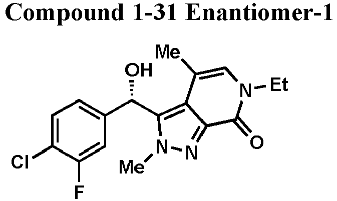

***(S)-3-((4-chloro-3-fluorophenyl)(hydroxy)methyl)-6-ethyl-2,4-dimethyl-2,6-dihydro-7H-pyrazolo[3,4-c]pyridin-7-one* (1-31) ¹H NMR (400 MHz, DMSO-*d₆*):** *δ* 7.55 (t, *J =* 8.0 Hz, 1H), 7.32 (d, *J =* 10.44 Hz, 1H), 6.98 (d, *J =* 8.28 Hz, 1H), 6.89 (s, 1H), 6.75 (d, *J =* 4.16 Hz, 1H), 6.36 (d, *J =* 4.24 Hz, 1H), 3.93-3.84 (m, 2H), 3.82 (s, 3H), 2.12 (s, 3H), 1.20 (t, *J =* 7.16 Hz, 3H) ppm. **LC-MS (ESI, m/z):** calcd for [M+H]⁺ 350.1; found: 350.1. **Retention time:** 6.02 min.

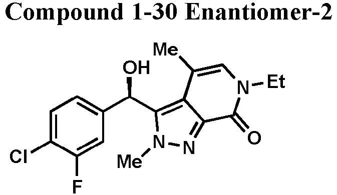

### (R)-3-((4-chloro-3-fluorophenyl)(hydroxy)methyl)-6-ethyl-2,4-dimethyl-2,6-dihydro-7H-pyrazolo[3,4-c]pyridin-7-one (1-30)

**¹H NMR (400 MHz, DMSO-*d₆*):** *δ* 7.55 (t, *J =* 8.0 Hz, 1H), 7.32 (d, *J =* 10.44 Hz, 1H), 6.98 (d, *J* = 8.28 Hz, 1H), 6.89 (s, 1H), 6.75 (d, *J =* 4.16 Hz, 1H), 6.36 (d, *J =* 4.24 Hz, 1H), 3.93-3.84 (m, 2H), 3.82 (s, 3H), 2.12 (s, 3H), 1.20 (t, *J =* 7.16 Hz, 3H) ppm. **LC-MS (ESI, m/z):** calcd for [M+H]⁺ 350.1; found: 350.17. **Retention time:** 7.19 min.

### Synthesis of compounds 1-32, 1-33, 1-36, 1-37, 1-42, 1-43

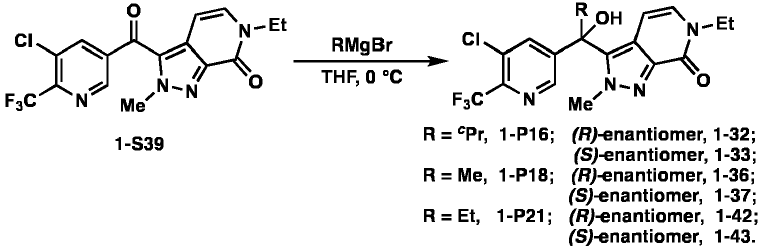

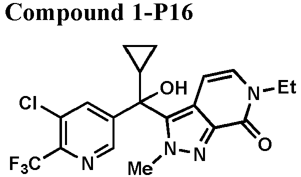

### 3-((5-chloro-6-(trifluoromethyl)pyridin-3-yl)(cyclopropyl)(hydroxy)methyl)-6-ethyl-2-methyl-2,6-dihydro-7H-pyrazolo[3,4-c]pyridin-7-one (1-P16)

Following **General Procedure F1** on 0.26 mmol scale with 3-(5-chloro-6-(trifluoromethyl)nicotinoyl)-6-ethyl-2-methyl-2,6-dihydro-7*H*-pyrazolo[3,4-*c*]pyridin-7-one (**1-S39**) and cyclopropyl magnesium bromide (3.0 equiv., 0.7 M in THF). Purification by CombiFlash chromatography (70% ethyl acetate in hexane) to afford 3-((5-chloro-6-(trifluoromethyl) pyridin-3-yl)(cyclopropyl)(hydroxy)methyl)-6-ethyl-2-methyl-2,6-dihydro-7*H*-pyrazolo[3,4-c]pyridin-7-one **(1-P16)** as an off-white solid (55 mg, 49%). **Physical State:** off-white solid.

The racemic compound **1-P16** was submitted for Chiral separation (Prep-HPLC-Chiral-NP) to afford enantiomer-1 **(1-33)** (15 mg) and enantiomer-2 **(1-32)** (15 mg).

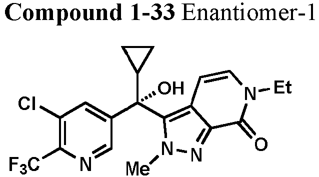

### (S)-3-((5-chloro-6-(trifluoromethyl)pyridin-3-yl)(cyclopropyl)(hydroxy)methyl)-6-ethyl-2-methyl-2, 6-dihydro-7H-pyrazolo[3,4-c]pyridin-7-one (1-33)

**¹H NMR (400 MHz, DMSO-*d₆*):** *δ* 8.52 (s, 1H), 7.98 (s, 1H), 7.13 (d, *J =* 7.6 Hz, 1H), 6.69 (d, *J* = 7.6 Hz, 1H), 6.38 (s, 1H), 3.96-3.94 (m, 2H), 3.70 (s, 3H), 1.90-1.80 (m, 1H) 1.22 (t, *J =* 6.8 Hz, 3H), 0.79-0.74 (m, 2H), 0.60-0.50 (m, 1H), 0.45-0.40 (m, 1H) ppm. **LC-MS (ESI, m/z):** calcd for [M+H]⁺ 427.1; found: 427.1. **Retention time:** 7.70 min.

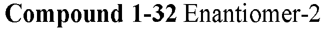

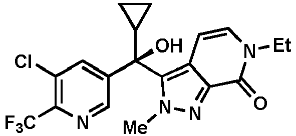

### (R)-3-((5-chloro-6-(trifluoromethyl)pyridin-3-yl)(cyclopropyl)(hydroxy)methyl)-6-ethyl-2-methyl-2, 6-dihydro-7H-pyrazolo[3,4-c]pyridin-7-one (1-32)

**¹H NMR (400 MHz, DMSO-*d₆*):** *δ* 8.52 (s, 1H), 7.98 (s, 1H), 7.13 (d, *J =* 7.6 Hz, 1H), 6.69 (d, *J* = 7.6 Hz, 1H), 6.38 (s, 1H), 3.96-3.94 (m, 2H), 3.70 (s, 3H), 1.90-1.80 (m, 1H) 1.22 (t, *J =* 6.8 Hz, 3H), 0.80-0.74 (m, 2H), 0.60-0.50 (m, 1H), 0.45-0.40 (m, 1H) ppm. **LC-MS (ESI,** m/z): calcd for [M+H]⁺ 427.1; found: 427.1. **Retention time:** 11.74 min.

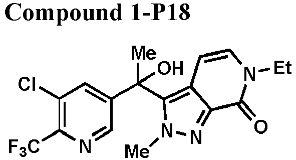

### 3-(1-(5-chloro-6-(trifluoromethyl)pyridin-3-yl)-1-hydroxyethyl)-6-ethyl-2-methyl-2,6-dihydro-7H-pyrazolo[3,4-c]pyridin-7-one (1-P18)

Following **General Procedure F1** on 0.26 mmol scale with 3-(5-chloro-6-(trifluoromethyl)nicotinoyl)-6-ethyl-2-methyl-2,6-dihydro-7*H*-pyrazolo[3,4-*c*]pyridin-7-one (**1-S39**) and MeMgBr (3.0 equiv., 3M in THF). Purification by CombiFlash chromatography (70% ethyl acetate in hexane) to afford 3-(1-(5-chloro-6-(trifluoromethyl)pyridin-3-yl)-1-hydroxyethyl)-6-ethyl-2-methyl-2,6-dihydro-7*H*-pyrazolo[3,4-*c*]pyridin-7-one **(1-P18)** as an off-white solid (65 mg, 62%). **Physical State:** off-white solid.

The racemic product **1-P18** was submitted for Chiral separation (Prep-HPLC-Chiral-NP) to afford enantiomer-1 **(1-37)** (12 mg) and enantiomer-2 **(1-36)** (12 mg).

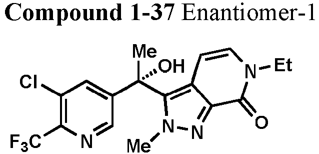

***(S)-3-(1-(5-chloro-6-(trifluoromethyl)pyridin-3-yl)-1-hydroxyethyl)-6-ethyl-2-methyl-2,6-dihydro-7H-pyrazolo[3,4-c]pyridin-7-one* (1-37) ¹H NMR (400 MHz, DMSO-*d₆*):** *δ* 8.57 (d, *J =* 1.3 Hz, 1H), 8.06 (s, 1H), 7.14 (d, *J =* 7.5 Hz, 1H), 6.94 (s, 1H), 6.50 (d, *J* = 7.5 Hz, 1H), 3.96-3.91 (m, 2H), 3.78 (s, 3H), 2.08 (s, 3H), 1.21 (t, *J =* 7.0 Hz, 3H) ppm. **LC-MS (ESI, m/z):** calcd for [M+H]⁺ 401.1; found: 401.1. **Retention time:** 5.24 min.

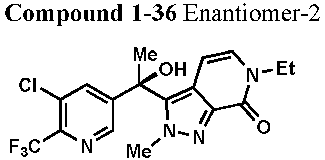

### (R)-3-(1-(5-chloro-6-(trifluoromethyl)pyridin-3-yl)-1-hydroxyethyl)-6-ethyl-2-methyl-2,6-dihydro-7H-pyrazolo[3,4-c]pyridin-7-one (1-36)

**¹H NMR (400 MHz, DMSO-*d₆*):** *δ* 8.57 (d, *J* = 1.3 Hz, 1H), 8.06 (s, 1H), 7.14 (d, *J* = 7.5 Hz, 1H), 6.94 (s, 1H), 6.50 (d, *J =* 7.5 Hz, 1H), 3.96-3.91 (m, 2H), 3.78 (s, 3H), 2.08 (s, 3H), 1.21 (t, *J* = 7.0 Hz, 3H) ppm. **LC-MS (ESI, m/z):** calcd for [M+H]⁺ 401.1; found: 401.1. **Retention time:** 6.17 min.

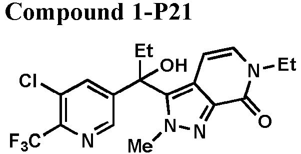

### 3-(1-(5-chloro-6-(trifluoromethyl)pyridin-3-yl)-1-hydroxypropyl)-6-ethyl-2-methyl-2,6-dihydro-7H-pyrazolo[3,4-c]pyridin-7-one (1-P21)

Following **General Procedure F1** on 0.52 mmol scale with 3-(5-chloro-6-(trifluoromethyl)nicotinoyl)-6-ethyl-2-methyl-2,6-dihydro-7*H*-pyrazolo[3,4-*c*]pyridin-7-one (**1-S39**) and EtMgBr (3M in THF). Purification by CombiFlash chromatography (70% ethyl acetate in hexane) to afford the 3-(1-(5-chloro-6-(trifluoromethyl)pyridin-3-yl)-1-hydroxypropyl)-6-ethyl-2-methyl-2,6-dihydro-7*H*-pyrazolo[3,4-*c*]pyridin-7-one **(1-P21)** as an off-white solid (70 mg, 32%). **Physical State:** off-white solid.

The racemic compound **1-P21** was submitted for Chiral separation (Prep-HPLC-Chiral-NP) to afford enantiomer-1 **(1-43)** (15 mg) and enantiomer-2 **(1-42)** (15 mg).

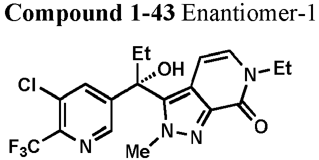

***(S)-3-(1-(5-chloro-6-(trifluoromethyl)pyridin-3-yl)-1-hydroxypropyl)-6-ethyl-2-methyl-2,6-dihydro-7H-pyrazolo[3,4-c]pyridin-7-one* (1-43) ¹H NMR (400 MHz, DMSO-*d₆*):** *δ* 8.55 (s, 1H), 8.00 (s, 1H), 7.14 (d, *J* = 7.5 Hz, 1H), 6.63 (s, 1H), 6.58 (d, *J =* 7.5 Hz, 1H), 3.94-3.92 (m, 2H), 3.79 (s, 3H), 2.55-2.44 (m, 2H), 1.22 (t, *J =* 6.8 Hz, 3H), 0.81 (t, *J =* 7.0 Hz, 3H) ppm. **LC-MS (ESI, m/z):** calcd for [M+H]⁺ 415.1; found: 415.1. **Retention time:** 4.99 min.

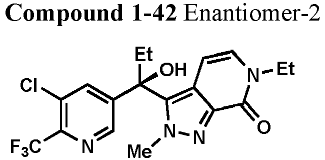

***(R)-3-(1-(5-chloro-6-(trifluoromethyl)pyridin-3-yl)-1-hydroxypropyl)-6-ethyl-2-methyl-2,6-dihydro-7H-pyrazolo[3,4-c]pyridin-7-one* (1-42) ¹H NMR (400 MHz, DMSO-*d₆*):** *δ* 8.55 (s, 1H), 8.00 (s, 1H), 7.14 (d, *J* = 7.5 Hz, 1H), 6.63 (s, 1H), 6.59 (d, *J =* 7.5 Hz, 1H), 3.95-3.92 (m, 2H), 3.79 (s, 3H), 2.55-2.44 (m, 2H), 1.22 (t, *J =* 6.8 Hz, 3H), 0.81 (t, *J =* 7.0 Hz, 3H) ppm. **LC-MS (ESI, m/z):** calcd for [M+H]⁺ 415.1; found: 415.1. **Retention time:** 7.25 min.

### Synthesis of compounds 1-34, 1-35, 1-38, 1-39, 1-40, 1-41

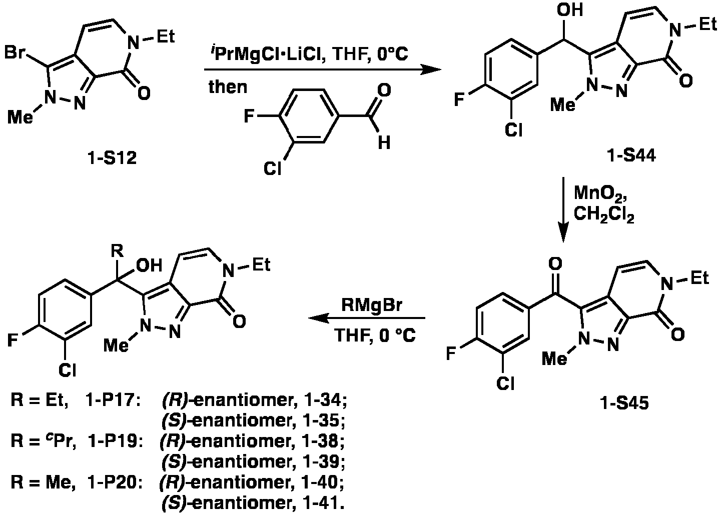

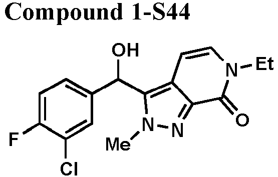

### 3-((3-chloro-4-fluorophenyl)(hydroxy)methyl)-6-ethyl-2-methyl-2,6-dihydro-7H-pyrazolo[3,4-c]pyridin-7-one (1-S44)

Following **General Procedure D1** on 0.75 mmol scale with 3-bromo-6-ethyl-2-methyl-2,6-dihydro-7H-pyrazolo[3,4-c]pyridin-7-one **(1-S12)** and 3-chloro-4-fluorobenzaldehyde. Purification by CombiFlash chromatography (70% ethyl acetate in hexane) to afford 3-((3-chloro-4-fluorophenyl)(hydroxy)methyl)-6-ethyl-2-methyl-2,6-dihydro-7*H*-pyrazolo[3,4-*c*]pyridin-7-one **(1-S44)** as an off-white solid (0.9 g, 69%). **Physical State:** off-white solid.

**¹H NMR (400 MHz, DMSO-*d₆*):** *δ* 7.60 (d, *J =* 6.92 Hz, 1H), 7.46-7.37 (m, 2H), 6.99 (d, *J =* 7.2 Hz, 1H), 6.53 (d, *J =* 4.8 Hz, 1H), 6.22 (d, *J =* 4.3 Hz, 1H), 5.87 (d, *J =* 7.32 Hz, 1H), 4.01 (s, 3H), 3.87 (q, *J* = 7.04 Hz, 2H), 1.17 (t, *J =* 7.04 Hz, 3H) ppm. **LC-MS (ESI, m/z):** calcd for [M+H]⁺ 336.1; found: 336.1.

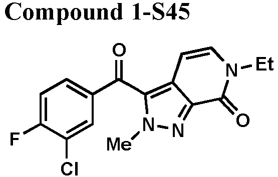

### 3-(3-chloro-4-fluorobenzoyl)-6-ethyl-2-methyl-2,6-dihydro-7H-pyrazolo[3,4-c]pyridine-7-one (1-S45)

Following **General Procedure E1** on 10.75 mmol scale with 3-((3-chloro-4-fluorophenyl)(hydroxy)methyl)-6-ethyl-2-methyl-2,6-dihydro-7*H*-pyrazolo[3,4-*c*]pyridine-7-one **(1-S44).** The reaction mixture was filtered through Celite bed and the filtrate was concentrated to afford 3-(3-chloro-4-fluorobenzoyl)-6-ethyl-2-methyl-2,6-dihydro-7H-pyrazolo[3,4-c]pyridine-7-one **(1-S45)** as an off-white solid (2 g, 56%). **Physical State:** off-white solid. **LC-MS (ESI, m/z):** calcd for [M+H]⁺ 334.1; found: 334.1.

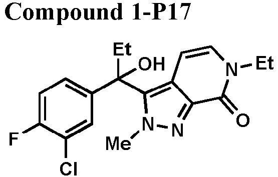

### 3-(1-(3-chloro-4-fluorophenyl)-1-hydroxypropyl)-6-ethyl-2-methyl-2,6-dihydro-7H-pyrazolo[3,4-c]pyridin-7-one (1-P17)

Following **General Procedure F1** on 0.45 mmol scale with 3-(3-chloro-4-fluorobenzoyl)-6-ethyl-2-methyl-2,6-dihydro-7*H*-pyrazolo[3,4-*c*]pyridine-7-one **(1-S45)** and EtMgBr (3 M in THF). Purification by CombiFlash chromatography (60% ethyl acetate in hexane) to afford 3-(1-(3-chloro-4-fluorophenyl)-1-hydroxypropyl)-6-ethyl-2-methyl-2,6-dihydro-7*H-*pyrazolo[3,4-c]pyridin-7-one **(1-P17)** as an off-white solid (60 mg, 37%). **Physical State:** off-white solid.

The racemic compound **1-P17** was submitted for chiral separation (Prep-HPLC-NP) to afford Enantiomer-1 **(1-35)** (20 mg) and Enantiomer-2 **(1-34)** (20 mg).

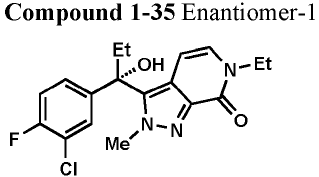

### (S)-3-(1-(3-chloro-4-fluorophenyl)-1-hydroxypropyl)-6-ethyl-2-methyl-2,6-dihydro-7H-pyrazolo[3,4-c]pyridin-7-one (1-35)

**¹H NMR (400 MHz, DMSO-*d₆*):** *δ* 7.43 (d, *J =* 7.0 Hz, 1H), 7.36 (t, *J =* 8.6 Hz, 1H), 7.14-7.10 (m, 2H), 6.56 (d, *J* = 7.56 Hz, 1H), 6.28 (s, 1H), 3.96-3.89 (m, 2H), 3.72 (s, 3H), 2.50-2.44 (m, 1H), 2.33-2.28 (m, 1H), 1.22 (t, *J =* 7.0 Hz, 3H), 0.79 (t, *J =* 6.8 Hz, 3H) ppm. N **LC-MS (ESI, m/z):** calcd for [M+H]⁺ 364.1; found: 364.1. **Retention time:** 5.35 min.

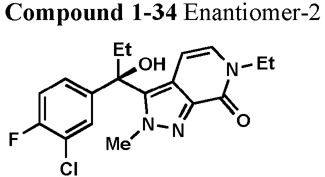

### (R)-3-(1-(3-chloro-4-fluorophenyl)-1-hydroxypropyl)-6-ethyl-2-methyl-2,6-dihydro-7H-pyrazolo[3,4-c]pyridin-7-one (1-34)

**¹H NMR (400 MHz, DMSO-*d₆*):** *δ* 7.43 (d, *J* = 7.0 Hz, 1H), 7.36 (t, *J* = 8.6 Hz, 1H), 7.14-7.10 (m, 2H), 6.56 (d, *J =* 7.56 Hz, 1H), 6.28 (s, 1H), 3.96-3.89 (m, 2H), 3.72 (s, 3H), 2.50-2.44 (m, 1H), 2.33-2.28 (m, 1H), 1.22 (t, *J =* 7.0 Hz, 3H), 0.79 (t, *J =* 6.8 Hz, 3H) ppm. **LC-MS (ESI, m/z):** calcd for [M+H]⁺ 364.1; found: 364.1. **Retention time:** 6.74 min.

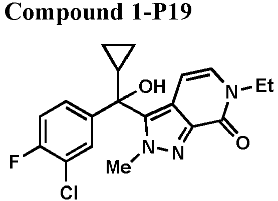

### 3-((3-chloro-4-fluorophenyl)(cyclopropyl)(hydroxy)methyl)-6-ethyl-2-methyl-2,6-dihydro-7H-pyrazolo[3,4-c]pyridin-7-one (1-P19)

Following **General Procedure F1** on 0.3 mmol scale with 3-(3-chloro-4-fluorobenzoyl)-6-ethyl-2-methyl-2,6-dihydro-7*H*-pyrazolo[3,4-c]pyridine-7-one **(1-S45)** and cyclopropyl magnesium bromide (0.7 M in THF). Purification by CombiFlash chromatography (60% ethyl acetate in hexane) to afford 3-((3-chloro-4-fluorophenyl)(cyclopropyl)(hydroxy)methyl)-6-ethyl-2-methyl-2,6-dihydro-7*H*-pyrazolo[3,4-*c*]pyridin-7-one **(1-P19)** as an off-white solid (80 mg, 71%). **Physical State:** off-white solid.

The racemic compound **1-P19** was submitted for enantiomer separation (Prep-HPLC-Chiral-NP) to afford Enantiomer-1 **(1-39)** and Enantiomer-2 **(1-38).**

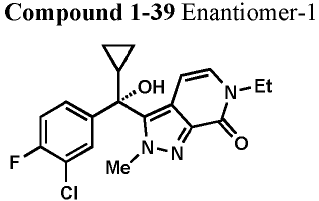

### (S)-3-((3-chloro-4-fluorophenyl)(cyclopropyl)(hydroxy)methyl)-6-ethyl-2-methyl-2,6-dihydro-7H-pyrazolo[3,4-c]pyridin-7-one (1-39)

**¹H NMR (400 MHz, DMSO-*d₆*):** *δ* 7.41 (dd, *J =* 7.12, 2.04 Hz, 1H), 7.36 (t, *J =* 8.92 Hz, 1H), 7.11-7.08 (m, 2H), 6.70 (d, *J =* 7.56 Hz, 1H), 6.08 (s, 1H), 3.95-3.93 (m, 2H), 3.65 (s, 3H), 1.80-1.70 (m, 1H), 1.22 (t, *J =* 6.96 Hz, 3H), 0.70-0.60 (m, 2H), 0.50-0.40 (m, 2H) ppm. **LC-MS (ESI, m/z):** calcd for [M+H]⁺ 376.1; found: 376.1. **Retention time:** 5.31 min.

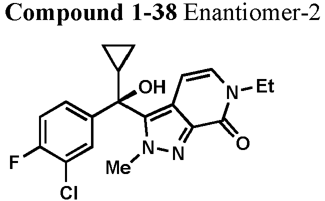

### (R)-3-((3-chloro-4-fluorophenyl)(cyclopropyl)(hydroxy)methyl)-6-ethyl-2-methyl-2,6-dihydro-7H-pyrazolo[3,4-c]pyridin-7-one (1-38)

**¹H NMR (400 MHz, DMSO-*d₆*):** *δ* 7.41 (dd, *J =* 7.12, 2.04 Hz, 1H), 7.36 (t, *J =* 8.92 Hz, 1H), 7.11-7.08 (m, 2H), 6.70 (d, *J =* 7.56 Hz, 1H), 6.08 (s, 1H), 3.95-3.93 (m, 2H), 3.65 (s, 3H), 1.80-1.70 (m, 1H), 1.22 (t, *J =* 6.96 Hz, 3H), 0.70-0.60 (m, 2H), 0.50-0.40 (m, 2H) ppm. **LC-MS (ESI, m/z):** calcd for [M+H]⁺ 376.1; found: 376.1. **Retention time:** 7.55 min.

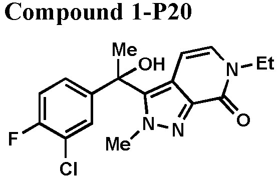

### 3-(1-(3-chloro-4-fluorophenyl)-1-hydroxyethyl)-6-ethyl-2-methyl-2,6-dihydro-7H-pyrazolo[3,4-c]pyridin-7-one (1-P20)

Following **General Procedure F1** on 0.36 mmol scale with 3-(3-chloro-4-fluorobenzoyl)-6-ethyl-2-methyl-2,6-dihydro-7*H*-pyrazolo[3,4-*c*]pyridine-7-one **(1-S45)** and MeMgBr (3M in THF). Purification by CombiFlash chromatography (70% ethyl acetate in hexane) to afford 3-(1-(3-chloro-4-fluorophenyl)-1-hydroxyethyl)-6-ethyl-2-methyl-2,6-dihydro-7*H-*pyrazolo[3,4-*c*]pyridin-7-one **(1-P20)** as an off-white solid (50 mg, 40%). **Physical State:** off-white solid.

The racemic compound **1-P20** was submitted for enantiomer separation (Prep-HPLC-Chiral-NP) to afford Enantiomer-1 **(1-41)** and Enantiomer-2 **(1-40).**

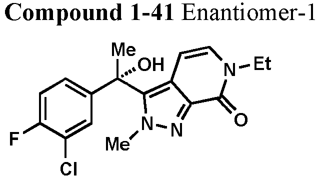

### (S)-3-(1-(3-chloro-4-fluorophenyl)-1-hydroxyethyl)-6-ethyl-2-methyl-2,6-dihydro-7H-pyrazolo[3,4-c]pyridin-7-one (1-41)

**¹H NMR (400 MHz, DMSO-*d₆*):** *δ* 7.49 (d, *J =* 6.8 Hz, 1H), 7.37 (t, *J =* 8.4 Hz, 1H), 7.20-7.15 (m, 1H), 7.09 (d, *J =* 7.6 Hz, 1H), 6.59 (s, 1H), 6.44 (d, *J =* 7.6 Hz, 1H), 3.94-3.91 (m, 2H), 3.73 (s, 3H), 1.99 (s, 3H), 1.21 (t, *J =* 7.6 Hz, 3H) ppm. **LC-MS (ESI, m/z):** calcd for [M+H]⁺ 350.1; found: 350.1. **Retention time:** 2.18 min.

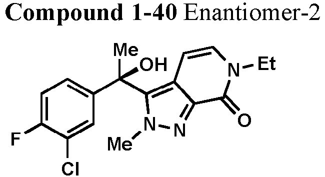

### (R)-3-(1-(3-chloro-4-fluorophenyl)-1-hydroxyethyl)-6-ethyl-2-methyl-2,6-dihydro-7H-pyrazolo[3,4-c]pyridin-7-one (1-40)

**¹H NMR (400 MHz, DMSO-*d₆*):** *δ* 7.49 (d, *J =* 6.8 Hz, 1H), 7.37 (t, *J =* 8.4 Hz, 1H), 7.20-7.15 (m, 1H), 7.09 (d, *J =* 7.6 Hz, 1H), 6.59 (s, 1H), 6.44 (d, *J =* 7.6 Hz, 1H), 3.94-3.91 (m, 2H), 3.73 (s, 3H), 1.99 (s, 3H), 1.21 (t, *J =* 7.6 Hz, 3H) ppm. **LC-MS (ESI, m/z):** calcd for [M+H]⁺ 350.1; found: 350.1. **Retention time:** 2.69 min.

### Synthesis of compounds 1-44, 1-45, 1-48, 1-49, 1-50, 1-51

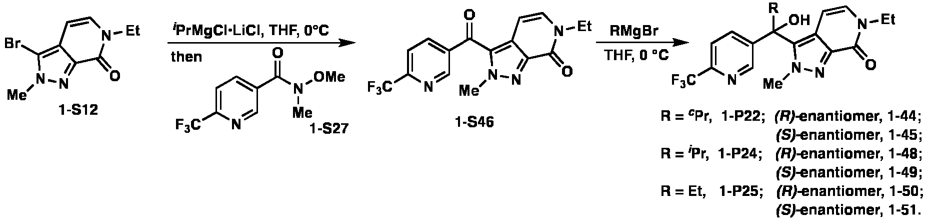

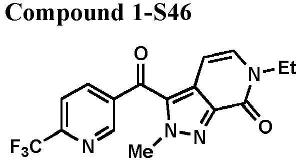

### 6-ethyl-2-methyl-3-[6-(trifluoromethyl)pyridine-3-carbonyl]pyrazolo[3,4-c]pyridin-7-one (1-S46)

Following **General Procedure C1 on** 7.81 mmol scale with 3-bromo-6-ethyl-2-methyl-pyrazolo[3,4-c]pyridin-7-one **(1-S12)** and *N*-methoxy-*N*-methyl-6-(trifluoromethyl)pyridine-3-carboxamide **(1-S27).** Purification by column chromatography (SiO₂ 100-200, 50% ethyl acetate in hexane) to afford 6-ethyl-2-methyl-3-[6-(trifluoromethyl)pyridine-3-carbonyl] pyrazolo[3,4-c]pyridin-7-one **(1-S46)** as a yellow solid (2.2 g, 80%). **Physical State:** yellow solid. **¹H NMR (400 MHz, CDCl₃)**: *δ* 9.08 (s, 1H), 8.29 (d, *J =* 8.52 Hz, 1H), 7.89 (d, *J =* 8.08 Hz, 1H), 6.88 (d, *J* = 7.4 Hz, 1H), 5.63 (d, *J =* 7.4 Hz, 1H), 4.45 (s, 3H), 4.04 (q, *J =* 7.12 Hz, 2H), 1.34 (t, *J* = 7.12 Hz, 3H) ppm. **LC-MS (ESI, m/z):** calcd for [M+H]⁺ 351.2; found: 351.18.

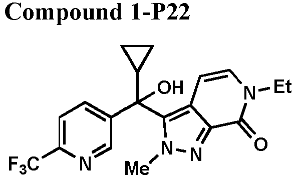

### 3-(cyclopropyl(hydroxy)(6-(trifluoromethyl)pyridin-3-yl)nwthyl)-6-ethyl-2-methyl-2,6-dihydro-7H-pyrazolo[3,4-c]pyridin-7-one (1-P22)

Following **General Procedure F1** on 0.86 mmol scale with 6-ethyl-2-methyl-3-(6-(trifluoromethyl)nicotinoyl)-2,6-dihydro-7*H*-pyrazolo[3,4-*c*]pyridin-7-one **(1-S46)** and cyclopropyl magnesium bromide (0.7 M in THF). Purification by CombiFlash chromatography (60% ethyl acetate in hexane) to afford 3-(cyclopropyl(hydroxy)(6-(trifluoromethyl)pyridin-3-yl)methyl)-6-ethyl-2-methyl-2,6-dihydro-7*H*-pyrazolo[3,4-*c*]pyridin-7-one **(1-P22)** as an off-white solid (320 mg, 65%). **Physical State:** off-white solid.

The racemic product **1-P22** was submitted for chiral separation (Prep-HPLC-SFC) to afford Enantiomer-1 **(1-45)** (10 mg) and Enantiomer-2 **(1-44)** (10 mg).

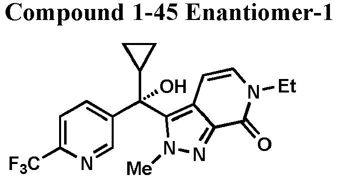

### (S)-3-(cyclopropyl(hydroxy)(6-(trifluoromethyl)pyridin-3-yl)methyl)-6-ethyl-2-methyl-2,6-dihydro-7H-pyrazolo[3,4-c]pyridin-7-one (1-45)

**¹H NMR (400 MHz, DMSO-*d₆*):** *δ* 8.67 (s, 1H), 7.87 (d, *J =* 8.2 Hz, 1H), 7.80 (d, *J =* 7.8 Hz, 1H), 7.12 (d, *J* = 7.6 Hz, 1H), 6.71 (d, *J =* 7.5 Hz, 1H), 6.29 (s, 1H), 3.96-3.90 (m, 2H), 3.66 (s, 3H), 1.84-1.79 (m, 1H), 1.23 (t, *J =* 7.0 Hz, 3H), 0.76-0.72 (m, 2H), 0.56-0.53 (m, 1H), 0.46-0.44 (m, 1H) ppm. **LC-MS (ESI, m/z):** calcd for [M+H]⁺ 393.1; found: 393.1. **Retention time:** 3.81 min.

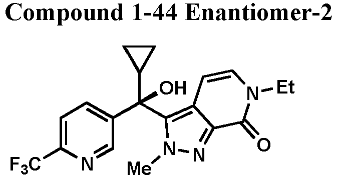

### (R)-3-(cyclopropyl(hydroxy)(6-(trifluoromethyl)pyridin-3-yl)methyl)-6-ethyl-2-methyl-2,6-dihydro-7H-pyrazolo[3,4-c]pyridin-7-one (1-44)

**¹H NMR (400 MHz, DMSO-*d₆*):** *δ* 8.67 (s, 1H), 7.87 (d, *J =* 8.2 Hz, 1H), 7.80 (d, *J =* 7.8 Hz, 1H), 7.12 (d, *J* = 7.6 Hz, 1H), 6.71 (d, *J =* 7.5 Hz, 1H), 6.29 (s, 1H), 3.96-3.90 (m, 2H), 3.66 (s, 3H), 1.84-1.79 (m, 1H), 1.23 (t, *J =* 7.0 Hz, 3H), 0.76-0.72 (m, 2H), 0.56-0.53 (m, 1H), 0.46-0.44 (m, 1H) ppm. **LC-MS (ESI, m/z):** calcd for [M+H]⁺ 393.1; found: 393.1. **Retention time:** 5.53 min.

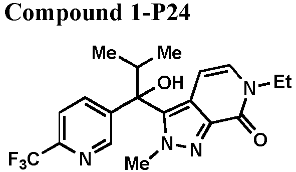

### 6-ethyl-3-(1-hydroxy-2-methyl-1-(6-(trifluoromethyl)pyridin-3-yl)propyl)-2-methyl-2,6-dihydro-7H-pyrazolo[3,4-c]pyridin-7-one (1-P24)

Following **General Procedure F1** on 1.14 mmol scale with 6-ethyl-2-methyl-3-(6-(trifluoromethyl)nicotinoyl)-2,6-dihydro-7*H*-pyrazolo[3,4-*c*]pyridin-7-one **(1-S46)** and *ⁱ*PrMgCl.LiCl (1.3M in THF). Purification by CombiFlash chromatography (70% ethyl acetate in hexane) to afford 6-ethyl-3-(1-hydroxy-2-methyl-1-(6-(trifluoromethyl)pyridin-3-yl)propyl)-2-methyl-2,6-dihydro-7H-pyrazolo[3,4-c]pyridin-7-one **(1-P24)** as an off-white solid (200 mg, 44%). **Physical State:** off-white solid.

The racemic compound **1-P24** (100 mg) was purified by prep-HPLC-SFC to afford enantiomer-1 **(1-49)** (35 mg) and enantiomer-2 **(1-48)** (35 mg).

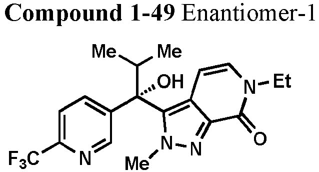

### (S)-6-ethyl-3-(1-hydroxy-2-methyl-1-(6-(trifluoromethyl)pyridin-3-yl)propyl)-2-methyl-2,6-dihydro-7H-pyrazolo[3,4-c]pyridin-7-one (1-49)

**¹H NMR (400 MHz, DMSO-*d₆*):** *δ* 8.68 (s, 1H), 7.90-7.85 (m, 2H), 7.18 (d, *J =* 7.2 Hz, 1H), 6.63 (d, *J* = 7.6 Hz, 1H), 6.35 (s, 1H), 4.06-3.86 (m, 2H), 3.82 (s, 3H), 3.01-2.94 (m, 1H), 1.23 (t, *J* = 7.2 Hz, 3H), 1.08 (d, *J =* 6.8 Hz, 3H), 0.67 (d, *J =* 6.4 Hz, 3H) ppm. **LC-MS (ESI, m/z):** calcd for [M+H]⁺ 395.1; found: 395.21. **Retention time:** 2.78 min.

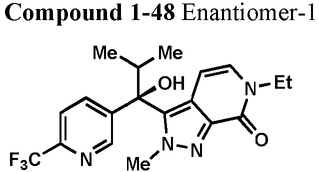

### (R)-6-ethyl-3-(1-hydroxy-2-methyl-1-(6-(trifluoromethyl)pyridin-3-yl)propyl)-2-methyl-2,6-dihydro-7H-pyrazolo[3,4-c]pyridin-7-one (1-48)

**¹H NMR (400 MHz, DMSO-*d₆*):** *δ* 8.68 (s, 1H), 7.90-7.85 (m, 2H), 7.18 (d, *J =* 7.2 Hz, 1H), 6.63 (d, *J* = 7.6 Hz, 1H), 6.35 (s, 1H), 4.06-3.86 (m, 2H), 3.82 (s, 3H), 3.01-2.94 (m, 1H), 1.23 (t, *J* = 7.2 Hz, 3H), 1.08 (d, *J =* 6.8 Hz, 3H), 0.67 (d, *J =* 6.4 Hz, 3H) ppm. **LC-MS (ESI, m/z):** calcd for [M+H]⁺ 395.1; found: 395.21. **Retention time:** 4.13 min.

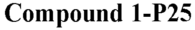

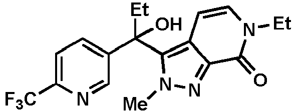

### 6-ethyl-3-{1-hydroxy-1-[6-(trifluoromethyl)pyridin-3-yl]propyl}-2-methyl-2H,6H,7H-pyrazolo[3,4-c]pyridin-7-one (1-P25)

Following **General Procedure F1** on 0.85 mmol scale with 6-ethyl-2-methyl-3-(6-(trifluoromethyl)nicotinoyl)-2,6-dihydro-7*H*-pyrazolo[3,4-*c*]pyridin-7-one **(1-S46)** and EtMgBr (3 M in THF). Purification by CombiFlash chromatography (70% ethyl acetate in hexane) to afford 6-ethyl-3-{1-hydroxy-1-[6-(trifluoromethyl)pyridin-3-yl]propyl }-2-methyl-2H,6H,7H-pyrazolo[3,4-c]pyridin-7-one **(1-P25)** as an off-white solid (112 mg, 34%). **Physical State:** off-white solid.

The racemic compound **1-P25** was purified by prep-HPLC Chiral (NP) to afford enantiomer-1 (**1-51**) (25 mg) and enantiomer-2 **(1-50)** (20 mg).

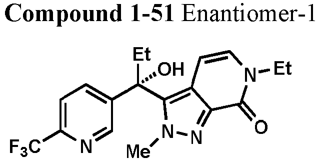

### (S)-6-ethyl-3-(1-hydroxy-1-(6-(trifluoromethyl)pyridin-3-yl)propyl)-2-methyl-2,6-dihydro-7H-pyrazolo[3,4-c]pyridin-7-one (1-51)

**¹H NMR (400 MHz, DMSO-*d₆*):** *δ* 8.69 (s, 1H), 7.86 (s, 2H), 7.14 (d, *J =* 7.60 Hz, 1H), 6.59 (d, *J* = 7.52 Hz, 1H), 6.52 (s, 1H), 3.96-3.88 (m, 2H), 3.74 (s, 3H), 2.58-2.52 (m, 1H), 2.42-2.37 (m, 1H), 1.22 (t, *J =* 7.10 Hz, 3H), 0.80 (t, *J =* 7.20 Hz, 3H)ppm. **LC-MS (ESI, m/z):** calcd for [M+H]⁺ 381.1; found: 381.2. **Retention time:** 4.40 min.

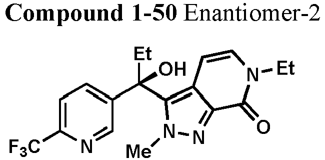

***(R)-6-ethyl-3-(1-hydroxy-1-(6-(trifluoromethyl)pyridin-3-yl)propyl)-2-methyl-2,6-dihydro-7H-pyrazolo[3,4-c]pyridin-7-one* (1-50) ¹H NMR (400 MHz, DMSO-*d₆*):** *δ* 8.68 (s, 1H), 7.86 (s, 2H), 7.12 (d, *J =* 7.60 Hz, 1H), 6.59 (d, *J =* 7.52 Hz, 1H), 6.52 (s, 1H), 3.98-3.88 (m, 2H), 3.74 (s, 3H), 2.58-2.52 (m, 1H), 2.49-2.32 (m, 1H), 1.22 (t, *J =* 7.10 Hz, 3H), 0.80 (t, *J =* 7.20 Hz, 3H)ppm. **LC-MS (ESI, m/z):** calcd for [M+H]⁺ 381.1; found: 381.2. **Retention time:** 7.56 min.

### Synthesis of compounds 1-46, 1-47

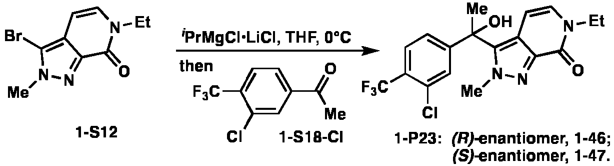

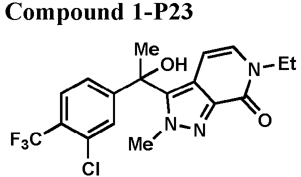

### 3-(1-(3-chloro-4-(trifluoromethyl)phenyl)-1-hydroxyethyl)-6-ethyl-2-methyl-2,6-dihydro-7H-pyrazolo[3,4-c]pyridin-7-one (1-P23)

Following **General Procedure D1** on 0.98 mmol scale with 3-bromo-6-ethyl-2-methyl-pyrazolo[3,4-c]pyridin-7-one **(1-S12)** and 1-(3-chloro-4-(trifluoromethyl)phenyl)-ethan-1-one **(1-S18-Cl).** Purification by preparative-HPLC (RP) to afford 3-(1-(3-chloro-4-(trifluoromethyl)phenyl)-1-hydroxyethyl)-6-ethyl-2-methyl-2,6-dihydro-7*H*-pyrazolo[3,4-*c*]pyridin-7-one **(1-P23)** as an off-white solid (80 mg). **Physical State:** off-white solid. The racemic compound **1-P23** was purified by prep-HPLC Chiral (SFC) to afford enantiomer-1 (**1-47**) (30 mg) and enantiomer-2 **(1-46)** (30 mg).

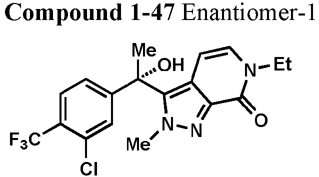

### (S)-3-(1-(3-chloro-4-(trifluoromethyl)phenyl)-1-hydroxyethyl)-6-ethyl-2-methyl-2,6-dihydro-7H-pyrazolo[3,4-c]pyridin-7-one (1-47)

**¹H NMR (400 MHz, DMSO-*d₆*):** *δ* 7.83 (d, *J =* 8.3 Hz, 1H), 7.64 (s, 1H), 7.31 (d, *J =* 8.0 Hz, 1H), 7.13 (d, *J= 7.6* Hz, 1H), 6.75 (*br.* s, 1H), 6.53 (d, *J =* 7.2 Hz, 1H), 3.97-3.91 (m, 2H), 3.71 (s, 3H), 2.03 (s, 3H), 1.22 (t, *J=* 6.8 Hz, 3H) ppm. **LC-MS (ESI, m/z):** calcd for [M+H]⁺ 400.1; found: 400.17. **Retention time:** 4.52 min.

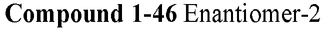

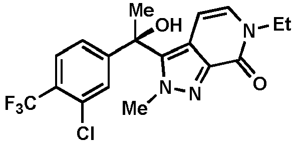

### (R)-3-(1-(3-chloro-4-(trifluoromethyl)phenyl)-1-hydroxyethyl)-6-ethyl-2-methyl-2,6-dihydro-7H-pyrazolo[3,4-c]pyridin-7-one (1-46)

**¹H NMR (400 MHz, DMSO-*d₆*):** *δ* 7.83 (d, *J* = 8.3 Hz, 1H), 7.64 (s, 1H), 7.31 (d, *J =* 8.0 Hz, 1H), 7.13 (d, *J =* 7.6 Hz, 1H), 6.77 (*br.* s, 1H), 6.53 (d, *J =* 7.2 Hz, 1H), 3.97-3.88 (m, 2H), 3.71 (s, 3H), 2.03 (s, 3H), 1.22 (t, *J =* 6.8 Hz, 3H) ppm. **LC-MS (ESI, m/z):** calcd for [M+H]⁺ 400.1; found: 400.21. **Retention time:** 4.93 min.

### Synthesis of compounds 2-1 to 2-20

### Synthesis of 2-1, 2-2

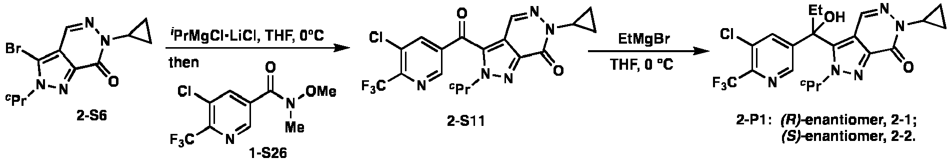

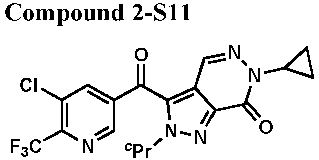

### 3-(5-chloro-6-(trifluoromethyl)nicotinoyl)-2,6-dicyclopropyl-2,6-dihydro-7H-pyrazolo[3,4-d]pyridazin-7-one (2-S11)

Following **General Procedure C2** on 1.80 mmol scale with 3-bromo-2,6-dicyclopropyl-2,6-dihydro-7*H*-pyrazolo[3,4-*d*]pyridazin-7-one **(2-S6)** and 5-chloro-*N*-methoxy-*N*-methyl-6-(trifluoromethyl)nicotinamide **(1-S26).** Purification by CombiFlash chromatography (80% ethyl acetate in hexane) to afford 3-(5-chloro-6-(trifluoromethyl)nicotinoyl)-2,6-dicyclopropyl-2,6-dihydro-7*H*-pyrazolo[3,4-*d*]pyridazin-7-one **(2-S11)** as an off-white solid (490 mg, 64%). **Physical state:** off-white solid. **¹H NMR (400 MHz, CDCl₃)**: *δ* 8.92 (s, 1H), 8.32 (s, 1H), 7.42 (s, 1H), 4.59-4.53 (m, 1H), 4.12-4.06 (m, 1H), 1.32-1.23 (m, 4H), 1.06-0.85 (m, 4H) ppm. **LC-MS (ESI, m/z):** calcd for [M+H]⁺ 424.1; found: 424.16.

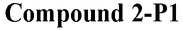

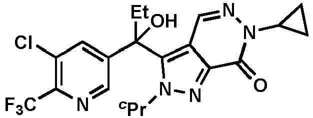

### 3-(1-(5-chloro-6-(trifluoromethyl)pyridin-3-yl)-1-hydroxypropyl)-2,6-dicyclopropyl-2,6-dihydro-7H-pyrazolo[3,4-d]pyridazin-7-one (2-P1)

Following **General Procedure F2** on 0.94 mmol scale with 3-(5-chloro-6-(trifluoromethyl)nicotinoyl)-2,6-dicyclopropyl-2,6-dihydro-7H-pyrazolo[3,4-*d*]pyridazin-7-one **(2-S11)** and EtMgBr (3M in diethyl ether). Purification by CombiFlash chromatography (70% ethyl acetate in hexane) to afford 3-(1-(5-chloro-6-(trifluoromethyl)pyridin-3-yl)-1-hydroxypropyl)-2,6-dicyclopropyl-2,6-dihydro-7*H*-pyrazolo[3,4-*d*]pyridazin-7-one **(2-P1)** as an off-white solid (100 mg, 23%). **Physical state:** off-white solid.

The racemic compound was submitted to preparative-HPLC-SFC to afford Enantiomer-1 **(2-2)** (40 mg) and Enantiomer-2 **(2-1)** (40 mg).

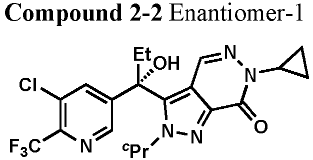

### (S)-3-(1-(5-chloro-6-(trifluoromethyl)pyridin-3-yl)-1-hydroxypropyl)-2,6-dicyclopropyl-2,6-dihydro-7H-pyrazolo[3,4-d]pyridazin-7-one (2-2)

**¹H NMR (400 MHz, DMSO-*d₆*):** *δ* 8.64 (d, *J* = 1.7 Hz, 1H), 8.46 (s, 1H), 8.13 (d, *J* = 1.4 Hz, 1H), 6.89 (s, 1H), 3.98-3.93 (m, 2H), 2.70-2.66 (m, 1H), 2.60-2.54 (m, 1H), 1.50-1.40 (m, 1H), 1.01-0.89 (m, 5H), 0.80 (t, *J =* 7.1 Hz, 3H), 0.51-0.48 (m, 1H), 0.45-0.35 (m, 1H) ppm. **LC-MS (ESI, m/z):** calcd for [M+H]⁺ 454.1; found: 454.35. **Retention time:** 1.09 min. [α]²⁵_{D} = - 58.798° (c = 0.2500 g/100ml, MeOH)

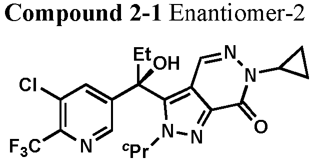

### (R)-3-(1-(5-chloro-6-(trifluoromethyl)pyridin-3-yl)-1-hydroxypropyl)-2,6-dicyclopropyl-2,6-dihydro-7H-pyrazolo[3,4-d]pyridazin-7-one (2-1)

**¹H NMR (400 MHz, DMSO-*d₆*):** *δ* 8.64 (d, *J =* 1.7 Hz, 1H), 8.46 (s, 1H), 8.13 (d, *J =* 1.4 Hz, 1H), 6.89 (s, 1H), 3.99-3.93 (m, 2H), 2.71-2.66 (m, 1H), 2.60-2.54 (m, 1H), 1.50-1.40 (m, 1H), 1.02-0.87 (m, 5H), 0.80 (t, *J =* 7.1 Hz, 3H), 0.51-0.48 (m, 1H), 0.45-0.35 (m, 1H) ppm. LC-**MS (ESI, m/z):** calcd for [M+H]⁺ 454.1; found: 454.35. **Retention time: 1.46** min.

[α]²⁵_{D} = +65.118° (c = 0.2565 g/100ml, MeOH)

### Synthesis of compounds 2-3, 2-4

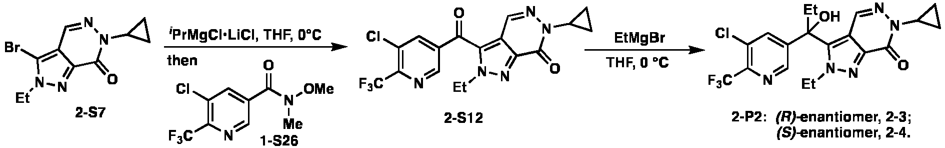

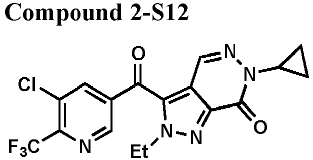

### 3-(5-chloro-6-(trifluoromethyl)nicotinoyl)-6-cyclopropyl-2-ethyl-2,6-dihydro-7H-pyrazolo[3,4-d]pyridazin-7-one (2-S12)

Following **General Procedure C2 on 1.58** mmol scale with 3-bromo-6-cyclopropyl-2-ethyl-2,6-dihydro-7H-pyrazolo[3,4-d]pyridazin-7-one **(2-S7)** and 5-chloro-*N*-methoxy-*N*-methyl-6-(trifluoromethyl)nicotinamide **(1-S26).** Purification by Combiflash chromatography (40% ethyl acetate in hexane) to afford 3-(5-chloro-6-(trifluoromethyl)nicotinoyl)-6-cyclopropyl-2-ethyl-2,6-dihydro-7H-pyrazolo[3,4-*d*]pyridazin-7-one **(2-S12)** as an off-white solid (270 mg, 41%). **Physical state:** off-white solid. **¹H NMR (400 MHz, DMSO-*d₆*):** *δ* 9.02 (s, 1H), 8.61 (s, 1H), 7.69 (s, 1H), 4.69 (q, *J =* 7.1 Hz, 2H), 4.04-3.95 (m, 1H), 1.54 (t, *J =* 7.1 Hz, 3H), 0.95-0.90 (m, 4H) ppm. **LC-MS (ESI, m/z):** calcd for [M+H]⁺ 412.1; found: 412.0.

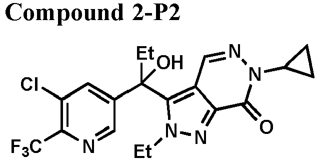

### 3-(1-(5-chloro-6-(trifluoromethyl)pyridin-3-yl)-1-hydroxypropyl)-6-cyclopropyl-2-ethyl-2,6-dihydro-7H-pyrazolo[3,4-d]pyridazin-7-one (2-P2)

Following **General Procedure F2** on 0.66 mmol scale with 3-(5-chloro-6-(trifluoromethyl)nicotinoyl)-6-cyclopropyl-2-ethyl-2,6-dihydro-7*H*-pyrazolo[3,4-d]pyridazin-7-one **(2-S12)** and EtMgBr (3M in diethyl ether) (2.0 equiv.). Purification by Combiflash chromatography (60% ethyl acetate in hexane) to afford 3-(1-(5-chloro-6-(trifluoromethyl)pyridin-3-yl)-1-hydroxypropyl)-6-cyclopropyl-2-ethyl-2,6-dihydro-7*H-*pyrazolo[3,4-*d*]pyridazin-7-one **(2-P2)** as an off-white solid (75 mg, 26%). **Physical state:** off-white solid.

The racemic compound **2-P2** was purified from Prep-HPLC-SFC to afford Enantiomer-1 **(2-4)** (20 mg) and Enantiomer-2 **(2-3)** (20 mg).

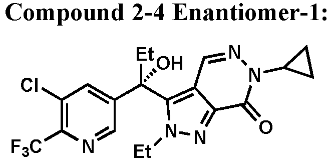

### (S)-3-(1-(5-chloro-6-(trifluoromethyl)pyridin-3-yl)-1-hydroxypropyl)-6-cyclopropyl-2-ethyl-2,6-dihydro-7H-pyrazolo[3,4-d]pyridazin-7-one (2-4)

**¹H NMR (400 MHz, DMSO-*d₆*): *δ*** 8.63 (s, 1H), 8.45 (s, 1H), 8.11 (s, 1H), 6.88 (s, 1H), 4.23-4.11 (m, 2H), 4.00-3.95 (m, 1H), 2.60-2.49 (m, 2H), 1.08 (t, J=7.0 Hz, 3H), 0.99-0.94 (m, 4H), 0.79 (t, *J* = 6.8 Hz, 3H) ppm. **LC-MS (ESI, m/z):** calcd for [M+H]⁺ 442.1; found: 442.2. **Retention time:** 1.87 min. [α]²⁵_{D} = -73.427° (c = 0.2574 g/100ml, MeOH)

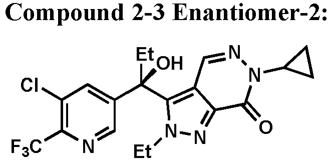

### (R)-3-(1-(5-chloro-6-(trifluoromethyl)pyridin-3-yl)-1-hydroxypropyl)-6-cyclopropyl-2-ethyl-2,6-dihydro-7H-pyrazolo[3,4-d]pyridazin-7-one (2-3)

**¹H NMR (400 MHz, DMSO-*d₆*):** *δ* 8.63 (s, 1H), 8.45 (s, 1H), 8.11 (s, 1H), 6.88 (s, 1H), 4.23-4.11 (m, 2H), 4.00-3.95 (m, 1H), 2.60-2.49 (m, 2H), 1.08 (t, *J*=7.0 Hz, 3H), 0.99-0.94 (m, 4H), 0.79 (t, *J* = 6.8 Hz, 3H) ppm. **LC-MS (ESI, m/z):** calcd for [M+H]⁺ 442.1; found: 442.2.

**Retention time:** 2.11 min. [α]²⁵_{D} = +54.900° (c = 0.2514 g/100 ml, MeOH)

### Synthesis of compounds 2-5, 2-6, 2-7, 2-8

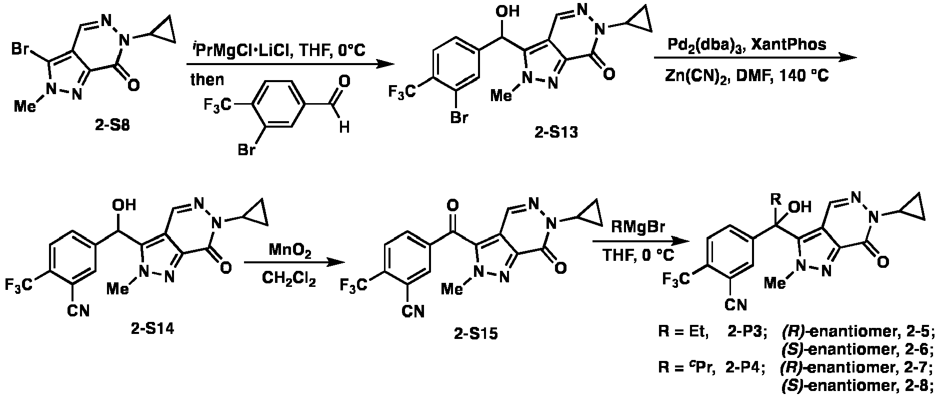

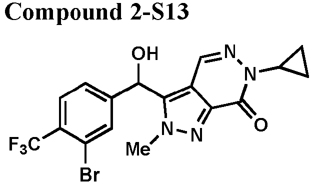

### 3-((3-bromo-4-(trifluoromethyl)phenyl)(hydroxy)methyl)-6-cyclopropyl-2-methyl-2,6-dihydro-7H-pyrazolo[3,4-d]pyridazin-7-one (2-S13)

Following **General Procedure D2** on 3.34 mmol scale with 3-bromo-6-cyclopropyl-2-methyl-2,6-dihydro-7*H*-pyrazolo[3,4-*d*]pyridazin-7-one **(2-S8)** and 3-bromo-4-(trifluoromethyl)-benzaldehyde. Purification by CombiFlash chromatography (80% ethyl acetate in hexane) to afford 3-((3-bromo-4-(trifluoromethyl)phenyl)(hydroxy)methyl)-6-cyclopropyl-2-methyl-2,6-dihydro-7*H*-pyrazolo[3,4-d]pyridazin-7-one **(2-S13)** as an off-white solid (1.3 g, 88%). **Physical state:** off-white solid. **¹H NMR (400 MHz, DMSO-*d₆*):** *δ* 7.96 (s, 1H), 7.88 (d, *J =* 8.0 Hz, 1H), 7.81 (s, 1H), 7.60 (d, *J =* 8.0 Hz, 1H), 6.93 (d, *J =* 4.6 Hz, 1H), 6.43 (d, *J =* 4.1 Hz, 1H), 4.06 (s, 3H), 3.95-3.90 (m, 1H), 0.93-0.88 (m, 4H) ppm. **LC-MS (ESI, m/z):** calcd for [M+H]⁺ 443.1; found: 443.2, 445.2 [M+2+H]⁺.

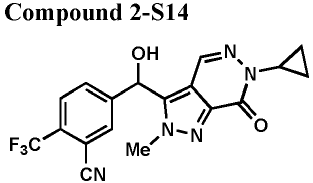

### 5-((6-cyclopropyl-2-methyl-7-oxo-6,7-dihydro-2H-pyrazolo[3,4-d]pyridazin-3-yl)(hydroxy)methyl)-2-(trifluoromethyl)benzonitrile (2-S14)

In a reaction tube, a stirred solution of 3-((3-bromo-4-(trifluoromethyl)phenyl)(hydroxy)methyl)-6-cyclopropyl-2-methyl-2,6-dihydro-7*H-*pyrazolo[3,4-*d*]pyridazin-7-one **(2-S13)** (800 mg, 1.81 mmol) in DMF (10 mL), was degassed with argon for 15 minutes and then Pd₂(dba)₃ (165 mg, 0.18 mmol) followed by Zn(CN)₂ (424 mg, 3.61 mmol) and Xantphos (209 mg, 0.36 mmol) were added. The tube was sealed and the reaction mixture was stirred at 120 °C for 14 h. After completion, the reaction mixture was cooled, diluted with ethyl acetate, washed with water, brine, dried over anhydrous Na₂SO₄ and concentrated. The crude product was purified by CombiFlash chromatography (70% ethyl acetate in hexane) to afford 5-((6-cyclopropyl-2-methyl-7-oxo-6,7-dihydro-2*H*-pyrazolo[3,4-*d*]pyridazin-3-yl)(hydroxy)methyl)-2-(trifluoromethyl)benzonitrile **(2-S14)** as an off white solid.

**Note.** The reaction was performed in another 500 mg scale (total 1.3 g scale) and (1 g, 88%) of 5-((6-cyclopropyl-2-methyl-7-oxo-6,7-dihydro-2H-pyrazolo[3,4-d]pyridazin-3-yl)(hydroxy)methyl) -2-(trifluoromethyl)benzonitrile **(2-S14)** was obtained. **Physical state:** off-white solid. **¹H NMR (400 MHz, DMSO-*d₆*): *δ*** 8.21 (s, 1H), 8.04 (d, *J* = 8.2 Hz, 1H), 7.97 (d, *J=* 8.1 Hz, 1H), 7.93 (s, 1H), 7.02 (d, ***J =*** 4.6 Hz, 1H), 6.48 (d, *J =* 4.0 Hz, 1H), 4.06 (s, 3H), 3.95-3.90 (m, 1H), 0.93-0.89 (m, 4H) ppm. **LC-MS (ESI, m/z):** calcd for [M+H]⁺ 390.1; found: 390.2.

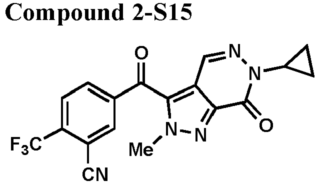

### 5-(6-cyclopropyl-2-methyl-7-oxo-6,7-dihydro-2H-pyrazolo[3,4-d]pyridazine-3-carbonyl)-2-(trifluoromethyl)benzonitrile (2-S15)

Following **General Procedure** E**2** on 2.57 mmol scale with 5-((6-cyclopropyl-2-methyl-7-oxo-6,7-dihydro-2*H*-pyrazolo[3,4-d]pyridazin-3-yl)(hydroxy)methyl)-2-(trifluoromethyl)benzonitrile **(2-S14).** Purification by CombiFlash chromatography (40% ethyl acetate in hexane) to afford 5-(6-cyclopropyl-2-methyl-7-oxo-6,7-dihydro-2*H*-pyrazolo[3,4-*d*]pyridazine-3-carbonyl)-2-(trifluoromethyl)benzonitrile **(5-S15)** as an off-white solid (0.65 g, 65%). **Physical state:** off-white solid. **¹H NMR (400 MHz,** DMSO-*d₆*): *δ* 8.56 (s, 1H), 8.30 (d, *J =* 8.2 Hz, 1H), 8.23 (d, *J =* 8.2 Hz, 1H), 7.56 (s, 1H), 4.35 (s, 3H), 4.01-3.96 (m, 1H), 0.95-0.90 (m, 4H) ppm. **LC-MS (ESI, m/z):** calcd for [M+H]⁺ 388.1; found: 388.2.

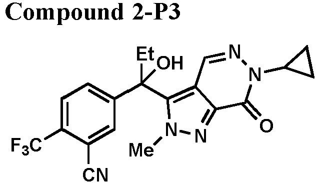

### 5-(1-(6-cyclopropyl-2-methyl-7-oxo-6,7-dihydro-2H-pyrazolo[3,4-d]pyridazin-3-yl)-1-hydroxypropyl)-2-(trifluoromethyl)benzonitrile (2-P3)

Following **General Procedure F2** on 1.03 mmol scale with 5-(6-cyclopropyl-2-methyl-7-oxo-6,7-dihydro-2*H*-pyrazolo[3,4-d]pyridazine-3-carbonyl)-2-(trifluoromethyl)benzonitrile **(2-S15)** and EtMgBr (3M in diethyl ether) (1.5 equiv.). Purification CombiFlash chromatography (70% ethyl acetate in hexane) to afford 5-(1-(6-cyclopropyl-2-methyl-7-oxo-6,7-dihydro-2*H-*pyrazolo[3,4-*d*]pyridazin-3-yl)-1-hydroxypropyl)-2-(trifluoromethyl)benzonitrile **(2-P3)** as an off-white solid (130 mg, 30%). **Physical state:** off-white solid.

The racemic compound **2-P3** was purified by preparative-HPLC-Chiral (SFC) to afford enantiomer-1 **(2-6)** (50 mg) and enantiomer-2 **(2-5)** (50 mg).

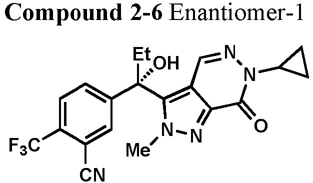

***(S)-5-(1-(6-cyclopropyl-2-methyl-7-oxo-6,7-dihydro-2H-pyrazolo[3,4-d]pyridazin-3-yl)-1-hydroxypropyl)-2-(trifluoromethyl)benzonitrile* (2-6) ¹H NMR (400 MHz, DMSO-*d₆*):** *δ* 8.46 (s, 1H), 8.05 (s, 1H), 7.99 (d, *J =* 8.4 Hz, 1H), 7.85 (d, *J =* 8.4 Hz, 1H), 6.73 (s, 1H), 4.01-3.97 (m, 1H), 3.74 (s, 3H), 2.58-2.50 (m, 2H), 1.02-0.94 (m, 4H), 0.76 (t, *J =* 7.1 Hz, 3H) ppm. **LC-MS (ESI, m/z):** calcd for [M+H]⁺ 418.1; found: 418.40. **Retention time:** 1.37 min.

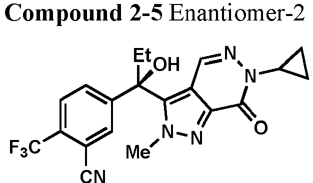

***(R)-5-(1-(6-cyclopropyl-2-methyl-7-oxo-6,7-dihydro-2H-pyrazolo[3,4-d]pyridazin-3-yl)-1-hydroxypropyl)-2-(trifluoromethyl)benzonitrile* (2-5) ¹H NMR (400 MHz, DMSO-*d₆*):** *δ* 8.46 (s, 1H), 8.05 (s, 1H), 7.99 (d, *J =* 8.4 Hz, 1H), 7.85 (d, *J =* 8.4 Hz, 1H), 6.73 (s, 1H), 4.01-3.97 (m, 1H), 3.74 (s, 3H), 2.60-2.50 (m, 2H), 1.03-0.94 (m, 4H), 0.76 (t, *J =* 7.1 Hz, 3H) ppm. **LC-MS (ESI, m/z):** calcd for [M+H]⁺ 418.1; found: 418.37. **Retention time:** 2.13 min.

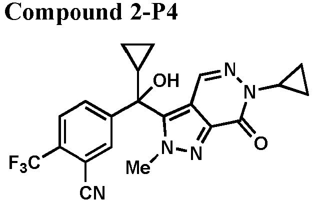

### 5-(cyclopropyl(6-cyclopropyl-2-methyl-7-oxo-6,7-dihydro-2H-pyrazolo[3,4-d]pyridazin-3-yl)(hydroxy)methyl)-2-(trifluoromethyl)benzonitrile (2-P4)

Following **General Procedure F2** on 0.65 mmol scale with 5-(6-cyclopropyl-2-methyl-7-oxo-6,7-dihydro-2H-pyrazolo[3,4-*d*]pyridazine-3-carbonyl)-2-(trifluoromethyl)benzonitrile **(2-S15)** and cyclopropylmagnesium bromide (1M in 2-methyl THF) (1.5 equiv.). Purification CombiFlash chromatography (70% ethyl acetate in hexane) to afford 5-(cyclopropyl(6-cyclopropyl-2-methyl-7-oxo-6,7-dihydro-2*H*-pyrazolo[3,4-d]pyridazin-3-yl)(hydroxy)methyl)-2-(trifluoromethyl)benzonitrile **(2-P4)** as an off-white solid (183 mg, 66%). **Physical state:** off-white solid.

The racemic compound **2-P4** was purified by preparative-HPLC-Chiral (SFC) to afford enantiomer-1 **(2-8)** (80 mg) and enantiomer-2 **(2-7)** (80 mg).

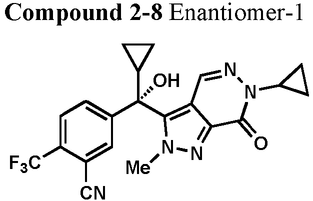

### (S)-5-(cyclopropyl(6-cyclopropyl-2-methyl-7-oxo-6,7-dihydro-2H-pyrazolo[3,4-d]pyridazin-3-yl)(hydroxy)methyl)-2-(trifluoromethyl)benzonitrile (2-8)

**¹H NMR (400 MHz, DMSO-*d₆*):** *δ* 8.54 (s, 1H), 8.03-7.98 (m, 2H), 7.80 (d, *J =* 8.4 Hz, 1H), 6.50 (s, 1H), 4.04-4.00 (m, 1H), 3.66 (s, 3H), 1.94-1.91 (m, 1H), 1.03-0.94 (m, 4H), 0.78-0.71 (m, 2H), 0.59-0.55 (m, 1H), 0.50-0.40 (m, 1H) ppm. **LC-MS (ESI, m/z):** calcd for [M+H]⁺ 430.1; found: 430.39. **Retention time:** 1.55 min.

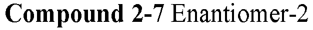

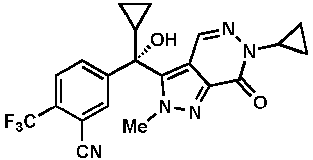

### (R)-5-(cyclopropyl(6-cyclopropyl-2-methyl-7-oxo-6,7-dihydro-2H-pyrazolo[3,4-d]pyridazin-3-yl)(hydroxy)methyl)-2-(trifluoromethyl)benzonitrile (2-7)

**¹H NMR (400 MHz, DMSO-*d₆*):** *δ* 8.54 (s, 1H), 8.03-7.98 (m, 2H), 7.80 (d, *J =* 8.4 Hz, 1H), 6.50 (s, 1H), 4.04-4.00 (m, 1H), 3.66 (s, 3H), 1.94-1.91 (m, 1H), 1.03-0.94 (m, 4H), 0.78-0.71 (m, 2H), 0.59-0.55 (m, 1H), 0.50-0.40 (m, 1H) **ppm.LC-MS (ESI, m/z):** calcd for [M+H]⁺ 430.1; found: 430.43. **Retention time:** 1.89 min.

### Synthesis of compounds 2-9, 2-10, 2-13, 2-14

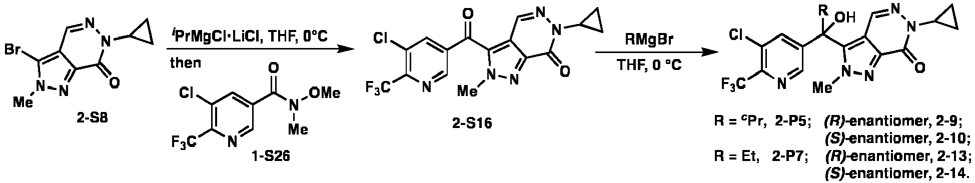

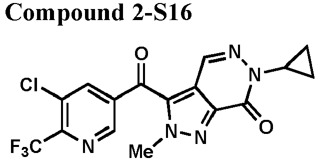

### 3-(5-chloro-6-(trifluoromethyl)nicotinoyl)-6-cyclopropyl-2-methyl-2,6-dihydro-7H-pyrazolo[3,4-d]pyridazin-7-one (2-S16)

Following **General Procedure C2** on 1.86 mmol scale with 3-bromo-6-cyclopropyl-2-methyl-2,6-dihydro-7*H*-pyrazolo[3,4-d]pyridazin-7-one **(2-S8)** and 5-chloro-*N*-methoxy-*N*-methyl-6-(trifluoromethyl)nicotinamide **(1-S26).** Purification by Combiflash chromatography (80% ethyl acetate in hexane) to afford 3-(5-chloro-6-(trifluoromethyl)nicotinoyl)-6-cyclopropyl-2-methyl-2,6-dihydro-7H-pyrazolo[3,4-*d*]pyridazin-7-one **(2-S16)** as an off-white solid (430 mg, 58%). **Physical state:** off-white solid. **¹H NMR (400 MHz, CDCl₃):** *δ* 8.91 (s, 1H), 8.31 (s, 1H), 7.43 (s, 1H), 4.48 (s, 3H), 4.11-4.05 (m, 1H), 1.07-1.05 (m, 2H), 1.02-1.00 (m, 2H)ppm. **LC-MS (ESI, m/z):** calcd for [M+H]⁺ 398.1; found: 398.0.

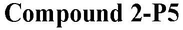

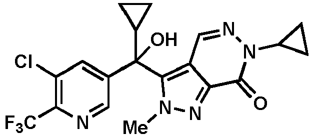

### 3-((5-chloro-6-(trifluoromethyl)pyridin-3-yl)(cyclopropyl)(hydroxy)methyl)-6-cyclopropyl-2-methyl-2,6-dihydro-7H-pyrazolo[3,4-d]pyridazin-7-one (2-P5)

Following **General Procedure F2** on 0.5 mmol scale with 3-(5-chloro-6-(trifluoromethyl)nicotinoyl)-6-cyclopropyl-2-methyl-2,6-dihydro-7*H*-pyrazolo[3,4-*d*]pyridazin-7-one **(2-S16)** and cyclopropylmagnesium bromide (1M in 2-methyl THF) (2.5 equiv.). Purification by Combi-flash chromatography (50% ethyl acetate in hexane) to afford 3-((5-chloro-6-(trifluoromethyl)pyridin-3-yl)(cyclopropyl)(hydroxy)methyl)-6-cyclopropyl-2-methyl-2,6-dihydro-7*H*-pyrazolo[3,4-d]pyridazin-7-one **(2-P5)** as an off-white solid (80 mg, 36%). **Physical state:** off-white solid.

The racemic compound **2-P5** was purified by preparative-HPLC-Chiral (NP) to afford enantiomer-1 (2-10) (25 mg) and enantiomer-2 (2-9) (25 mg).

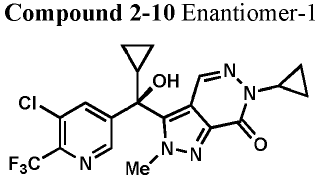

***(R)-3-((5-chloro-6-(trifluoromethyl)pyridin-3-yl)(cyclopropyl)(hydroxy)methyl)-6-cyclopropyl-2-methyl-2,6-dihydro-7H-pyrazolo[3,4-d]pyridazin-7-one* (2-10) ¹H NMR (400 MHz, DMSO-*d₆*):** *δ* 8.61 (s, 1H), 8.52 (s, 1H), 8.08 (s, 1H), 6.57 (s, 1H), 4.05-4.00 (m, 1H), 3.74 (s, 3H), 2.00-1.90 (m, 1H), 1.00-0.94 (m, 4H), 0.80-0.70 (m, 2H), 0.65-0.55 (m, 1H), 0.50-0.40 (m, 1H) ppm. **LC-MS (ESI, m/z):** calcd for [M+H]⁺ 440.1; found: 440.18. **Retention time:** 5.60 min.

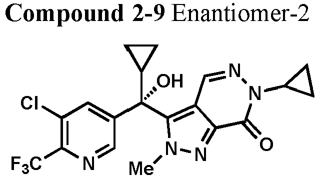

***(S)-3-((5-chloro-6-(trifluoromethyl)pyridin-3-yl)(cyclopropyl)(hydroxy)methyl)-6-cyclopropyl-2-methyl-2,6-dihydro-7H-pyrazolo[3,4-d]pyridazin-7-one* (2-9) ¹H NMR (400 MHz, DMSO-*d₆*):** *δ* 8.61 (s, 1H), 8.52 (s, 1H), 8.08 (s, 1H), 6.57 (s, 1H), 4.04-3.99 (m, 1H), 3.74 (s, 3H), 2.01-1.94 (m, 1H), 1.01-0.92 (m, 4H), 0.80-0.74 (m, 2H), 0.62-0.59 (m, 1H), 0.50-0.40 (m, 1H) ppm. **LC-MS (ESI, m/z):** calcd for [M+H]⁺ 440.1; found: 440.18. **Retention time:** 6.28 min.

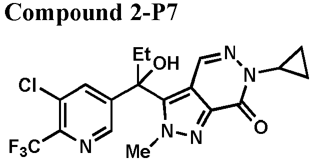

### 3-(1-(5-chloro-6-(trifluoromethyl)pyridin-3-yl)-1-hydroxypropyl)-6-cyclopropyl-2-methyl-2,6-dihydro-7H-pyrazolo[3,4-d]pyridazin-7-one (2-P7)

Following **General Procedure F2** on 0.55 mmol scale with 3-(5-chloro-6-(trifluoromethyl)nicotinoyl)-6-cyclopropyl-2-ethyl-2,6-dihydro-7*H*-pyrazolo[3,4-d]pyridazin-7-one **(2-S16)** and EtMgBr (3M in diethyl ether) (1.6 equiv.). Purification by Combiflash chromatography (50% ethyl acetate in hexane) to afford 3-(1-(5-chloro-6-(trifluoromethyl)pyridin-3-yl)-1-hydroxypropyl)-6-cyclopropyl-2-methyl-2,6-dihydro-7*H-*pyrazolo[3,4-*d*]pyridazin-7-one **(2-P7)** as an off-white solid (65 mg, 27%). **Physical state:** off-white solid.

The racemic compound **2-P7** was purified by preparative-HPLC-Chiral (NP) to afford enantiomer-1 **(2-14)** (25 mg) and enantiomer-2 **(2-13)** (25 mg).

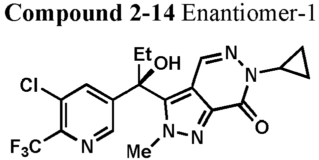

***(R)-3-(1-(5-chloro-6-(trifluoromethyl)pyridin-3-yl)-1-hydroxypropyl)-6-cyclopropyl-2-methyl-2,6-dihydro-7H-pyrazolo[3,4-d]pyridazin-7-one* (2-14) ¹H NMR (400 MHz, DMSO-**d*₆*): *δ* 8.64 (s, 1H), 8.44 (s, 1H), 8.11 (s, 1H), 6.81 (s, 1H), 4.00-3.95 (m, 1H), 3.82 (s, 3H), 2.60-2.54 (m, 2H), 1.05-0.99 (m, 2H), 0.95-0.93 (m, 2H), 0.79 (t, *J =* 7.08 Hz, 3H) ppm. **LC-MS (ESI, m/z):** calcd for [M+H]⁺ 428.1; found: 428.26. **Retention time:** 4.48 min.

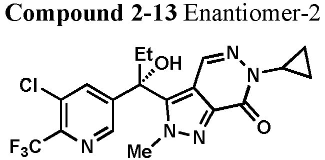

***(S)-3-(1-(5-chloro-6-(trifluoromethyl)pyridin-3-yl)-1-hydroxypropyl)-6-cyclopropyl-2-methyl-2,6-dihydro-7H-pyrazolo[3,4-d]pyridazin-7-one* (2-13) ¹H NMR (400 MHz, DMSO-d*₆*): *δ* 8.64** (s, 1H), 8.44 (s, 1H), 8.10 (s, 1H), 6.81 (s, 1H), 4.02-3.96 (m, 1H), 3.81 (s, 3H), **2.67-2.54** (m, 2H), 1.03-0.99 (m, 2H), 0.97-0.92 (m, 2H), 0.79 (t, *J =* 7.08 Hz, 3H) ppm. **LC-MS (ESI, m/z):** calcd for [M+H]⁺ 428.1; found: 428.26. **Retention time:** 5.34 min.

### Synthesis of compounds 2-11, 2-12, 2-15, 2-16

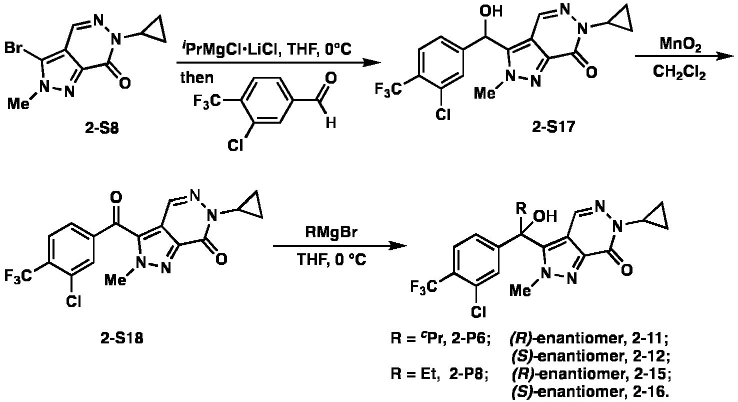

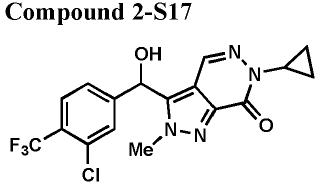

### 3-((3-chloro-4-(trifluoromethyl)phenyl)(hydroxy)methyl)-6-cyclopropyl-2-methyl-2,6-dihydro-7H-pyrazolo[3,4-d]pyridazin-7-one (2-S17)

Following **General Procedure D2** on 1.49 mmol scale with 3-bromo-6-cyclopropyl-2-methyl-2,6-dihydro-7*H*-pyrazolo[3,4-*d*]pyridazin-7-one **(2-S8)** and 3-chloro-4-(trifluoromethyl)-benzaldehyde. Purification by Combiflash chromatography (80% ethyl acetate in hexane) to afford 3-((3-chloro-4-(trifluoromethyl)phenyl)(hydroxy)methyl)-6-cyclopropyl-2-methyl-2,6-dihydro-7*H*-pyrazolo[3,4-d]pyridazin-7-one **(2-S17)** as an off-white solid (330 mg, 56%). **Physical state:** off-white solid. **¹H NMR (400 MHz, DMSO-*d₆*):** *δ* 7.89 (d, *J =* 8.2 Hz, 1H), 7.83 (s, 1H), 7.79 (s, 1H), 7.57 (d, *J=* 8.2 Hz, 1H), 6.94 (d, *J =* 4.76 Hz, 1H), 6.43 (d, *J =* 4.44 Hz, 1H), 4.06 (s, 3H), 3.96-3.90 (m, 1H), 0.96-0.86 (m, 4H) ppm. **LC-MS (ESI, m/z):** calcd for [M+H]⁺ 399.1; found: 399.16.

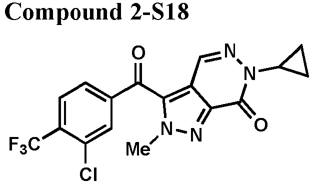

### 3-(3-chloro-4-(trifluoromethyl)benzoyl)-6-cyclopropyl-2-methyl-2,6-dihydro-7H-pyrazolo[3,4-d]pyridazin-7-one (2-S18)

Following **General Procedure E2** on 0.83 mmol scale with 3-((3-chloro-4-(trifluoromethyl)phenyl)(hydroxy)methyl)-6-cyclopropyl-2-methyl-2,6-dihydro-7*H*-pyrazolo-[3,4-*d*]pyridazin-7-one **(2-S17).** After completion, the reaction mixture was filtered and concentrated to afford 3-(3-chloro-4-(trifluoromethyl)benzoyl)-6-cyclopropyl-2-methyl-2,6-dihydro-7*H*-pyrazolo[3,4-d]pyridazin-7-one **(2-S18)** (325 mg, 99%) as an off-white solid. **Physical state:** off-white solid. **¹H NMR (400 MHz, CDCl₃):** *δ* 7.97 (s, 1H), 7.91 (d, *J =* 8.16 Hz, 1H), 7.76 (d, *J =* 8.24 Hz, 1H), 7.41 (s, 1H), 4.46 (s, 3H), 4.11-4.07 (m, 1H), 1.06-1.04 (m, 2H), 1.02-0.99 (m, 2H) ppm. **LC-MS (ESI, m/z):** calcd for [M+H]⁺ 399.1; found: 397.17.

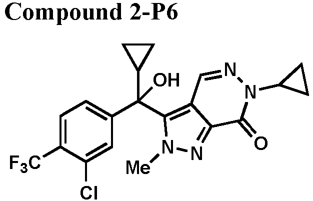

### 3-((3-chloro-4-(trifluoromethyl)phenyl)(cyclopropyl)(hydroxy)methyl)-6-cyclopropyl-2-methyl-2,6-dihydro-7H-pyrazolo[3,4-d]pyridazin-7-one (2-P6)

Following **General Procedure F2** on 0.35 mmol scale with 3-(3-chloro-4-(trifluoromethyl)benzoyl)-6-cyclopropyl-2-methyl-2,6-dihydro-7H-pyrazolo[3,4-*d*]pyridazin-7-one **(2-S18)** and cyclopropylmagnesium bromide (1M in 2-methyl THF) (2.5 equiv.). Purification by Combiflash chromatography (50% ethyl acetate in hexane) to afford 3-((3-chloro-4-(trifluoromethyl)phenyl)(cyclopropyl)(hydroxy)methyl)-6-cyclopropyl-2-methyl-2,6-dihydro-7*H*-pyrazolo[3,4-d]pyridazin-7-one **(2-P6)** as an off-white solid (90 mg, 58%). **Physical state:** off-white solid.

The racemic compound **2-P6** was purified by preparative-HPLC-Chiral (NP) to afford enantiomer-1 **(2-12)** (35 mg) and enantiomer-2 **(2-11)** (35 mg).

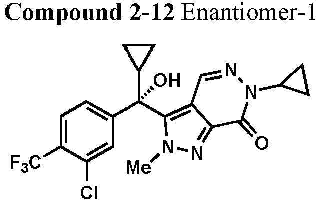

***(S)-3-((3-chloro-4-(trifluoromethyl)phenyl)(cyclopropyl)(hydroxy)methyl)-6-cyclopropyl-2-methyl-2,6-dihydro-7H-pyrazolo[3,4-d]pyridazin-7-one* (2-12) ¹H NMR (400 MHz, DMSO-**d*₆*): *δ* 8.53 (s, 1H), 7.83 (d, *J* = 8.4 Hz, 1H), 7.62 (s, 1H), 7.34 (d, *J* = 8.4 Hz, 1H), 6.41 (s, 1H), 4.03-3.99 (m, 1H), 3.68 (s, 3H), 1.92-1.90 (m, 1H), 1.03-0.99 (m, 2H), 0.96-0.92 (m, 2H), 0.75-0.69 (m, 2H), 0.59-0.56 (m, 1H), 0.50-0.45 (m, 1H) ppm. **LC-MS (ESI, m/z):** calcd for [M+H]⁺ 439.1; found: 439.28. **Retention time:** 4.14 min.

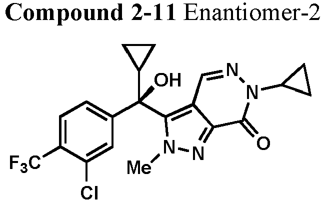

***(R)-3-((3-chloro-4-(trifluoromethyl)phenyl)(cyclopropyl)(hydroxy)methyl)-6-cyclopropyl-2-methyl-2,6-dihydro-7H-pyrazolo[3,4-d]pyridazin-7-one* (2-11) ¹H NMR (400 MHz, DMSO-d*₆*):** *δ* 8.53 (s, 1H), 7.83 (d, *J* = 8.4 Hz, 1H), 7.62 (s, 1H), 7.34 (d, *J* = 8.4 Hz, 1H), 6.41 (s, 1H), 4.03-3.99 (m, 1H), 3.68 (s, 3H), 1.92-1.90 (m, 1H), 1.03-0.99 (m, 2H), 0.96-0.92 (m, 2H), 0.75-0.69 (m, 2H), 0.59-0.56 (m, 1H), 0.50-0.45 (m, 1H) ppm. **LC-MS (ESI, m/z):** calcd for [M+H]⁺ 439.1; found: 439.29. **Retention time:** 4.70 min.

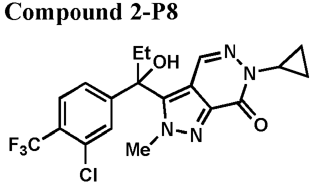

### 3-(1-(3-chloro-4-(trifluoromethyl)phenyl)-1-hydroxypropyl)-6-cyclopropyl-2-methyl-2,6-dihydro-7H-pyrazolo[3,4-d]pyridazin-7-one (2-P8)

Following **General Procedure F2** on 0.40 mmol scale with 3-(3-chloro-4-(trifluoromethyl)benzoyl)-6-cyclopropyl-2-methyl-2,6-dihydro-7*H*-pyrazolo[3,4-*d*]pyridazin-7-one **(2-S18)** and EtMgBr (3M in diethyl ether) (2.5 equiv.). Purification by Combiflash chromatography (50% ethyl acetate in hexane) to afford 3-(1-(3-chloro-4-(trifluoromethyl)phenyl)-1-hydroxypropyl)-6-cyclopropyl-2-methyl-2,6-dihydro-7*H-*pyrazolo[3,4-*d*]pyridazin-7-one **(2-P8)** as an off-white solid (40 mg, 23%). **Physical state:** off-white solid.

The racemic compound **2-P8** was purified by preparative-HPLC-Chiral (NP) to afford enantiomer-1 **(2-16)** (15 mg) and enantiomer-2 **(2-15)** (15 mg).

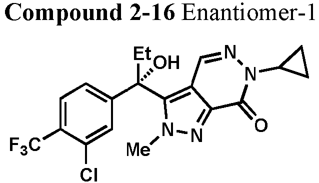

***(S)-3-(1-(3-chloro-4-(trifluoromethyl)phenyl)-1-hydroxypropyl)-6-cyclopropyl-2-methyl-2,6-dihydro-7H-pyrazolo[3,4-d]pyridazin-7-one* (2-16) ¹H NMR (400 MHz, DMSO-*d₆*):** *δ* 8.45 (s, 1H), 7.83 (d, *J =* 8.32 Hz, 1H), 7.65 (s, 1H), 7.40 (d, *J =* 8.24 Hz, 1H), 6.65 (s, 1H), 4.01-3.96 (m, 1H), 3.76 (s, 3H), 2.58-2.44 (m, 2H), 1.00-0.99 (m, 2H), 0.96-0.93 (m, 2H), 0.77 (t, *J* = 7.16 Hz, 3H) ppm. **LC-MS (ESI, m/z):** calcd for [M+H]⁺ 427.1; found: 427.27. **Retention time:** 4.13 min.

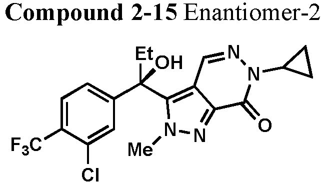

### (R)-3-(1-(3-chloro-4-(trifluoromethyl)phenyl)-1-hydroxypropyl)-6-cyclopropyl-2-methyl-2,6-dihydro-7H-pyrazolo[3,4-d]pyridazin-7-one (2-15)

**¹H NMR (400 MHz, DMSO-*d₆*):** *δ* 8.45 (s, 1H), 7.83 (d, *J =* 8.32 Hz, 1H), 7.65 (s, 1H), 7.40 (d, *J=* 8.24 Hz, 1H), 6.67 (s, 1H), 4.01-3.96 (m, 1H), 3.76 (s, 3H), 2.58-2.42 (m, 2H), 1.03-0.99 (m, 2H), 0.97-0.93 (m, 2H), 0.77 (t, *J=* 7.16 Hz, 3H) ppm. **LC-MS (ESI, m/z):** calcd for [M+H]⁺ 427.1; found: 427.25. **Retention time:** 4.61 min.

### Synthesis of compounds 2-17, 2-18

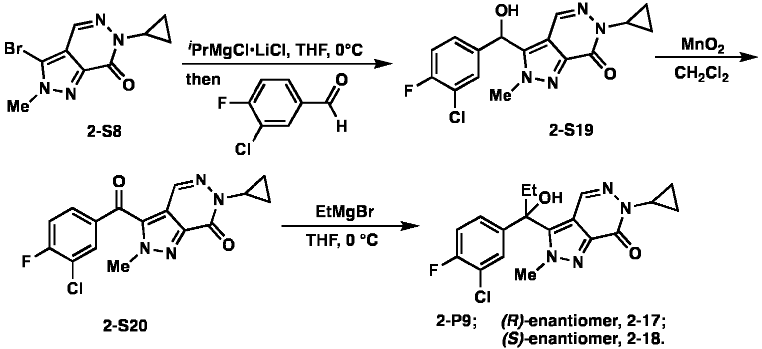

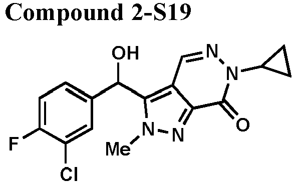

### 3-((3-chloro-4-fluorophenyl)(hydroxy)methyl)-6-cyclopropyl-2-methyl-2,6-dihydro-7H-pyrazolo[3,4-d]pyridazin-7-one (2-S19)

Following **General Procedure D2** on 3.34 mmol scale with 3-bromo-6-cyclopropyl-2-methyl-2,6-dihydro-7H-pyrazolo[3,4-d]pyridazin-7-one **(2-S8)** and 3-chloro-4-fluorobenzaldehyde. Purification by Combiflash chromatography (80% ethyl acetate in hexane) to afford 3-((3-chloro-4-fluorophenyl)(hydroxy)methyl)-6-cyclopropyl-2-methyl-2,6-dihydro-7*H-*pyrazolo[3,4-d]pyridazin-7-one (2-S19) as an off-white solid (757 mg, 83%). **Physical state:** off-white solid. **¹H NMR (400 MHz, DMSO-*d₆*):** *δ* 7.76 (s, 1H), 7.67-7.65 (m, 1H), 7.45-7.41 (m, 2H), 6.78 (d, *J =* 4.68 Hz, 1H), 6.31 (d, *J =* 4.28 Hz, 1H), 4.04 (s, 3H), 3.95-3.90 (m, 1H), 0.93-0.88 (m, 4H) ppm. **LC-MS (ESI, m/z):** calcd for [M+H]⁺ 349.1; found: 349.1.

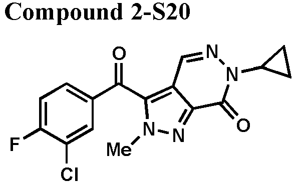

### 3-(3-chloro-4-fluorobenzoyl)-6-cyclopropyl-2-methyl-2,6-dihydro-7H-pyrazolo[3,4-d]pyridazin-7-one (2-S20)

Following **General Procedure E2** on 2.17 mmol scale with 3-((3-chloro-4-fluorophenyl)(hydroxy)methyl)-6-cyclopropyl-2-methyl-2,6-dihydro-7*H*-pyrazolo[3,4-*d*]pyrida-zin-7-one **(2-S19).** After completion, the reaction mixture was filtered and concentrated to afford 3-(3-chloro-4-fluorobenzoyl)-6-cyclopropyl-2-methyl-2,6-dihydro-7*H-*pyrazolo[3,4-*d*]pyridazin-7-one **(2-S20)** as an off-white solid (750 mg, 99%). **Physical state:** off-white solid. **¹H NMR (400 MHz, CDCl₃):** *δ* 7.98-7.96 (m, 1H), 7.73-7.72 (m, 1H), 7.50 (s, 1H), 7.34 (t, *J =* 8.44 Hz, 1H), 4.41 (s, 3H), 4.09-4.07 (m, 1H), 1.08-1.06 (m, 2H), 1.01-0.97 (m, 2H) ppm. **LC-MS (ESI, m/z):** calcd for [M+H]⁺ 347.1; found: 347.29.

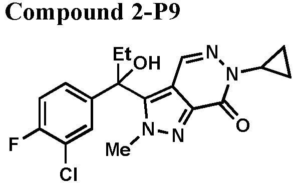

### 3-(1-(3-chloro-4-fluorophenyl)-1-hydroxypropyl)-6-cyclopropyl-2-methyl-2,6-dihydro-7H-pyrazolo[3,4-d]pyridazin-7-one (2-P9)

Following **General Procedure F2** on 0.58 mmol scale with 3-(3-chloro-4-(trifluoromethyl)benzoyl)-6-cyclopropyl-2-methyl-2,6-dihydro-7*H*-pyrazolo[3,4-d]pyridazin-7-one **(2-S20)** and EtMgBr (3M in diethyl ether) (1.5 equiv.). Purification by Combiflash chromatography (50% ethyl acetate in hexane) to afford 3-(1-(3-chloro-4-fluorophenyl)-1-hydroxypropyl)-6-cyclopropyl-2-methyl-2,6-dihydro-7H-pyrazolo[3,4-*d*]pyridazin-7-one (2-**P9)** as an off-white solid (46 mg, 21%). **Physical state:** off-white solid.

The racemic compound **2-P9** was purified by preparative-HPLC-Chiral (NP) to afford enantiomer-1 **(2-18)** (20 mg) and enantiomer-2 **(2-17)** (20 mg).

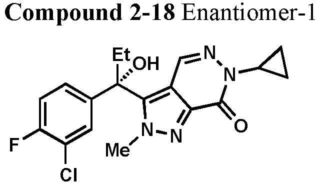

***(S)-3-(1-(3-chloro-4-fluorophenyl)-1-hydroxypropyl)-6-cyclopropyl-2-methyl-2,6-dihydro-7H-pyrazolo[3,4-d]pyridazin-7-one* (2-18) ¹H NMR (400 MHz, DMSO-*d₆*): *δ*** 8.39 (s, 1H), 7.51 (d, *J =* 5.32 Hz, 1H), 7.38 (t, J = 8.88 Hz, 1H), 7.25-7.24 (m, 1H), 6.46 (s, 1H), 3.99-3.96 (m, 1H), 3.76 (s, 3H), 2.50-2.39 (m, 2H), 0.99-0.93 (m, 4H), 0.77 (t, *J =* 6.96 Hz, 3H) ppm. **LC-MS (ESI, m/z):** calcd for [M+H]⁺ 377.1; found: 377.25. **Retention time:** 5.63 min.

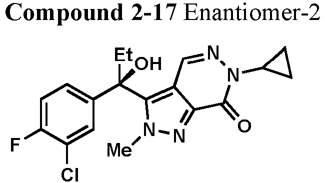

***(R)-3-(1-(3-chloro-4-fluorophenyl)-1-hydroxypropyl)-6-cyclopropyl-2-methyl-2,6-dihydro-7H-pyrazolo[3,4-d]pyridazin-7-one* (2-17) ¹H NMR (400 MHz, DMSO-*d₆*):** *δ* 8.39 (s, 1H), 7.51 (d, *J =* 5.32 Hz, 1H), 7.38 (t, *J* = 8.88 Hz, 1H), 7.26-7.22 (m, 1H), 6.46 (s, 1H), 3.99-3.95 (m, 1H), 3.76 (s, 3H), 2.50-2.39 (m, 2H), 1.01-0.98 (m, 2 H), 0.95-0.92 (m, 2 H), 0.77 (t, *J =* 6.96 Hz, 3H) ppm. **LC-MS (ESI, m/z):** calcd for [M+H]⁺ 377.1; found: 377.25. **Retention time:** 6.90 min.

### Synthesis of compounds 2-19, 2-20

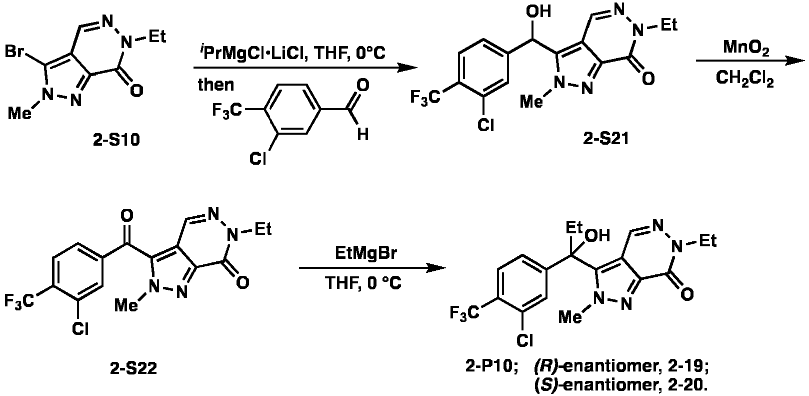

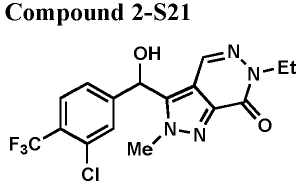

### 3-((3-chloro-4-(trifluoromethyl)phenyl)(hydroxy)methyl)-6-ethyl-2-methyl-2,6-dihydro-7H-pyrazolo[3,4-d]pyridazin-7-one (2-S21)

Following **General Procedure D2** on 3.89 mmol scale with 3-bromo-6-ethyl-2-methyl-2,6-dihydro-7*H*-pyrazolo[3,4-*d*]pyridazin-7-one **(2-S10)** and 3-chloro-4-(trifluoromethyl)-benzaldehyde. Purification by CombiFlash Chromatography (50% ethyl acetate in hexane) to afford 3-((3-chloro-4-(trifluoromethyl)phenyl)(hydroxy)methyl)-6-ethyl-2-methyl-2,6-dihydro-7*H*-pyrazolo[3,4-*d*]pyridazin-7-one **(2-S21)** as an off-white solid (0.83 g, 55%).

**Physical state:** off-white solid. **¹H NMR (400 MHz, DMSO-*d₆*):** *δ* 7.90 (d, *J =* 8.2 Hz, 1H), 7.87 (s, 1H), 7.80 (s, 1H), 7.58 (d, *J =* 8.16 Hz, 1H), 6.94 (d, *J =* 4.8 Hz, 1H), 6.44 (d, *J =* 4.64 Hz, 1H), 4.12-4.03 (m, 5H), 1.22 (t, *J* = 7.16 Hz, 3H) ppm. **LC-MS (ESI, m/z):** calcd for [M+H]⁺ 387.1; found: 386.93.

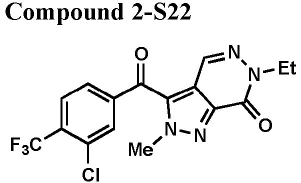

### 3-(3-chloro-4-(trifluoromethyl)benzoyl)-6-ethyl-2-methyl-2,6-dihydro-7H-pyrazolo[3,4-d]pyridazin-7-one (2-S22)

Following **General Procedure E2** on 1.81 mmol scale with 3-((3-chloro-4-(trifluoromethyl)phenyl)(hydroxy)methyl)-6-ethyl-2-methyl-2,6-dihydro-7*H*-pyrazolo[3,4-*d*]pyridazin-7-one **(2-S21).** Purification by CombiFlash Chromatography (30% ethyl acetate in hexane) to afford 3-(3-chloro-4-(trifluoromethyl)benzoyl)-6-ethyl-2-methyl-2,6-dihydro-7*H-*pyrazolo[3,4-*d*]pyridazin-7-one **(2-S22)** as an off-white solid (0.6 g, 86%). **Physical state:** off-white solid. **¹H NMR (400 MHz, CDCl₃):** *δ* 7.98 (s, 1H), 7.92 (d, *J =* 8.0 Hz, 1H), 7.78 (d, *J* = 8.0 Hz, 1H), 7.47 (s, 1H), 4.46 (s, 3H), 4.27 (q, *J =* 7.2 Hz, 2H), 1.37 (t, *J =* 7.2 Hz, 3H) ppm. **LC-MS (ESI, m/z):** calcd for [M+H]⁺ 385.1; found: 384.81.

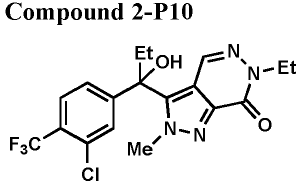

### 3-(1-(3-chloro-4-(trifluoromethyl)phenyl)-1-hydroxypropyl)-6-ethyl-2-methyl-2,6-dihydro-7H-pyrazolo[3,4-d]pyridazin-7-one (2-P10)

Following **General Procedure F2** on 0.26 mmol scale with 3-(3-chloro-4-(trifluoromethyl)benzoyl)-6-ethyl-2-methyl-2,6-dihydro-7H-pyrazolo[3,4-*d*]pyridazin-7-one **(2-S22)** and EtMgBr (3M in diethyl ether) (1.5 equiv.) at -5 °C. Purification by CombiFlash Chromatography (70% ethyl acetate in hexane) to afford 3-(1-(3-chloro-4-(trifluoromethyl)phenyl)-1-hydroxypropyl)-6-ethyl-2-methyl-2,6-dihydro-7*H*-pyrazolo[3,4-*d*]pyridazin-7-one **(2-P10)** as an off-white solid (70 mg). **Physical state:** off-white solid.

The racemic compound **2-P10** was purified by preparative Chiral-HPLC to afford Enantiomer-1 **(2-20)** (20 mg) and Enantiomer-2 **(2-19)** (20 mg).

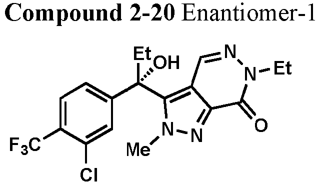

### (S)-3-(1-(3-chloro-4-(trifluoromethyl)phenyl)-1-hydroxypropyl)-6-ethyl-2-methyl-2,6-dihydro-7H-pyrazolo[3,4-d]pyridazin-7-one (2-20)

**¹H NMR (400 MHz, DMSO-*d₆*):** *δ* 8.48 (s, 1H), 7.84 (d, *J =* 8.4 Hz, 1H), 7.67 (s, 1H), 7.42 (d, *J =* 8.0 Hz, 1H), 6.63 (s, 1H), 4.15-4.12 (m, 2H), 3.76 (s, 3H), 2.58-2.56 (m, 2H), 1.28 (t, *J =* 7.20 Hz, 3H), 0.79 (t, *J =* 7.20 Hz, 3H) ppm. **LC-MS (ESI, m/z):** calcd for [M+H]⁺ 415.1; found: 414.90. **Retention time:** 4.45 min.

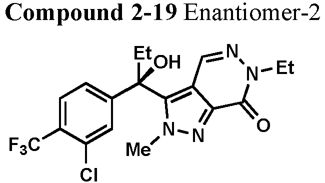

*(R)-3-(1-(3-chloro-4-(trifluoromethyl)phenyl)-1-hydroxypropyl)-6-ethyl-2-methyl-2,6-dihydro-7H-pyrazolo[3,4-d]pyridazin-7-one* (2-19) **¹H** NMR (400 MHz, DMSO-*d₆*): *δ* 8.48 (s, 1H), 7.84 (d, *J =* 8.4 Hz, 1H), 7.67 (s, 1H), 7.42 (d, *J =* 8.0 Hz, 1H), 6.63 (s, 1H), 4.15-4.12 (m, 2H), 3.76 (s, 3H), 2.58-2.56 (m, 2H), 1.28 (t, *J =* 7.20 Hz, 3H), 0.79 (t, *J =* 7.20 Hz, 3H) ppm. **LC-MS (ESI, m/z):** calcd for [M+H]⁺ 415.1; found: 414.97. **Retention time:** 5.21 min.

### Synthesis of compounds 3-1 to 3-12

### Synthesis of compounds 3-1, 3-2, 3-3, 3-4, 3-5, 3-6

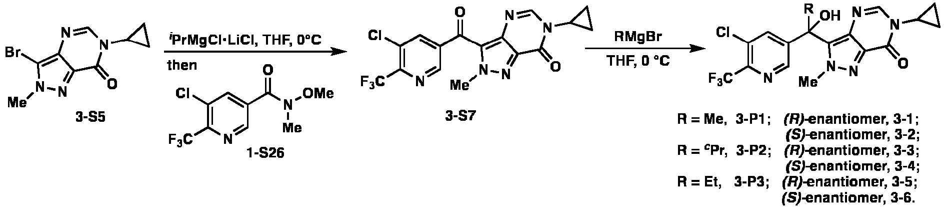

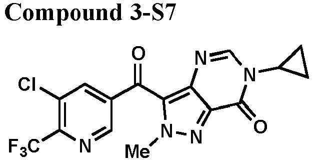

### 3-(5-chloro-6-(trifluoromethyl)nicotinoyl)-6-cyclopropyl-2-methyl-2,6-dihydro-7H-pyrazolo[4,3-d]pyrimidin-7-one (3-S7)

Following **General Procedure C3** on 5.57 mmol scale with 3-bromo-6-cyclopropyl-2-methyl-2,6-dihydro-7*H*-pyrazolo[4,3-d]pyrimidin-7-one **(3-S5)** and 5-chloro-*N*-methoxy-*N*-methyl-6-(trifluoromethyl)nicotinamide **(1-S26).** Purification by CombiFlash chromatography (80% ethyl acetate in hexane) to afford 3-(5-chloro-6-(trifluoromethyl)nicotinoyl)-6-cyclopropyl-2-methyl-2,6-dihydro-7*H*-pyrazolo[4,3-*d*]pyrimidin-7-one **(3-S7)** as a light yellow solid (0.45 g, 20%). **Physical state:** light yellow solid. **¹H NMR (400 MHz, CDCl₃):** *δ* 8.95 (s, 1H), 8.33 (s, 1H), 7.90 (s, 1H), 4.45 (s, 3H), 3.25-3.20 (m, 1H), 1.25-1.15 (m, 2H), 0.92-0.85 (m, 2H) ppm. **LC-MS (ESI, m/z):** calcd for [M+H]⁺ 398.1; found: 398.19.

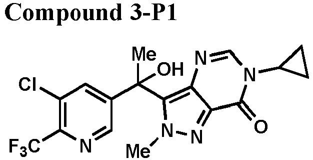

### 3-(1-(5-chloro-6-(trifluoromethyl)pyridin-3-yl)-1-hydroxyethyl)-6-cyclopropyl-2-methyl-2,6-dihydro-7H-pyrazolo[4,3-d]pyrimidin-7-one (3-P1)

Following **General Procedure F3** on 0.43 mmol scale with 3-(5-chloro-6-(trifluoromethyl)nicotinoyl)-6-cyclopropyl-2-methyl-2,6-dihydro-7*H*-pyrazolo[4,3-*d*]pyrimidin-7-one **(3-S7)** and MeMgBr (3M in diethyl ether) (1.4 equiv.). Purification by CombiFlash chromatography (50% ethyl acetate in hexane) to afford 3-(1-(5-chloro-6-(trifluoromethyl)pyridin-3-yl)-1-hydroxyethyl)-6-cyclopropyl-2-methyl-2,6-dihydro-7*H-*pyrazolo[4,3-*d*]pyrimidin-7-one **(3-P1)** as an off-white solid (100 mg, 57%). **Physical state:** off-white solid.

The racemic compound **3-P1** was purified from Preparative-HPLC-Chiral (SFC) to afford enantiomer-1 **(3-2)** (35 mg) and enantiomer-2 **(3-1)** (35 mg).

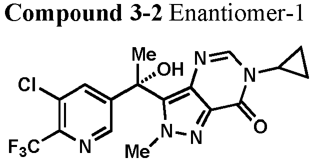

***(S)-3-(1-(5-chloro-6-(trifluoromethyl)pyridin-3-yl)-1-hydroxyethyl)-6-cyclopropyl-2-methyl-2,6-dihydro-7H-pyrazolo[4,3-d]pyrimidin-7-one* (3-2) ¹H NMR (400 MHz, DMSO-*d₆*):** *δ* 8.57 (d, *J=* 1.4 Hz, 1H), 8.06 (s, 2H), 6.99 (br s, 1H), 3.87 (s, 3H), 3.19-3.16 (m, 1H), 2.19 (s, 3H), 1.06-1.01 (m, 2H), 0.93-0.89 (m, 2H) ppm. **LC-MS (ESI, m/z):** calcd for [M+H]⁺ 414.1; found: 414.1. **Retention time:** 1.58 min. **[α]_{D}²⁰** = -102.29 (c = 0.26, CHCl₃).

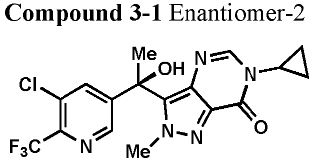

***(R)-3-(1-(5-chloro-6-(trifluoromethyl)pyridin-3-yl)-1-hydroxyethyl)-6-cyclopropyl-2-methyl-2,6-dihydro-7H-pyrazolo[4,3-d]pyrimidin-7-one* (3-1) ¹H NMR (400 MHz, DMSO-*d₆*):** *δ* 8.57 (s, 1H), 8.06 (s, 2H), 6.99 *(br.,* 1H), 3.87 (s, 3H), 3.19-3.16 (m, 1H), 2.19 (s, 3H), 1.06-1.01 (m, 2H), 0.93-0.89 (m, 2H) ppm. **LC-MS (ESI, m/z):** calcd for [M+H]⁺ 414.1; found: 414.1. **Retention time:** 3.31 min.

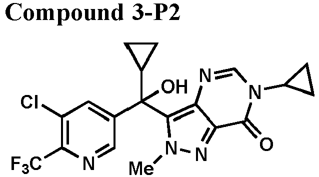

### 3-((5-chloro-6-(trifluoromethyl)pyridin-3-yl)(cyclopropyl) (hydroxy) methyl)-6-cyclopropyl-2-methyl-2,6-dihydro-7H-pyrazolo[4,3-d]pyrimidin-7-one (3-P2)

Following **General Procedure F3** on 0.252 mmol scale with 3-(5-chloro-6-(trifluoromethyl)nicotinoyl)-6-cyclopropyl-2-methyl-2,6-dihydro-7*H*-pyrazolo[4,3-*d*]pyrimidin-7-one **(3-S7)** and cyclopropylmagnesium bromide (0.7M in THF) (1.0 equiv.). Purification by Combiflash chromatography (60% ethyl acetate in hexane) to afford 3-((5-chloro-6-(trifluoromethyl)pyridin-3-yl)(cyclopropyl)(hydroxy)methyl)-6-cyclopropyl-2-methyl-2,6-dihydro-7*H*-pyrazolo[4,3-*d*]pyrimidin-7-one **(3-P2)** as an off-white solid (75 mg, 68%). **Physical state:** off-white solid.

The racemic compound **3-P2** was purified by preparative-HPLC-Chiral (NP) to afford enantiomer-1 **(3-4)** (25 mg) and enantiomer-2 **(3-3)** (25 mg).

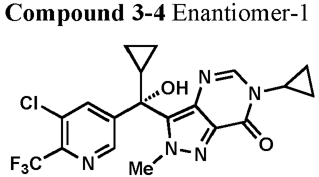

***(S)-3-((5-chloro-6-(trifluoromethyl)pyridin-3-yl)(cyclopropyl)(hydroxy)methyl)-6-cyclopropyl-2-methyl-2,6-dihydro-7H-pyrazolo[4,3-d]pyrimidin-7-one* (3-4) ¹H NMR (400 MHz, DMSO-*d₆*):** *δ* 8.59 (s, 1H), 8.03-8.00 (m, 2H), 6.41 (s, 1H), 3.91 (s, 3H), 3.20-3.15 (m, 1H), 2.35-2.33 (m, 1H), 1.03-1.00 (m, 2H), 0.95-0.90 (m, 2H), 0.75-0.72 (m, 1H), 0.60-0.50 (m, 1H), 0.47-0.44 (m, 2H) ppm. **LC-MS (ESI, m/z):** calcd for [M+H]⁺ 440.1; found: 440.1. **Retention time:** 3.57 min. **[α]_{D}²⁰** = -42.490 (c **=** 0.24, CHCl₃).

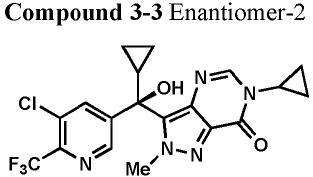

***(R)-3-((5-chloro-6-(trifluoromethyl)pyridin-3-yl)(cyclopropyl)(hydroxy)methyl)-6-cyclopropyl-2-methyl-2,6-dihydro-7H-pyrazolo[4,3-d]pyrimidin-7-one* (3-3) ¹H NMR (400 MHz, DMSO-*d₆*):** *δ* 8.59 (s, 1H), 8.03-8.00 (m, 2H), 6.41 (s, 1H), 3.91 (s, 3H), 3.20-3.15 (m, 1H), 2.35-2.33 (m, 1H), 1.03-1.00 (m, 2H), 0.95-0.90 (m, 2H), 0.75-0.72 (m, 1H), 0.60-0.50 (m, 1H), 0.47-0.44 (m, 2H) ppm. **LC-MS (ESI, m/z):** calcd for [M+H]⁺ 440.1; found: 440.1. **Retention time:** 5.24 min.

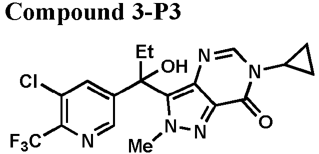

### 3-(1-(5-chloro-6-(trifluoromethyl)pyridin-3-yl)-1-hydroxypropyl)-6-cyclopropyl-2-methyl-2,6-dihydro-7H-pyrazolo[4,3-d]pyrimidin-7-one (3-P3)

Following **General Procedure F3** on 0.18 mmol scale with 3-(5-chloro-6-(trifluoromethyl)nicotinoyl)-6-cyclopropyl-2-methyl-2,6-dihydro-7*H*-pyrazolo[4,3-*d*]pyrimidin-7-one **(3-S7)** and EtMgBr (3M in diethyl ether) (1.0 equiv.). Purification by Combiflash chromatography (75% ethyl acetate in hexane) to afford 3-(1-(5-chloro-6-(trifluoromethyl)pyridin-3-yl)-1-hydroxypropyl)-6-cyclopropyl-2-methyl-2,6-dihydro-7*H-*pyrazolo[4,3-*d*]pyrimidin-7-one **(3-P3)** as an off-white solid (40 mg, 53%). **Physical state:** off-white solid. The racemic compound **3-P3** was purified by preparative-HPLC-Chiral (NP) to afford enantiomer-1 **(3-6)** (15 mg) and enantiomer-2 **(3-5)** (15 mg).

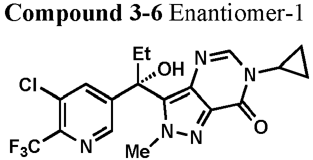

### (S)-3-(1-(5-chloro-6-(trifluoromethyl)pyridin-3-yl)-1-hydroxypropyl)-6-cyclopropyl-2-methyl-2,6-dihydro-7H-pyrazolo[4,3-d]pyrimidin-7-one (3-6)

**¹H NMR (400 MHz, DMSO-*d₆*):** *δ* 8.56 *(d, J=* 1.4 Hz, 1H), 8.08 (s, 1H), 8.03 (s, 1H), 6.71 (s, 1H), 3.90 (s, 3H), 3.18-3.16 (m, 1H), 2.79-2.75 (m, 1H), 2.60-2.57 (m, 1H), 1.03-1.00 (m, 2H), 0.94-0.91 (m, 2H), 0.82 (t, *J =* 7.24 Hz, 3H) ppm. **LC-MS (ESI, m/z):** calcd for [M+H]⁺ 428.1; found: 428.1. **Retention time:** 4.79 min.

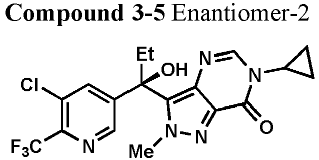

***(R)-3-(1-(5-chloro-6-(trifluoromethyl)pyridin-3-yl)-1-hydroxypropyl)-6-cyclopropyl-2-methyl-2,6-dihydro-7H-pyrazolo[4,3-d]pyrimidin-7-one* (3-5) ¹H NMR (400 MHz, DMSO-d*₆*):** *δ* 8.56 (d, *J=* 1.48 Hz, 1H), 8.08 (s, 1H), 8.03 (s, 1H), 6.71 (s, 1H), 3.90 (s, 3H), 3.18-3.16 (m, 1H), 2.79-2.75 (m, 1H), 2.61-2.57 (m, 1H), 1.03-1.00 (m, 2H), 0.94-0.91 (m, 2H), 0.82 (t, *J =* 7.24 Hz, 3H) ppm. **LC-MS (ESI, m/z):** calcd for [M+H]⁺ 428.1; found: 428.1. **Retention time:** 5.12 min.

### Synthesis of compounds 3-7, 3-8, 3-9, 3-10

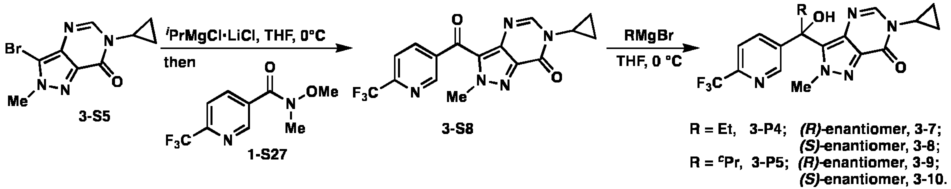

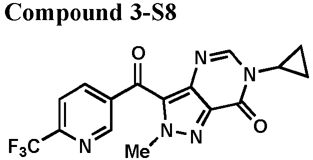

### 6-cyclopropyl-2-methyl-3-(6-(trifluoromethyl)nicotinoyl)-2,6-dihydro-7H-pyrazolo[4,3-d]pyrimidin-7-one (3-S8)

Following **General Procedure** C3 on 4.46 mmol scale with 3-bromo-6-cyclopropyl-2-methyl-2,6-dihydro-7*H*-pyrazolo[4,3-d]pyrimidin-7-one **(3-S5)** and *N*-methoxy-*N*-methyl-6-(trifluoromethyl)nicotinamide **(1-S27).** Purification by Combiflash chromatography (80% ethyl acetate in hexane) to afford 6-cyclopropyl-2-methyl-3-(6-(trifluoromethyl)nicotinoyl)-2,6-dihydro-7H-pyrazolo[4,3-*d*]pyrimidin-7-one **(3-S8)** as an off-white solid (0.22 g, 14%).

**Physical** state: off-white solid. **¹H NMR (400 MHz, CDCl₃):** *δ* 9.13 (s, 1H), 8.37-8.35 (m, 1H), 7.88 (s, 1H), 7.83 (d, *J =* 8.08 Hz, 1H), 4.45 (s, 3H), 3.25-3.19 (m, 1H), 1.28-1.19 (m, 2H), 0.93-0.88 (m, 2H) ppm. **LC-MS (ESI, m/z):** calcd for [M+H]⁺ 364.1; found: 364.2.

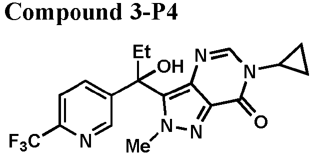

### 6-cyclopropyl-3-(1-hydroxy-1-(6-(trijluoromethyl)pyridin-3-yl)propyl)-2-methyl-2,6-dihydro-7H-pyrazolo[4,3-d]pyrimidin-7-one (3-P4)

Following **General Procedure F3** on 0.58 mmol scale with 6-cyclopropyl-2-methyl-3-(6-(trifluoromethyl)nicotinoyl)-2,6-dihydro-7*H*-pyrazolo[4,3-d]pyrimidin-7-one **(3-S8)** and EtMgBr (3M in diethyl ether) (1.5 equiv.). Purification by Combiflash chromatography (50% ethyl acetate in hexane) to afford 6-cyclopropyl-3-(1-hydroxy-1-(6-(trifluoromethyl)pyridin-3-yl)propyl)-2-methyl-2,6-dihydro-7*H*-pyrazolo[4,3-d]pyrimidin-7-one **(3-P4)** as an off-white solid (60 mg, 26%). **Physical state:** off-white solid.

The racemic compound **3-P4** was purified by preparative-HPLC-Chiral (NP) to afford enantiomer-1 **(3-8)** (15 mg) and enantiomer-2 **(3-7)** (15 mg).

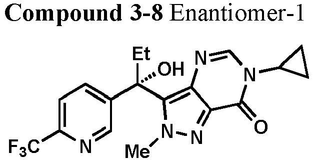

***(S)-6-cyclopropyl-3-(1-hydroxy-1-(6-(trifluoromethyl)pyridin-3-yl)propyl)-2-methyl-2,6-dihydro-7H-pyrazolo[4,3-d]pyrimidin-7-one* (3-8) ¹H NMR (400 MHz, DMSO-*d₆*): *δ*** 8.71 (s, 1H), 8.08 (s, 1H), 7.91 (dd, *J =* 8.2*,* 1.6 Hz, 1H), 7.86 (d, *J =* 8.24 Hz, 1H), 6.57 (s, 1H), 3.85 (s, 3H), 3.21-3.16 (m, 1H), 2.82-2.74 (m, 1H), 2.61-2.54 (m, 1H), 1.08-1.01 (m, 2H), 0.97-0.90 (m, 2H), 0.81 (t, *J =* 7.2 Hz, 3H) ppm. **LC-MS (ESI, m/z):** calcd for [M+H]⁺ 394.1; found: 394.3. **Retention time:** 5.51 min.

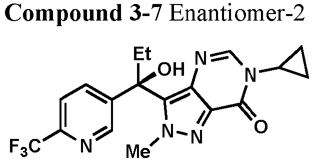

***(R)-6-cyclopropyl-3-(1-hydroxy-1-(6-(trifluoromethyl)pyridin-3-yl)propyl)-2-methyl-2,6-dihydro-7H-pyrazolo[4,3-d]pyrimidin-7-one* (3-7) ¹H NMR (400 MHz, DMSO-*d₆*): *δ* 8.71 (s,** 1H), 8.09 (s, 1H), 7.92-7.85 (m, 2 H), 6.59 (s, 1H), 3.85 (s, 3H), 3.20-3.15 (m, 1H), 2.81-2.76 (m, 1H), 2.61-2.50 (m, 1H), 1.10-1.01 (m, 2H), 0.97-0.90 (m, 2H), 0.81 (t, *J =* 7.2 Hz, 3H) ppm. **LC-MS (ESI, m/z): calcd** for [M+H]⁺ 394.1; found: 394.3. **Retention time:** 6.23 min.

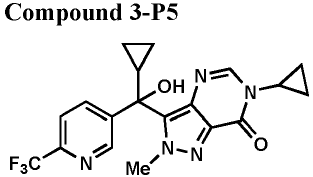

### 6-cyclopropyl-3-(cyclopropyl(hydroxy)(6-(trifluoromethyl)pyridin-3-yl)methyl)-2-methyl-2,6-dihydro-7H-pyrazolo[4,3-d]pyrimidin-7-one (3-P5)

Following **General Procedure F3** on 0.58 mmol scale with 6-cyclopropyl-2-methyl-3-(6-(trifluoromethyl)nicotinoyl)-2,6-dihydro-7*H*-pyrazolo[4,3-d]pyrimidin-7-one **(3-S8)** and cyclopropylmagnesium bromide (1M in 2-methyl THF) (2.5 equiv.). Purification by Combiflash chromatography (50% ethyl acetate in hexane) to afford 6-cyclopropyl-3-(cyclopropyl(hydroxy)(6-(trifluoromethyl)pyridin-3-yl)methyl)-2-methyl-2,6-dihydro-7*H-*pyrazolo[4,3-d]pyrimidin-7-one **(3-P5)** as an off-white solid (70 mg, 30%). **Physical state:** off-white solid.

The racemic compound **3-P5** was purified by preparative-HPLC-Chiral (NP) to afford enantiomer-1 **(3-10)** (30 mg) and enantiomer-2 **(3-9)** (25 mg).

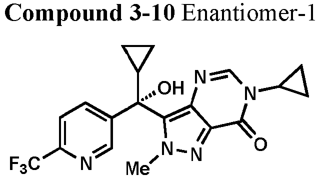

### (S)-6-cyclopropyl-3-(cyclopropyl(hydroxy)(6-(trifluoromethyl)pyridin-3-yl)methyl)-2-methyl-2,6-dihydro-7H-pyrazolo[4,3-d]pyrimidin-7-one (3-10)

**¹H NMR (400 MHz, DMSO-*d₆*):** *δ* 8.70 (s, 1H), 8.02 (s, 1H), 7.89-7.85 (m, 2H), 6.28 (s, 1H), 3.85 (s, 3H), 3.19-3.16 (m, 1H), 2.39-2.37 (m, 1H), 1.05-1.00 (m, 2H), 0.93-0.91 (m, 2H), 0.73-0.69 (m, 1H), 0.56-0.51 (m, 1H), 0.48-0.44 (m, 2H) ppm. **LC-MS (ESI, m/z):** calcd for [M+H]⁺ 406.1; found: 406.33. **Retention time:** 5.36 min.

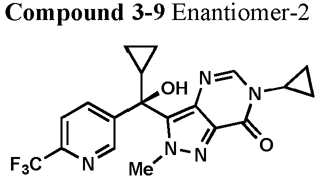

### (R)-6-cyclopropyl-3-(cyclopropyl(hydroxy)(6-(trifluoromethyl)pyridin-3-yl)methyl)-2-methyl-2,6-dihydro-7H-pyrazolo[4,3-d]pyrimidin-7-one (3-9)

**¹H NMR (400 MHz, DMSO-*d₆*): *δ* 8.70** (s, 1H), 8.02 (s, 1H), 7.87-7.85 (m, 2H), 6.28 (s, 1H), 3.85 (s, 3H), 3.19-3.16 (m, 1H), 2.39-2.37 (m, 1H), 1.05-1.00 (m, 2H), 0.93-0.91 (m, 2H), 0.73-0.69 (m, 1H), 0.56-0.51 (m, 1H), 0.48-0.44 (m, 2H) ppm. **LC-MS (ESI,** m/z): calcd for [M+H]⁺ 406.1; found: 406.33. **Retention time:** 6.28 min.

### Synthesis of compounds 3-11, 3-12

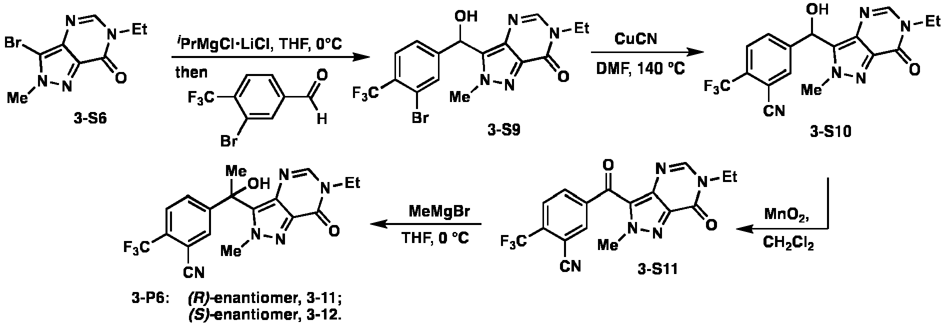

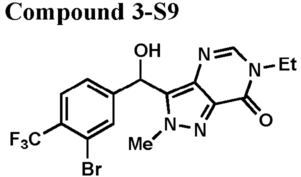

### 3-((3-bromo-4-(trifluoromethyl)phenyl)(hydroxy)methyl)-6-ethyl-2-methyl-2,6-dihydro-7H-pyrazolo[4,3-d]pyrimidin-7-one (3-S9)

Following **General Procedure D3 on** 7.00 mmol scale with 3-bromo-6-ethyl-2-methyl-2,6-dihydro-7*H*-pyrazolo[4,3-d]pyrimidin-7-one **(3-S6)** and 3-bromo-4-(trifluoromethyl)benzaldehyde. Purification by Combiflash chromatography (80% ethyl acetate in hexane) to afford 3-((3-bromo-4-(trifluoromethyl)phenyl)(hydroxy)methyl)-6-ethyl-2-methyl-2,6-dihydro-7*H-*pyrazolo[4,3-*d*]pyrimidin-7-one **(3-S9)** as an off-white solid (2 g, 66%). **Physical state:** off-white solid. **¹H NMR (400 MHz, DMSO-*d₆*):** *δ* 8.10 (s, 1H), 7.89 (s, 1H), 7.82 (d, J= 8.20 Hz, 1H), 7.51 (d, *J =* 8.08 Hz, 1H), 6.80 (d, *J =* 4.76 Hz, 1H), 6.36 (d, *J =* 4.56 Hz, 1H), 4.00-3.92 (m, 5H), 1.24 (t, *J =* 7.08 Hz, 3H) ppm. **LC-MS (ESI, m/z):** calcd for [M+H]⁺ 431.1; found: 430.86, 432.85[M+H+2]⁺.

**¹H NMR (400 MHz, DMSO-*d₆*):** *δ* 8.10 (s, 1H), 7.89 (s, 1H), 7.82 (d, *J =* 8.20 Hz, 1H), 7.51 (d, *J =* 8.08 Hz, 1H), 6.78 (d, *J =* 4.44 Hz, 1H), 6.36 (*br.,* 1H), 4.00-3.92 (m, 5H), 1.24 (t, *J =* 6.84 Hz, 3H) ppm. **LC-MS (ESI, m/z):** calcd for [M+H]⁺ 431.1; found: 431.14, 433.12[M+H+2]⁺.

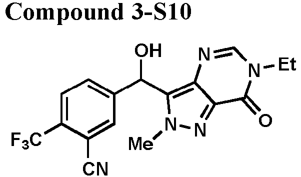

### 5-((6-ethyl-2-methyl-7-oxo-6,7-dihydro-2H-pyrazolo[4,3-d]pyrimidin-3-yl)(hydroxy)methyl)-2-(trifluoromethyl)benzonitrile (3-S10)

In a reaction tube, to a stirred solution of 3-((3-bromo-4-(trifluoromethyl)phenyl)(hydroxy)methyl)-6-ethyl-2-methyl-2,6-dihydro-7H-pyrazolo[4,3-d]-pyrimidin-7-one **(3-S9)** (1 g, 2.32 mmol) in anhydrous DMF (10 mL), CuCN (1.04 g, 11.6 mmol) was added. The tube was sealed and the mixture was stirred at 150 °C for 12 h. After completion, the reaction mixture was extracted with ethyl acetate, washed with aqueous saturated NH₄Cl solution, brine, dried over anhydrous Na₂SO₄ and concentrated. The crude product was purified by Combiflash chromatography (70% ethyl acetate in hexane) to afford 5-((6-ethyl-2-methyl-7-oxo-6,7-dihydro-2*H*-pyrazolo[4,3-*d*]pyrimidin-3-yl)(hydroxy)methyl)-2-(trifluoromethyl)benzo-nitrile **(3-S10)** as an off-white solid (0.4 g, 46%). **Physical state:** off-white solid. **¹H NMR (400 MHz, DMSO-*d₆*):** *δ* 8.13 (s, 1H), 8.06 (s, 1H), 8.01 (d, *J =* 8.20 Hz, 1H), 7.92 (d, *J =* 8.08 Hz, 1H), 6.86 (d, *J =* 4.68 Hz, 1H), 6.40 (d, *J =* 3.92 Hz, 1H), 4.01 (s, 3H), 3.96-3.91 (m, 2H), 1.23 (t, *J =* 6.96 Hz, 3H) ppm. **LC-MS (ESI, m/z):** calcd for [M+H]⁺ 378.1; found: 378.2.

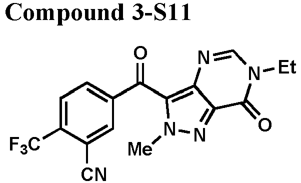

### 5-(6-ethyl-2-methyl-7-oxo-6,7-dihydro-2H-pyrazolo[4,3-d]pyrimidine-3-carbonyl)-2-(trifluoromethyl)benzonitrile (3-S11)

Following **General Procedure E3** on 0.795 mmol scale with 5-((6-ethyl-2-methyl-7-oxo-6,7-dihydro-2*H*-pyrazolo[4,3-d]pyrimidin-3-yl)(hydroxy)methyl)-2-(trifluoromethyl)benzonitrile **(3-S10).** Purification by Combiflash chromatography (30% ethyl acetate in hexane) to afford 5-(6-ethyl-2-methyl-7-oxo-6,7-dihydro-2*H*-pyrazolo[4,3-d]pyrimidine-3-carbonyl)-2-(trifluoromethyl)benzonitrile **(3-S11)** as an off-white solid (290 mg, 97%). **Physical state:** off-white solid. **¹H NMR (400 MHz, DMSO-*d₆*):** *δ* 8.56 (d, *J =* 6.44 Hz, 1H), 8.32 (d, *J* = 8.28 Hz, 1H), 8.22-8.20 (m, 2H), 4.35 (s, 3H), 3.97 (q, *J* = 7.16 Hz, 2H), 1.25 (t, *J=* 7.04 Hz, 3H) ppm. **LC-MS (ESI, m/z):** calcd for [M+H]⁺ 376.1; found: 375.95.

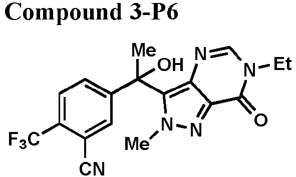

### 5-(1-(6-ethyl-2-methyl-7-oxo-6,7-dihydro-2H-pyrazolo[4,3-d]pyrimidin-3-yl)-1-hydroxyethyl)-2-(trifluoromethyl)benzonitrile (3-P6)

Following **General Procedure F3** on 0.533 mmol scale with 5-(6-ethyl-2-methyl-7-oxo-6,7-dihydro-2*H*-pyrazolo[4,3-d]pyrimidine-3-carbonyl)-2-(trifluoromethyl)benzonitrile **(3-S11)** and MeMgBr (3M in THF) (1.5 equiv.). Purification by Combiflash chromatography (70% ethyl acetate in hexane) to afford 5-(1-(6-ethyl-2-methyl-7-oxo-6,7-dihydro-2*H*-pyrazolo[4,3-*d*]pyrimidin-3-yl)-1-hydroxyethyl)-2-(trifluoromethyl)benzonitrile **(3-P6)** as an off-white solid (100 mg, 48%). **Physical state:** off-white solid.

The racemic compound **3-P6** was submitted for chiral separation to afford enantiomer-1 **(3-12)** (40 mg) and enantiomer-2 **(6-11)** (40 mg).

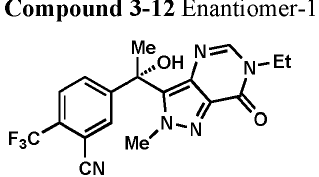

### (S)-5-(1-(6-ethyl-2-methyl-7-oxo-6,7-dihydro-2H-pyrazolo[4,3-d]pyrimidin-3-yl)-1-hydroxyethyl)-2-(trifluoromethyl)benzonitrile (3-12)

**¹H NMR (400 MHz, DMSO-*d₆*):** *δ* 8.18 (s, 1H), 8.05 (s, 1H), 7.97 (d, *J =* 8.0 Hz, 1H), 7.76 (d, *J=* 8.4 Hz, 1H), 6.91 (s, 1H), 4.00 (q, *J =* 6.8 Hz, 2H), 3.77 (s, 3H), 2.16 (s, 3H), 1.27 (t, *J =* 6.8 Hz, 3H) ppm. **LC-MS (ESI, m/z):** calcd for [M+H]⁺ 392.1; found: 392.23. **Retention time:** 7.07 min.

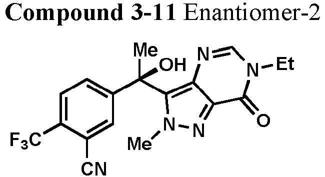

***(R)-5-(1-(6-ethyl-2-methyl-7-oxo-6,7-dihydro-2H-pyrazolo[4,3-d]pyrimidin-3-yl)-1-hydroxyethyl)-2-(trifluoromethyl)benzonitrile* (3-11) ¹H NMR (400 MHz, DMSO-*d₆*):** *δ* 8.18 (s, 1H), 8.05 (s, 1H), 7.97 (d, *J =* 8.4 Hz, 1H), 7.76 (d, *J =* 8.0 Hz, 1H), 6.91 (s, 1H), 4.00 (q, *J* = 7.2 Hz, 2H), 3.77 (s, 3H), 2.16 (s, 3H), 1.28 (t, *J =* 6.8 Hz, 3H) ppm. **LC-MS (ESI, m/z):** calcd for [M+H]⁺ 392.1; found: 392.23. **Retention time:** 9.54 min.

### Synthesis of compounds 4-1 to 4-28

### Synthesis of compounds 4-1, 4-2

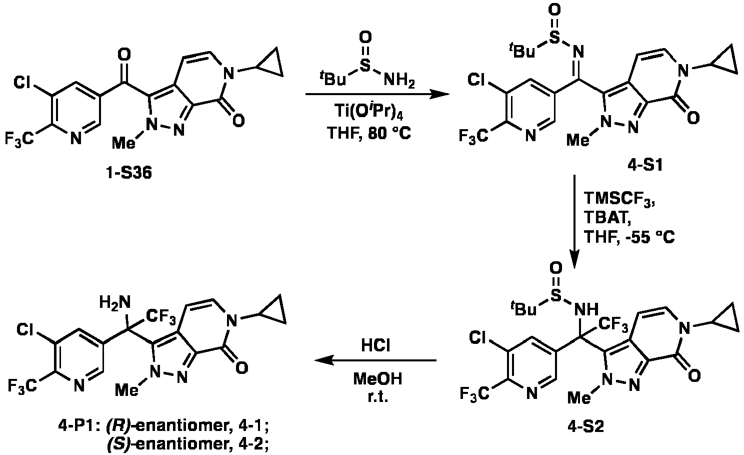

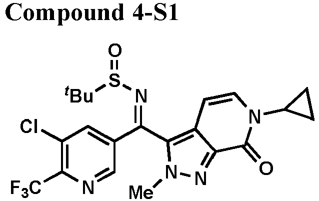

### N-((5-chloro-6-(trifluoromethyl)pyridin-3-yl)(6-cyclopropyl-2-methyl-7-oxo-6,7-dihydro-2H-pyrazolo[3,4-c]pyridin-3-yl)methylene)-2-methylpropane-2-sulfinamide (4-S1)

Following **General Procedure F4a on** 1.26 mmol scale with 3-(5-chloro-6-(trifluoromethyl)nicotinoyl)-6-cyclopropyl-2-methyl-2,6-dihydro-7*H*-pyrazolo[3,4-c]pyridin-7-one **(1-S36).** Purification by CombiFlash chromatography (65% ethyl acetate in hexane) to afford *N*-((5-chloro-6-(trifluoromethyl)pyridin-3-yl)(6-cyclopropyl-2-methyl-7-oxo-6,7-dihydro-2*H*-pyrazolo[3,4-c]pyridin-3-yl)methylene)-2-methylpropane-2-sulfinamide **(4-S1)** as a yellow solid (550 mg, 87%). **Physical State:** yellow solid. **LC-MS (ESI, m/z):** calcd for [M+H]⁺ 500.1; found: 500.1, 499.9.

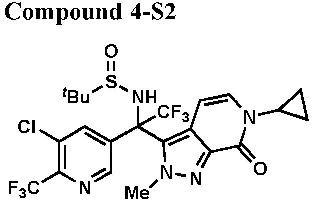

### N-(1-(5-chloro-6-(trifluoromethyl)pyridin-3-yl)-1-(6-cyclopropyl-2-methyl-7-oxo-6,7-dihydro-2H-pyrazolo[3,4-c]pyridin-3-yl)-2,2,2-trifluoroethyl)-2-methylpropane-2-sulfinamide (4-S2)

To a stirred solution of *N*-((5-chloro-6-(trifluoromethyl)pyridin-3-yl)(6-cyclopropyl-2-methyl-7-oxo-6,7-dihydro-2*H*-pyrazolo[3,4-c]pyridin-3-yl)methylene)-2-methylpropane-2-sulfinamide **(4-S1)** (550 mg, 1.1 mmol) in anhydrous THF (5 mL), TMSCF₃ (0.2 mL, 1.32 mmol) followed by TBAT (652 mg, 1.21 mmol) in anhydrous THF (3 mL) was added dropwise at -55 °C and the reaction mixture was stirred at that temperature for 1 h. After completion, the reaction mixture was quenched with aqueous saturated NH₄Cl solution, extracted with ethyl acetate, washed with brine, dried over anhydrous Na₂SO₄ and concentrated. The crude *N*-(1-(5-chloro-6-(trifluoromethyl)pyridin-3-yl)-1-(6-cyclopropyl-2-methyl-7-oxo-6,7-dihydro-2*H-*pyrazolo[3,4-c]pyridin-3-yl)-2,2,2-trifluoroethyl)-2-methylpropane-2-sulfinamide **(4-S2)** was directly taken for the next step (600 mg, crude).

**LC-MS (ESI, m/z):** calcd for [M+H]⁺ 570.1; found: 570.0.

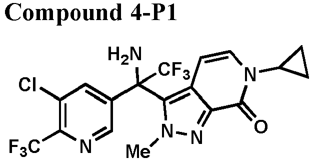

### 3-(1-amino-1-(5-chloro-6-(trifluoromethyl)pyridin-3-yl)-2,2,2-trifluoroethyl)-6-cyclopropyl-2-methyl-2,6-dihydro-7H-pyrazolo[3,4-c]pyridin-7-one (4-P1)

Following **General Procedure H4a** on 1.05 mmol scale with *N*-(1-(5-chloro-6-(trifluoromethyl)pyridin-3-yl)-1-(6-cyclopropyl-2-methyl-7-oxo-6,7-dihydro-2*H-*pyrazolo[3,4-c]pyridin-3-yl)-2,2,2-trifluoroethyl)-2-methylpropane-2-sulfinamide **(4-S2)** and HCl (4M, 10 mL). Purification by CombiFlash chromatography (70% ethyl acetate in hexane) to afford 3-(1-amino-1-(5-chloro-6-(trifluoromethyl)pyridin-3-yl)-2,2,2-trifluoroethyl)-6-cyclopropyl-2-methyl-2,6-dihydro-7*H*-pyrazolo[3,4-c]pyridin-7-one **(4-P1)** as an off-white solid (76 mg, 15%)

The racemic compound **4-P1** was purified from preparative-HPLC-SFC to afford enantiomer-**1 4-2** (25 mg) and enantiomer-2 **4-1(20** mg).

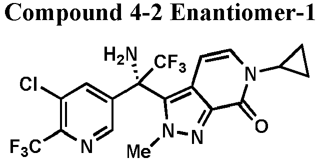

***(S)-3-(1-amino-1-(5-chloro-6-(trifluoromethyl)pyridin-3-yl)-2,2,2-trifluoroethyl)-6-cyclopropyl-2-methyl-2,6-dihydro-7H-pyrazolo[3,4-c]pyridin-7-one* (4-2). ¹H NMR (400 MHz, DMSO-*d₆*):** *δ* 8.52 (s, 1H), 8.32 (s, 1H), 7.03 (d, *J =* 7.8 Hz, 1H), 6.05 (d, *J =* 7.4 Hz, 1H), 3.83 (br s, 5H), 3.27-3.24 (m, 1H), 1.00-0.95 (m, 2H), 0.82-0.79 (m, 2H) ppm. **LC-MS (ESI, m/z):** calcd for [M+H]⁺ 466.1; found: 466.1. **Retention time:** 1.42 min.

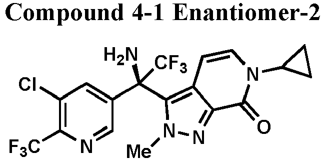

***(R)-3-(1-amino-1-(5-chloro-6-(trifluoromethyl)pyridin-3-yl)-2,2,2-trifluoroethyl)-6-cyclopropyl-2-methyl-2,6-dihydro-7H-pyrazolo[3,4-c]pyridin-7-one* (4-1) ¹H NMR (400 MHz, DMSO-*d₆*):** *δ* 8.52 (s, 1H), 8.32 (s, 1H), 7.03 (d, *J =* 7.8 Hz, 1H), 6.05 (d, *J =* 7.4 Hz, 1H), 3.83 (br s, 5H), 3.27-3.23 (m, 1H), 1.01-0.98 (m, 2H), 0.82-0.78 (m, 2H) ppm. **LC-MS (ESI, m/z):** calcd for [M+H]⁺ 466.1; found: 466.2. **Retention time:** 1.77 min.

### Synthesis of compounds 4-3, 4-4, 4-7, 4-8

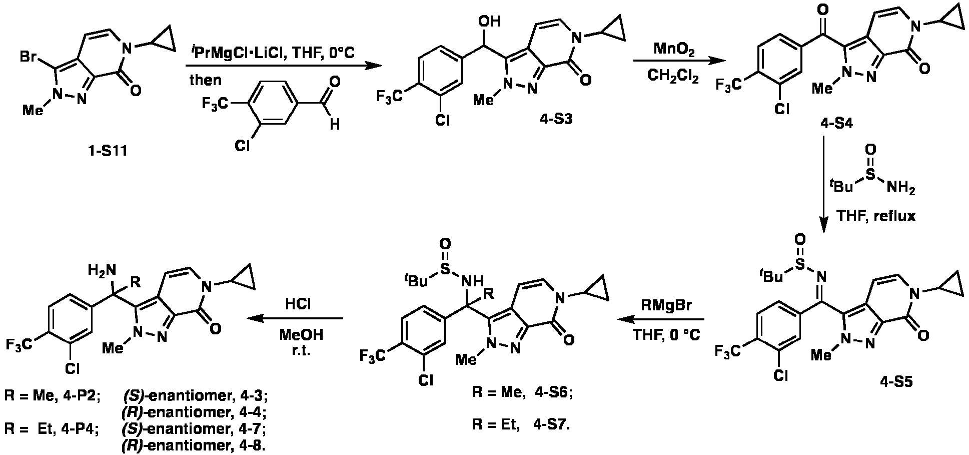

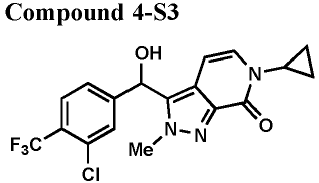

### 3-((3-chloro-4-(trifluoromethyl)phenyl)(hydroxy)methyl)-6-cyclopropyl-2-methyl-2,6-dihydro-7H-pyrazolo[3,4-c]pyridin-7-one (4-S3)

Following **General Procedure D1** on 5.59 mmol scale with 3-bromo-6-cyclopropyl-2-methyl-2,6-dihydro-7*H*-pyrazolo[3,4-c]pyridin-7-one **(1-S11)** and of 3-chloro-4-(trifluoromethyl)benzaldehyde. Purification by CombiFlash chromatography (85% ethyl acetate in hexane) to afford 3-((3-chloro-4-(trifluoromethyl)phenyl)(hydroxy)methyl)-6-cyclopropyl-2-methyl-2,6-dihydro-7*H*-pyrazolo[3,4-c]pyridin-7-one **(4-S3)** as an off-white solid (1 g, 44%). **Physical State:** off-white solid. **¹H NMR (400 MHz, DMSO-*d₆*):** *δ* 7.88 (d, *J=* 8.1 Hz, 1H), 7.72 (s, 1H), 7.52 (d, *J =* 7.9 Hz, 1H), 6.89 (d, *J=* 7.5 Hz, 1H), 6.68 (d, *J=* 7.4 Hz, 1H), 6.33 (br s, 1H), 5.85 (d, *J =* 7.4 Hz, 1H) 4.05 (s, 3H), 3.23-3.19 (m, 1H), 0.95-0.93 (m, 2H), 0.76-0.75 (m, 2H) ppm. **LC-MS (ESI, m/z):** calcd for [M+H]⁺ 398.1; found: 397.8.

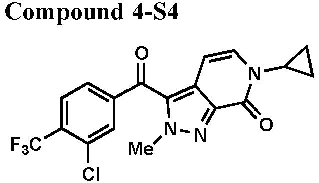

### 3-(3-chloro-4-(trifluoromethyl)benzoyl)-6-cyclopropyl-2-methyl-2,6-dihydro-7H-pyrazolo[3,4-c]pyridin-7-one (4-S4)

Following **General Procedure E1** on 1.76 mmol scale with 3-((3-chloro-4-(trifluoromethyl)phenyl)(hydroxy)methyl)-6-cyclopropyl-2-methyl-2,6-dihydro-7*H-*pyrazolo[3,4-*c*]pyridin-7-one **(4-S3).** Purification by CombiFlash chromatography (70% ethyl acetate in hexane) to afford 3-(3-chloro-4-(trifluoromethyl)benzoyl)-6-cyclopropyl-2-methyl-2,6-dihydro-7*H*-pyrazolo [3,4-*c*]pyridin-7-one **(4-S4)** as a yellow solid (0.65 g, 93%). **Physical State:** yellow solid. **¹H NMR (400 MHz, DMSO-*d₆*):** *δ* 8.10 (d, *J =* 7.9 Hz, 1H), 8.05 (s, 1H), 7.88 (d, *J=* 8.0 Hz, 1H), 7.14 (d, *J=* 7.6 Hz, 1H), 5.50 (d, *J*=7.5 Hz, 1H), 4.31 (s, 3H), 3.26-3.22 (m, 1H), 1.00-0.95 (m, 2H), 0.79 (br s, 2H) ppm. **LC-MS (ESI, m/z):** calcd for [M+H]⁺ 396.1; found: 395.8.

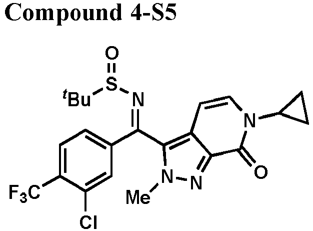

### N-((3-chloro-4-(trifluoromethyl)phenyl)(6-cyclopropyl-2-methyl-7-oxo-6,7-dihydro-2H-pyrazolo[3,4-c]pyridin-3-yl)methylene)-2-methylpropane-2-sulfinamide (4-S5)

Following **General Procedure F4a** on 1.64 mmol scale with 3-(3-chloro-4-(trifluoromethyl)benzoyl)-6-cyclopropyl-2-methyl-2,6-dihydro-7*H*-pyrazolo[3,4-c]pyridin-7-one **(4-S4).** Purification by CombiFlash chromatography (75% ethyl acetate in hexane) to afford N-((3-chloro-4-(trifluoromethyl)phenyl)(6-cyclopropyl-2-methyl-7-oxo-6,7-dihydro-2*H-*pyrazolo-[3,4-c] pyridin-3-yl)methylene)-2-methylpropane-2-sulfinamide **(4-S5)** as a yellow solid (580 mg, 70%). **Physical State:** yellow solid. **LC-MS (ESI, m/z):** calcd for [M+H]⁺ 499.1; found: 499.4.

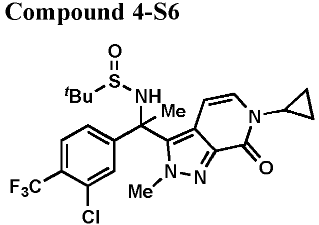

### N-(1-(3-chloro-4-(trifluoromethyl)phenyl)-1-(6-cyclopropyl-2-methyl-7-oxo-6,7-dihydro-2H-pyrazolo[3,4-c]pyridin-3-yl)ethyl)-2-methylpropane-2-sulfinamide (4-S6)

Following **General Procedure G4a** on 0.60 mmol scale with *N*-((3-chloro-4-(trifluoromethyl)phenyl)(6-cyclopropyl-2-methyl-7-oxo-6,7-dihydro-2*H*-pyrazolo[3,4-*c*]pyridin-3-yl)methylene)-2-methylpropane-2-sulfinamide **(4-S5)** and MeMgBr (3 M in THF). After completion, the reaction mixture was quenched with aqueous saturated ammonium chloride solution, extracted with ethyl acetate, washed with brine, dried over anhydrous Na₂SO₄ and concentrated to afford *N*-(1-(3-chloro-4-(trifluoromethyl)phenyl)-1-(6-cyclopropyl-2-methyl-7-oxo-6,7-dihydro-2*H*-pyrazolo[3,4-c]pyridin-3-yl)ethyl)-2-methylpropane-2-sulfinamide **(4-S6)** as a yellow solid, which was taken directly for the next step (250 mg). **LC-MS (ESI, m/z):** calcd for [M+H]⁺ 515.1; found: 515.1.

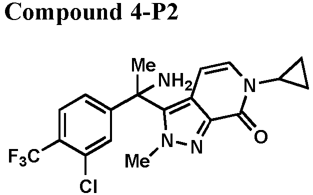

### 3-(1-amino-1-(3-chloro-4-(trifluoromethyl)phenyl)ethyl)-6-cyclopropyl-2-methyl-2,6-dihydro-7H-pyrazolo[3,4-c]pyridin-7-one (4-P2)

Following **General Procedure H4a** on 0.48 mmol scale with *N*-(1-(3-chloro-4-(trifluoromethyl)phenyl)-1-(6-cyclopropyl-2-methyl-7-oxo-6,7-dihydro-2H-pyrazolo[3,4-c]pyridin-3-yl)ethyl)-2-methylpropane-2-sulfinamide **(4-S6)** and HCl (4M, 10 mL). Purification by Combiflash chromatography (70% ethyl acetate in hexane) to afford 3-(1-amino-1-(3-chloro-4-(trifluoromethyl)phenyl)ethyl)-6-cyclopropyl-2-methyl-2,6-dihydro-7H-pyrazolo[3,4-c]pyridin-7-one **(4-P2)** as an off-white solid (86 mg, 43%). **Physical State:** off-white solid.

The racemic compound **4-P2** was purified from Prep-HPLC-SFC chiral to afford enantiomer-1 **(4-4)** (20 mg) and enantiomer-2 **(4-3)** (20 mg).

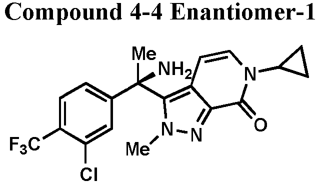

***(R)-3-(1-amino-1-(3-chloro-4-(trifluoromethyl)phenyl)ethyl)-6-cyclopropyl-2-methyl-2,6-dihydro-7H-pyrazolo[3,4-c]pyridin-7-one* (4-4) ¹H NMR (400 MHz, DMSO-*d₆*):** *δ* 7.80 (d, *J* = 8.3 Hz, 1H), 7.73 (s, 1H), 7.28 (d, *J =* 8.2 Hz, 1H), 6.93 (d, *J =* 7.6 Hz, 1H), 6.38 (d, *J =* 7.6 Hz, 1H), 3.72 (s, 3H), 3.27-3.23 (m, 1H), 2.85 (s, 2H), 1.93 (s, 3H), 1.01-0.96 (m, 2H), 0.81-0.77 (m, 2H) ppm. **LC-MS (ESI, m/z):** calcd for [M+H]⁺ 411.1; found: 411.2. **Retention time:** 1.50 min.

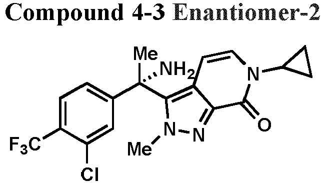

***(S)-3-(1-amino-1-(3-chloro-4-(trifluoromethyl)phenyl)ethyl)-6-cyclopropyl-2-methyl-2,6-dihydro-7H-pyrazolo[3,4-c]pyridin-7-one* (4-3) ¹H NMR (400 MHz, DMSO-*d₆*):** *δ* 7.80 (d, *J* = 8.3 Hz, 1H), 7.73 (s, 1H), 7.28 (d, *J =* 8.2 Hz, 1H), 6.93 (d, *J =* 7.6 Hz, 1H), 6.38 (d, *J =* 7.6 Hz, 1H), 3.72 (s, 3H), 3.27-3.23 (m, 1H), 2.85 (s, 2H), 1.93 (s, 3H), 1.01-0.96 (m, 2H), 0.81-0.77 (m, 2H) ppm. **LC-MS (ESI, m/z):** calcd for [M+H]⁺ 411.1; found: 411.2. **Retention time:** 1.85 min.

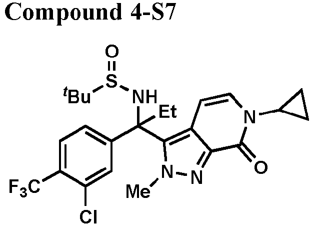

### N-(1-(3-chloro-4-(trifluoromethyl)phenyl)-1-(6-cyclopropyl-2-methyl-7-oxo-6,7-dihydro-2H-pyrazolo[3,4-c]pyridin-3-yl)propyl)-2-methylpropane-2-sulfinamide (4-S7)

Following **General Procedure G4a** on 0.60 mmol scale with *N*-((3-chloro-4-(trifluoromethyl)phenyl)(6-cyclopropyl-2-methyl-7-oxo-6,7-dihydro-2*H*-pyrazolo[3,4-*c*]pyridin-3-yl)methylene)-2-methylpropane-2-sulfinamide **(4-S5)** and EtMgBr (3M in THF). After completion, the reaction mixture was quenched with aqueous saturated NH₄Cl solution, extracted with ethyl acetate, washed with brine, dried over anhydrous Na₂SO₄ and concentrated. The crude *N*-(1-(3-chloro-4-(trifluoromethyl)phenyl)-1-(6-cyclopropyl-2-methyl-7-oxo-6,7-dihydro-2H-pyrazolo[3,4-c]pyridin-3-yl)propyl)-2-methylpropane-2-sulfinamide **(4-S7)** was taken directly for the next step (214 mg, 67%). **LC-MS (ESI, m/z):** calcd for [M+H]⁺ 529.1; found: 529.4.

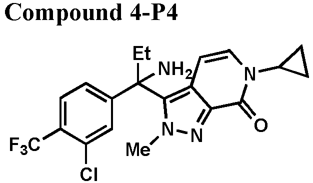

### 3-(1-amino-1-(3-chloro-4-(trifluoromethyl)phenyl)propyl)-6-cyclopropyl-2-methyl-2,6-dihydro-7H-pyrazolo[3,4-c]pyridin-7-one (4-P4)

Following **General Procedure G4a on** 0.47 mmol scale with *N*-(1-(3-chloro-4-(trifluoromethyl)phenyl)-1-(6-cyclopropyl-2-methyl-7-oxo-6,7-dihydro-2*H*-pyrazolo[3,4-*c*]pyridin-3-yl)propyl)-2-methylpropane-2-sulfinamide **(4-S7)** and methanolic HCl (4M, 5 mL). Purification by CombiFlash chromatography (85% ethyl acetate in hexane) to afford 3-(1-amino-1-(3-chloro-4-(trifluoromethyl)phenyl)propyl)-6-cyclopropyl-2-methyl-2,6-dihydro-7H-pyrazolo[3,4-c]pyridin-7-one **(4-P4)** as an off-white solid (60 mg, 35%). **Physical State:** off-white solid.

The racemic compound **4-P4** was purified from preparative-HPLC-Chiral (SFC) to afford enantiomer-1 **(4-8)** (20 mg) and enantiomer-2 **(4-7)** (20 mg).

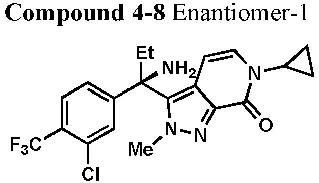

***(R)-3-(1-amino-1-(3-chloro-4-(trifluoromethyl)phenyl)propyl)-6-cyclopropyl-2-methyl-2,6-dihydro-7H-pyrazolo[3,4-c]pyridin-7-one* (4-8) ¹H NMR (400 MHz, DMSO-*d₆*):** *δ* 7.79 (d, *J* = 8.0 Hz, 1H), 7.65 (s, 1H), 7.25 (d, *J =* 8.0 Hz, 1H), 6.94 (d, *J =* 7.7 Hz, 1H), 6.47 (d, *J =* 7.7 Hz, 1H), 3.70 (s, 3H), 3.26-3.22 (m, 1H), 2.70-2.67 (m, 2H), 2.45-2.40 (m, 1H), 2.32-2.23 (m, 1H), 1.01-0.97 (m, 2H ), 0.82-0.74 (m, 5H) ppm. **LC-MS (ESI, m/z):** calcd for [M+H]⁺ 425.1; found: **425.1. Retention time:** 5.15 min.

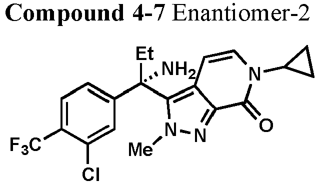

***(S)-3-(1-amino-1-(3-chloro-4-(trifluoromethyl)phenyl)propyl)-6-cyclopropyl-2-methyl-2,6-dihydro-7H-pyrazolo[3,4-c]pyridin-7-one* (4-7) ¹H NMR (400 MHz, DMSO-*d₆*): *δ*** 7.79 (d, *J* = 8.0 Hz, 1H), 7.65 (s, 1H), 7.25 (d, *J= 8.0* Hz, 1H), 6.94 (d, *J=* 7.7 Hz, 1H), 6.47 (d, *J= 7.7* Hz, 1H), 3.70 (s, 3H), 3.26-3.22 (m, 1H), 2.70-2.67 (m, 2H), 2.45-2.40 (m, 1H), 2.32-2.23 (m, 1H), 1.01-0.97 (m, 2H ), 0.82-0.74 (m, 5H) ppm. **LC-MS (ESI, m/z):** calcd for [M+H]⁺ 425.1; found: 425.1. **Retention time:** 6.35 min.

### Synthesis of compounds 4-5, 4-6

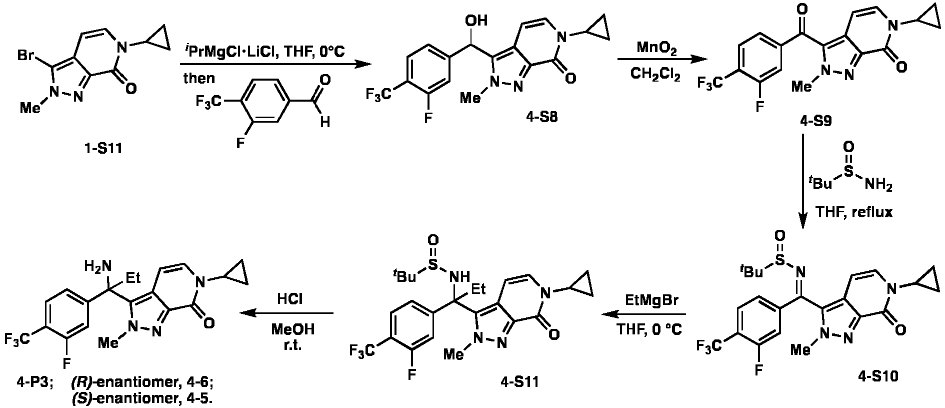

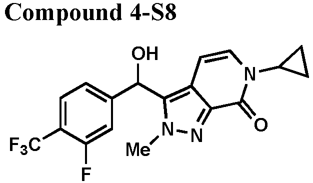

### 6-cyclopropyl-3-((3-fluoro-4-(trifluoromethyl)phenyl)(hydroxy)methyl)-2-methyl-2,6-dihydro-7H-pyrazolo[3,4-c]pyridin-7-one (4-S8)

Following **General Procedure D1** on 2.24 mmol scale with 3-bromo-6-cyclopropyl-2-methyl-2,6-dihydro-7*H*-pyrazolo[3,4-c]pyridin-7-one **(1-S11)** and 3-fluoro-4-(trifluoromethyl)benzaldehyde. Purification by CombiFlash chromatography (75% ethyl acetate in hexane) to afford 6-cyclopropyl-3-((3-fluoro-4-(trifluoromethyl)phenyl)(hydroxy)methyl)-2-methyl-2,6-dihydro-7*H*-pyrazolo[3,4-c]pyridin-7-one **(4-S8)** as an off-white solid (450 mg, 53%). **Physical State:** off-white solid. **¹H NMR (400 MHz, DMSO-*d₆*):** *δ* 7.80 (t, *J =* 7.8 Hz, 1H), 7.52 (d, *J =* 11.8 Hz, 1H), 7.38 (d, *J =* 8.0 Hz, 1H), 6.89 (d, *J =* 7.4 Hz, 1H), 6.68 (d, *J =* 4.76 Hz, 1H), 6.33 (br s, 1H), 5.87 (d, *J=* 7.4 Hz, 1H), 4.06 (s, 3H), 3.23-3.18 (m, 1H), 1.08-0.93 (m, 2H), 0.86-0.75 (m, 2H) ppm. **LC-MS (ESI, m/z):** calcd for [M+H]⁺ 382.1; found: 382.1.

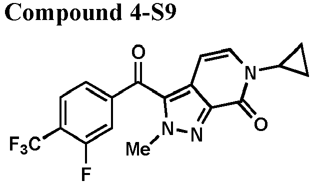

### 6-cyclopropyl-3-(3-fluoro-4-(trifluoromethyl)benzoyl)-2-methyl-2,6-dihydro-7H-pyrazolo[3,4-c]pyridin-7-one (4-S9)

Following **General Procedure E1** on 1.19 mmol scale with 6-cyclopropyl-3-((3-fluoro-4-(trifluoromethyl)phenyl)(hydroxy)methyl)-2-methyl-2,6-dihydro-7*H*-pyrazolo[3,4-c]pyridin-7-one **(4-S8).** After completion, the reaction mixture was filtered through Celite bed, washed with dichloromethane and the filtrate was concentrated to afford 6-cyclopropyl-3-(3-fluoro-4-(trifluoromethyl)benzoyl)-2-methyl-2,6-dihydro-7*H*-pyrazolo[3,4-c]pyridin-7-one **(4-S9)** as a yellow solid (400 mg, 89%). **Physical State:** yellow solid. **¹H NMR (400 MHz, DMSO-*d₆*):** *δ* 8.06-8.02 (m, 1H), 7.88 (*d, J =* 10.8 Hz, 1H), 7.77 (d, *J =* 8.0 Hz, 1H), 7.13 (d, *J=* 7.5 Hz, 1H), 5.51 (d, *J=* 7.5 Hz, 1H), 4.30 (s, 3H), 3.29-3.25 (m, 1H), 1.00-0.95 (m, 2H), 0.80-0.75 (m, 2H) ppm. **LC-MS (ESI, m/z):** calcd for [M+H]⁺ 380.1; found: 380.1.

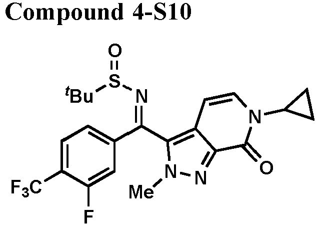

### N-((6-cyclopropyl-2-methyl-7-oxo-6,7-dihydro-2H-pyrazolo[3,4-c]pyridin-3-yl)(3-fluoro-4-(trifluoromethyl)phenyl)methylene)-2-methylpropane-2-sulfinamide (4-S10)

Following **General Procedure G4a** on 1.05 mmol scale with 6-cyclopropyl-3-(3-fluoro-4-(trifluoromethyl)benzoyl)-2-methyl-2,6-dihydro-7*H*-pyrazolo[3,4-c]pyridin-7-one **(4-S9).** Purification by CombiFlash chromatography (70% ethyl acetate in hexane) to afford N-((6-cyclopropyl-2-methyl-7-oxo-6,7-dihydro-2*H*-pyrazolo[3,4-c]pyridin-3-yl)(3-fluoro-4-(trifluoromethyl)phenyl)methylene)-2-methylpropane-2-sulfinamide **(4-S10)** as a yellow solid (500 mg, 98%). **Physical State:** yellow solid. **LC-MS (ESI, m/z):** calcd for [M+H]⁺ 483.1; found: 482.8.

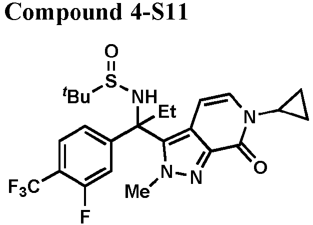

### N-(1-(6-cyclopropyl-2-methyl-7-oxo-6,7-dihydro-2H-pyrazolo[3,4-c]pyridin-3-yl)-1-(3-fluoro-4-(trifluoromethyl)phenyl)propyl)-2-methylpropane-2-sulfinamide (4-S11)

Following **General Procedure G4a** on 0.52 mmol scale with *N*-((6-cyclopropyl-2-methyl-7-oxo-6,7-dihydro-2*H*-pyrazolo[3,4-c]pyridin-3-yl)(3-fluoro-4-(trifluoromethyl)phenyl)methylene)-2-methylpropane-2-sulfinamide **(4-S10)** and EtMgBr (3 M in THF). After completion, the reaction mixture was quenched with aqueous saturated NH₄Cl solution, extracted with ethyl acetate, washed with brine, dried over anhydrous Na₂SO₄ and concentrated. The crude *N*-(1-(6-cyclopropyl-2-methyl-7-oxo-6,7-dihydro-2H-pyrazolo[3,4-*c*]pyridin-3-yl)-1-(3-fluoro-4-(trifluoromethyl)phenyl)propyl)-2-methylpropane-2-sulfinamide **(4-S11)** was taken directly for the next step (250 mg, crude). **LC-MS (ESI,** m/z): calcd for [M+H]⁺ 513. 1; found: 512.9.

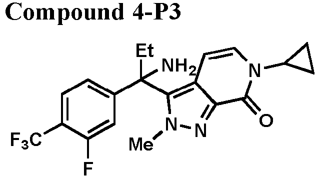

### 3-(1-amino-1-(3-fluoro-4-(trifluoromethyl)phenyl)propyl)-6-cyclopropyl-2-methyl-2,6-dihydro-7H-pyrazolo[3,4-c]pyridin-7-one (4-P3)

**Following General Procedure H4a on** 0.49 mmol scale with *N*-(1-(6-cyclopropyl-2-methyl-7-oxo-6,7-dihydro-2*H*-pyrazolo[3,4-c]pyridin-3-yl)-1-(3-fluoro-4-(trifluoromethyl)phenyl)propyl)-2-methylpropane-2-sulfinamide **(4-S11)** and methanolic HCl (4M, 5 mL). Purification by CombiFlash chromatography (80% ethyl acetate in hexane) to afford 3-(1-amino-1-(3-fluoro-4-(trifluoromethyl)phenyl)propyl)-6-cyclopropyl-2-methyl-2,6-dihydro-7*H*-pyrazolo[3,4-*c*]pyridin-7-one **(4-P3)** as an off-white solid (60 mg, 30%). **Physical State:** off-white solid.

The racemic compound **4-P3** was purified from preparative-HPLC-Chiral (SFC) to afford enantiomer-1 **(4-6)** (12 mg) and enantiomer-2 **(4-5)** (12 mg).

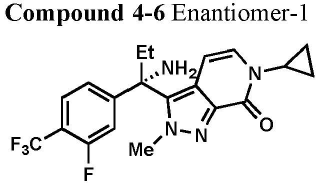

***(S)-3-(1-amino-1-(3-fluoro-4-(trifluoromethyl)phenyl)propyl)-6-cyclopropyl-2-methyl-2,6-dihydro-7H-pyrazolo[3,4-c]pyridin-7-one* (4-6) ¹H NMR (400 MHz, DMSO-*d₆*): *δ*** 7.72 (t, *J* = 7.9 Hz, 1H), 7.46 (d, *J=* 12.6 Hz, 1H), 7.11 (d, *J =* 8.2 Hz, 1H), 6.94 (d, *J =* 7.6 Hz, 1H), 6.48 (d, *J=* 7.7 Hz, 1H), 3.70 (s, 3H), 3.25-3.23 (m, 1H), 2.70-2.65 (m, 2H), 2.45-2.39 (m, 1H), 2.30-2.26 (m, 1H), 1.00-0.90 (m, 2H), 0.82-0.74 (m, 5H) ppm. **LC-MS (ESI, m/z):** calcd for [M+H]⁺ 409.1; found: 409.5. **Retention time:** 2.22 min.

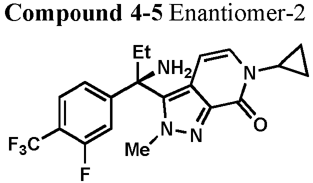

### (R)-3-(1-amino-1-(3-fluoro-4-(trifluoromethyl)phenyl)propyl)-6-cyclopropyl-2-methyl-2,6-dihydro-7H-pyrazolo[3,4-c]pyridin-7-one (4-5)

**¹H NMR (400 MHz, DMSO-*d₆*):** *δ* 7.72 (t, *J* = 7.9 Hz, 1H), 7.46 (d, *J* = 12.6 Hz, 1H), 7.11 (d, *J =* 8.2 Hz, 1H), 6.94 (d, *J =* 7.6 Hz, 1H), 6.48 (d, *J=* 7.7 Hz, 1H), 3.70 (s, 3H), 3.26-3.22 (m, 1H), 2.70-2.65 (m, 2H), 2.42-2.38 (m, 1H), 2.29-2.25 (m, 1H), 1.00-0.90 (m, 2H), 0.82-0.74 (m, 5H) ppm. **LC-MS (ESI, m/z):** calcd for [M+H]⁺ 409.1; found: 409.5. **Retention time:** 2.87 min.

### Synthesis of compounds 4-9, 4-10, 4-11, 4-12

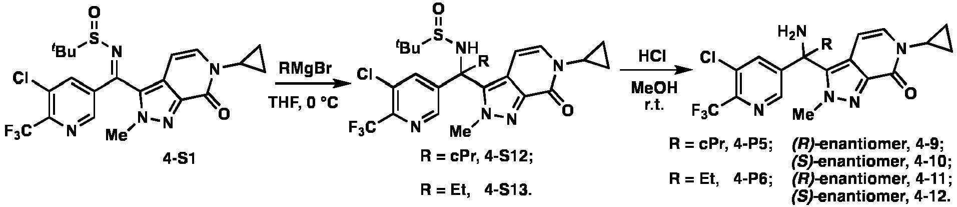

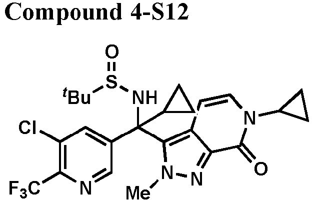

### N-((5-chloro-6-(trifluoromethyl)pyridin-3-yl)(cyclopropyl)(6-cyclopropyl-2-methyl-7-oxo-6, 7-dihydro-2H-pyrazolo[3,4-c]pyridin-3-yl)methyl)-2-methylpropane-2-sulfinamide (4-S12)

Following **General Procedure G4a** on 0.60 mmol scale with *N*-((5-chloro-6-(trifluoromethyl)pyridin-3-yl)(6-cyclopropyl-2-methyl-7-oxo-6,7-dihydro-2*H*-pyrazolo[3,4-*c*]pyridin-3-yl)methylene)-2-methylpropane-2-sulfinamide **(4-S1)** and cyclopropyl magnesium bromide (0.7 M in THF). After completion, the reaction mixture was quenched with aqueous saturated NH₄Cl solution, extracted with ethyl acetate, washed with brine, dried over anhydrous Na₂SO₄ and concentrated. The crude *N*-((5-chloro-6-(trifluoromethyl)pyridin-3-yl)(cyclopropyl)(6-cyclopropyl-2-methyl-7-oxo-6,7-dihydro-2*H*-pyrazolo[3,4-c]pyridin-3-yl)methyl)-2-methylpropane-2-sulfinamide **(4-S12)** (320 mg, crude) was taken directly for the next step. **LC-MS (ESI, m/z): calcd** for [M+H]⁺ 542.1; found: 542.0.

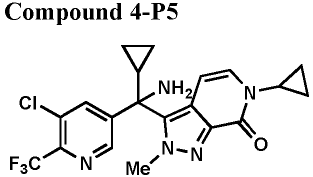

### 3-(amino(5-chloro-6-(trifluoromethyl)pyridin-3-yl)(cyclopropyl) methyl)-6-cyclopropyl-2-methyl-2,6-dihydro-7H-pyrazolo[3,4-c]pyridin-7-one (4-P5)

Following **General Procedure H4a on 0.59 mmol scale with** *N*-((5-chloro-6-(trifluoromethyl)pyridin-3-yl)(cyclopropyl)(6-cyclopropyl-2-methyl-7-oxo-6,7-dihydro-2*H-*pyrazolo[3,4-c]pyridin-3-yl)methyl)-2-methylpropane-2-sulfinamide **(4-S12)** and methanolic HCl (4M, 10 mL). Purification by CombiFlash chromatography (80% ethyl acetate in hexane) to afford 3-(amino(5-chloro-6-(trifluoromethyl)pyridin-3-yl)(cyclopropyl)methyl)-6-cyclopropyl-2-methyl-2,6-dihydro-7H-pyrazolo[3,4-c]pyridin-7-one as an off-white solid **(4-P5)** (18 mg, 19%). **Physical State:** off-white solid.

The racemic compound **4-P5** was purified from Preparative-HPLC-Chiral (NP) to afford enantiomer-1 **(4-10)** (4 mg) and enantiomer-2 **(4-9)** (4 mg).

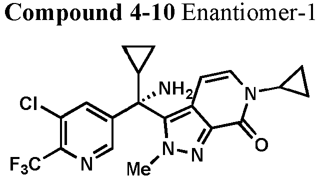

***(S)-3-(amino(5-chloro-6-(trifluoromethyl)pyridin-3-yl)(cyclopropyl) methyl)-6-cyclopropyl-2-methyl-2,6-dihydro-7H-pyrazolo[3,4-c]pyridin-7-one* (4-10) ¹H NMR (400 MHz, DMSO-**d*₆*): *δ* 8.46 (s, 1 H), 8.09 (s, 1 H), 6.93 (d, *J =* 7.4 Hz, 1 H), 6.56 (d, *J* =7.7 Hz, 1 H), 3.74 (s, 3 H), 3.30-3.25 (m, 1 H), 2.98-2.85 (m, 2 H), 1.79 (br s, 1 H), 1.00-0.98 (m, 2 H), 0.90-0.75 (m, 3 H), 0.70-0.60 (m, 2 H), 0.49-0.45 (m, 1 H) ppm. **LC-MS (ESI, m/z):** calcd for [M+H]⁺ 438.1; found: 438.0. **Retention time:** 5.75 min.

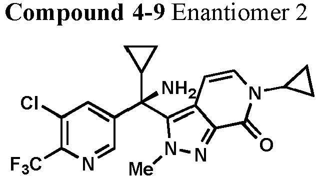

***(R)-3-(amino(5-chloro-6-(trifluoromethyl)pyridin-3-yl)(cyclopropyl)methyl)-6-cyclopropyl-2-methyl-2,6-dihydro-7H-pyrazolo[3,4-c]pyridin-7-one* (4-9) ¹H NMR (400 MHz, DMSO-d*₆*):** *δ* 8.46 (s, 1 H), 8.09 (s, 1 H), 6.93 (d, *J=* 7.4 Hz, 1 H), 6.56 (d, *J* =7.7 Hz, 1 H), 3.74 (s, 3 H), 3.30-3.25 (m, 1 H), 2.98-2.85 (m, 2 H), 1.79 (br s, 1 H), 1.00-0.98 (m, 2 H), 0.90-0.75 (m, 3 H), 0.70-0.60 (m, 2 H), 0.49-0.45 (m, 1 H) ppm. **LC-MS (ESI, m/z):** calcd for [M+H]⁺ 438.1; found: 438.0. **Retention time:** 7.35 min.

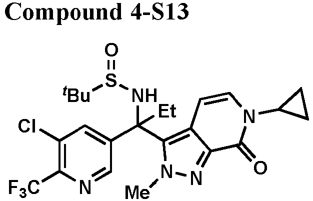

### N-(1-(5-chloro-6-(trifluoromethyl)pyridin-3-yl)-1-(6-cyclopropyl-2-methyl-7-oxo-6,7-dihydro-2H-pyrazolo[3,4-c]pyridin-3-yl)propyl)-2-methylpropane-2-sulfinamide (4-S13)

Following **General Procedure G4a** on 0.50 mmol scale with *N*-((5-chloro-6-(trifluoromethyl)pyridin-3-yl)(6-cyclopropyl-2-methyl-7-oxo-6,7-dihydro-2*H*-pyrazolo[3,4-c]pyridin-3-yl)methylene)-2-methylpropane-2-sulfinamide **(4-S1)** and EtMgBr (3 M in THF). After completion, the reaction mixture was quenched with aqueous saturated NH₄Cl solution, extracted with ethyl acetate, washed with brine, dried over anhydrous Na₂SO₄ and concentrated.

The crude *N*-(1-(5-chloro-6-(trifluoromethyl)pyridin-3-yl)-1-(6-cyclopropyl-2-methyl-7-oxo-6,7-dihydro-2H-pyrazolo[3,4-*c*]pyridin-3-yl)propyl)-2-methylpropane-2-sulfinamide **(4-S13)** (220 mg, crude) was taken directly for the next step. **LC-MS (ESI, m/z):** calcd for [M+H]⁺ 530.1; found: 530.2.

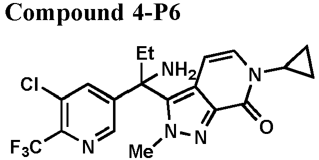

### 3-(1-amino-1-(5-chloro-6-(trifluoromethyl)pyridin-3-yl)propyl)-6-cyclopropyl-2-methyl-2,6-dihydro-7H-pyrazolo[3,4-c]pyridin-7-one (4-P6)

Following **General Procedure H4a** on 0.42 mmol scale with *N*-(1-(5-chloro-6-(trifluoromethyl)pyridin-3-yl)-1-(6-cyclopropyl-2-methyl-7-oxo-6,7-dihydro-2*H-*pyrazolo[3,4-*c*]pyridin-3-yl)propyl)-2-methylpropane-2-sulfinamide **(4-S13)** and methanolic HCl (4M, 10 mL). Purification by CombiFlash chromatography (80% ethyl acetate in hexane) to afford 3-(1-amino-1-(5-chloro-6-(trifluoromethyl)pyridin-3-yl)propyl)-6-cyclopropyl-2-methyl-2,6-dihydro-7*H*-pyrazolo[3,4-c]pyridin-7-one **(4-P6)** as an off-white solid (65 mg, 37%). **Physical State:** off-white solid.

The racemic compound **4-P6** was purified from Preparative-HPLC-Chiral (NP) to afford enantiomer-1 **(4-12)** (8 mg) and enantiomer-2 **(4-11)** (8 mg).

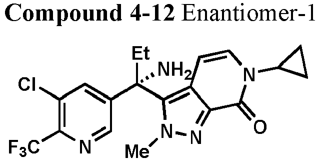

### (S)-3-(1-amino-1-(5-chloro-6-(trifluoromethyl)pyridin-3-yl)propyl)-6-cyclopropyl-2-methyl-2,6-dihydro-7H-pyrazolo[3,4-c]pyridin-7-one (4-12)

**¹H NMR (400 MHz, DMSO-*d₆*):** *δ* 8.48 (d, *J* = 1.5 Hz, 1H), 8.10 (s, 1H), 6.93 (d, *J* = 7.7 Hz, 1H), 6.38 (d, *J =* 7.7 Hz, 1H), 3.79 (s, 3H), 3.29-3.20 (m, 1H), 2.81 (br s, 2H), 2.49-2.44 (m, 2H), 1.00-0.97 (m, 2H), 0.80-0.76 (m, 5H) ppm. **LC-MS (ESI, m/z):** calcd for [M+H]⁺ 426.1; found: 426.1. **Retention time:** 5.72 min.

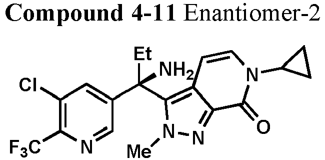

***(R)-3-(1-amino-1-(5-chloro-6-(trifluoromethyl)pyridin-3-yl)propyl)-6-cyclopropyl-2-methyl-2,6-dihydro-7H-pyrazolo[3,4-c]pyridin-7-one*** (**4-11) ¹H NMR (400 MHz, DMSO-*d₆*):** *δ* 8.48 *(d, J= 1.5 Hz,* 1H), 8.10 (s, 1H), 6.93 (d, *J* = 7.7 Hz, 1H), 6.38 (d, *J =* 7.7 Hz, 1H), 3.79 (s, 3H), 3.29-3.20 (m, 1H), 2.81 (br s, 2H), 2.49-2.44 (m, 2H), 1.00-0.97 (m, 2H), 0.80-0.76 (m, 5H) ppm. **LC-MS (ESI, m/z):** calcd for [M+H]⁺ 426.1; found: 426.1. **Retention time:** 8.04 min.

### Synthesis of compounds 4-13, 4-14

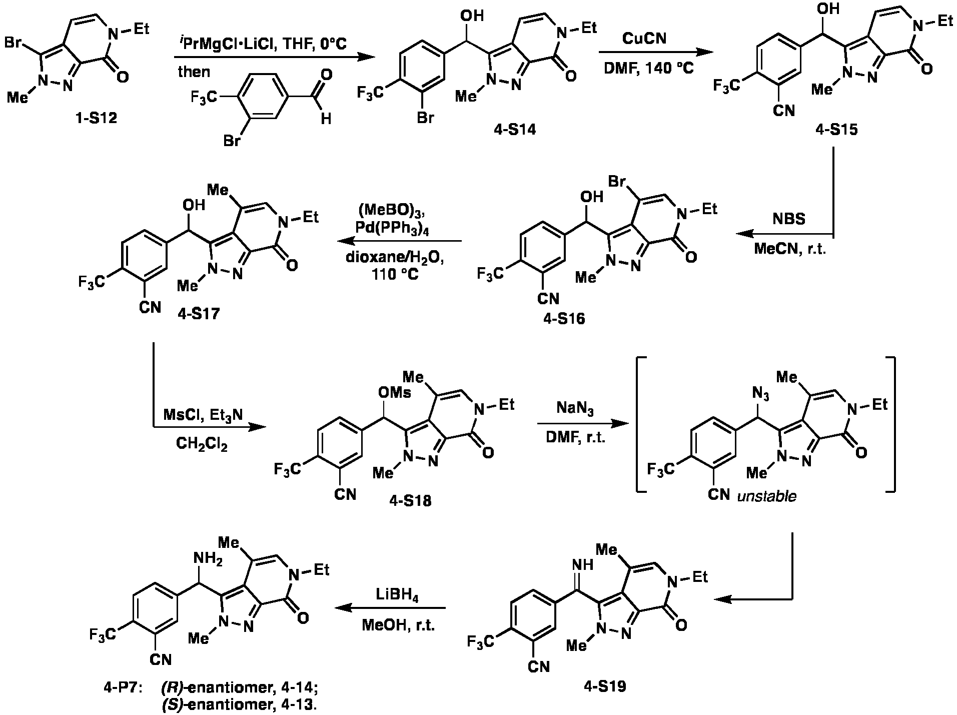

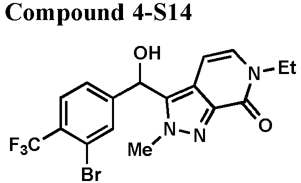

### 3-((3-bromo-4-(trifluoromethyl)phenyl)(hydroxy)methyl)-6-ethyl-2-methyl-2,6-dihydro-7H-pyrazolo[3,4-c]pyridin-7-one (4-S14)

Following **General Procedure D1** on 7.81 mmol scale with 3-bromo-6-ethyl-2-methyl-pyrazolo[3,4-*c*]pyridin-7-one **(1-S12)** and 3-bromo-4-(trifluoromethyl)benzaldehyde. Purification by column chromatography (SiO₂ 100-200, 80% ethyl acetate in hexane) to afford 3-((3-bromo-4-(trifluoromethyl)phenyl)(hydroxy)methyl)-6-ethyl-2-methyl-2,6-dihydro-7*H-*pyrazolo[3,4-c]-pyridin-7-one **(4-S14)** as an off-white solid (2.6 g, 77%). **Physical State:** off-white solid. **¹H NMR (400 MHz, DMSO-*d₆*):** *δ* 7.91-7.86 (m, 2 H), 7.57 (d, *J =* 7.84 Hz, 1 H), 7.02 (d, *J =* 7.12 Hz, 1 H), 6.68 (d, *J =* 4.52 Hz, 1 H), 6.34-6.33 (m, 1 H), 5.90 (d, *J =* 7.4 Hz, 1 H), 4.03 (s, 3 H), 3.92-3.87 (m, 2 H), 1.22-1.17 (m, 3 H) ppm. **LC-MS (ESI, m/z):** calcd for [M+H]⁺ 430.0; found: 429.9.

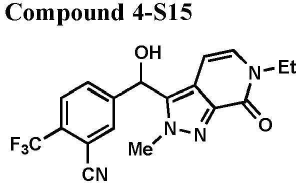

### 5-((6-ethyl-2-methyl-7-oxo-6,7-dihydro-2H-pyrazolo[3,4-c]pyridin-3-yl)(hydroxy)methyl)-2-(trifluoromethyl)benzonitrile (4-S15)

To a stirred solution of 3-{[3-bromo-4-(trifluoromethyl)phenyl](hydroxy)methyl}-6-ethyl-2-methyl-2*H*,6*H*,7*H*-pyrazolo[3,4-c]pyridin-7-one **(4-S14)** (5.81 mmol, 1.0 equiv.) in DMF (0.15 M), CuCN (5.0 equiv.) was added and the reaction mixture was stirred at 140-150 °C for 10-14 h. After completion, the reaction mixture was cooled, extracted with 20% 2-propanol in dichloromethane, washed with water, brine, dried over anhydrous Na₂SO₄ and concentrated. The crude product was purified by column chromatography (SiO₂ 100-200, 80% ethyl acetate in hexane) to afford 5-((6-ethyl-2-methyl-7-oxo-6,7-dihydro-2H-pyrazolo[3,4-c]pyridin-3-yl)(hydroxy)methyl)-2-(trifluoromethyl)benzonitrile **(4-S15)** as an off-white solid (1.42 g, 65%). **Physical State:** off-white solid. **¹H NMR (400 MHz, DMSO-*d₆*):** *δ* 8.16 (s, 1 H), 8.05 (d, *J =* 7.84 Hz, 1 H), 7.92 (d, *J =* 8.12 Hz, 1 H), 7.01 (d, *J =* 7.56 Hz, 1 H), 6.78 (d, *J =* 4.12 Hz, 1 H), 6.36 (br s, 1 H), 5.93 (d, *J =* 7.2 Hz, 1 H), 4.04 (s, 3 H), 3.88 (q, *J =* 7.12 Hz, 2 H), 1.17 (t, *J=* 6.8 Hz, 3 H) ppm. LC-MS (ESI, m/z): calcd for [M+H]⁺ 377.1; found: 377.18.

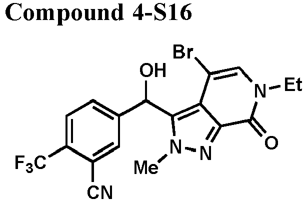

### 5-({4-bromo-6-ethyl-2-methyl-7-oxo-2H,6H,7H-pyrazolo[3,4-c]pyridin-3-yl}(hydroxy)-methyl)-2-(trifluoromethyl)benzonitrile (4-S16)

To a stirred solution of 5-((6-ethyl-2-methyl-7-oxo-6,7-dihydro-2H-pyrazolo[3,4-c]pyridin-3-yl)(hydroxy)methyl)-2-(trifluoromethyl)benzonitrile **(4-S15)** (3.77 mmol, 1.0 equiv.) in acetonitrile (0.1 M), NBS (1.2 equiv.) was added and the mixture was stirred at room temperature for 2 h. After completion, ice cold water was added to the reaction mixture and an off white solid precipitate was appeared, which was filtered and dried to afford 5-({4-bromo-6-ethyl-2-methyl-7-oxo-2*H*,6*H*,7*H*-pyrazolo[3,4-*c*]pyridin-3-yl}(hydroxy)methyl)-2-(trifluoromethyl)-benzonitrile **(4-S16)** as an off-white solid (1.2 g, 70%). **Physical State:** off-white solid. **¹H NMR (400 MHz, DMSO-*d₆*):** *δ* 8.06 (s, 1 H), 8.01 (d, *J =* 8.24 Hz, 1 H), 7.76 (d, *J=* 7.8 Hz, 1 H), 7.59 (br s, 1 H), 7.15 (d, *J =* 3.56 Hz, 1 H), 6.94 (br s, 1 H), 4.02-3.95 (m, 2 H), 3.80 (s, 3 H), 1.25-1.15 (m, 3 H) ppm. **LC-MS (ESI, m/z):** calcd for [M+H]⁺ 455.0; found: 455.30, [M+2+H] 457.29.

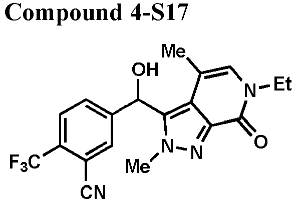

### 5-({6-ethyl-2,4-dimethyl-7-oxo-2H,6H,7H-pyrazolo[3,4-c]pyridin-3-yl}(hydroxy)methyl)-2-(trifluoromethyl)benzonitrile (4-S17)

In a reaction tube, to a stirred solution of 5-({4-bromo-6-ethyl-2-methyl-7-oxo-2*H*,6*H*,7*H-*pyrazolo[3,4-*c*]pyridin-3-yl}(hydroxy)methyl)-2-(trifluoromethyl)benzonitrile **(4-S16)** (1.54 mmol, 1.0 equiv.) in 1,4-dioxane-water (0.1 M, 3:1) (12 mL), K₂CO₃ (3.0 equiv.) followed by trimethylboraxine (4.0 equiv.) was added. The mixture was degassed with argon for 15 minutes and then Pd(PPh₃)₄ (5 mol%) was added. The tube was sealed and the reaction mixture was stirred at 110 °C for 3-16 h. After completion, the reaction mixture was extracted with ethyl acetate, washed with water, brine, dried over anhydrous Na₂SO₄ and concentrated. The crude product was purified by column chromatography (SiO₂ 100-200, 2% methanol in dichloromethane) to afford 5-({6-ethyl-2,4-dimethyl-7-oxo-2*H*,6*H*,7*H*-pyrazolo[3,4-*c*]pyridin-3-yl}(hydroxy)methyl)-2-(trifluoromethyl)benzonitrile **(4-S17)** as an off-white solid (200 mg, 33%). **Physical State:** off-white solid. **¹H NMR (400 MHz, DMSO-*d₆*):** *δ* 8.02-7.99 (m, 2 H), 7.75 (d, *J =* 8.24 Hz, 1 H), 6.98 (br s, 1 H), 6.90 (s, 1 H), 6.47 (s, 1 H), 3.88 (q, *J =* 7.36 Hz, 2 H), 3.82 (s, 3 H), 2.09 (s, 3 H), 1.20 (t, *J =* 7.04 Hz, 3 H) ppm. **LC-MS (ESI, m/z):** calcd for [M+H]⁺ 391.1; found: 391.26

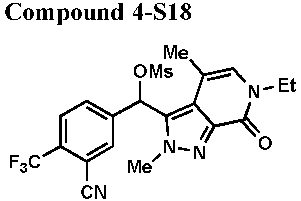

### [[3-cyano-4-(trifluoromethyl)phenyl]-(6-ethyl-2,4-dimethyl-7-oxo-pyrazolo[3,4-c]pyridin-3-yl)methyl] methanesulfonate (4-S18)

To a stirring solution of 5-({6-ethyl-2,4-dimethyl-7-oxo-2*H*,6*H*,7*H*-pyrazolo[3,4-*c*]pyridin-3-yl}(hydroxy)methyl)-2-(trifluoromethyl)benzonitrile **(4-S17)** (60 mg, 0.15 mmol) in dichloromethane (3 mL), triethylamine (0.11 mL, 0.77 mmol) followed by mesyl chloride (0.02 mL, 0.3 mmol) was added dropwise at 0 °C and stirred at that temperature for 1 h. After completion, the reaction mixture was extracted with dichloromethane, washed aqueous saturated NaHCO₃ solution, brine, dried over anhydrous Na₂SO₄ and concentrated. The crude [[3-cyano-4-(trifluoromethyl)phenyl]-(6-ethyl-2,4-dimethyl-7-oxo-pyrazolo[3,4-c]pyridin-3-yl)methyl] methanesulfonate **(4-S18)** was taken directly for the next step (70 mg, crude).

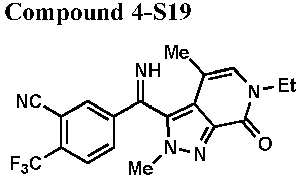

### 5-(6-ethyl-2,4-dimethyl-7-oxo-pyrazolo[3,4-c]pyridine-3-carboximidoyl)-2-(trifluoromethyl)benzonitrile (4-S19)

To a stirred solution of [[3-cyano-4-(trifluoromethyl)phenyl]-(6-ethyl-2,4-dimethyl-7-oxo-pyrazolo[3,4-c]pyridin-3-yl)methyl] methanesulfonate **(4-S18)** (70 mg, 0.15 mmol) in DMF (3 mL), NaN₃ (49 mg, 0.75 mmol) was added and the reaction mixture was stirred at room temperature for 1 h (TLC monitoring). After completion, the reaction mixture was extracted with ethyl acetate, washed with water, brine, dried over anhydrous Na₂SO₄ and concentrated to afford 5-[azido-(6-ethyl-2,4-dimethyl-7-oxo-pyrazolo[3,4-c]pyridin-3-yl)methyl]-2-(trifluoromethyl)-benzonitrile (azide intermediate) (unstable) which readily decomposes to 5-(6-ethyl-2,4-dimethyl-7-oxo-pyrazolo[3,4-c]pyridine-3-carboximidoyl)-2-(trifluoromethyl)benzonitrile **(4-S19)** (70 mg, crude). **LC-MS (ESI, m/z):** calcd for [M+H]⁺ 388.2; found: 388.21.

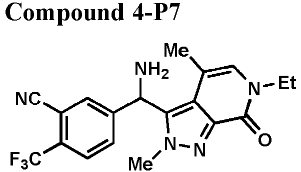

### 5-[amino-(6-ethyl-2,4-dimethyl-7-oxo-pyrazolo[3,4-c]pyridin-3-yl)methyl]-2-(trifluoromethyl)-benzonitrile (4-P7)

To a stirring solution of 5-(6-ethyl-2,4-dimethyl-7-oxo-pyrazolo[3,4-c]pyridine-3-carboximidoyl)-2-(trifluoromethyl)benzonitrile **(4-S19)** (70 mg, 0.18 mmol) in methanol (3 mL), LiBH₄ (20 mg, 0.9 mmol) was added at 0 °C and the reaction mixture was stirred at room temperature for 14 h. After completion, the reaction was quenched by the addition of water, extracted with 10% 2-propanol in dichloromethane, washed with brine, dried over anhydrous Na₂SO₄ and concentrated. The crude product was purified by preparative-HPLC to afford 5-[amino-(6-ethyl-2,4-dimethyl-7-oxo-pyrazolo[3,4-c]pyridin-3-yl)methyl]-2-(trifluor-methyl) benzonitrile **(4-P7)** as an off-white solid (10 mg, 14%). **Physical state:** off-white solid.

**¹H NMR (400 MHz, DMSO-*d₆*): *δ*** 8.14 (s, 1H), 7.97 (d, *J =* 8.32 Hz, 1H), 7.73 (d, *J =* 8.4 Hz, 1H), 6.85 (s, 1H), 5.91 (s, 1H), 3.88 (q, *J =* 7.08 Hz, 2H), 3.81 (s, 3H), 2.80 (br., 2H), 2.09 (s, 3H), 1.19 (t, *J =* 7.08 Hz, 3H) ppm. **LC-MS (ESI, m/z):** calcd for [M+H]⁺ 390.2; found: 390.3. **Note:** 120 mg of **4-P7** was synthesized from another 500 mg scale reaction (25%). Both enantiomers were separated by preparative chiral HPLC to afford enantiomer-1 **4-14** (52 mg) and enantiomer-2 **4-13** (42 mg)

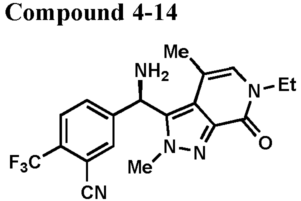

### (R)-5-(amino(6-ethyl-2,4-dimethyl-7-oxo-6,7-dihydro-2H-pyrazolo[3,4-c]pyridin-3-yl)methyl)-2-(trifluoromethyl)benzonitrile (4-14)

**¹H NMR (400 MHz, DMSO-*d₆*):** *δ* 8.14 (s, 1 H), 7.97 (d, *J =* 8.32 Hz, 1 H), 7.73 (d, *J =* 8.4 Hz, 1 H), 6.85 (s, 1 H), 5.91 (s, 1 H), 3.88 (q, *J =* 7.08 Hz, 2 H), 3.81 (s, 3 H), 2.80 (br s, 2 H), 2.09 (s, 3 H), 1.19 (t, *J =* 7.08 Hz, 3 H) ppm. **LC-MS (ESI, m/z):** calcd for [M+H]⁺ 390.2; found: 390.24. **Retention time:** 2.98 min.

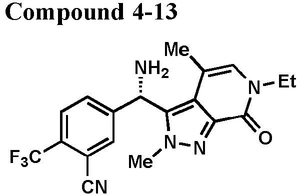

***(S)-5-(amino(6-ethyl-2,4-dimethyl-7-oxo-6,7-dihydro-2H-pyrazolo[3,4-c]pyridin-3-yl)methyl)-2-(trifluoromethyl)benzonitrile* (4-13) ¹H NMR (400 MHz, DMSO-*d₆*): *δ*** 8.14 (s, 1 H), 7.97 (d, *J =* 8.32 Hz, 1 H), 7.73 (d, *J =* 8.4 Hz, 1 H), 6.85 (s, 1 H), 5.91 (s, 1 H), 3.88 (q, *J =* 7.08 Hz, 2 H), 3.81 (s, 3 H), 2.80 (br s, 2 H), 2.09 (s, 3 H), 1.19 (t, *J =* 7.08 Hz, 3 H) ppm. **LC-MS (ESI, m/z):** calcd for [M+H]⁺ 390.2; found: 390.24. **Retention time:** 5.19 min.

### Synthesis of compounds 4-15, 4-16, 4-17, 4-18

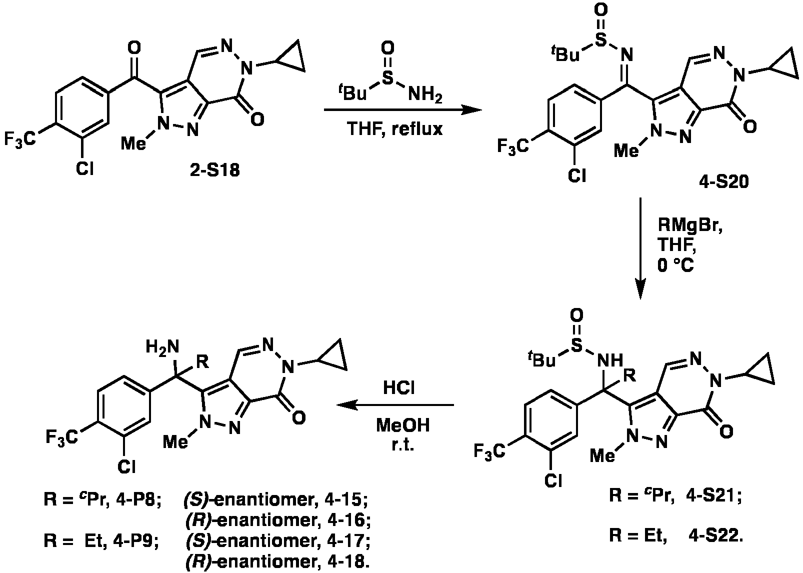

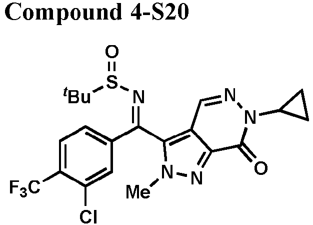

### N-((3-chloro-4-(trifluoromethyl)phenyl)(6-cyclopropyl-2-methyl-7-oxo-6,7-dihydro-2H-pyrazolo[3,4-d]pyridazin-3-yl)methylene)-2-methylpropane-2-sulfinamide (4-S20)

Following **General Procedure F4b** on 1.76 mmol scale with 3-(3-chloro-4-(trifluoromethyl)benzoyl)-6-cyclopropyl-2-methyl-2,6-dihydro-7*H*-pyrazolo[3,4-d]pyridazin-7-one **(2-S18)** and *tert*-butanesulfinamide (3.0 equiv.), Ti(O*ⁱ*Pr)₄ (7.0 equiv.). Purification by Combiflash chromatography (60% ethyl acetate in hexane) to afford *N*-((3-chloro-4-(trifluoromethyl)phenyl)(6-cyclopropyl-2-methyl-7-oxo-6,7-dihydro-2*H*-pyrazolo[3,4-*d*]pyrida-zin-3-yl)methylene)-2-methylpropane-2-sulfinamide **(4-S20)** (800 mg, 91%) as a yellow solid (E and Z mixture), which was taken directly for the next step. **Physical state:** yellow solid. **LC-MS (ESI, m/z):** calcd for [M+H]⁺ 500.1; found: 500.1.

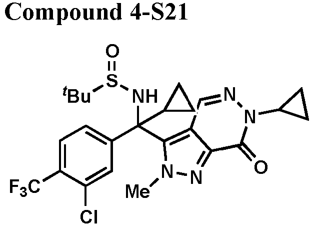

### N-((3-chloro-4-(trifluoromethyl)phenyl)(cyclopropyl) (6-cyclopropyl-2-methyl- 7-oxo-6,7-dihydro-2H-pyrazolo[3,4-d]pyridazin-3-yl)methyl)-2-methylpropane-2-sulfinamide (4-S21)

Following **General Procedure G4b** on 0.64 mmol scale with *N*-((3-chloro-4-(trifluoromethyl)phenyl)(6-cyclopropyl-2-methyl-7-oxo-6,7-dihydro-2*H*-pyrazolo[3,4-*d*]pyrida-zin-3-yl)methylene)-2-methylpropane-2-sulfinamide **(4-S20)** and cyclopropylmagnesium bromide (0.7M) (1.2 equiv.). After completion, the reaction mixture was quenched by the addition of aqueous saturated NH₄Cl solution, extracted with ethyl acetate, washed with brine, dried over anhydrous Na₂SO₄ and concentrated. The crude N-((3-chloro-4-(trifluoromethyl)phenyl)(cyclopropyl)(6-cyclopropyl-2-methyl-7-oxo-6,7-dihydro-2H-pyrazolo-[3,4-*d*]pyridazin-3-yl)methyl)-2-methylpropane-2-sulfinamide **(4-S21)** (340 mg, crude) was taken directly for the next step. **LC-MS (ESI, m/z):** calcd for [M+H]⁺ 542.1; found: 542.0.

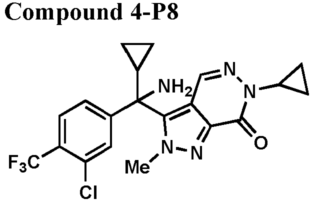

### 3-(amino(3-chloro-4-(trifluoromethyl)phenyl)(cyclopropyl)methyl)-6-cyclopropyl-2-methyl-2,6-dihydro-7H-pyrazolo[3,4-d]pyridazin-7-one (4-P8)

Following **General Procedure H4b** on 0.64 mmol scale with *N*-((3-chloro-4-(trifluoromethyl)phenyl)(cyclopropyl)(6-cyclopropyl-2-methyl-7-oxo-6,7-dihydro-2*H-*pyrazolo[3,4-*d*]pyridazin-3-yl)methyl)-2-methylpropane-2-sulfinamide **(4-S21)** and methanolic HCl (4M, 7 mL). Purification by Combiflash chromatography (70% ethyl acetate in hexane) to afford 3-(amino(3-chloro-4-(trifluoromethyl)phenyl)(cyclopropyl)methyl)-6-cyclopropyl-2-methyl-2,6-dihydro-7H-pyrazolo[3,4-d]pyridazin-7-one **(4-P8)** as an off-white solid (30 mg, 11%). **Physical state:** off-white solid.

The racemic compound **4-P8** was purified by preparative-HPLC-Chiral (NP) to afford enantiomer-1 **(4-16)** (13 mg) and enantiomer-2 **(4-15)** (13 mg).

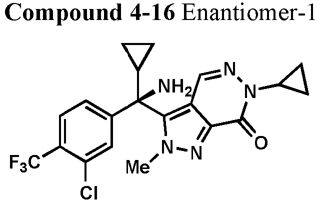

### (R)-3-(amino(3-chloro-4-(trifluoromethyl)phenyl)(cyclopropyl)methyl)-6-cyclopropyl-2-methyl-2,6-dihydro-7H-pyrazolo[3,4-d]pyridazin-7-one (4-16)

**¹H NMR (400 MHz, DMSO-*d₆*): *δ*** 8.50 (s, 1H), 7.81 (d, *J =* 8.16 Hz, 1H), 7.70 (s, 1H), 7.28 (d, *J =* 8.24 Hz, 1H), 4.02-3.97 (m, 1H), 3.71 (s, 3H), 1.85-1.80 (m, 1H), 1.01-0.93 (m, 4H), 0.80-0.60 (m, 3H), 0.50-0.40 (m, 1H) ppm. **LC-MS (ESI, m/z):** calcd for [M+H]⁺ 438.1; found: 438.1. **Retention time:** 4.25 min.

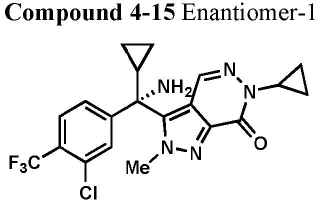

### (S)-3-(amino(3-chloro-4-(trifluoromethyl)phenyl)(cyclopropyl)methyl)-6-cyclopropyl-2-methyl-2,6-dihydro-7H-pyrazolo[3,4-d]pyridazin-7-one (4-15)

**¹H NMR (400 MHz, DMSO-*d₆*):** *δ* 8.51 (s, 1H), 7.80 (d, *J =* 8.32 Hz, 1H), 7.70 (s, 1H), 7.27 (d, *J* = 8.28 Hz, 1H), 4.01-3.98 (m, 1H), 3.70 (s, 3H), 1.84-1.81 (m, 1H), 1.01-0.93 (m, 4H), 0.75-0.64 (m, 3H), 0.45-0.40 (m, 1H) ppm. **LC-MS (ESI, m/z):** calcd for [M+H]⁺ 438.1; found: 438.1. **Retention time:** 4.62 min.

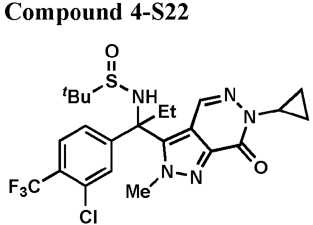

### N-(1-(3-chloro-4-(trifluoromethyl)phenyl)-1-(6-cyclopropyl-2-methyl-7-oxo-6,7-dihydro-2H-pyrazolo[3,4-d]pyridazin-3-yl)propyl)-2-methylpropane-2-sulfinamide (4-S22)

Following **General Procedure G4b** on 0.64 mmol scale with *N*-((3-chloro-4-(trifluoromethyl)phenyl)(6-cyclopropyl-2-methyl-7-oxo-6,7-dihydro-2*H*-pyrazolo[3,4-*d*]pyrida-zin-3-yl)methylene)-2-methylpropane-2-sulfinamide (**4-S20**) and EtMgBr (3M in diethyl ether) (1.2 equiv.) at -5 °C. After completion, the reaction mixture was quenched by the addition of aqueous saturated NH₄Cl solution, extracted with ethyl acetate, washed with brine, dried over anhydrous Na₂SO₄ and concentrated. The crude *N*-(1-(3-chloro-4-(trifluoromethyl)phenyl)-1-(6-cyclopropyl-2-methyl-7-oxo-6,7-dihydro-2*H*-pyrazolo[3,4-*d*]pyridazin-3-yl)propyl)-2-methyl-propane-2-sulfinamide (**4-S22**) was directly taken for the next step (330 mg, crude). **LC-MS (ESI, m/z):** calcd for [M+H]⁺ 530.1; found: 529.9.

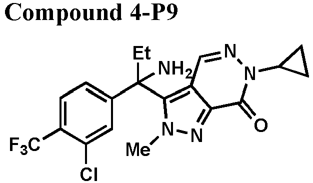

### 3-(1-amino-1-(3-chloro-4-(trifluoromethyl)phenyl)propyl)-6-cyclopropyl-2-methyl-2,6-dihydro-7H-pyrazolo[3,4-d]pyridazin-7-one (4-P9)

Following **General Procedure H4b** on 0.57 mmol scale with *N*-(1-(3-chloro-4-(trifluoromethyl)phenyl)-1-(6-cyclopropyl-2-methyl-7-oxo-6,7-dihydro-2*H*-pyrazolo[3,4-*d*]pyridazin-3-yl)propyl)-2-methylpropane-2-sulfinamide (**4-S22**) and methanolic HCl (4M, 7 mL). Purification by Combiflash chromatography (70% ethyl acetate in hexane) to afford 3-(1-amino-1-(3-chloro-4-(trifluoromethyl)phenyl)propyl)-6-cyclopropyl-2-methyl-2,6-dihydro-7*H*-pyrazolo[3,4-*d*]pyridazin-7-one (**4-P9**) as an off-white solid (90 mg, 37%). **Physical state:** off-white solid.

The racemic compound **4-P9** was purified by preparative-HPLC-Chiral (NP) to afford enantiomer-1 (**4-18**) (20 mg) and enantiomer-2 (**4-17**) (20 mg).

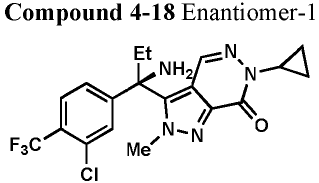

***(R)-3-(1-amino-1-(3-chloro-4-(trifluoromethyl)phenyl)propyl)-6-cyclopropyl-2-methyl-2,6-dihydro-7H-pyrazolo[3,4-d]pyridazin-7-one* (4-18) ¹H NMR (400 MHz, DMSO-*d₆*):** *δ* 8.42 (s, 1H), 7.81 (d, *J* = 8.2 Hz, 1H), 7.69 (s, 1H), 7.33 (d, *J =* 8.2 Hz, 1H), 3.99-3.96 (m, 1H), 3.69 (s, 3H), 2.83 (*br*., 2H), 2.50-2.33 (m, 2H), 0.98-0.92 (m, 4H), 0.74 (t, *J =* 7.0 Hz, 3H) ppm. **LC-MS (ESI, m/z):** calcd for [M+H]⁺ 426.1; found: 426.1. **Retention time:** 2.36 min.

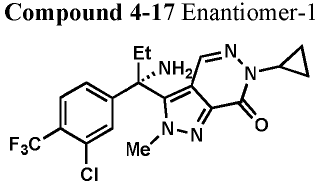

***(S)-3-(1-amino-1-(3-chloro-4-(trifluoromethyl)phenyl)propyl)-6-cyclopropyl-2-methyl-2,6-dihydro-7H-pyrazolo[3,4-d]pyridazin-7-one* (4-17) ¹H NMR (400 MHz, DMSO-*d₆*):** *δ* 8.42 (s, 1H), 7.81 (d, *J* = 8.2 Hz, 1H), 7.69 (s, 1H), 7.33 (d, *J =* 8.2 Hz, 1H), 3.99-3.96 (m, 1H), 3.69 (s, 3H), 2.83 (*br.,* 2H), 2.50-2.33 (m, 2H), 0.98-0.92 (m, 4H), 0.74 (t, *J =* 7.12 Hz, 3H) ppm. **LC-MS (ESI, m/z):** calcd for [M+H]⁺ 426.1; found: 426.1. **Retention time:** 2.52 min.

### Synthesis of compounds 4-19, 4-20, 4-23, 4-24, 4-25, 4-26

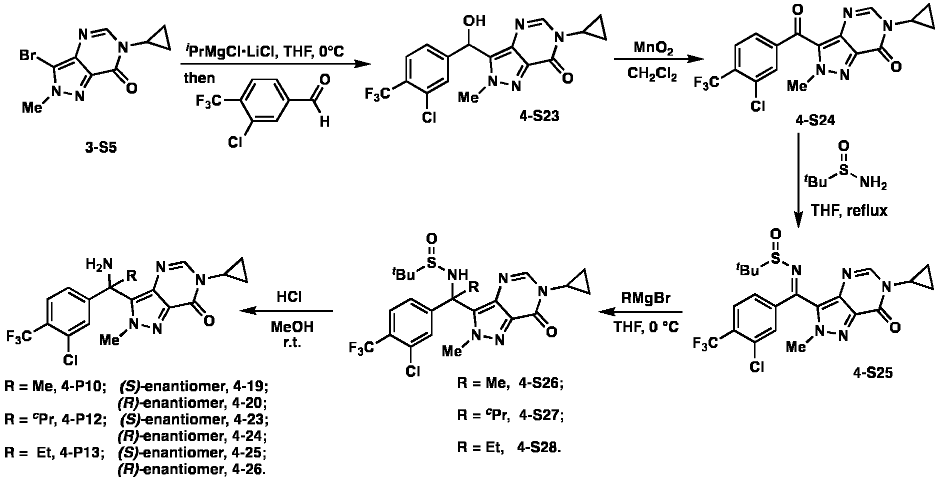

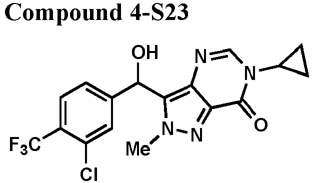

### 3-((3-chloro-4-(trifluoromethyl)phenyl)(hydroxy)methyl)-6-cyclopropyl-2-methyl-2,6-dihydro-7H-pyrazolo[4,3-d]pyrimidin-7-one (4-S23)

Following **General Procedure D3** on 1.86 mmol scale with 3-bromo-6-cyclopropyl-2-methyl-2,6-dihydro-7*H*-pyrazolo[4,3-*d*]pyrimidin-7-one (**3-S5**) and 3-chloro-4-(trifluoromethyl)-benzaldehyde. Purification by CombiFlash chromatography (80% ethyl acetate in hexane) to afford 3-((3-chloro-4-(trifluoromethyl)phenyl)(hydroxy)methyl)-6-cyclopropyl-2-methyl-2,6-dihydro-7*H*-pyrazolo[4,3-*d*]pyrimidin-7-one (**4-S23**) as an off-white solid (367 mg, 50%). **Physical state:** off-white solid. **¹H NMR (400 MHz, CDCl₃):** *δ* 7.81 (s, 1H), 7.64 (d, *J =* 8.2 Hz, 1H), 7.53 (s, 1H), 7.32 (d, *J =* 8.2 Hz, 1H), 6.37 (s, 1H), 3.92 (s, 3H), 3.22-3.18 (m, 1H), 1.26-1.21 (m, 2H), 1.02-0.91 (m, 2H) ppm. **LC-MS (ESI, m/z):** calcd for [M+H]⁺ 399.1; found: 399.1.

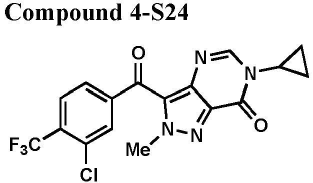

### 3-(3-chloro-4-(trifluoromethyl)benzoyl)-6-cyclopropyl-2-methyl-2,6-dihydro-7H-pyrazolo[4,3-d]pyrimidin-7-one (4-S24)

Following **General Procedure E3** on 0.92 mmol scale with 3-((3-chloro-4-(trifluoromethyl)phenyl)(hydroxy)methyl)-6-cyclopropyl-2-methyl-2,6-dihydro-7H-pyrazolo[4,3-d]pyrimidin-7-one (**4-S23**). Purification by CombiFlash chromatography (40% ethyl acetate in hexane) to afford 3-(3-chloro-4-(trifluoromethyl)benzoyl)-6-cyclopropyl-2-methyl-2,6-dihydro-7*H*-pyrazolo[4,3-*d*]pyrimidin-7-one (**4-S24**) as an off-white solid (339 g, 93%). **Physical state:** off-white solid. **¹H NMR (400 MHz, CDCl₃):** *δ* 8.02 (s, 1H), 7.89-7.81 (m, 3H), 4.41 (s, 3H), 3.25-3.20 (m, 1H), 1.25-1.21 (m, 2H), 0.95-0.90 (m, 2H) ppm. **LC-MS (ESI, m/z):** calcd for [M+H]⁺ 397.1; found: 397.22.

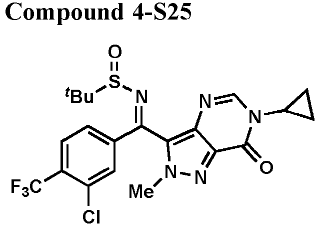

### N-((3-chloro-4-(trifluoromethyl)phenyl)(6-cyclopropyl-2-methyl-7-oxo-6,7-dihydro-2H-pyrazolo[4,3-d]pyrimidin-3-yl)methylene)-2-methylpropane-2-sulfinamide (4-S25):

Following **General Procedure F4c** on 0.85 mmol scale with 3-(3-chloro-4-(trifluoromethyl)benzoyl)-6-cyclopropyl-2-methyl-2,6-dihydro-7*H*-pyrazolo[4,3-*d*]pyrimidin-7-one (**4-S24**) and *tert*-butanesulfinamide (3.0 equiv.), Ti(O*ⁱ*Pr)₄ (7.0 equiv.). Purification by Combiflash chromatography (60% ethyl acetate in hexane) to afford *N*-((3-chloro-4-(trifluoromethyl)phenyl)(6-cyclopropyl-2-methyl-7-oxo-6,7-dihydro-2*H*-pyrazolo[4,3-*d*]pyrimidin-3-yl)methylene)-2-methylpropane-2-sulfinamide (**4-S25**) as a yellow solid (E and Z mixture) (370 mg, 87%). **Physical state:** yellow solid. **LC-MS (ESI, m/z):** calcd for [M+H]⁺ 500.1; found: 500.1.

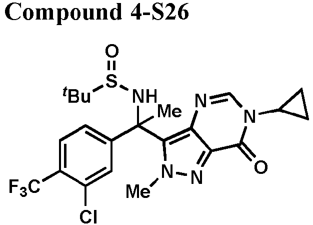

### N-(1-(3-chloro-4-(trifluoromethyl)phenyl)-1-(6-cyclopropyl-2-methyl-7-oxo-6,7-dihydro-2H-pyrazolo[4,3-d]pyrimidin-3-yl)ethyl)-2-methylpropane-2-sulfinamide (4-S26)

Following **General Procedure G4c** on 0.7 mmol scale with *N*-((3-chloro-4-(trifluoromethyl)phenyl)(6-cyclopropyl-2-methyl-7-oxo-6,7-dihydro-2*H*-pyrazolo[4,3-*d*]pyrimidin-3-yl)methylene)-2-methylpropane-2-sulfinamide (**4-S25**) and MeMgBr (3M in diethyl ether) (1.3 equiv.). After completion, the reaction mixture was quenched with aqueous saturated NH₄Cl solution, extracted with ethyl acetate, washed with brine, dried over anhydrous Na₂SO₄ and concentrated. The crude *N*-(1-(3-chloro-4-(trifluoromethyl)phenyl)-1-(6-cyclopropyl-2-methyl-7-oxo-6,7-dihydro-2*H*-pyrazolo[4,3-*d*]pyrimidin-3-yl)ethyl)-2-methylpropane-2-sulfinamide (**4-S26**) was directly taken for the next step (360 mg, crude). **LC-MS (ESI, m/z):** calcd for [M+H]⁺ 516.1; found: 516.34.

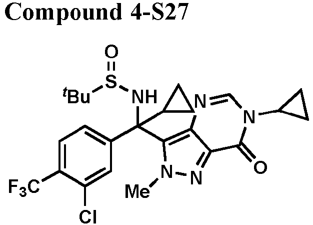

### N-((3-chloro-4-(trifluoromethyl)phenyl)(cyclopropyl) (6-cyclopropyl-2-methyl- 7-oxo-6,7-dihydro-2H-pyrazolo[4,3-d]pyrimidin-3-yl)methyl)-2-methylpropane-2-sulfinamide (4-S27)

Following **General Procedure G4c** on 0.36 mmol scale with *N*-((3-chloro-4-(trifluoromethyl)phenyl)(6-cyclopropyl-2-methyl-7-oxo-6,7-dihydro-2*H*-pyrazolo[4,3-*d*]pyrimidin-3-yl)methylene)-2-methylpropane-2-sulfinamide (**4-S25**) and cyclopropylmagnesium bromide (0.7M in THF) (1.5 equiv.). After completion, the reaction mixture was quenched with aqueous saturated NH₄Cl solution, extracted with ethyl acetate, washed with brine, dried over anhydrous Na₂SO₄ and concentrated. The crude *N*-((3-chloro-4-(trifluoromethyl)phenyl)(cyclopropyl)(6-cyclopropyl-2-methyl-7-oxo-6,7-dihydro-2*H-*pyrazolo[4,3-*d*]pyrimidin-3-yl)methyl)-2-methylpropane-2-sulfinamide (**4-S27**) was directly taken for the next step (190 mg, crude). **LC-MS (ESI, m/z):** calcd for [M+H]⁺ 542.1; found: 542.0.

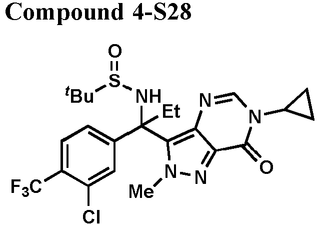

### N-(1-(3-chloro-4-(trifluoromethyl)phenyl)-1-(6-cyclopropyl-2-methyl-7-oxo-6,7-dihydro-2H-pyrazolo[4,3-d]pyrimidin-3-yl)propyl)-2-methylpropane-2-sulfinamide (4-S28)

Following **General Procedure G4c** on 0.36 mmol scale with *N*-((3-chloro-4-(trifluoromethyl)phenyl)(6-cyclopropyl-2-methyl-7-oxo-6,7-dihydro-2*H*-pyrazolo[4,3-*d*]pyrimidin-3-yl)methylene)-2-methylpropane-2-sulfinamide (**4-S25**) and EtMgBr (3M in diethyl ether) (1.5 equiv.). After completion, the reaction mixture was quenched with aqueous saturated NH₄Cl solution, extracted with ethyl acetate, washed with brine, dried over anhydrous Na₂SO₄ and concentrated. The crude *N*-(1-(3-chloro-4-(trifluoromethyl)phenyl)-1-(6-cyclopropyl-2-methyl-7-oxo-6,7-dihydro-2*H*-pyrazolo[4,3-*d*]pyrimidin-3-yl)propyl)-2-methylpropane-2-sulfinamide (**4-S28**) was directly taken for the next step (190 mg, crude). **LC-MS (ESI, m/z):** calcd for [M+H]⁺ 530.1; found: 530.2.

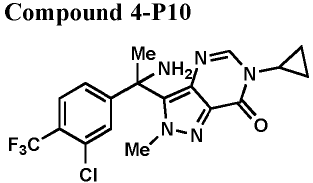

### 3-(1-amino-1-(3-chloro-4-(trifluoromethyl)phenyl)ethyl)-6-cyclopropyl-2-methyl-2,6-dihydro-7H-pyrazolo[4,3-d]pyrimidin-7-one (4-P10)

Following **General Procedure H4c** on 0.7 mmol scale with *N*-(1-(3-chloro-4-(trifluoromethyl)phenyl)-1-(6-cyclopropyl-2-methyl-7-oxo-6,7-dihydro-2*H*-pyrazolo[4,3-*d*]pyrimidin-3-yl)ethyl)-2-methylpropane-2-sulfinamide (**4-S26**). Purification by CombiFlash chromatography (70% ethyl acetate in hexane) to afford 3-(1-amino-1-(3-chloro-4-(trifluoromethyl)phenyl)ethyl)-6-cyclopropyl-2-methyl-2,6-dihydro-7*H*-pyrazolo[4,3-*d*]pyrimidin-7-one (**4-P10**) as an off-white solid (120 mg, 42%). **Physical state:** off-white solid. The racemic compound **4-P10** was purified by preparative-HPLC-Chiral (NP) to afford enantiomer-1 (**4-20**) (50 mg) and enantiomer-2 (**4-19**) (50 mg).

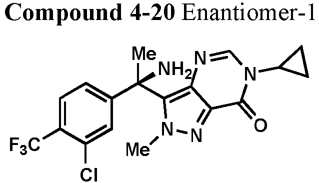

***(R)-3-(1-amino-1-(3-chloro-4-(trifluoromethyl)phenyl)ethyl)-6-cyclopropyl-2-methyl-2,6-dihydro-7H-pyrazolo[4,3-d]pyrimidin-7-one* (4-20) ¹H NMR (400 MHz, DMSO-*d₆*):** *δ* 8.03 (s, 1H), 7.78 (d, *J* = 8.3 Hz, 1H), 7.75 (s, 1H), 7.28 (d, *J =* 8.2 Hz, 1H), 3.74 (s, 3H), 3.19-3.16 (m, 1H), 2.90 (*br*., 2H), 2.08 (s, 3H), 1.05-1.00 (m, 2H), 0.92-0.88 (m, 2H) ppm. **LC-MS (ESI, m/z):** calcd for [M+H]⁺ 412.1; found: 412.3. **Retention time:** 1.18 min. **[α]_{D}²⁰** = +92.125 (c = 0.28, CHCl₃).

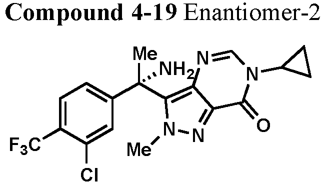

***(S)-3-(1-amino-1-(3-chloro-4-(trifluoromethyl)phenyl)ethyl)-6-cyclopropyl-2-methyl-2,6-dihydro-7H-pyrazolo[4,3-d]pyrimidin-7-one* (4-19) ¹H NMR (400 MHz, DMSO-*d₆*):** *δ* 8.03 (s, 1H), 7.78 (d, *J =* 8.3 Hz, 1H), 7.75 (s, 1H), 7.28 (d, *J =* 8.2 Hz, 1H), 3.74 (s, 3H), 3.21-3.15 (m, 1H), 2.90 (*br*., 2H), 2.08 (s, 3H), 1.05-1.00 (m, 2H), 0.92-0.88 (m, 2H) ppm. **LC-MS (ESI, m/z):** calcd for [M+H]⁺ 412.1; found: 412.3. **Retention time:** 1.42 min. **[α]_{D}²⁰** = -95.215 (c = 0.21, CHCl₃).

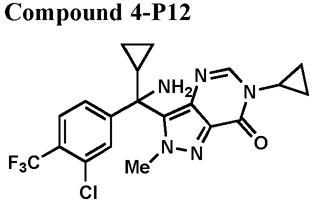

### 3-(amino(3-chloro-4-(trifluoromethyl)phenyl)(cyclopropyl)methyl)-6-cyclopropyl-2-methyl-2,6-dihydro-7H-pyrazolo[4,3-d]pyrimidin-7-one (4-P12)

Following **General Procedure H4c** on 0.35 mmol scale with *N*-((3-chloro-4-(trifluoromethyl)phenyl)(cyclopropyl)(6-cyclopropyl-2-methyl-7-oxo-6,7-dihydro-2*H-*pyrazolo[4,3-*d*]pyrimidin-3-yl)methyl)-2-methylpropane-2-sulfinamide (**4-S27**). Purification by CombiFlash chromatography (70% ethyl acetate in hexane) to afford 3-(amino(3-chloro-4-(trifluoromethyl)phenyl)(cyclopropyl)methyl)-6-cyclopropyl-2-methyl-2,6-dihydro-7*H-*pyrazolo[4,3-*d*]pyrimidin-7-one (**4-P12**) as an off-white solid (90 mg, 59%).

The racemic compound **4-P12** was purified from Preparative-HPLC-Chiral (SFC) to afford enantiomer-1 (**4-24**) (30 mg) and enantiomer-2 (**4-23**) (30 mg).

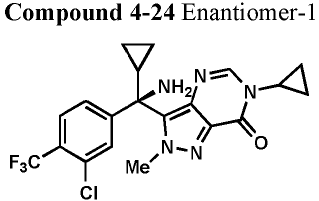

***(R)-3-(amino(3-chloro-4-(trifluoromethyl)phenyl)(cyclopropyl)methyl)-6-cyclopropyl-2-methyl-2,6-dihydro-7H-pyrazolo[4,3-d]pyrimidin-7-one* (4-24) ¹H NMR (400 MHz, DMSO-*d₆*):** *δ* 7.99 (s, 1H), 7.78 (d, *J* = 8.3 Hz, 1H), 7.68 (s, 1H), 7.30 (d, *J* = 8.1 Hz, 1H), 3.81 (s, 3H), 3.19-3.14 (m, 1H), 2.50-2.48 (m, 2H), 2.20-2.17 (m, 1H), 1.03-1.00 (m, 2H), 0.95-0.90 (m, 2H), 0.59-0.57 (m, 2H), 0.52-0.47 (m, 1H), 0.40-0.35 (m, 1H) ppm. **LC-MS (ESI, m/z):** calcd for [M+H]⁺ 438.1; found: 438.1. **Retention time:** 1.66 min.

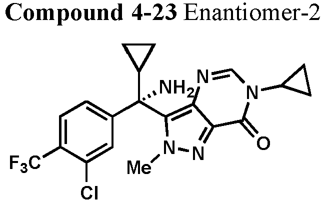

***(S)-3-(amino(3-chloro-4-(trifluoromethyl)phenyl)(cyclopropyl)methyl)-6-cyclopropyl-2-methyl-2,6-dihydro-7H-pyrazolo[4,3-d]pyrimidin-7-one* (4-23) ¹H NMR (400 MHz, DMSO-*d₆*):** *δ* 7.99 (s, 1H), 7.79 (d, *J* = 8.3 Hz, 1H), 7.68 (s, 1H), 7.30 (d, *J* = 8.1 Hz, 1H), 3.81 (s, 3H), 3.18-3.16 (m, 1H), 2.50-2.48 (m, 2H), 2.20-2.15 (m, 1H), 1.03-1.00 (m, 2H), 0.95-0.90 (m, 2H), 0.59-0.57 (m, 2H), 0.52-0.47 (m, 1H), 0.40-0.35 (m, 1H) ppm. **LC-MS (ESI, m/z):** calcd for [M+H]⁺ 438.1; found: 438.1. **Retention time:** 5.04 min.

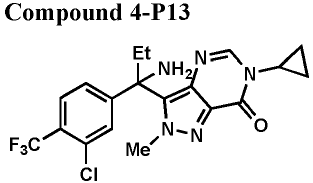

### 3-(1-amino-1-(3-chloro-4-(trifluoromethyl)phenyl)propyl)-6-cyclopropyl-2-methyl-2,6-dihydro-7H-pyrazolo[4,3-d]pyrimidin-7-one (4-P13)

Following **General Procedure H4c** on 0.36 mmol scale with *N*-(1-(3-chloro-4-(trifluoromethyl)phenyl)-1-(6-cyclopropyl-2-methyl-7-oxo-6,7-dihydro-2*H*-pyrazolo[4,3-*d*]pyrimidin-3-yl)propyl)-2-methylpropane-2-sulfinamide (**4-S28**). Purification by CombiFlash chromatography (70% ethyl acetate in hexane) to afford 3-(1-amino-1-(3-chloro-4-(trifluoromethyl)phenyl)propyl)-6-cyclopropyl-2-methyl-2,6-dihydro-7*H*-pyrazolo[4,3-*d*]pyrimidin-7-one (**4-P13**) as an off-white solid (50 mg, 33%).

The racemic compound **4-P13** was purified from Preparative-HPLC-Chiral (SFC) to afford enantiomer-1 (**4-26**) (10 mg) and enantiomer-2 (**4-25**) (10 mg).

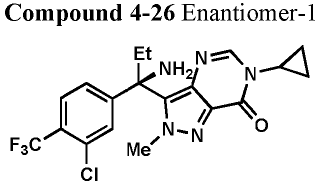

***(R)-3-(1-amino-1-(3-chloro-4-(trifluoromethyl)phenyl)propyl)-6-cyclopropyl-2-methyl-2,6-dihydro-7H-pyrazolo[4,3-d]pyrimidin-7-one* (4-26) ¹H NMR (400 MHz, DMSO-*d₆*):** *δ* 8.05 (s, 1H), 7.78 (d, *J* = 8.3 Hz, 1H), 7.70 (s, 1H), 7.27 (d, *J =* 8.2 Hz, 1H), 3.79 (s, 3H), 3.19-3.16 (m, 1H), 2.78-2.67 (m, 3H), 2.38-2.33 (m, 1H), 1.05-1.00 (m, 2H), 0.95-0.90 (m, 2H), 0.79 (t, *J =* 7.1 Hz, 3H) ppm. **LC-MS (ESI, m/z):** calcd for [M+H]⁺ 426.1; found: 426.2. **Retention time:** 2.12 min.

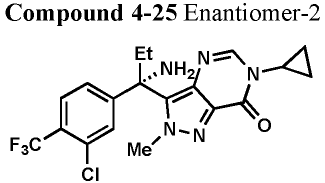

***(S)-3-(1-amino-1-(3-chloro-4-(trifluoromethyl)phenyl)propyl)-6-cyclopropyl-2-methyl-2,6-dihydro-7H-pyrazolo[4,3-d]pyrimidin-7-one* (4-25) ¹H NMR (400 MHz, DMSO-*d₆*):** *δ* 8.05 (s, 1H), 7.78 (d, *J* = 8.3 Hz, 1H), 7.70 (s, 1H), 7.27 (d, *J =* 8.2 Hz, 1H), 3.79 (s, 3H), 3.19-3.16 (m, 1H), 2.78-2.67 (m, 3H), 2.38-2.33 (m, 1H), 1.05-1.00 (m, 2H), 0.95-0.90 (m, 2H), 0.78 (t, *J* = 7.1 Hz, 3H) ppm. **LC-MS (ESI, m/z):** calcd for [M+H]⁺ 426.1; found: 426.2. **Retention time:** 3.83 min.

### Synthesis of compounds 4-21, 4-22

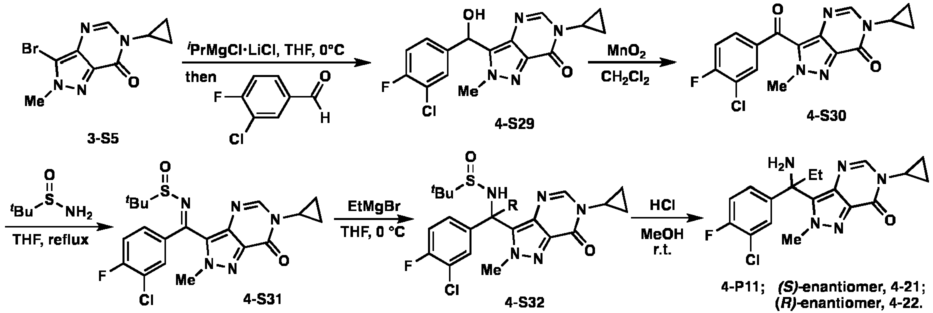

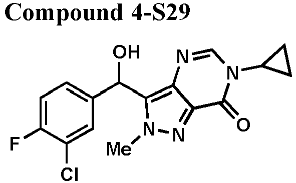

### 3-((3-chloro-4-fluorophenyl)(hydroxy)methyl)-6-cyclopropyl-2-methyl-2, 6-dihydro-7H-pyrazolo[4,3-d]pyrimidin-7-one (4-S29)

Following **General Procedure D3** on 2.97 mmol scale with 3-bromo-6-cyclopropyl-2-methyl-2,6-dihydro-7*H*-pyrazolo[4,3-*d*]pyrimidin-7-one (**3-S5**) and 3-chloro-4-fluorobenzaldehyde. Purification by CombiFlash chromatography (80% ethyl acetate in hexane) to afford 3-((3-*chloro-4-fluorophenyl)(hydroxy)methyl)-6-cyclopropyl-2-methyl-2,6-dihydro-7H-*pyrazolo[4,3-*d*]pyrimidin-7-one (**4-S29**) as an off-white solid (454 mg, 44%).

**Physical state:** off-white solid. **¹H NMR (400 MHz, DMSO-*d₆*):** δ 7.99 (s, 1H), 7.55 (d, *J =* 6.0 Hz, 1H), 7.38 (t, *J =* 9.0 Hz, 1H), 7.30-7.25 (m, 1H), 6.71 (*br*., 1H), 6.26 (s, 1H), 3.94 (s, 3H), 3.16-3.14 (m, 1H), 1.05-1.00 (m, 2H), 0.90-0.85 (m, 2H) ppm. **LC-MS (ESI, m/z):** calcd for [M+H]⁺ 349.1; found: 349.1.

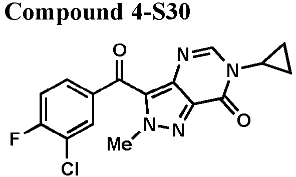

### 3-(3-chloro-4-fluorobenzoyl)-6-cyclopropyl-2-methyl-2,6-dihydro-7H-pyrazolo[4,3-d]pyrimidin-7-one (4-S30)

Following **General Procedure E3** on 1.22 mmol scale with 3-((3-chloro-4-fluorophenyl)(hydroxy)methyl)-6-cyclopropyl-2-methyl-2,6-dihydro-7*H*-pyrazolo[4,3-*d*]pyrimidin-7-one (**4-S29**). The crude 3-(3-chloro-4-fluorobenzoyl)-6-cyclopropyl-2-methyl-2,6-dihydro-7*H*-pyrazolo[4,3-*d*]pyrimidin-7-one (**4-S30**) was taken directly for the next step (410 mg, 97%). **LC-MS (ESI, m/z):** calcd for [M+H]⁺ 347.1; found: 347.0.

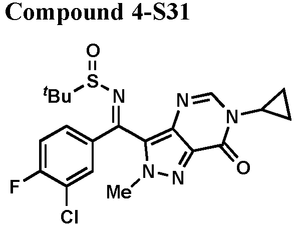

### N-((3-chloro-4-fluorophenyl)(6-cyclopropyl-2-methyl-7-oxo-6,7-dihydro-2H-pyrazolo[4,3-d]pyrimidin-3-yl)methylene)-2-methylpropane-2-sulfinamide (4-S31)

Following **General Procedure F4c** on 1.64 mmol scale with 3-(3-chloro-4-fluorobenzoyl)-6-cyclopropyl-2-methyl-2,6-dihydro-7*H*-pyrazolo[4,3-*d*]pyrimidin-7-one (**4-S30**) and *tert-*butanesulfinamide (3.0 equiv.), Ti(O*ⁱ*Pr)₄ (7.0 equiv.). Purification by CombiFlash chromatography (60% ethyl acetate in hexane) to afford *N*-((3-chloro-4-fluorophenyl)(6-cyclopropyl-2-methyl-7-oxo-6,7-dihydro-2*H*-pyrazolo[4,3-*d*]pyrimidin-3-yl)methylene)-2-methylpropane-2-sulfinamide (**4-S31**) as a yellow solid (230 mg, 93%). **Physical state:** yellow solid. **LC-MS (ESI, m/z):** calcd for [M+H]⁺ 450.1; found: 450.1.

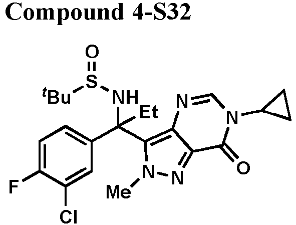

### N-(1-(3-chloro-4-fluorophenyl)-1-(6-cyclopropyl-2-methyl-7-oxo-6,7-dihydro-2H-pyrazolo[4,3-d]pyrimidin-3-yl)propyl)-2-methylpropane-2-sulfinamide (4-S32)

Following **General Procedure G4c** on 0.45 mmol scale with *N*-((3-chloro-4-fluorophenyl)(6-cyclopropyl-2-methyl-7-oxo-6,7-dihydro-2*H*-pyrazolo[4,3-*d*]pyrimidin-3-yl)methylene)-2-methylpropane-2-sulfinamide (**4-S31**) and EtMgBr (3M in diethyl ether) (1.5 equiv.). After completion, the reaction mixture was quenched with aqueous saturated NH₄Cl solution, extracted with ethyl acetate, washed with brine, dried over anhydrous Na₂SO₄ and concentrated. The crude *N*-(1-(3-chloro-4-fluorophenyl)-1-(6-cyclopropyl-2-methyl-7-oxo-6,7-dihydro-2*H-*pyrazolo[4,3-*d*]pyrimidin-3-yl)propyl)-2-methylpropane-2-sulfinamide (**4-S32**) was directly taken for the next step (210 mg, crude). **LC-MS (ESI, m/z):** calcd for [M+H]⁺ 480.1; found: 480.22.

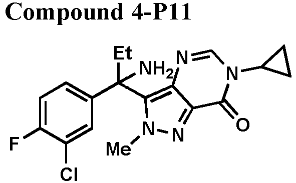

### 3-(1-amino-1-(3-chloro-4-fluorophenyl)propyl)-6-cyclopropyl-2-methyl-2,6-dihydro-7H-pyrazolo[4,3-d]pyrimidin-7-one (4-P11)

Following **General Procedure H4c** on 0.40 mmol scale with *N*-(1-(3-chloro-4-fluorophenyl)-1-(6-cyclopropyl-2-methyl-7-oxo-6,7-dihydro-2*H*-pyrazolo[4,3-*d*]pyrimidin-3-yl)propyl)-2-methylpropane-2-sulfinamide (**4-S32**). Purification by CombiFlash chromatography (70% ethyl acetate in hexane) to afford 3-(1-amino-1-(3-chloro-4-fluorophenyl)propyl)-6-cyclopropyl-2-methyl-2,6-dihydro-7*H*-pyrazolo[4,3-*d*]pyrimidin-7-one (**4-P11**) as an off-white solid (35 mg, 24%). **Physical state:** off-white solid.

The racemic compound **4-P11** was purified from Preparative-HPLC-Chiral (SFC) to afford enantiomer-1 (**4-22**) (10 mg) and enantiomer-2 (**4-21**) (10 mg).

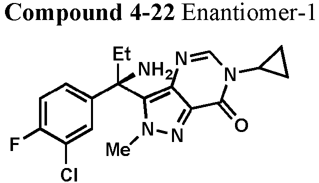

### (R)-3-(1-amino-1-(3-chloro-4-fluorophenyl)propyl)-6-cyclopropyl-2-methyl-2,6-dihydro-7H-pyrazolo[4,3-d]pyrimidin-7-one (4-22)

**¹H NMR (400 MHz, DMSO-*d₆*):** *δ* 8.03 (s, 1H), 7.53 (d, *J* = 5.8 Hz, 1H), 7.32 (t, *J =* 8.9 Hz, 1H), 7.15-7.10 (m, 1H), 3.77 (s, 3H), 3.20-3.10 (m, 1H), 2.80-2.60 (m, 3H), 2.38-2.32 (m, 1H), 1.05-1.00 (m, 2H), 0.95-0.90 (m, 2H), 0.77 (t, *J =* 7.0 Hz, 3H) ppm. **LC-MS (ESI, m/z):** calcd for [M+H]⁺ 376.1; found: 376.3. **Retention time:** 1.46 min.

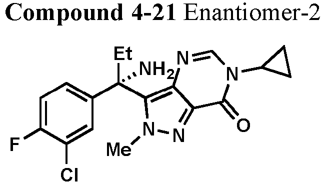

***(S)-3-(1-amino-1-(3-chloro-4-fluorophenyl)propyl)-6-cyclopropyl-2-methyl-2,6-dihydro-7H-pyrazolo[4,3-d]pyrimidin-7-one* (4-21) ¹H NMR (400 MHz, DMSO-*d₆*):** *δ* 8.03 (s, 1H), 7.54 (d, *J =* 6.3 Hz, 1H), 7.33 (t, *J =* 8.5 Hz, 1H), 7.12 (*br*., 1H), 3.76 (s, 3H), 3.20-3.10 (m, 1H), 2.80-2.60 (m, 3H), 2.37-2.33 (m, 1H), 1.05-1.00 (m, 2H), 0.95-0.90 (m, 2H), 0.77 (t, *J =* 6.6 Hz, 3H) ppm. **LC-MS (ESI, m/z):** calcd for [M+H]⁺ 376.1; found: 376.3. **Retention time:** 2.46 min.

### Synthesis of compounds 4-27, 4-28

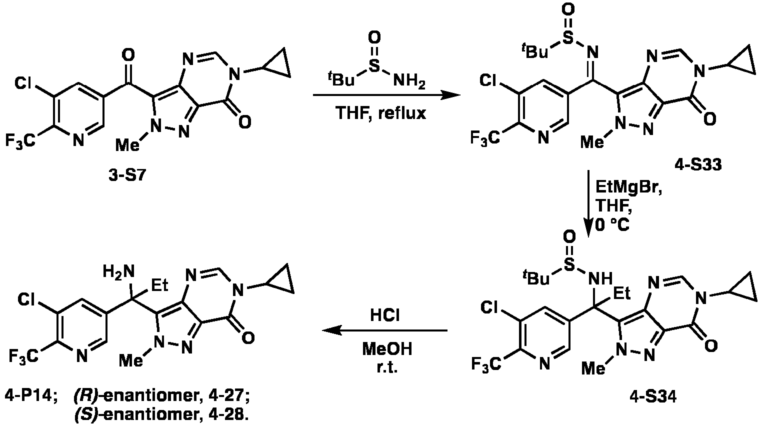

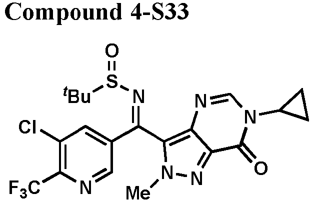

### N-((5-chloro-6-(trifluoromethyl)pyridin-3-yl)(6-cyclopropyl-2-methyl-7-oxo-6,7-dihydro-2H-pyrazolo[4,3-d]pyrimidin-3-yl)methylene)-2-methylpropane-2-sulfinamide (4-S33)

Following **General Procedure F4c** on 0.63 mmol scale with 3-(5-chloro-6-(trifluoromethyl)nicotinoyl)-6-cyclopropyl-2-methyl-2,6-dihydro-7*H*-pyrazolo[4,3-*d*]pyrimidin-7-one (**3-S7**) and *tert*-butanesulfinamide (3.0 equiv.), Ti(O*ⁱ*Pr)₄ (7.0 equiv.). Purification by Combiflash chromatography (60% ethyl acetate in hexane) to afford *N*-((5-chloro-6-(trifluoromethyl)pyridin-3-yl)(6-cyclopropyl-2-methyl-7-oxo-6,7-dihydro-2*H-*pyrazolo[4,3-*d*]pyrimidin-3-yl)methylene)-2-methylpropane-2-sulfinamide (**4-S33**) as a yellow solid (290 mg, 92%). **Physical state:** yellow solid. **LC-MS (ESI, m/z):** calcd for [M+H]⁺ 501.1; found: 501.1.

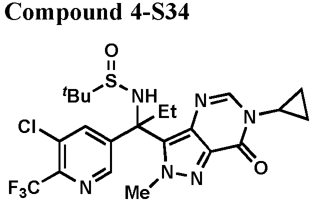

### N-(1-(5-chloro-6-(trifluoromethyl)pyridin-3-yl)-1-(6-cyclopropyl-2-methyl-7-oxo-6,7-dihydro-2H-pyrazolo[4,3-d]pyrimidin-3-yl)propyl)-2-methylpropane-2-sulfinamide (4-S34)

Following **General Procedure G4c** on 0.56 mmol scale with *N*-((5-chloro-6-(trifluoromethyl)pyridin-3-yl)(6-cyclopropyl-2-methyl-7-oxo-6,7-dihydro-2*H*-pyrazolo[4,3-*d*]pyrimidin-3-yl)methylene)-2-methylpropane-2-sulfinamide (**4-S33**) and EtMgBr (3M in diethyl ether) (1.1 equiv.). After completion, the reaction mixture was quenched with aqueous saturated NH₄Cl solution, extracted with ethyl acetate, washed with brine, dried over anhydrous Na₂SO₄ and concentrated. The crude *N*-(1-(5-chloro-6-(trifluoromethyl)pyridin-3-yl)-1-(6-cyclopropyl-2-methyl-7-oxo-6,7-dihydro-2*H*-pyrazolo[4,3-*d*]pyrimidin-3-yl)propyl)-2-methylpropane-2-sulfinamide (**4-S34**) was directly taken for the next step (290 mg, crude). **LC-MS (ESI, m/z):** calcd for [M+H]⁺ 531.1; found: 531.0.

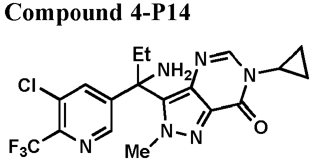

### 3-(1-amino-1-(5-chloro-6-(trifluoromethyl)pyridin-3-yl)propyl)-6-cyclopropyl-2-methyl-2,6-dihydro-7H-pyrazolo[4,3-d]pyrimidin-7-one (4-P14)

Following **General Procedure H4c** on 0.51 mmol scale with *N*-(1-(5-chloro-6-(trifluoromethyl)pyridin-3-yl)-1-(6-cyclopropyl-2-methyl-7-oxo-6,7-dihydro-2*H-*pyrazolo[4,3-*d*]pyrimidin-3-yl)propyl)-2-methylpropane-2-sulfinamide (**4-S34**). Purification by Combiflash chromatography (70% ethyl acetate in hexane) to afford 3-(1-amino-1-(5-chloro-6-(trifluoromethyl)pyridin-3-yl)propyl)-6-cyclopropyl-2-methyl-2,6-dihydro-7*H-*pyrazolo[4,3-*d*]pyrimidin-7-one (**4-P14**) as an off-white solid (64 mg, 29%). **Physical state:** off-white solid.

The racemic compound **4-P14** was purified by preparative-HPLC-Chiral (NP) to afford enantiomer-1 (**4-28**) (25 mg) and enantiomer-2 (**4-27**) (25 mg).

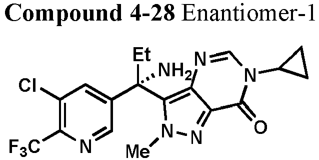

***(S)-3-(1-amino-1-(5-chloro-6-(trifluoromethyl)pyridin-3-yl)propyl)-6-cyclopropyl-2-methyl-2,6-dihydro-7H-pyrazolo[4,3-d]pyrimidin-7-one* (4-28)¹H NMR (400 MHz, DMSO-*d₆*):** *δ* 8.49 (d, *J =* 1.16 Hz, 1H), 8.13 (s, 1H), 8.02 (s, 1H), 3.91 (s, 3H), 3.19-3.14 (m, 1H), 2.88-2.84 (m, 2H), 2.82-2.77 (m, 1H), 2.40-2.33 (m, 1H), 1.05-1.00 (m, 2H), 0.92-0.88 (m, 2H), 0.81 (t, *J =* 7.24 Hz, 3H) ppm. **LC-MS (ESI, m/z):** calcd for [M+H]⁺ 427.1; found: 427.2. **Retention time:** 3.51 min. [α]²⁵_{D} = -93.544° (c = 0.2512 g/100ml, MeOH).

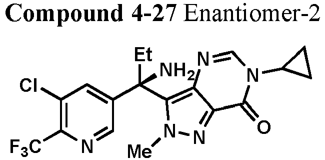

***(R)-3-(1-amino-1-(5-chloro-6-(trifluoromethyl)pyridin-3-yl)propyl)-6-cyclopropyl-2-methyl-2,6-dihydro-7H-pyrazolo[4,3-d]pyrimidin-7-one* (4-27) ¹H NMR (400 MHz, DMSO-*d₆*):** *δ* 8.49 (s, 1H), 8.13 (s, 1H), 8.02 (s, 1H), 3.91 (s, 3H), 3.19-3.14 (m, 1H), 2.88-2.84 (m, 2H), 2.82-2.77 (m, 1H), 2.41-2.33 (m, 1H), 1.05-1.00 (m, 2H), 0.92-0.88 (m, 2H), 0.81 (t, *J =* 7.24 Hz, 3H) ppm. **LC-MS (ESI, m/z):** calcd for [M+H]⁺ 427.1; found: 427.1. **Retention time:** 5.21 min. [α]²⁵_{D} = +93.057° (c = 0.2536 g/100ml, MeOH).

### Synthesis of compounds 5-1 to 5-12

### Synthesis of compounds 5-1, 5-2

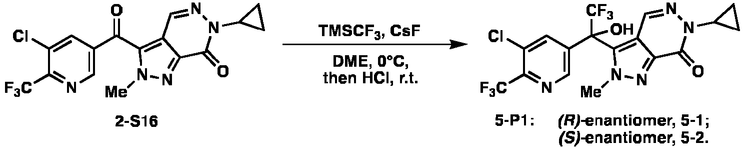

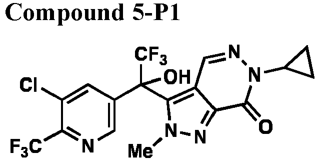

### 3-(1-(5-chloro-6-(trifluoromethyl)pyridin-3-yl)-2,2,2-trifluoro-1-hydroxyethyl)-6-cyclopropyl-2-methyl-2,6-dihydro-7H-pyrazolo[3,4-d]pyridazin-7-one

Following **General Procedure F5** on 0.55 mmol scale with 3-(5-chloro-6-(trifluoromethyl)nicotinoyl)-6-cyclopropyl-2-methyl-2,6-dihydro-7*H*-pyrazolo[3,4-*d*]pyridazin-7-one (**2-S16**). Purification by CombiFlash chromatography (70% ethyl acetate in hexane) to afford 3-(1-(5-chloro-6-(trifluoromethyl)pyridin-3-yl)-2,2,2-trifluoro-1-hydroxyethyl)-6-cyclopropyl-2-methyl-2,6-dihydro-7*H-*pyrazolo[3,4-*d*]pyridazin-7-one (**5-P1**) as an off-white solid (120 mg, 46%). **Physical State:** off-white solid.

The racemic compound **5-P1** was submitted to preparative-HPLC-SFC to afford Enantiomer-1 (**5-2**) (45 mg) and Enantiomer-2 (**5-1**) (45 mg).

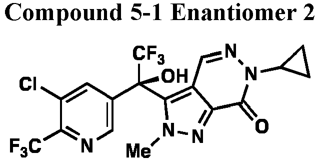

### (R)-3-(1-(5-chloro-6-(trifluoromethyl)pyridin-3-yl)-2,2,2-trifluoro-1-hydroxyethyl)-6-cyclopropyl-2-methyl-2,6-dihydro-7H-pyrazolo[3,4-d]pyridazin-7-one (5-1)

**m.p.:** 203-205 °C. ¹H **NMR (400 MHz, DMSO-*d₆*):** *δ* 8.98 (s, 1H), 8.75 (s, 1H), 8.28 (s, 1H), 8.04 (s, 1H), 4.02-3.97 (m, 1H), 3.80 (s, 3H), 1.04-0.95 (m, 4H) ppm. ¹H **NMR (600 MHz, CDCl₃):** δ 8.44 (*br.* 1H), 8.13 - 7.92 (m, 2H), 7.64 (s, 1H), 4.29 (tt, *J* = 7.8, 4.2 Hz, 1H), 3.84 (s, 3H), 1.21 - 1.08 (m, 2H), 1.05 - 0.94 (m, 2H) ppm. ¹³C **NMR (151 MHz, CDCl₃):** δ 156.70, 146.07 (q, *J* = 34.8 Hz), 145.62, 139.47, 138.95, 136.27, 133.27, 132.06 (q, *J =* 7.5 Hz), 131.04, 123.65 (d, *J =* 287.4 Hz), 120.69 (d, *J* = 275.8 Hz), 115.72, 73.62 (q, *J =* 32.1 Hz), 41.80, 31.38, 5.88, 5.85 ppm. **¹⁹F NMR (376 MHz, CDCl₃):** δ -66.31, -75.35 ppm. **HRMS (ESI-TOF):** calc'd for C₁₇H₁₂ClF₆N₅O₂ [M+H]+: 468.0657, found: 468.0653. **LC-MS (ESI, m/z):** calcd for [M+H]⁺ 468.1; found: 468.28. **TLC:** R*_{f}* = 0.51 (4:1 methylene chloride: acetone). **[α]_{D}²⁰** = +68.320 (c = 0.24, CHCl₃). **Retention time:** 4.29 min. **[α]_{D}²⁰** = +68.320 (c = 0.24, CHCl₃). [α]²⁵_{D} = +84.511° (c = 0.5171 g/100ml, MeOH).

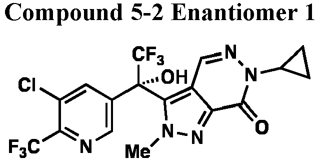

***(S)-3-(1-(5-chloro-6-(trifluoromethyl)pyridin-3-yl)-2,2,2-trifluoro-1-hydroxyethyl)-6-cyclopropyl-2-methyl-2,6-dihydro-7H-pyrazolo[3,4-d]pyridazin-7-one* (5-2) ¹H NMR (400 MHz, DMSO-*d₆*)**: *δ* 8.98 (s, 1H), 8.75 (s, 1H), 8.28 (s, 1H), 8.04 (s, 1H), 4.01-3.97 (m, 1H), 3.80 (s, 3H), 1.04-0.95 (m, 4H) ppm. **LC-MS (ESI, m/z):** calcd for [M+H]⁺ 468.1; found: 468.30. **Retention time:** 3.06 min. [α]²⁵_{D} = -74.072° (c = 0.5090 g/100ml, MeOH).

### Synthesis of compounds 5-3, 5-4

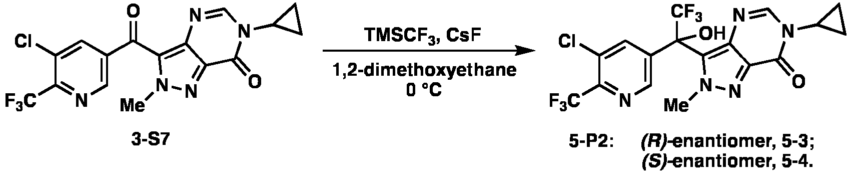

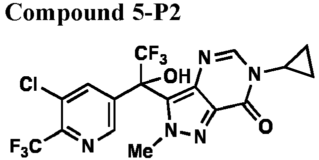

### 3-(1-(5-chloro-6-(trifluoromethyl)pyridin-3-yl)-2,2,2-trifluoro-1-hydroxyethyl)-6-cyclopropyl-2-methyl-2,6-dihydro-7H-pyrazolo[4,3-d]pyrimidin-7-one (5-P2)

Following **General Procedure F5** on 0.62 mmol scale with 3-(5-chloro-6-(trifluoromethyl)nicotinoyl)-6-cyclopropyl-2-methyl-2,6-dihydro-7*H*-pyrazolo[4,3-*d*]pyrimidin-7-one (**3-S7**). Purification by CombiFlash chromatography (70% ethyl acetate in hexane) to afford 3-(1-(5-chloro-6-(trifluoromethyl)pyridin-3-yl)-2,2,2-trifluoro-1-hydroxyethyl)-6-cyclopropyl-2-methyl-2,6-dihydro-7*H*-pyrazolo[4,3-*d*]pyrimidin-7-one (**5-P2**) as a white solid (200 mg, 69%). **Physical State:** white solid.

The racemic compound **5-P2** was purified by preparative-HPLC-Chiral (SFC) to afford enantiomer-1 (**5-4**) (90 mg) and enantiomer-2 (**5-3**) (90 mg).

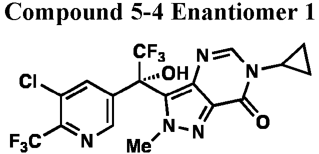

***(S)-3-(1-(5-chloro-6-(trifluoromethyl)pyridin-3-yl)-2,2,2-trifluoro-1-hydroxyethyl)-6-cyclo propyl-2-methyl-2,6-dihydro-7H-pyrazolo[4,3-d]pyrimidin-7-one* (5-4) ¹H NMR (400 MHz, DMSO-*d₆*):** *δ* 8.69 (br s, 2H), 8.20 (s, 1H), 8.02 (s, 1H), 3.91 (s, 3H), 3.17-3.14 (m, 1H), 1.05-1.00 (m, 2H), 0.95-0.90 (m, 2H) ppm. ¹H **NMR (600 MHz, CDCl₃):** δ 8.70 (d, *J =* 1.9 Hz, 1H), 8.07 - 8.01 (m, 1H), 7.91 (s, 1H), 7.47 (s, 1H), 3.69 (s, 3H), 3.23 (tt, *J =* 7.4, 4.0 Hz, 1H), 1.26 - 1.17 (m, 2H), 1.03 - 0.87 (m, 2H) ppm. ¹³C **NMR (151 MHz, CDCl₃):** δ 157.20, 146.59, 145,74, 145.74 (q, *J =* 34.8 Hz), 139.37, 136.62, 136.50, 134.37, 130.91, 130.02, 123.93 (q, *J* = 286.8 Hz), 120.68 (q, *J* = 275.7 Hz), 75.97 (q, *J =* 32.4 Hz), 41.52, 29.30, 7.18, 6.92 ppm. **¹⁹F NMR (376 MHz, CDCl₃):** δ -66.33, -74.92 ppm. **LC-MS (ESI, m/z):** calcd for [M+H]⁺ 468.1; found: 468.27. **[α]_{D}²⁰** = +25.710 (*c* = 0.21, CHCl₃). **[α]_{D}²⁰** = -21.424 (*c* = 0.28, EtOH). **Retention time:** 2.22 min. [α]²⁵_{D} = -26.793° (c = 0.5263 g/100ml, MeOH).

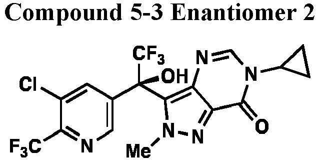

***(R)-3-(1-(5-chloro-6-(trifluoromethyl)pyridin-3-yl)-2,2,2-trifluoro-1-hydroxyethyl)-6-cyclopropyl-2-methyl-2,6-dihydro-7H-pyrazolo[4,3-d]pyrimidin-7-one (5-3)* Physical State:** white solid. **m.p.:** 104-106 °C. **¹H NMR (400 MHz, DMSO-*d₆*):** *δ* 8.69 (br s, 2H), 8.20 (s, 1H), 8.03 (s, 1H), 3.91 (s, 3H), 3.17-3.14 (m, 1H), 1.05-1.00 (m, 2H), 0.95-0.90 (m, 2H) ppm;
**¹H NMR (600 MHz, CDCl₃):** δ 8.72 (d, *J =* 2.0 Hz, 1H), 8.04 (d, *J =* 1.9 Hz, 1H), 7.92 (s, 1H), 7.51 (s, 1H), 3.69 (s, 3H), 3.23 (tt, *J =* 7.2, 4.0 Hz, 1H), 1.30 - 1.21 (m, 2H), 1.01 - 0.88 (m, 2H) ppm. **¹³C NMR (151 MHz, CDCl₃):** δ 157.10, 145.76 (q, *J =* 34.9 Hz), 146.69, 145.73, 139.40, 136.70, 136.58, 134.47, 130.93, 129.91, 123.99 (q, *J =* 286.8 Hz), 120.67 (q, *J* = 275.5 Hz), 76.05 (q, *J* = 32.6 Hz), 41.47, 29.33, 7.17, 6.97 ppm. **¹⁹F NMR (376 MHz, CDCl₃):** δ -66.34, -74.93 ppm. **HRMS (ESI-TOF):** calc'd for C₁₇H₁₂ClF₆N₅O₂ [M+H]+: 468.0657, found: 468.0661. **TLC:** R*_{f}* = 0.67 (2:1 methylene chloride: acetone). **[α]_{D}²⁰** = -33.326 (*c* = 0.15, CHCl₃). **Retention time:** 2.63 min. [α]²⁵_{D} = +23.556° (c = 0.5221 g/100ml, MeOH).

### Synthesis of compounds 5-5, 5-6

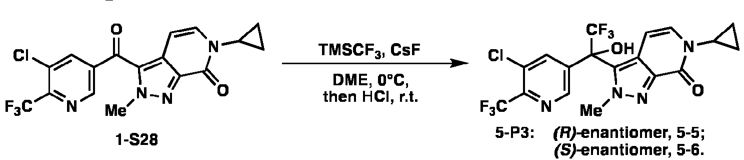

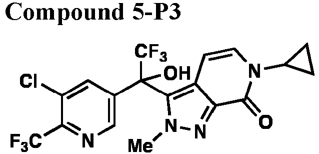

### 3-(1-(5-chloro-6-(trifluoromethyl)pyridin-3-yl)-2,2,2-trifluoro-1-hydroxyethyl)-6-cyclopropyl-2-methyl-2,6-dihydro-7H-pyrazolo[3,4-c]pyridin-7-one (5-P3)

Following **General Procedure F5** on 0.30 mmol scale with 3-(5-chloro-6-(trifluoromethyl)nicotinoyl)-6-cyclopropyl-2-methyl-2,6-dihydro-7*H*-pyrazolo[3,4-*c*]pyridin-7-one **(1-S28).** Purification by Combiflash chromatography (50% ethyl acetate in hexane) to afford 3-(1-(5-chloro-6-(trifluoromethyl)pyridin-3-yl)-2,2,2-trifluoro-1-hydroxyethyl)-6-cyclopropyl-2-methyl-2,6-dihydro-7*H*-pyrazolo[3,4-*c*]pyridin-7-one (**5-P3**) as an off-white solid (53 mg, 37%). **Physical state:** off-white solid.

The racemic compound **5-P3** was purified from Prep-HPLC-SFC to afford enantiomer-1 (**5-6**) (13 mg) and enantiomer-2 (**5-5**) (12 mg).

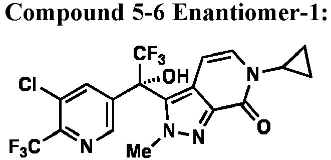

***(S)-3-(1-(5-chloro-6-(trifluoromethyl)pyridin-3-yl)-2,2,2-trifluoro-1-hydroxyethyl)-6-cyclopropyl-2-methyl-2,6-dihydro-7H-pyrazolo[3,4-c]pyridin-7-one* (5-6) ¹H NMR (400 MHz, DMSO-*d₆*):** *δ* 8.72 (s, 1H), 8.64 (s, 1H), 8.18 (s, 1H), 7.08 (d, *J*=7.8 Hz, 1H), 6.10 (d, *J* =7.6 Hz, 1H), 3.78 (s, 3H), 3.32-3.25 (m, 1H), 1.02-0.98 (m, 2H), 0.83-0.81 (m, 2H) ppm. **LC-MS (ESI, m/z):** calcd for [M+H]⁺ 467.1; found: 467.1. **Retention time:** 2.18 min. [α]²⁵_{D} = - 99.800° (c = 0.2535 g/100ml, MeOH).

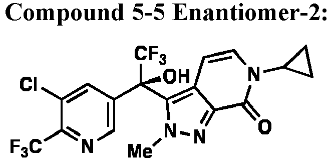

***(R)-3-(1-(5-chloro-6-(trifluoromethyl)pyridin-3-yl)-2,2,2-trifluoro-1-hydroxyethyl)-6-cyclopropyl-2-methyl-2,6-dihydro-7H-pyrazolo[3,4-c]pyridin-7-one* (5-5) ¹H NMR (400 MHz, DMSO-*d₆*):** *δ* 8.72 (s, 1H), 8.64 (s, 1H), 8.18 (s, 1H), 7.08 (d, *J*=7.8 Hz, 1H), 6.10 (d, *J* =7.6 Hz, 1H), 3.78 (s, 3H), 3.32-3.25 (m, 1H), 1.02-0.98 (m, 2H), 0.83-0.81 (m, 2H) ppm. **LC-MS (ESI, m/z):** calcd for [M+H]⁺ 467.1; found: 467.1. **Retention time:** 2.63 min. [α]²⁵_{D} = +104.480° (c = 0.2565 g/100ml, MeOH).

### Synthesis of compounds 5-7, 5-8

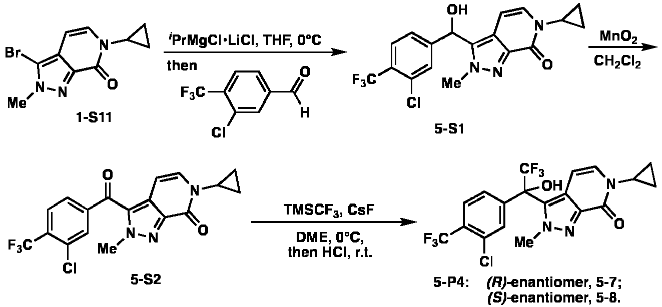

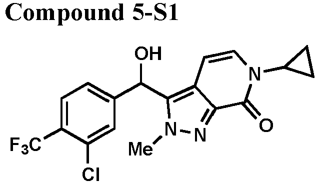

### 3-((3-chloro-4-(trifluoromethyl)phenyl)(hydroxy)methyl)-6-cyclopropyl-2-methyl-2,6-dihydro-7H-pyrazolo[3,4-c]pyridin-7-one (5-S1)

To a stirred solution of 3-bromo-6-cyclopropyl-2-methyl-2,6-dihydro-7*H*-pyrazolo[3,4-*c*]pyridin-7-one (**1-S11**) (1000 mg, 3.73 mmol) in anhydrous THF (10 mL), iPrMgCl•LiCl (1.3M in THF) (3.73 mL, 4.84 mmol) was added dropwise at 0 °C and stirred for 10 minutes. After that 3-chloro-4-(trifluoromethyl)benzaldehyde (780 mg, 3.73 mmol) dissolved in anhydrous THF (5 mL) was added and the reaction mixture was stirred at 0 °C for 30 minutes. After completion, the reaction mixture was quenched with aqueous saturated NH₄Cl solution, extracted with ethyl acetate, washed with brine, dried over anhydrous Na₂SO₄ and concentrated. The crude product was purified by CombiFlash chromatography (90% ethyl acetate in hexane) to afford 3-((3-chloro-4-(trifluoromethyl)phenyl)-(hydroxy)methyl)-6-cyclopropyl-2-methyl-2,6-dihydro-7*H*-pyrazolo[3,4-c]pyridin-7-one (**5-S1**) as an off-white solid (550 mg, 37%). **LC-MS (ESI, m/z):** calcd for [M+H]⁺ 398.1; found: 398.1.

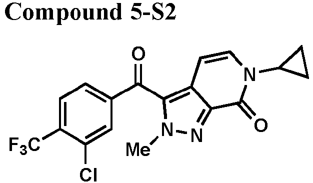

### 3-(3-chloro-4-(trifluoromethyl)benzoyl)-6-cyclopropyl-2-methyl-2,6-dihydro-7H-pyrazolo[3,4-c]pyridin-7-one (5-S2)

To a stirred solution of 3-((3-chloro-4-(trifluoromethyl)phenyl)(hydroxy)methyl)-6-cyclopropyl-2-methyl-2,6-dihydro-7*H*-pyrazolo[3,4-*c*]pyridin-7-one (**5-S1**) (550 mg, 1.38 mmol) in dichloromethane (15 mL), MnO₂ (1202 mg, 13.82 mmol) was added and the mixture was stirred at room temperature for 14 h. After completion, the reaction mixture was filtered through Celite bed, washed with dichloromethane and the filtrate was concentrated to afford 3-(3-chloro-4-(trifluoromethyl)benzoyl)-6-cyclopropyl-2-methyl-2,6-dihydro-7*H*-pyrazolo[3,4-*c*]pyridin-7-one **(5-S2)** as a yellow solid (460 mg, 84%). **¹H NMR (400 MHz, DMSO-*d₆*):** *δ* 8.10 (d, *J* = 8.0 Hz, 1H), 8.05 (s, 1H), 7.88 (d, *J* = 8.1 Hz, 1H), 7.14 (d, *J* = 7.5 Hz, 1H), 5.50 (d, *J* = 7.4 Hz, 1H), 4.31 (s, 3H), 3.29-3.23 (m, 1H), 1.00-0.95 (m, 2H), 0.80-0.70 (m, 2H) ppm. **LC-MS (ESI, m/z):** calcd for [M+H]⁺ 396.1; found: 396.28.

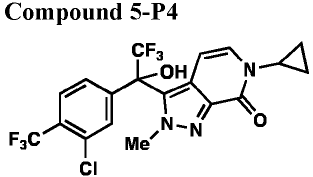

### 3-(1-(3-chloro-4-(trifluoromethyl)phenyl)-2,2,2-trifluoro-1-hydroxyethyl)-6-cyclopropyl-2-methyl-2,6-dihydro-7H-pyrazolo[3,4-c]pyridin-7-one (5-P4)

Following **General Procedure F5** on 0.38 mmol scale with 3-(3-chloro-4-(trifluoromethyl)benzoyl)-6-cyclopropyl-2-methyl-2,6-dihydro-7*H*-pyrazolo[3,4-*c*]pyridin-7-one (**5-S2**). Purification CombiFlash chromatography (70% ethyl acetate in hexane) to afford 3-(1-(3-chloro-4-(trifluoromethyl)phenyl)-2,2,2-trifluoro-1-hydroxyethyl)-6-cyclopropyl-2-methyl-2,6-dihydro-7*H*-pyrazolo[3,4-*c*]pyridin-7-one (**5-P4**) as an off-white solid (120 mg, 68%). **Physical state:** off-white solid.

The racemic compound **5-P4** was purified from Prep-HPLC-SFC to afford Enantiomer-1 (**5-8**) (50 mg) and Enantiomer-2 (**5-7**) (50 mg).

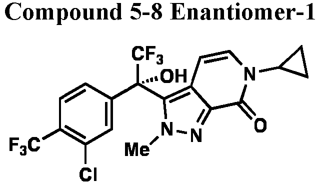

***(S)-6-cyclopropyl-2-methyl-3-(2,2,2-trifluoro-1-hydroxy-1-(6-(trifluoromethyl)pyridin-3-yl)ethyl)-2,6-dihydro-7H-pyrazolo[3,4-c]pyridin-7-one* (5-8) ¹H NMR (400 MHz, DMSO-*d₆*):** *δ* 8.54 (s, 1H), 7.94 (d, *J* = 8.3 Hz, 1H), 7.71 (s, 1H), 7.40 (d, *J* = 8.2 Hz, 1H), 7.08 (d,*J* = 7.8 Hz, 1H), 6.16 (d, *J* = 7.4 Hz, 1H), 3.70 (s, 3H), 3.29-3.24 (m, 1H), 1.02-0.99 (m, 2H), 0.83-0.81 (m, 2H) ppm. **LC-MS (ESI, m/z):** calcd for [M+H]⁺ 466.1; found: 466.1. **Retention time:** 2.68 min.

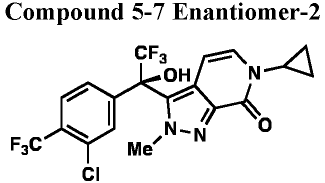

***(R)-6-cyclopropyl-2-methyl-3-(2,2,2-trifluoro-1-hydroxy-1-(6-(trifluoromethyl)pyridin-3-yl)ethyl)-2,6-dihydro-7H-pyrazolo[3,4-c]pyridin-7-one* (5-7) ¹H NMR (400 MHz, DMSO-d*₆*):** *δ* 8.54 (s, 1H), 7.94 (d, *J* = 8.3 Hz, 1H), 7.71 (s, 1H), 7.40 (d, *J* = 8.2 Hz, 1H), 7.08 (d, *J =* 7.8 Hz, 1H), 6.16 (d, *J* = 7.4 Hz, 1H), 3.70 (s, 3H), 3.29-3.25 (m, 1H), 1.02-0.99 (m, 2H), 0.83-0.81 (m, 2H) ppm. **LC-MS (ESI, m/z):** calcd for [M+H]⁺ 466.1; found: 466.1. **Retention time:** 3.37 min.

### Synthesis of compounds 5-9, 5-10

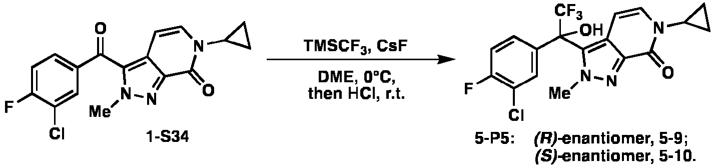

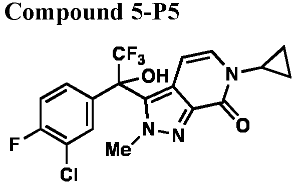

### 3-(1-(3-chloro-4-fluorophenyl)-2,2,2-trifluoro-1-hydroxyethyl)-6-cyclopropyl-2-methyl-2,6-dihydro-7H-pyrazolo[3,4-c]pyridin-7-one (5-P5)

Following **General Procedure F5** on 0.83 mmol scale with 3-(3-chloro-4-fluorobenzoyl)-6-cyclopropyl-2-methyl-2,6-dihydro-7*H*-pyrazolo[3,4-*c*]pyridin-7-one (**1-S34**). TMSCF₃ (0.24 mL, 1.65 mmol) followed by cesium fluoride (13 mg, 0.08 mmol) was added at 0 °C and the mixture was stirred at room temperature for 16 h and quenched with aqueous saturated NH₄Cl solution. Purification CombiFlash chromatography (70% ethyl acetate in hexane) to afford 3-(1-(3-chloro-4-fluorophenyl)-2,2,2-trifluoro-1-hydroxyethyl)-6-cyclopropyl-2-methyl-2,6-dihydro-7*H*-pyrazolo-[3,4-*c*]pyridin-7-one (60 mg, 17%) as an off-white solid. **Physical state:** off-white solid.

The racemic compound **5-P5** was submitted for chiral separation to afford enantiomer-1 (**5-10**) (20 mg) and enantiomer-2 (**5-9**) (20 mg).

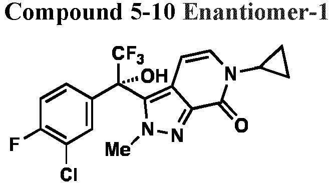

***(S)-3-(1-(3-chloro-4-fluorophenyl)-2,2,2-trifluoro-1-trifluoro-1-hydroxyethyl)-6-cyclopropyl-2-methyl-2,6-dihydro-7H-pyrazolo[3,4-c]pyridin-7-one* (5-10) ¹H NMR (400 MHz, DMSO-*d₆*):** *δ* 8.33 (s, 1H), 7.56 (d, *J* = 5.4 Hz, 1H), 7.48 (t, *J =* 8.9 Hz, 1H), 7.25-7.20 (m, 1H), 7.07 (d, *J =* 7.8 Hz, 1H), 6.14 (d, *J =* 7.5 Hz, 1H), 3.71 (s, 3H), 3.29-3.24 (m, 1H), 1.02-0.97 (m, 2H), 0.85-0.80 (m, 2H) ppm. **LC-MS (ESI, m/z):** calcd for [M+H]⁺ 416.1; found: 416.22. **Retention time:** 3.83 min.

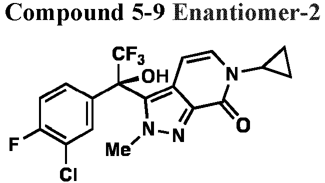

***(R)-3-(1-(3-chloro-4-fluorophenyl)-2,2,2-trifluoro-1-hydroxyethyl)-6-cyclopropyl-2-methyl-2,6-dihydro-7H-pyrazolo[3,4-c]pyridin-7-one* (5-9) ¹H NMR (400 MHz, DMSO-*d₆*):** *δ* 8.33 (s, 1H), 7.56 (d, *J* = 5.4 Hz, 1H), 7.48 (t, *J =* 8.9 Hz, 1H), 7.25-7.20 (m, 1H), 7.07 (d, *J =* 7.8 Hz, 1H), 6.14 (d, *J =* 7.5 Hz, 1H), 3.71 (s, 3H), 3.29-3.24 (m, 1H), 1.04-0.99 (m, 2H), 0.86-0.81 (m, 2H) ppm. **LC-MS (ESI, m/z):** calcd for [M+H]⁺ 416.1; found: 416.26. **Retention time:** 4.38 min.

### Synthesis of compounds 5-11, 5-12

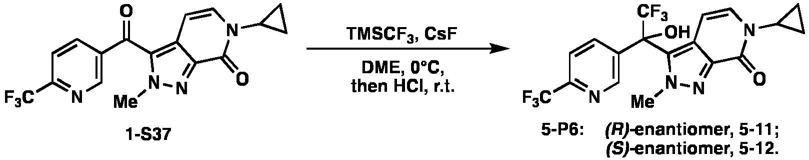

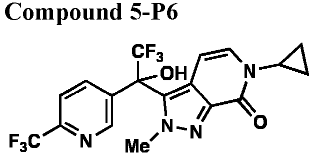

### 6-cyclopropyl-2-methyl-3-(2,2,2-trifluoro-1-hydroxy-1-(6-(trifluoromethyl)pyridin-3-yl)ethyl)-2,6-dihydro-7H-pyrazolo[3,4-c]pyridin-7-one (5-P6)

Following **General Procedure F5** on 1.24 mmol scale with 6-cyclopropyl-2-methyl-3-(6-(trifluoromethyl)nicotinoyl)-2,6-dihydro-7*H*-pyrazolo[3,4-*c*]pyridin-7-one (**1-S37**). TMSCF₃ (0.24 mL, 1.65 mmol) followed by cesium fluoride (13 mg, 0.08 mmol) was added at 0 °C and the mixture was stirred at room temperature for 16 h and quenched with aqueous saturated NH₄Cl solution. Purification by CombiFlash chromatography (70% ethyl acetate in hexane) to afford 6-cyclopropyl-2-methyl-3-(2,2,2-trifluoro-1-hydroxy-1-(6-(trifluoromethyl)pyridin-3-yl)ethyl)-2,6-dihydro-7*H*-pyrazolo[3,4-*c*]pyridin-7-one (**5-P6**) (40 mg, 8%) as an off-white solid. **Physical state:** off-white solid.

The racemic compound **5-P6** was purified from Prep-HPLC-SFC to afford enantiomer-1 (**5-12**) (11 mg) and enantiomer-2 (**5-11**) (12 mg).

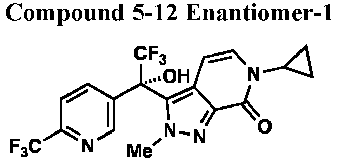

### (S)-6-cyclopropyl-2-methyl-3-(2,2,2-trifluoro-1-hydroxy-1-(6-(trifluoromethyl)pyridin-3-yl)ethyl)-2,6-dihydro-7H-pyrazolo[3,4-c]pyridin-7-one (5-12)

**¹H NMR (400 MHz, DMSO-*d₆*):** *δ* 8.79 (s, 1H), 8.61 (s, 1H), 8.00 (s, 2H), 7.07 (d, *J =* 7.8 Hz, 1H), 6.07 (d, *J =* 7.3 Hz, 1H) 3.75 (s, 3H), 3.29-3.25 (m, 1H), 1.01-0.98 (m, 2H), 0.85-0.80 (m, 2H) ppm. **LC-MS (ESI, m/z):** calcd for [M+H]⁺ 433.1; found: 433.1. **Retention time:** 1.30 min.

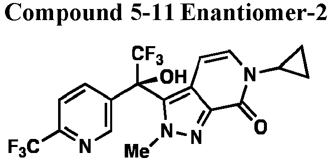

***(R)-6-cyclopropyl-2-methyl-3-(2,2,2-trifluoro-1-hydroxy-1-(6-(trifluoromethyl)pyridin-3-yl)ethyl)-2,6-dihydro-7H-pyrazolo[3,4-c]pyridin-7-one* (5-11) ¹H NMR (400 MHz, DMSO-*d₆*):** *δ* 8.79 (s, 1H), 8.61 (s, 1H), 8.00 (s, 2H), 7.07 (d, *J =* 7.8 Hz, 1H), 6.07 (d, *J =* 7.3 Hz, 1H) 3.75 (s, 3H), 3.28-3.25 (m, 1H), 1.00-0.98 (m, 2H), 0.85-0.80 (m, 2H) ppm. **LC-MS (ESI, m/z):** calcd for [M+H]⁺ 433.1; found: 433.1. **Retention time:** 1.68 min.

### Synthesis of compounds 5-13 and 5-14

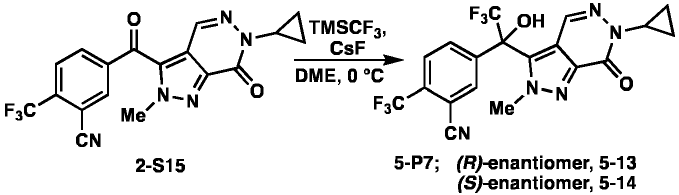

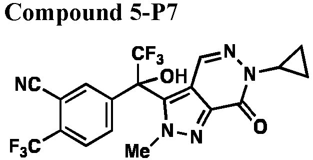

### 5-(1-(6-cyclopropyl-2-methyl-7-oxo-6,7-dihydro-2H-pyrazolo[3,4-d]pyridazin-3-yl)-2,2,2-trifluoro-1-hydroxyethyl)-2-(trifluoromethyl)benzonitrile (5-P7)

To a stirred solution of 5-(6-cyclopropyl-2-methyl-7-oxo-6,7-dihydro-2*H*-pyrazolo[3,4-*d*]pyridazine-3-carbonyl)-2-(trifluoromethyl)benzonitrile (**2-S15**) (300 mg, 0.78 mmol) in DME (5 mL), TMSCF₃ (0.23 mL, 1.55 mmol) followed by CsF (12 mg, 0.08 mmol) was added at 0 °C and the reaction mixture was stirred at room temperature for 16 h. After that 5N HCl (0.3 mL) was added and stirred at room temperature for 2 h. After completion, the reaction mixture was concentrated, neutralized with aqueous NaHCO₃ solution, extracted with ethyl acetate, washed with brine, dried over anhydrous Na₂SO₄ and concentrated. The crude product was purified by Combiflash chromatography (70% ethyl acetate in hexane) to afford 5-(1-(6-cyclopropyl-2-methyl-7-oxo-6,7-dihydro-2*H*-pyrazolo[3,4-*d*]pyridazin-3-yl)-2,2,2-trifluoro-1-hydroxyethyl)-2-(trifluoromethyl)benzonitrile (**5-P7**) as an off white solid (220 mg, 62%).

**Physical State:** off white solid. The racemic compound **5-P7** was purified from preparative-HPLC-SFC to afford Enantiomer-1 (**5-14**) (60 mg) and Enantiomer-2 (**5-13**) (60 mg).

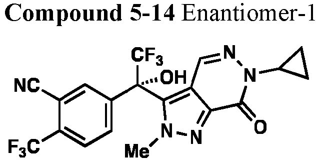

### (S)-5-(1-(6-cyclopropyl-2-methyl-7-oxo-6,7-dihydro-2H-pyrazolo[3,4-d]pyridazin-3-yl)-2,2,2-trifluoro-1-hydroxyethyl)-2-(trifluoromethyl)benzonitrile (5-14)

**¹H NMR (400 MHz, DMSO-*d₆*):** *δ* 8.88 (s, 1H), 8.18 (s, 1H), 8.10 (d, *J =* 8.4 Hz, 1H), 8.06 (s, 1H), 8.00 (d, *J* = 8.2 Hz, 1H), 4.05-3.95 (m, 1H), 3.71 (s, 3H), 1.00-0.95 (m, 4H) ppm. **LC-MS** (ESI, **m/z):** calcd for [M+H]⁺ 458.2; found: 458.3.

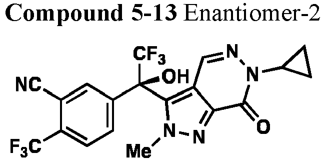

### (R)-3-(1-(6-cyclopropyl-2-methyl-7-oxo-6,7-dihydro-2H-pyrazolo[3,4-d]pyridazin-3-yl)-2,2,2-trifluoro-1-hydroxyethyl)-2-(trifluoromethyl)benzonitrile (5-13)

**¹H NMR (400 MHz, DMSO-*d₆*):** *δ* 8.88 (s, 1H), 8.18 (s, 1H), 8.10 (d, *J =* 8.4 Hz, 1H), 8.06 (s, 1H), 8.00 (d, *J* = 8.2 Hz, 1H), 4.05-3.95 (m, 1H), 3.71 (s, 3H), 1.02-0.95 (m, 4H) ppm. **LC-MS (ESI, m/z):** calcd for [M+H]⁺ 458.2; found: 458.3.

### Synthesis of compounds 5-15 and 5-16

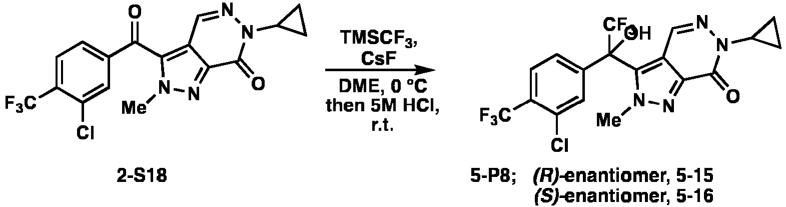

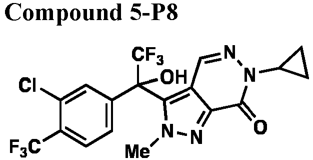

### 3-(1-(3-chloro-4-(trifluoromethyl)phenyl)-2,2,2-trifluoro-1-hydroxyethyl)-6-cyclopropyl-2-methyl-2,6-dihydro-7H-pyrazolo[3,4-d]pyridazin-7-one (5-P8)

To a stirred solution of 3-(3-chloro-4-(trifluoromethyl)benzoyl)-6-cyclopropyl-2-methyl-2,6-dihydro-7*H*-pyrazolo[3,4-*d*]pyridazin-7-one (**2-S18**) (395 mg, 0.10 mmol) in DME (5 mL), TMSCF₃ (0.29 mL, 1.99 mmol) followed by CsF (76 mg, 0.50 mmol) was added at 0 °C and the mixture was stirred at room temperature for 16 h. After that 5N HCl (0.4 mL) was added and stirred at room temperature for 2 h. After completion, the reaction mixture was concentrated, neutralized with aqueous NaHCO₃ solution, extracted with ethyl acetate, washed with brine, dried over anhydrous Na₂SO₄ and concentrated. The crude product was purified by Combiflash chromatography (70% ethyl acetate in hexane) to afford 3-(1-(3-chloro-4-(trifluoro methyl)phenyl)-2,2,2-trifluoro-1-hydroxyethyl)-6-cyclopropyl-2-methyl-2,6-dihydro-7*H-*pyrazolo[3,4-*d*]pyridazin-7-one (**5-P8**) as an off white solid (160 mg, 34%). **Physical State:** off white solid. The racemic compound **5-P8** was purified from preparative-HPLC-SFC to afford Enantiomer-1 (**5-16**) (70 mg) and Enantiomer-2 (**5-15**) (70 mg).

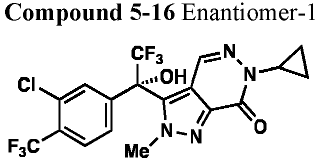

### (S)-3-(1-(3-chloro-4-(trifluoromethyl)phenyl)-2,2,2-trifluoro-1-hydroxyethyl)-6-cyclopropyl-2-methyl-2,6-dihydro-7H-pyrazolo[3,4-d]pyridazin-7-one (5-16)

**¹H NMR (400 MHz, DMSO-*d₆*):** *δ* 8.79 (s, 1H), 8.03 (s, 1H), 7.94 (d, *J =* 8.3 Hz, 1H), 7.79 (s, 1H), 7.52 (d, *J =* 7.8 Hz, 1H), 4.00-3.95 (m, 1H), 3.73 (s, 3H), 1.00-0.95 (m, 4H) ppm. **LC-MS (ESI, m/z):** calcd for [M+H]⁺ 467.1; found: 467.3.

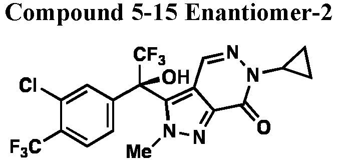

### (R)-3-(1-(3-chloro-4-(trifluoromethyl)phenyl)-2,2,2-trifluoro-1-hydroxyethyl)-6-cyclopropyl-2-methyl-2,6-dihydro-7H-pyrazolo[3,4-d]pyridazin-7-one (5-15)

**¹H NMR (400 MHz, DMSO-*d₆*):** *δ* 8.79 (s, 1H), 8.03 (s, 1H), 7.94 (d, *J =* 8.3 Hz, 1H), 7.79 (s, 1H), 7.52 (d, *J* = 7.8 Hz, 1H), 4.00-3.95 (m, 1H), 3.73 (s, 3H), 1.00-0.95 (m, 4H) ppm. **LC-MS (ESI, m/z):** calcd for [M+H]⁺ 467.1; found: 467.2.

### Synthesis of compounds 5-17 and 5-18

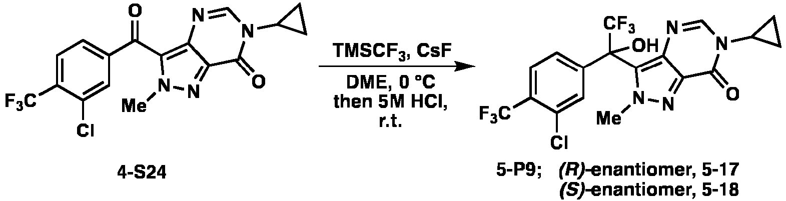

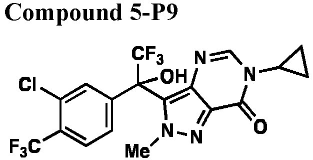

### 3-(1-(3-chloro-4-(trifluoromethyl)phenyl)-2,2,2-trifluoro-1-hydroxyethyl)-6-cyclopropyl-2-methyl-2,6-dihydro-7H-pyrazolo[4,3-d]pyrimidin-7-one (5-P9) (Target-88)

To a stirred solution of 3-(3-chloro-4-(trifluoromethyl)benzoyl)-6-cyclopropyl-2-methyl-2,6-dihydro-7*H*-pyrazolo[4,3-*d*]pyrimidin-7-one (**4-S24**) (242 mg, 0.61 mmol) in DME (5 mL), TMSCF₃ (0.11 mL, 0.73 mmol) followed by CsF (9 mg, 0.06 mmol) was added at 0 °C and the reaction mixture was stirred at room temperature for 2 h. After that 5N hydrochloric acid (0.5 mL) was added and stirred at 50 °C for 2 h. After completion, the reaction mixture was concentrated, neutralized with aqueous NaHCO₃ solution, extracted with ethyl acetate, washed with brine, dried over anhydrous Na₂SO₄ and concentrated. The crude product was purified by Combi-flash chromatography (70% ethyl acetate in hexane) to afford 3-(1-(3-chloro-4-(trifluoromethyl)phenyl)-2,2,2-trifluoro-1-hydroxyethyl)-6-cyclopropyl-2-methyl-2,6-dihydro-7*H*-pyrazolo[4,3-*d*]pyrimidin-7-one (**5-P9**) as an off white solid (120 mg, 42%). **Physical State:** off white solid. The racemic compound **5-P9** was purified from preparative HPLC (SFC) to afford Enantiomer-1 **(5-18)** (50 mg) and Enantiomer-2 **(5-17)** (50 mg).

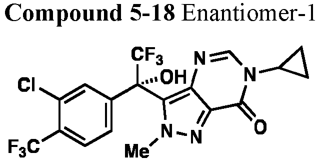

### (S)-3-(1-(3-chloro-4-(trifluoromethyl)phenyl)-2,2,2-trifluoro-1-hydroxyethyl)-6-cyclopropyl-2-methyl-2,6-dihydro-7H-pyrazolo[4,3-d]pyrimidin-7-one (5-18)

**¹H NMR (400 MHz, DMSO-*d₆*):** *δ* 8.48 (s, 1H), 8.10 (s, 1H), 7.93 (d, *J =* 8.3 Hz, 1H), 7.71 (s, 1H), 7.44 (d, *J =* 8.2 Hz, 1H), 3.72 (s, 3H), 3.20-3.15 (m, 1H), 1.05-1.00 (m, 2H), 0.95-0.90 (m, 2H) ppm. **LC-MS (ESI, m/z):** calcd for [M+H]⁺ 467.2; found: 467.2. **Retention time:** 3.07 min. **[α]²⁵_{D}** = -79.889° (c = 0.2503 g/100 cm³, CH₃OH)

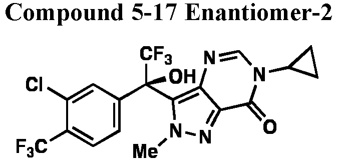

### (R)-3-(1-(3-chloro-4-(trifluoromethyl)phenyl)-2,2,2-trifluoro-1-hydroxyethyl)-6-cyclopropyl-2-methyl-2,6-dihydro-7H-pyrazolo[4,3-d]pyrimidin-7-one (5-17)

**¹H NMR (400 MHz, DMSO-*d₆*):** *δ* 8.48 (s, 1H), 8.09 (s, 1H), 7.93 (d, *J =* 8.3 Hz, 1H), 7.71 (s, 1H), 7.44 (d, *J =* 8.2 Hz, 1H), 3.71 (s, 3H), 3.21-3.15 (m, 1H), 1.05-1.00 (m, 2H), 0.95-0.90 (m, 2H) ppm. **LC-MS (ESI, m/z):** calcd for [M+H]⁺ 467.2; found: 467.2.

**Retention time:** 3.54 min. **[α]²⁵_{D}** = +79.598° (c = 0.2500 g/100 cm³, CH₃OH)

### Synthesis of compounds 5-19 and 5-20

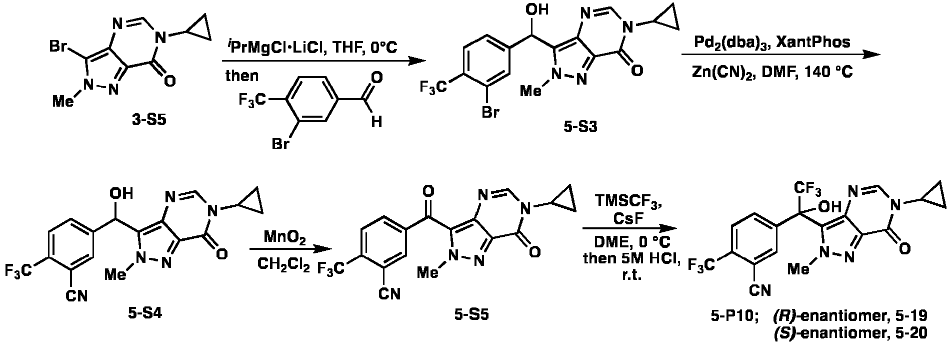

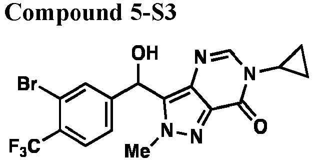

### 3-((3-bromo-4-(trifluoromethyl)phenyl)(hydroxy)methyl)-6-cyclopropyl-2-methyl-2,6-dihydro-7H-pyrazolo[4,3-d]pyrimidin-7-one (5-S3)

To a stirred solution of 3-bromo-6-cyclopropyl-2-methyl-2,6-dihydro-7*H*-pyrazolo[3,4-*c*]pyridin-7-one (**3-S5**) (800 mg, 2.97 mmol) in anhydrous THF (7 mL), iPrMgCl•LiCl (2.97 mL, 3.86 mmol) was added dropwise at 0 °C and stirred for 10 minutes. After that 3-bromo-4-(trifluoromethyl)benzaldehyde (752 mg, 2.97 mmol) dissolved in anhydrous THF (3 mL) was added and stirred at 0 °C for 30 minutes. After completion, the reaction mixture was quenched with aqueous saturated NH₄Cl solution, extracted with ethyl acetate, washed with brine, dried over anhydrous Na₂SO₄ and concentrated. The crude product was purified by Combi-Flash chromatography (65% ethyl acetate in hexane) to afford 3-((3-bromo-4-(trifluoromethyl)phenyl)(hydroxy)methyl)-6-cyclopropyl-2-methyl-2,6-dihydro-7*H*pyrazolo [4,3-*d*]pyrimidin-7-one (**5-S3**) as an off white solid (700 mg, 53%). **Physical State:** off white **solid.¹H NMR (400 MHz, DMSO-*d₆*):** *δ* 7.99 (s, 1H), 7.86 (s, 1H), 7.82 (d, *J =* 8.2 Hz, 1H), 7.50 (d, *J =* 8.2 Hz, 1H), 6.80 (d, *J =* 4.4 Hz, 1H), 6.34 (d, *J =* 3.6 Hz, 1H), 3.96 (s, 3H), 3.16-3.12 (m, 1H), 1.01-0.99 (m, 2H), 0.89-0.86 (m, 2H) ppm. **LC-MS (ESI, m/z):** calcd for [M+H]⁺ 443.2; found: 443.2.

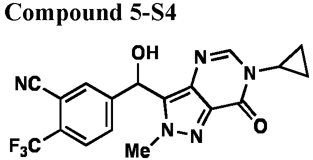

### 5-((6-cyclopropyl-2-methyl-7-oxo-6,7-dihydro-2H-pyrazolo[4,3-d]pyrimidin-3-yl)(hydroxy)-methyl)-2-(trifluoromethyl)benzonitrile (5-S4)

In a reaction tube, a stirred solution of 3-((3-bromo-4-(trifluoromethyl)phenyl)(hydroxy)methyl)-6-cyclopropyl-2-methyl-2,6-dihydro-7*H-*pyrazolo[4,3-*d*]pyrimidin-7-one (**5-S3**) (250 mg, 0.56 mmol) in anhydrous DMF (5 mL) was degassed with argon for 15 minutes. After that Zn(CN)₂ (166 mg, 1.41 mmol) followed by Pd₂dba₃ (52 mg, 0.05 mmol) and Xantphos (65 mg, 0.11 mmol) were added. The tube was sealed and the reaction mixture was stirred at 140 °C for 16 h. After completion, the reaction mixture was diluted by water, extracted with ethyl acetate, washed with brine, dried over anhydrous Na₂SO₄ and concentrated. The crude product was purified by Combi-Flash chromatography (2-5% methanol in dichloromethane) to afford 5-((6-cyclopropyl-2-methyl-7-oxo-6,7-dihydro-2*H*-pyrazolo[4,3-*d*]pyrimidin-3-yl)(hydroxy)methyl)-2-(trifluoromethyl)-benzonitrile **(5-S4)** as an off white solid (100 mg, 45%). **Physical State:** off white **solid.¹H NMR (400 MHz, DMSO-*d₆*):** *δ* 8.10 (s, 1H), 8.01 (d, *J* = 8.2 Hz, 1H), 7.95 (s, 1H), 7.90 (d, *J* = 8.2 Hz, 1H), 6.86 (d, *J* = 4.6 Hz, 1H), 6.38 (d, *J =* 4.3 Hz, 1H), 4.00 (s, 3H), 3.15-3.10 (m, 1H), 1.01-0.99 (m, 2H), 0.87-0.86 (m, 2H) ppm. **LC-MS (ESI, m/z):** calcd for [M+H]⁺ 390.2; found: 390.2.

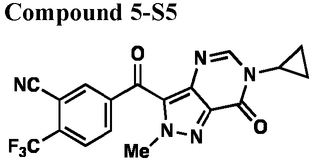

### 5-(6-cyclopropyl-2-methyl-7-oxo-6,7-dihydro-2H-pyrazolo[4,3-d]pyrimidine-3-carbonyl)-2-(trifluoromethyl)benzonitrile (5-S5)

To a stirred solution of 5-((6-cyclopropyl-2-methyl-7-oxo-6,7-dihydro-2*H*-pyrazolo[4,3-*d*]pyrimidin-3-yl)(hydroxy)methyl)-2-(trifluoromethyl)benzonitrile **(5-S4)** (250 mg, 0.64 mmol) in DCM (10 mL), MnO₂ (558 mg, 6.42 mmol) was added and the reaction mixture was stirred at room temperature for 16 h. After completion, the reaction mixture was filtered through Celite bed, washed with dichloromethane and the filtrate was concentrated to afford 5-(6-cyclopropyl-2-methyl-7-oxo-6,7-dihydro-2*H*-pyrazolo[4,3-*d*]pyrimidine-3-carbonyl)-2-(trifluoro methyl)benzonitrile (**5-S5**) as an off white solid (200 mg, 80%). **Physical State:** off white solid.

**¹H NMR (400 MHz, DMSO-d*₆*):** *δ* 8.54 (s, 1H), 8.30 (d, *J =* 8.2 Hz, 1H), 8.20 (d, *J =* 8.2 Hz, 1H), 8.11 (s, 1H), 4.33 (s, 3H), 3.20-3.15 (m, 1H), 1.03-0.98 (m, 2H), 0.90-0.86 (m, 2H) ppm. **LC-MS (ESI, m/z):** calcd for [M+H]⁺ 388.2; found: 388.3.

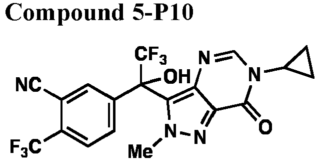

### 5-(1-(6-cyclopropyl-2-methyl-7-oxo-6,7-dihydro-2H-pyrazolo[4,3-d]pyrimidin-3-yl)-2,2,2-trifluoro-1-hydroxyethyl)-2-(trifluoromethyl)benzonitrile (5-P10)

To a stirred solution of 5-(6-cyclopropyl-2-methyl-7-oxo-6,7-dihydro-2*H*-pyrazolo[4,3-*d*]pyrimidine-3-carbonyl)-2-(trifluoromethyl)benzonitrile (**5-S5**) (190 mg, 0.49 mmol) in DME (5 mL), (trifluoromethyl)trimethylsilane ( 0.08 mL, 0.58 mmol) followed by cesium fluoride (7 mg, 0.04 mmol) was added at 0 °C and the reaction mixture was stirred at room temperature for overnight. After that 5 M HCl (0.2 mL) was added and the mixture was stirred at room temperature for 2 h. After completion, the reaction mixture was concentrated, neutralized with aqueous NaHCO₃ solution, extracted with ethyl acetate, washed with brine, dried over anhydrous Na₂SO₄ and concentrated. The crude product was purified by Combi-Flash chromatography (70% ethyl acetate in hexane) to afford 5-(1-(6-cyclopropyl-2-methyl-7-oxo-6,7-dihydro-2*H*-pyrazolo[4,3-*d*]pyrimidin-3-yl)-2,2,2-trifluoro-1-hydroxyethyl)-2-(trifluoro methyl)benzonitrile **(5-P10)** as an off white solid (65 mg, 28%). **Physical State:** off white solid. The racemic compound **5-P10** was purified from Prep-HPLC-SFC to afford Enantiomer-1 (**5-20**) (15 mg) and Enantiomer-2 (**5-19**) (16 mg).

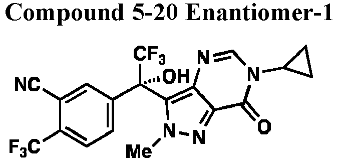

### (S)-5-(1-(6-cyclopropyl-2-methyl-7-oxo-6,7-dihydro-2H-pyrazolo[4,3-d]pyrimidin-3-yl)-2,2,2-trifluoro-1-hydroxyethyl)-2-(trifluoromethyl)benzonitrile (5-20)

**¹H NMR (400 MHz, DMSO-d*₆*):** *δ* 8.58 (s, 1H), 8.11-8.08 (m, 3H), 7.88 (d, *J* = 8.7 Hz, 1H),3.72 (s, 3H), 3.20-3.15 (m, 1H), 1.05-1.00 (m, 2H), 0.93-0.90 (m, 2H) ppm. **LC-MS (ESI, m/z):** calcd for [M+H]⁺ 458.2; found: 458.2. **Retention time:** 2.28 min

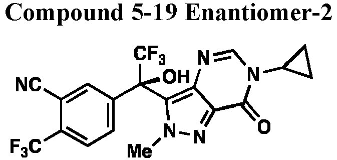

### (R)-5-(1-(6-cyclopropyl-2-methyl-7-oxo-6,7-dihydro-2H-pyrazolo[4,3-d]pyrimidin-3-yl)-2,2,2-trifluoro-1-hydroxyethyl)-2-(trifluoromethyl)benzonitrile (5-19)

**¹H NMR (400 MHz, DMSO-d*₆*):** *δ* 8.59 (s, 1H), 8.11-8.08 (m, 3H), 7.88 (d, *J =* 8.7 Hz, 1H), 3.72 (s, 3H), 3.20-3.15 (m, 1H), 1.05-1.00 (m, 2H), 0.93-0.90 (m, 2H) ppm. **LC-MS (ESI, m/z):** calcd for [M+H]⁺ 458.2; found: 458.2. **Retention time:** 2.57 min

### Synthesis of compounds 5-21 and 5-22

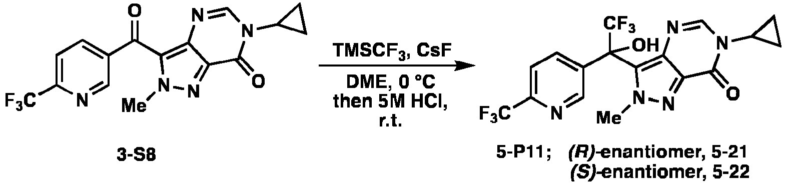

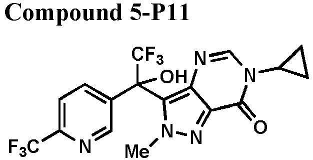

### 6-cyclopropyl-2-methyl-3-(2,2,2-trifluoro-1-hydroxy-1-(6-(trifluoromethyl)pyridin-3-yl)ethyl)-2,6-dihydro-7H-pyrazolo[4,3-d]pyrimidin-7-one (5-P11):

To a stirred solution of 6-cyclopropyl-2-methyl-3-(6-(trifluoromethyl)nicotinoyl)-2,6-dihydro-7*H*-pyrazolo[4,3-*d*]pyrimidin-7-one (**3-S8**) (100 mg, 0.28 mmol) in DME (3 mL), CsF (8 mg, 0.06 mmol) followed by TMSCF₃ (0.06 mL, 0.41 mmol) was added at 0 °C and the reaction mixture was stirred at room temperature for 2 h. After that 5N HCl (0.5 mL) was added and the reaction mixture was stirred at 40 °C for 1 h (TLC-monitoring). After completion, the reaction mixture was concentrated, neutralized with aqueous NaHCO₃ solution, extracted with ethyl acetate, washed with brine, dried over anhydrous Na₂SO₄ and concentrated. The crude product was purified by CombiFlash chromatography (50% ethyl acetate in hexane) to afford 6-cyclopropyl-2-methyl-3-(2,2,2-trifluoro-1-hydroxy-1-(6-(trifluoromethyl)pyridin-3-yl)ethyl)-2,6-dihydro-7*H*-pyrazolo[4,3-*d*]pyrimidin-7-one **(5-P11)** as an off white solid (65 mg, 55%).

**Physical State:** off white solid. The racemic compound **5-P11** was purified from prep-HPLC-SFC to afford Enantiomer-1 (**5-22**) (20 mg) (RT 2.14 min) and Enantiomer-2 (**5**-**21**) (20 mg) (RT 2.51 min).

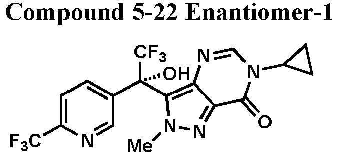

### (S)-6-cyclopropyl-2-methyl-3-(2,2,2-trifluoro-1-hydroxy-1-(6-(trifluoromethyl)pyridin-3-yl)ethyl)-2,6-dihydro-7H-pyrazolo[4,3-d]pyrimidin-7-one (5-170-enantiomer)

**¹H NMR (400 MHz, DMSO-*d₆*):** *δ* 8.79 (s, 1H), 8.56 (br s, 1H), 8.05-8.00 (m, 2H), 7.98 (d, *J* = 8.3 Hz, 1H), 3.83 (s, 3H), 3.19-3.14 (m, 1H), 1.05-1.00 (m, 2H), 0.95-0.90 (m, 2H) ppm. **LC-MS (ESI, m/z):** calcd for [M+H]⁺ 434.2; found: 434.3. Retention time: 2.14 min.

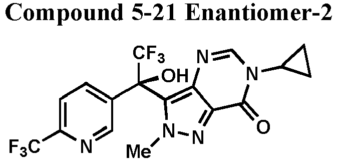

### (R)-6-cyclopropyl-2-methyl-3-(2,2,2-trifluoro-1-hydroxy-1-(6-(trifluoromethyl)pyridin-3-yl)ethyl)-2,6-dihydro-7H-pyrazolo[4,3-d]pyrimidin-7-one (5-21)

**¹H NMR (400 MHz, DMSO-*d₆*):** *δ* 8.79 (s, 1H), 8.54 (br s, 1H), 8.05-8.00 (m, 2H), 7.98 (d, *J* = 8.3 Hz, 1H), 3.83 (s, 3H), 3.19-3.14 (m, 1H), 1.04-1.00 (m, 2H), 0.92-0.88 (m, 2H) ppm. **LC-MS (ESI, m/z):** calcd for [M+H]⁺ 434.2; found: 434.3. Retention time: 2.51 min.

### Synthesis of compounds 5-22 and 5-23

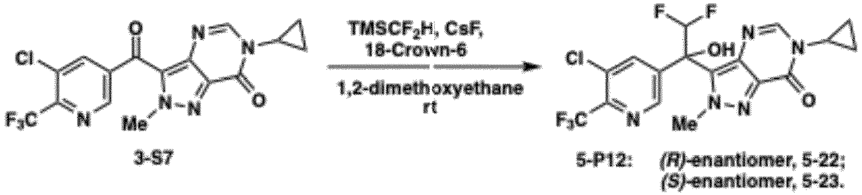

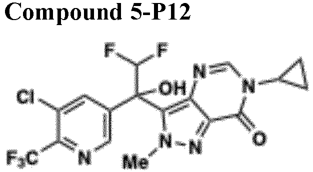

### 3-(1-(5-chloro-6-(trifluoromethyl)pyridin-3-yl)-2,2-difluoro-1-hydroxyethyl)-6-cyclopropyl-2-methyl-2,6-dihydro-7H-pyrazolo[4,3-d]pyrimidin-7-one (5-P12):

To a stirred solution of 3-(5-chloro-6-(trifluoromethyl)nicotinoyl)-6-cyclopropyl-2-methyl-2,6-dihydro-7*H*-pyrazolo[4,3-*d*]pyrimidin-7-one (**3-S7**) (150 mg, 0.38 mmol) in DME (3 mL), 18-Crown-6 (50 mg, 0.19 mmol) followed by CsF (29 mg, 0.19 mmol) and (difluoromethyl)trimethylsilane (70 mg, 0.57 mmol) were added at 0 °C and the mixture was stirred at room temperature for 16 h. After completion, the reaction mixture was quenched with aqueous saturated NH₄Cl solution, extracted with ethyl acetate, washed with water, brine, dried over anhydrous Na₂SO₄ and concentrated. The crude product was purified by CombiFlash chromatography (40% ethyl acetate in hexane) to afford 3-(1-(5-chloro-6-(trifluoromethyl)pyridin-3-yl)-2,2-difluoro-1-hydroxyethyl)-6-cyclopropyl-2-methyl-2,6-dihydro-7*H*-pyrazolo[4,3-*d*]pyrimidin-7-one (**5-P12**) as an off white solid (50 mg, 29%).

**Physical State:** off white solid. The racemic compound **5-P12** was purified from preparative-HPLC-SFC to afford Enantiomer-1 (**5-23**) (15 mg) and Enantiomer-2 (**5-22**) (15 mg).

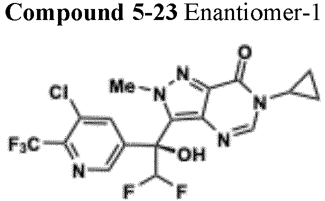

***(S)-3-(1-(5-chloro-6-(trifluoromethyl)pyridin-3-yl)-2,2-difluoro-1-hydroxyethyl)-6-cyclopropyl-2-methyl-2,6-dihydro-7H-pyrazolo[4,3-d]pyrimidin-7-one* (5-23) ¹H NMR (400 MHz, DMSO-*d₆*):** *δ* 8.61 (s, 1H), 8.12 (d, *J =* 8.1 Hz, 2H), 7.92 (s, 1H), 7.32 (t, *J =* 53.7 Hz, 1H), 3.83 (s, 3H), 3.21-3.17 (m, 1H), 1.05-1.00 (m, 2H), 0.95-0.90 (m, 2H) ppm. **LC-MS (ESI, m/z):** calcd for [M+H]⁺ 450.1; found: 450.2.

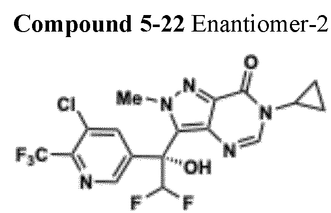

### (R)-3-(1-(5-chloro-6-(trifluoromethyl)pyridin-3-yl)-2,2-difluoro-1-hydroxyethyl)-6-cyclopropyl-2-methyl-2,6-dihydro-7H-pyrazolo[4,3-d]pyrimidin-7-one (5-22)

**¹H NMR (400 MHz, DMSO-*d₆*):** *δ* 8.61 (s, 1H), 8.12 (d, *J =* 7.7 Hz, 2H), 7.92 (s, 1H), 7.32 (t, *J =* 53.7 Hz, 1H), 3.83 (s, 3H), 3.22-3.18 (m, 1H), 1.05-1.00 (m, 2H), 0.95-0.90 (m, 2H) ppm. **LC-MS (ESI, m/z):** calcd for [M+H]⁺ 450.1; found: 450.2.

### Synthesis of compounds 5-26 and 5-27

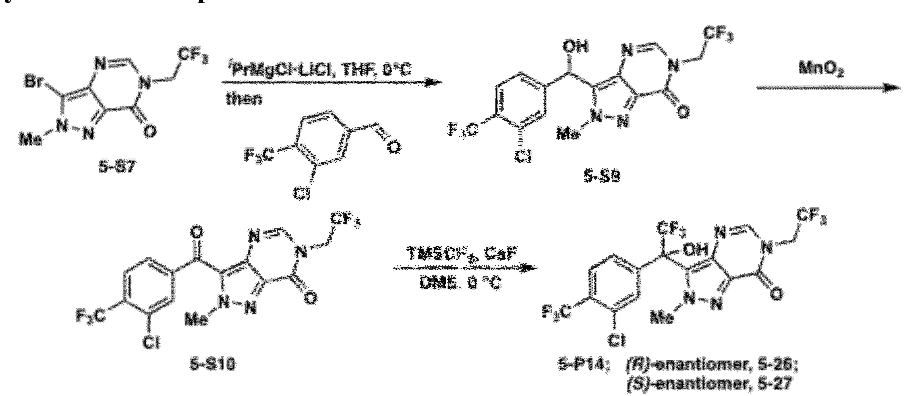

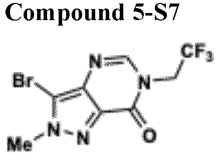

### 3-bromo-2-methyl-6-(2,2,2-trifluoroethyl)-2,6-dihydro-7H-pyrazolo[4,3-d]pyrimidin-7-one (5-S7)

To a stirred solution of 3-bromo-2-methyl-2,4-dihydro-7*H*-pyrazolo[4,3-*d*]pyrimidin-7-one (**3-S4**) (2.5 gm, 10.91 mmol) in anhydrous DMF (20 mL), Cs₂CO₃ (7.11 gm, 21.83 mmol) followed by 1,1,1-trifluoro-2-iodoethane (1.61 mL, 16.37 mmol) was added and the reaction mixture was stirred at 80°C for 16 h. After completion, the reaction mixture was diluted with cold water, extracted with ethyl acetate, washed with brine, dried over anhydrous Na₂SO₄ and concentrated. The crude product was purified by CombiFlash chromatography (40% ethyl acetate in hexane) to afford 3-bromo-2-methyl-6-(2,2,2-trifluoroethyl)-2,6-dihydro-7*H*-pyrazolo[4,3-d]pyrimidin-7-one (**5-S7**) (0.75 g, 22%) as an off white solid. **¹H NMR (400 MHz, DMSO-*d₆*)** *δ* 8.21 (s, 1H), 4.93 (q, *J =* 9.2 Hz, 2H), 4.09 (s, 3H) ppm; **LC-MS (ESI, m/z):** calcd for [M+H]⁺ 311.0; found: 311.0.

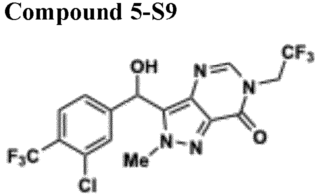

### 3-((3-chloro-4-(trifluoromethyl)phenyl)(hydroxy)methyl)-2-methyl-6-(2,2,2-trifluoroethyl)-2,6-dihydro-7H-pyrazolo[4,3-d]pyrimidin-7-one (5-S9):

To a stirred solution of 3-bromo-2-methyl-6-(2,2,2-trifluoroethyl)-2,6-dihydro-7*H-*pyrazolo[4,3-*d*]pyrimidin-7-one (**5-S7**) (300 mg, 0.96 mmol) in anhydrous THF (5 mL), was added iPrMgCl.LiCl (1.3 M in THF) (1 mL, 1.25 mmol) at 0 °C and stirred for 15 minutes. After that 3-chloro-4-(trifluoromethyl)benzaldehyde (201 mg, 0.96 mmol) in anhydrous THF (2 mL) was added and stirred at room temperature for 1 h. After completion, the reaction mixture was quenched with aqueous saturated NH₄Cl solution, extracted with ethyl acetate, washed with brine, dried over anhydrous Na₂SO₄ and concentrated. The crude product was purified by CombiFlash chromatography (50% ethyl acetate in hexane) to afford 3-((3-chloro-4-(trifluoromethyl)phenyl)(hydroxy)methyl)-2-methyl-6-(2,2,2-trifluoroethyl)-2,6-dihydro-7*H*-pyrazolo[4,3-*d*]pyrimidin-7-one (**5-S9**) as an off white solid (255 mg, 60%). **¹H NMR (400 MHz, CDCl₃):** *δ* 7.81 (s, 1H), 7.68 (d, *J* = 8.4 Hz, 1H), 7.57 (s, 1H), 7.33 (d, *J* = 9.0 Hz, 1H), 6.43 (s, 1H), 4.67 (q, *J =* 8.5 Hz, 2H), 3.95 (s, 3H) ppm. **LC-MS (ESI, m/z):** calcd for [M+H]⁺ 441.1; found: 441.1.

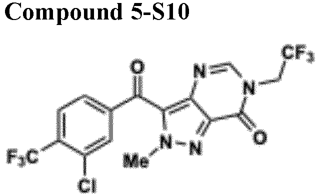

### 3-(3-chloro-4-(trifluoromethyl)benzoyl)-2-methyl-6-(2,2,2-trifluoroethyl)-2,6-dihydro-7H-pyrazolo[4,3-d]pyrimidin-7-one (5-S10):

To a stirred solution of 3-((3-chloro-4-(trifluoromethyl)phenyl)(hydroxy)methyl)-2-methyl-6-(2,2,2-trifluoroethyl)-2,6-dihydro-7*H*-pyrazolo[4,3-*d*]pyrimidin-7-one (**5-S9**) (250 mg, 0.57 mmol) in DCM (10 mL), MnO₂ (493 mg, 5.67 mmol) was added and the reaction mixture was stirred at room temperature for 4 h. After completion, the reaction mixture was filtered through celite bed, washed with dichloromethane and the filtrate was concentrated to afford 3-(3-chloro-4-(trifluoromethyl)benzoyl)-2-methyl-6-(2,2,2-trifluoroethyl)-2,6-dihydro-7*H*-pyrazolo[4,3-*d*]pyrimidin-7-one (**5-S10**) (230 mg, 92%) as an off white solid. **¹H NMR (400 MHz, CDCl₃):** *δ* 8.02 (s, 1H), 7.88-7.83 (m, 2H), 7.80 (s, 1H), 4.67 (q, *J =* 8.4 Hz, 2H), 4.42 (s, 3H) ppm. **LC-MS (ESI, m/z):** calcd for [M+H]⁺ 439.0; found: 439.2.

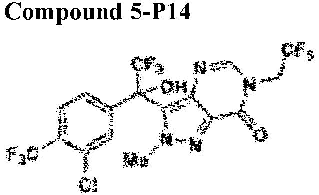

### 3-(1-(3-chloro-4-(trifluoromethyl)phenyl)-2,2,2-trifluoro-1-hydroxyethyl)-2-methyl-6-(2,2,2-trifluoroethyl)-2,6-dihydro-7H-pyrazolo[4,3-d]pyrimidin-7-one (5-P14):

To a stirred solution of 3-(3-chloro-4-(trifluoromethyl)benzoyl)-2-methyl-6-(2,2,2-trifluoroethyl)-2,6-dihydro-7*H*-pyrazolo[4,3-*d*]pyrimidin-7-one (**5-S10**) (210 mg, 0.48 mmol) in DME (5 mL), TMSCF₃ (0.11 mL, 0.72 mmol) followed by CsF (36 mg, 0.24 mmol) was added at 0 °C and the reaction mixture was stirred at room temperature for 2 h. After completion, the reaction mixture was quenched with aqueous saturated NH₄Cl solution, extracted with ethyl acetate, washed with brine, dried over anhydrous Na₂SO₄ and concentrated. The crude product was purified by Combiflash chromatography (50% ethyl acetate in hexane) to afford 3-(1-(3-chloro-4-(trifluoromethyl)phenyl)-2,2,2-trifluoro-1-hydroxyethyl)-2-methyl-6-(2,2,2-trifluoroethyl)-2,6-dihydro-7*H*-pyrazolo[4,3-*d*]pyrimidin-7-one (**5-P14**) as an off white solid (200 mg, 82%). The racemic compound (200 mg) was purified from prep-HPLC-SFC to afford Enantiomer-1 (80 mg) (RT 1.272 min) and Enantiomer-2 (80 mg) (RT 1.407 min).

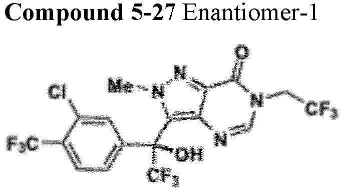

***(S)-3-(1-(3-chloro-4-(trifluoromethyl)phenyl)-2,2,2-trifluoro-1-hydroxyethyl)-2-methyl-6-(2,2,2-trifluoroethyl)-2,6-dihydro-7H-pyrazolo[4,3-d]pyrimidin-7-one (5-27).* ¹H NMR (400 MHz, DMSO-*d₆*):** *δ* 8.52 (s, 1H), 8.19 (s, 1H), 7.93 (d, *J =* 8.4 Hz, 1H), 7.77 (s, 1H), 7.48 (d, *J* = 8.2 Hz, 1H), 4.97-4.90 (m, 2H), 3.73 (s, 3H) ppm. **LC-MS (ESI, m/z):** calcd for [M+H]⁺ 509.0; found: 509.2. **[α]²⁵_{D}** = -82.936° (c = 0.2532 g/100 cm³, CH₃OH).

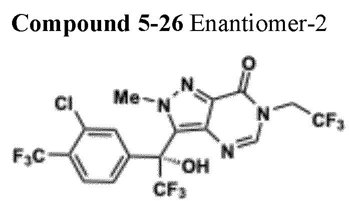

### (R)-3-(1-(3-chloro-4-(trifluoromethyl)phenyl)-2,2,2-trifluoro-1-hydroxyethyl)-2-methyl-6-(2,2,2-trifluoroethyl)-2,6-dihydro-7H-pyrazolo[4,3-d]pyrimidin-7-one (5-26)

**¹H NMR (400 MHz, DMSO-*d₆*):** *δ* 8.52 (s, 1H), 8.19 (s, 1H), 7.93 (d, *J =* 8.4 Hz, 1H), 7.77 (s, 1H), 7.48 (d, *J =* 8.2 Hz, 1H), 4.97-4.90 (m, 2H), 3.73 (s, 3H) ppm. **LC-MS (ESI, m/z):** calcd for [M+H]⁺ 509.0; found: 509.2. **[α]²⁵_{D}** = +68.807° (c = 0.2514 g/100 cm³, CH₃OH).

### Synthesis of compounds 5-32 and 5-33

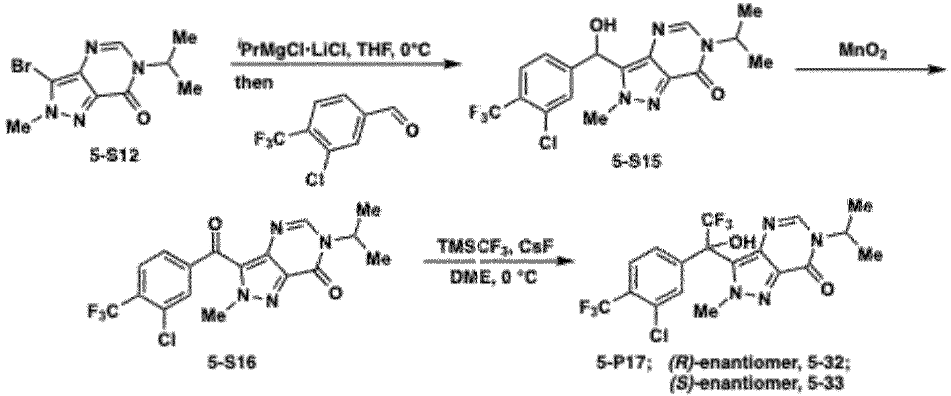

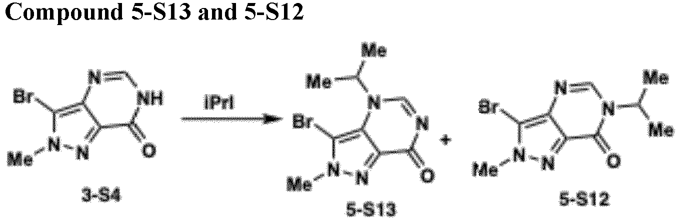

### 3-bromo-6-isopropyl-2-methyl-2,6-dihydro-7H-pyrazolo[4,3-d]pyrimidin-7-one (5-S12)

To a stirred solution of 3-bromo-2-methyl-2,4-dihydro-7*H*-pyrazolo[4,3-*d*]pyrimidin-7-one (**3-S4**) (3 g, 13.1 mmol) in DMF (15 mL), Cs₂CO₃ (8.54 g, 26.2 mmol) followed by 2-iodopropane **(2)** (1.95 mL, 19.65 mmol) was added and the reaction mixture was stirred at room temperature for 24 h. After completion, the reaction mixture was quenched with cold water, extracted with ethyl acetate, washed with aqueous saturated Na₂S₂O₃ solution, brine, dried over anhydrous Na₂SO₄ and concentrated. The crude product was purified by Combiflash chromatography (50% ethyl acetate in hexane) to afford 3-bromo-6-isopropyl-2-methyl-2,6-dihydro-7*H-*pyrazolo[4,3-*d*]pyrimidin-7-one (**5-S12**) (2 g, 56%) along with undesired regioisomer 3-bromo-4-isopropyl-2-methylene-7-oxo-4,7-dihydro-2*H*-pyrazolo[4,3-*d*]pyrimidin-2-ium (**5-S13**) (0.6 g, 17%) as an off white solid.

### Compound 5-S12:

**¹H NMR (400 MHz, DMSO-*d₆*)** *δ* 8.24 (s, 1H), 5.04-4.97 (m, 1H), 4.08 (s, 3H), 1.39 (d, *J =* 6.8 Hz, 6H) ppm. **LC-MS (ESI, m/z):** calcd for [M+H]⁺ 271.2; found: 271.1.

### Compound 5-S13:

**¹H NMR (400 MHz, DMSO-*d₆*)** *δ* 8.47 (s, 1H), 5.62-5.56 (m, 1H), 4.19 (s, 3H), 1.40 (d, *J =* 6.2 Hz, 6H) ppm. **LC-MS (ESI, m/z):** calcd for [M+H]⁺ 271.2; found: 271.1.

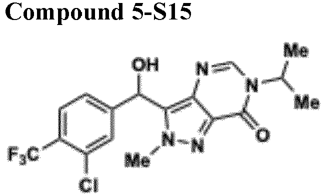

### 3-((3-chloro-4-(trifluoromethyl)phenyl)(hydroxy)methyl)-6-isopropyl-2-methyl-2,6-dihydro-7H-pyrazolo[4,3-d]pyrimidin-7-one (5-S15):

To a stirred solution of 3-bromo-6-isopropyl-2-methyl-2,6-dihydro-7*H*-pyrazolo[4,3-d]pyrimidin-7-one **(5-S12)** (400 mg, 1.48 mmol) in anhydrous THF (5 mL), iPrMgCl•LiCl (1.3 M) (1.5 mL, 1.92 mmol) was added at -5 °C and stirred for 15 minutes. After that 3-chloro-4-(trifluoromethyl)benzaldehyde (308 mg, 1.48 mmol) in anhydrous THF (2 mL) was added and stirred at room temperature for 1 h. After completion, the reaction mixture was quenched with aqueous saturated NH₄Cl solution, extracted with ethyl acetate, washed with brine, dried over anhydrous Na₂SO₄ and concentrated. The crude product was purified by Combiflash chromatography (50% ethyl acetate in hexane) to afford 3-((3-chloro-4-(trifluoromethyl)phenyl)(hydroxy)methyl)-6-isopropyl-2-methyl-2,6-dihydro-7*H*-pyrazolo [4,3-*d*]pyrimidin-7-one (**5-S15**) (425 mg, 72%) as an off white solid.**¹H NMR (400 MHz, DMSO-*d₆*)** *δ* 8.15 (s, 1H), 7.84 (d, *J* = 8.2 Hz, 1H), 7.74 (s, 1H), 7.49 (d, *J* = 8.2 Hz, 1H), 6.78 (d, *J =* 4.5 Hz, 1H), 6.36 (d, *J =* 3.8 Hz, 1H), 5.05-4.98 (m, 1H), 3.99 (s, 3H), 1.38 (d, *J =* 6.8 Hz, 6H) ppm. **LC-MS (ESI, m/z):** calcd for [M+H]⁺ 401.1; found: 401.2.

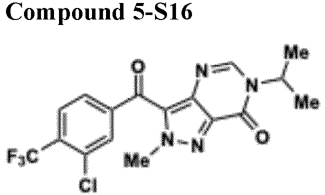

### 3-(3-chloro-4-(trifluoromethyl)benzoyl)-6-isopropyl-2-methyl-2,6-dihydro-7H-pyrazolo[4,3-d]pyrimidin-7-one (5-S16)

To a stirred solution of 3-((3-chloro-4-(trifluoromethyl)phenyl)(hydroxy)methyl)-6-isopropyl-2-methyl-2,6-dihydro-7*H*-pyrazolo[4,3-*d*]pyrimidin-7-one (**5-S15**) (350 mg, 0.87 mmol) in DCM (15 mL), MnO₂ (1518 mg, 17.47 mmol) was added and the reaction mixture was stirred at room temperature for 4 h. After completion, the reaction mixture was filtered through Celite bed, washed with dichloromethane and the filtrate was concentrated to afford 3-(3-chloro-4-(trifluoromethyl)benzoyl)-6-isopropyl-2-methyl-2,6-dihydro-7*H*-pyrazolo[4,3-*d*]pyrimidin-7-one (**5-S16**) (330 mg, 95%) as an off white solid. **¹H NMR (400 MHz, CDCl₃)** *δ* 8.03 (s, 1H), 7.89-7.87 (m, 2H), 7.83 (d, *J =* 8.2 Hz, 1H), 5.28-5.24 (m, 1H), 4.42 (s, 3H), 1.45 (d, *J* = 6.9 Hz, 6H) ppm. **LC-MS (ESI, m/z):** calcd for [M+H]⁺ 399.1; found: 399.2.

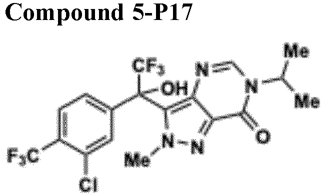

### 3-(1-(3-chloro-4-(trifluoromethyl)phenyl)-2,2,2-trifluoro-1-hydroxyethyl)-6-isopropyl-2-methyl-2,6-dihydro-7H-pyrazolo[4,3-d]pyrimidin-7-one (5-P17):

To a stirred solution of 3-(3-chloro-4-(trifluoromethyl)benzoyl)-6-isopropyl-2-methyl-2,6-dihydro-7*H*-pyrazolo[4,3-*d*]pyrimidin-7-one (**5-S10**) (200 mg, 0.5 mmol) in DME (5 mL), CsF (38 mg, 0.25 mmol) followed by TMSCF₃ (0.11 mL, 0.75 mmol) was added at 0°C and the reaction mixture was stirred at room temperature for 2 h. After completion, the reaction mixture was quenched with aqueous saturated NH₄Cl solution, extracted with ethyl acetate, washed with brine, dried over anhydrous Na₂SO₄ and concentrated. The crude product was purified by Combiflash chromatography (50% ethyl acetate in hexane) to afford 3-(1-(3-chloro-4-(trifluoromethyl)phenyl)-2,2,2-trifluoro-1-hydroxyethyl)-6-isopropyl-2-methyl-2,6-dihydro-7*H*-pyrazolo[4,3-*d*]pyrimidin-7-one (**5-P17**) as an off white solid (180 mg, 77%). **Physical State:** off white solid. The racemic compound **5-P17** (180 mg) was purified from preparative-HPLC-SFC to afford Enantiomer-1 (**5-33**) (60 mg) and Enantiomer-2 (**5-32**) (60 mg).

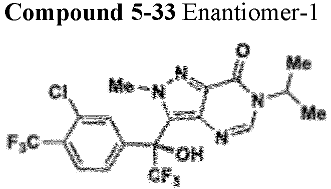

### (S)-3-(1-(3-chloro-4-(trifluoromethyl)phenyl)-2,2,2-trifluoro-1-hydroxyethyl)-6-isopropyl-2-methyl-2,6-dihydro-7H-pyrazolo[4,3-d]pyrimidin-7-one (5-33)

**¹H NMR (400 MHz, DMSO-*d₆*):** *δ* 8.46 (s, 1H), 8.30 (s, 1H), 7.93 (d, *J =* 8.4 Hz, 1H), 7.75 (s, 1H), 7.45 (d, *J =* 8.2 Hz, 1H), 5.06-5.00 (m, 1H), 3.71 (s, 3H), 1.42-1.39 (m, 6H) ppm. **LC-MS (ESI, m/z):** calcd for [M+H]⁺ 469.1; found: 469.2. **[α]²⁵_{D}** = -90.289° (c = 0.2592 g/100 cm³, CH₃OH).

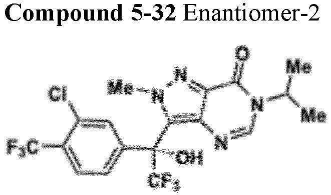

***(R)-3-(1-(3-chloro-4-(trifluoromethyl)phenyl)-2,2,2-trifluoro-1-hydroxyethyl)-6-isopropyl-2-methyl-2,6-dihydro-7H-pyrazolo[4,3-d]pyrimidin-7-one* (5-32) ¹H NMR (400 MHz, DMSO-*d₆*):** *δ* 8.72 (s, 1H), 8.68 (s, 1H), 8.23 (s, 2H), 5.04-4.97 (m, 1H), 3.90 (s, 3H), 1.39 (d, *J* = 6.8 Hz, 6H) ppm. **LC-MS (ESI, m/z):** calcd for [M+H]⁺ 469.1; found: 469.2. **[α]²⁵_{D}** = +86.885° (c = 0.2532 g/100 cm³, CH₃OH).

### Synthesis of intermediate compounds 5-40 and 5-41

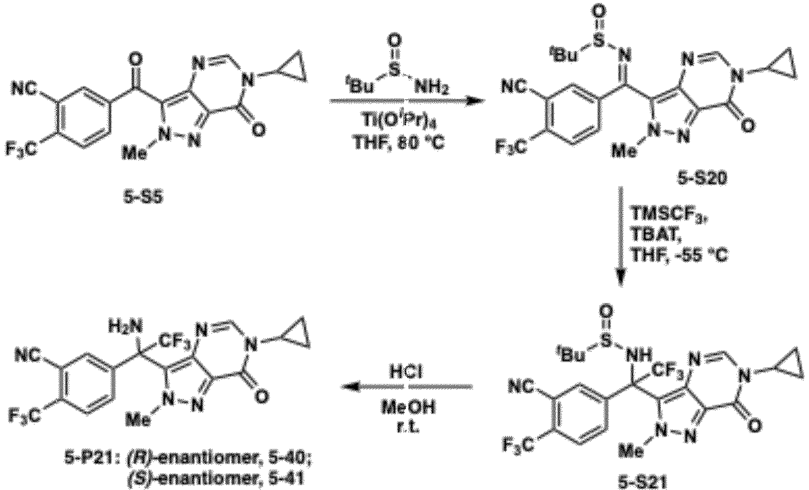

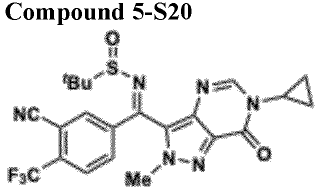

### N-((3-cyano-4-(trifluoromethyl)phenyl)(6-cyclopropyl-2-methyl-7-oxo-6,7-dihydro-2H-pyrazolo[4,3-d]pyrimidin-3-yl)methylene)-2-methylpropane-2-sulfinamide (5-S20):

To a stirred solution of 5-(6-cyclopropyl-2-methyl-7-oxo-6,7-dihydro-2*H*-pyrazolo[4,3-*d*]pyrimidine-3-carbonyl)-2-(trifluoromethyl)benzonitrile (**5-S5**) (390 mg, 1.01 mmol) and 2-methylpropane-2-sulfinamide (244 mg, 2.01 mmol) in anhydrous THF (10 mL), Ti(OiPr)₄ (2.14 mL, 7.05 mmol) was added and the reaction mixture was stirred at 80 °C for 14 h. After completion, the reaction mixture was quenched with aqueous saturated NH₄Cl solution, extracted with ethyl acetate, washed with water, brine, dried over anhydrous Na₂SO₄ and concentrated. The crude product was purified by CombiFlash chromatography (70% ethyl acetate in hexane) to afford *N*-((3-cyano-4-(trifluoromethyl)phenyl)(6-cyclopropyl-2-methyl-7-oxo-6,7-dihydro-2*H*-pyrazolo[4,3-*d*]pyrimidin-3-yl)methylene)-2-methylpropane-2-sulfinamide (**5-S20**) as a yellow solid (430 mg, 87%). **LC-MS (ESI, m/z):** calcd for [M+H]⁺ 491.2; found: 491.0.

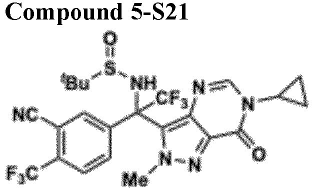

### N-(1-(3-cyano-4-(trifluoromethyl)phenyl)-1-(6-cyclopropyl-2-methyl-7-oxo-6,7-dihydro-2H-pyrazolo[4,3-d]pyrimidin-3-yl)-2,2,2-trifluoroethyl)-2-methylpropane-2-sulfinamide (5-S21):

To a stirred solution of *N*-((3-cyano-4-(trifluoromethyl)phenyl)(6-cyclopropyl-2-methyl-7-oxo-6,7-dihydro-2*H*-pyrazolo[4,3-*d*]pyrimidin-3-yl)methylene)-2-methylpropane-2-sulfinamide (**5-S20**) (300 mg, 0.61 mmol) and TBAT (495 mg, 0.917 mmol) in anhydrous THF (7 mL), TMSCF₃ (0.14 mL, 0.92 mmol) in anhydrous THF (0.5 mL) was added at -55 °C and the reaction mixture was stirred at that temperature for 1 h (TLC-monitoring). After completion, the reaction mixture was quenched with aqueous saturated NH₄Cl solution, extracted with ethyl acetate, washed with brine, dried over anhydrous Na₂SO₄ and concentrated. The crude *N*-(1-(3-cyano-4-(trifluoromethyl)phenyl)-1-(6-cyclopropyl-2-methyl-7-oxo-6,7-dihydro-2*H-*pyrazolo[4,3-*d*]pyrimidin-3-yl)-2,2,2-trifluoroethyl)-2-methylpropane-2-sulfinamide (**5-S21**) was directly taken for the next step (340 mg, crude). **LC-MS (ESI, m/z):** calcd for [M+H]⁺ 561.2; found: 561.2.

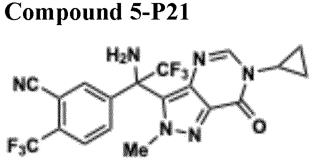

### 5-(1-amino-1-(6-cyclopropyl-2-methyl-7-oxo-6,7-dihydro-2H-pyrazolo[4,3-d]pyrimidin-3-yl)-2,2,2-trifluoroethyl)-2-(trifluoromethyl)benzonitrile (5-P21):

A solution of*N*-(1-(3-cyano-4-(trifluoromethyl)phenyl)-1-(6-cyclopropyl-2-methyl-7-oxo-6,7-dihydro-2*H*-pyrazolo[4,3-*d*]pyrimidin-3-yl)-2,2,2-trifluoroethyl)-2-methylpropane-2-sulfinamide (**5-S21**) (500 mg, 0.89 mmol) in HCl in dioxane (4M) (10 mL) was stirred at room temperature for 1 h. After completion, the reaction mixture was concentrated, quenched with aqueous NaHCO₃ solution, extracted with ethyl acetate, washed with brine, dried over anhydrous Na₂SO₄ and concentrated. The crude product was purified by CombiFlash chromatography (50% ethyl acetate in hexane) to afford 5-(1-amino-1-(6-cyclopropyl-2-methyl-7-oxo-6,7-dihydro-2*H*-pyrazolo[4,3-*d*]pyrimidin-3-yl)-2,2,2-trifluoroethyl)-2-(trifluoromethyl)benzonitrile (**5-P21**) as an off white solid (125 mg, 31%). **Physical State:** off white solid. The racemic compound **5-P21** (150 mg) was purified from preparative-HPLC-SFC to afford Enantiomer-1 (**5-41**) (45 mg) and Enantiomer-2 (**5-40**) (45 mg).

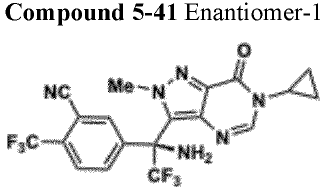

### (S)-5-(1-amino-1-(6-cyclopropyl-2-methyl-7-oxo-6,7-dihydro-2H-pyrazolo[4,3-d]pyrimidin-3-yl)-2,2,2-trifluoroethyl)-2-(trifluoromethyl)benzonitrile (5-41)

¹H **NMR (400 MHz, DMSO-*d₆*):** *δ* 8.30 (s, 1H), 8.07-8.04 (m, 2H), 7.76 (d, *J =* 8.3 Hz, 1H), 3.79 (s, 2H), 3.70 (s, 3H), 3.20-3.16 (m, 1H), 1.05-1.00 (m, 2H), 0.92-0.90 (m, 2H) ppm. **LC-MS (ESI, m/z):** calcd for [M+H]⁺ 457.1; found: 457.3. **[α]²⁵_{D}** = -60.767° (c = 0.2600 g/100 cm³, CH₃OH).

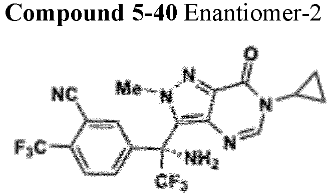

### (R)-5-(1-amino-1-(6-cyclopropyl-2-methyl-7-oxo-6,7-dihydro-2H-pyrazolo[4,3-d]pyrimidin-3-yl)-2,2,2-trifluoroethyl)-2-(trifluoromethyl)benzonitrile (5-40)

**¹H NMR (400 MHz, DMSO-*d₆*):** *δ* 8.30 (s, 1H), 8.07-8.04 (m, 2H), 7.76 (d, *J =* 8.3 Hz, 1H), 3.79 (s, 2H), 3.70 (s, 3H), 3.20-3.16 (m, 1H), 1.05-1.00 (m, 2H), 0.92-0.90 (m, 2H) ppm. **LC-MS (ESI, m/z):** calcd for [M+H]⁺ 457.1; found: 457.3. **[α]²⁵_{D}** = +58.779° (c = 0.2552 g/100 cm³, CH₃OH).

### Synthesis of intermediate compounds 5-44 and 5-45

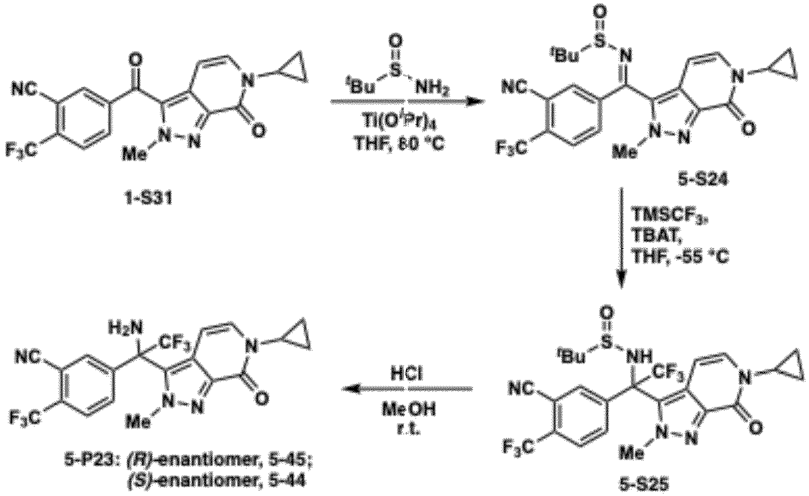

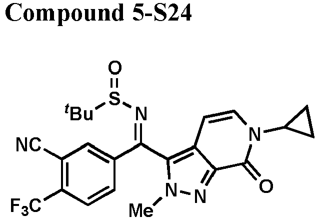

### Synthesis of N-((3-cyano-4-(trifluoromethyl)phenyl)(6-cyclopropyl-2-methyl-7-oxo-6,7-dihydro-2H-pyrazolo[3,4-c]pyridin-3-yl)methylene)-2-methylpropane-2-sulfinamide (5-S24):

To a stirred solution of 5-(6-cyclopropyl-2-methyl-7-oxo-6,7-dihydro-2*H*-pyrazolo[3,4-*c*]pyridine-3-carbonyl)-2-(trifluoromethyl)benzonitrile (**1-S31**) (410 mg, 1.06 mmol) and 2-methylpropane-2-sulfinamide (257 mg, 2.12 mmol) in anhydrous THF (10 mL), Ti(OiPr)₄ (2.25 mL, 7.43 mmol) was added and the reaction mixture was stirred at 80 °C for 16 h. After completion, the reaction mixture was quenched with aqueous saturated NH₄Cl solution, extracted with ethyl acetate, washed with water, brine, dried over anhydrous Na₂SO₄ and concentrated. The crude product was purified by CombiFlash chromatography (70% ethyl acetate in hexane) to afford *N*-((3-cyano-4-(trifluoromethyl)phenyl)(6-cyclopropyl-2-methyl-7-oxo-6,7-dihydro-2*H*-pyrazolo[3,4-*c*]pyridin-3-yl)methylene)-2-methylpropane-2-sulfinamide (**5-S24**) as a yellow solid (500 mg, 96%). **LC-MS (ESI, m/z):** calcd for [M+H]⁺ 490.2; found: 490.3.

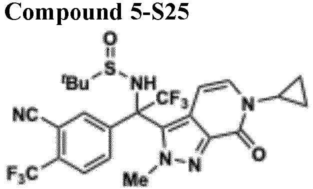

### N-(1-(3-cyano-4-(trifluoromethyl)phenyl)-1-(6-cyclopropyl-2-methyl-7-oxo-6,7-dihydro-2H-pyrazolo[3,4-c]pyridin-3-yl)-2,2,2-trifluoroethyl)-2-methylpropane-2-sulfinamide (5-S25):

To a stirred solution of *N*-((3-cyano-4-(trifluoromethyl)phenyl)(6-cyclopropyl-2-methyl-7-oxo-6,7-dihydro-2*H*-pyrazolo[3,4-*c*]pyridin-3-yl)methylene)-2-methylpropane-2-sulfinamide (**5-S24**) (286 mg, 0.58 mmol) and TBAT (473 mg, 0.88 mmol) in anhydrous THF (7 mL), TMSCF₃ (0.13 mL, 0.88 mmol) in anhydrous THF (0.5 mL) was added at -55 °C and the reaction mixture was stirred at that temperature for 1 h (TLC-monitoring). After completion, the reaction mixture was quenched with aqueous saturated NH₄Cl solution, extracted with ethyl acetate, washed with brine, dried over anhydrous Na₂SO₄ and concentrated. The crude product **5-S25** was directly taken for the next step (325 mg). **LC-MS (ESI, m/z):** calcd for [M+H]⁺ 560.2; found: 560.0.

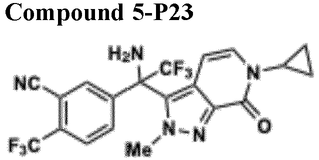

### 5-(1-amino-1-(6-cyclopropyl-2-methyl-7-oxo-6,7-dihydro-2H-pyrazolo[3,4-c]pyridin-3-yl)-2,2,2-trifluoroethyl)-2-(trifluoromethyl)benzonitrile (5-P23):

A solution of*N*-(1-(3-cyano-4-(trifluoromethyl)phenyl)-1-(6-cyclopropyl-2-methyl-7-oxo-6,7-dihydro-2*H*-pyrazolo[3,4-*c*]pyridin-3-yl)-2,2,2-trifluoroethyl)-2-methylpropane-2-sulfinamide (**5-S25**) (325 mg, 0.58 mmol) in HCl in dioxane (4M) (10 mL) was stirred at room temperature for 1 h. After completion, the reaction mixture was concentrated, quenched with aqueous NaHCO₃ solution, extracted with ethyl acetate, washed with brine, dried over anhydrous Na₂SO₄ and concentrated. The crude product was purified by CombiFlash chromatography (70% ethyl acetate in hexane) to afford 5-(1-amino-1-(6-cyclopropyl-2-methyl-7-oxo-6,7-dihydro-2*H*-pyrazolo[3,4-*c*]pyridin-3-yl)-2,2,2-trifluoroethyl)-2-(trifluoromethyl)benzonitrile (**5-P23**) as an off white solid (115 mg, 43%). **Physical State:** off white solid.

The racemic compound **5-P25** (115 mg) was purified from preparative-HPLC-SFC to afford Enantiomer-1 (**5-44**) (45 mg) and Enantiomer-2 (**5-45**) (45 mg).

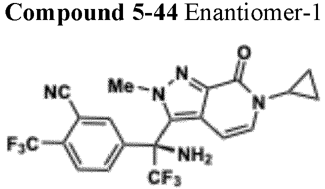

### (S)-5-(1-amino-1-(6-cyclopropyl-2-methyl-7-oxo-6,7-dihydro-2H-pyrazolo[3,4-c]pyridin-3-yl)-2,2,2-trifluoroethyl)-2-(trifluoromethyl)benzonitrile (5-44)

**¹H NMR (400 MHz, DMSO-*d₆*):** *δ* 8.34 (s, 1H), 8.05 (d, *J* = 8.5 Hz, 1H), 7.67 (d, *J =* 8.4 Hz, 1H), 7.06 (d, *J =* 7.9 Hz, 1H), 6.17 (d, *J =* 7.4 Hz, 1H), 3.79 (s, 2H), 3.70 (s, 3H), 3.29-3.23 (m, 1H), 1.03-0.98 (m, 2H), 0.83-0.78 (m, 2H) ppm. **LC-MS (ESI, m/z):** calcd for [M+H]⁺ 456.1; found: 456.1. **[α]²⁵_{D}** = -108.984° (c = 0.2606 g/100 cm³, CH₃OH).

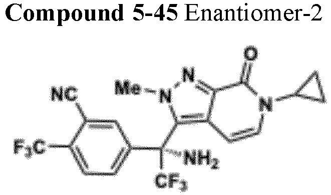

### (R)-5-(1-amino-1-(6-cyclopropyl-2-methyl-7-oxo-6,7-dihydro-2H-pyrazolo[3,4-c]pyridin-3-yl)-2,2,2-trifluoroethyl)-2-(trifluoromethyl)benzonitrile (5-45)

**¹H NMR (400 MHz, DMSO-*d₆*):** *δ* 8.05 (s, 1H), 7.94 (s, 1H), 7.90 (d, *J =* 8.4 Hz, 1H), 7.37 (d, *J =* 8.2 Hz, 1H), 4.00-3.95 (m, 1H), 3.87 (s, 2H), 3.74 (s, 3H), 0.99-0.94 (m, 4H) ppm. **LC-MS (ESI, m/z):** calcd for [M+H]⁺ 456.1; found: 456.1. **[α]²⁵_{D}** = +117.689° (c = 0.2600 g/100 cm³, CH₃OH).

### Synthesis of intermediate compounds 5-48 and 5-49

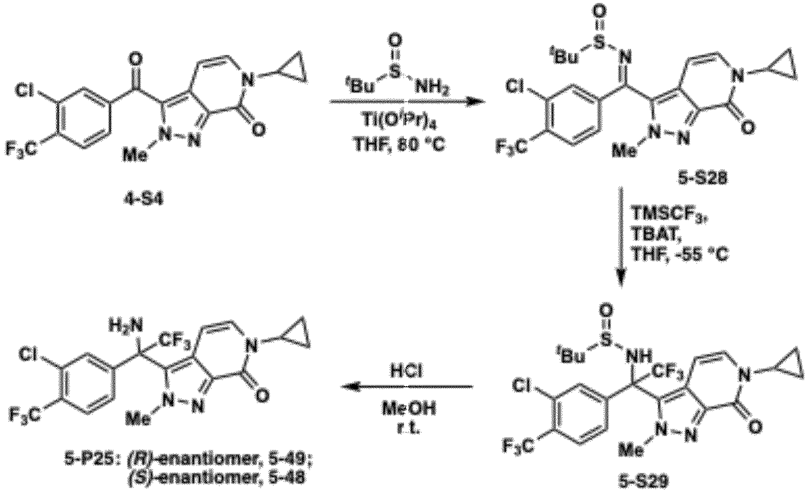

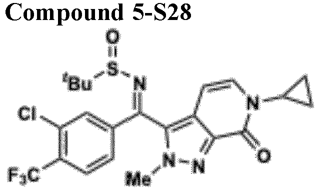

### N-((3-chloro-4-(trifluoromethyl)phenyl)(6-cyclopropyl-2-methyl-7-oxo-6,7-dihydro-2H-pyrazolo[3,4-c]pyridin-3-yl)methylene)-2-methylpropane-2-sulfinamide (5-S28):

To a stirred solution of 3-(3-chloro-4-(trifluoromethyl)benzoyl)-6-cyclopropyl-2-methyl-2,6-dihydro-7*H*-pyrazolo[3,4-*c*]pyridin-7-one (**4-S4**) (460 mg, 1.16 mmol) and 2-methylpropane-2-sulfinamide (423 mg, 3.49 mmol) in anhydrous THF (10 mL), Ti(OiPr)₄ (2.47 mL, 8.14 mmol) was added and the reaction mixture was stirred at 80 °C for overnight. After completion, the reaction mixture was quenched with aqueous saturated NH₄Cl solution, extracted with ethyl acetate, washed with water, brine, dried over anhydrous Na₂SO₄ and concentrated. The crude product was purified by CombiFlash chromatography (70% ethyl acetate in hexane) to afford N-((3-chloro-4-(trifluoromethyl)phenyl)(6-cyclopropyl-2-methyl-7-oxo-6,7-dihydro-2*H-*pyrazolo[3,4-*c*]pyridin-3-yl)methylene)-2-methylpropane-2-sulfinamide (**5-S28**) as a yellow gum (550 mg, 95%). **LC-MS (ESI, m/z):** calcd for [M+H]⁺ 499.1; found: 499.3.

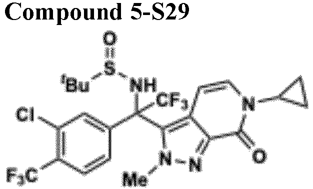

### N-(1-(3-chloro-4-(trifluoromethyl)phenyl)-1-(6-cyclopropyl-2-methyl-7-oxo-6,7-dihydro-2H-pyrazolo[3,4-c]pyridin-3-yl)-2,2,2-trifluoroethyl)-2-methylpropane-2-sulfinamide (5-S29):

To a stirred solution of the *N*-((3-chloro-4-(trifluoromethyl)phenyl)(6-cyclopropyl-2-methyl-7-oxo-6,7-dihydro-2*H*-pyrazolo[3,4-*c*]pyridin-3-yl)methylene)-2-methylpropane-2-sulfinamide (**5-S28**) (300 mg, 0.60 mmol) and TBAT (357 mg, 0.66 mmol) in anhydrous THF (5 mL), TMSCF₃ (0.11 mL, 0.72 mmol) in anhydrous THF was added at -55 °C and the reaction mixture was stirred at that temperature for 1 h (TLC-monitoring). After completion, the reaction mixture was quenched with aqueous saturated NH₄Cl solution, extracted with ethyl acetate, washed with brine, dried over anhydrous Na₂SO₄ and concentrated. The crude *N*-(1-(3-chloro-4-(trifluoromethyl)phenyl)-1-(6-cyclopropyl-2-methyl-7-oxo-6,7-dihydro-2*H*-pyrazolo[3,4-*c*]pyridin-3-yl)-2,2,2-trifluoroethyl)-2-methylpropane-2-sulfinamide (**5-S29**) was directly taken for the next step (340 mg, crude). **LC-MS (ESI, m/z):** calcd for [M+H]⁺ 569.1; found: 569.2.

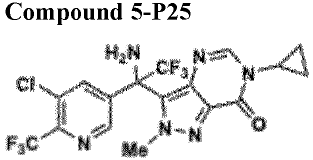

### 3-(1-amino-1-(3-chloro-4-(trifluoromethyl)phenyl)-2,2,2-trifluoroethyl)-6-cyclopropyl-2-methyl-2,6-dihydro-7H-pyrazolo[3,4-c]pyridin-7-one (5-P25):

A solution of *N*-(1-(3-chloro-4-(trifluoromethyl)phenyl)-1-(6-cyclopropyl-2-methyl-7-oxo-6,7-dihydro-2*H*-pyrazolo[3,4-*c*]pyridin-3-yl)-2,2,2-trifluoroethyl)-2-methylpropane-2-sulfinamide (**5-S29**) (600 mg, 1.055 mmol) in methanolic HCl (4N) (7 mL) was stirred at room temperature for 1 h. After completion, the reaction mixture was concentrated, quenched with aqueous NaHCO₃ solution, extracted with ethyl acetate, washed with brine, dried over anhydrous Na₂SO₄ and concentrated. The crude product was purified by CombiFlash chromatography (60% ethyl acetate in hexane) to afford 3-(1-amino-1-(3-chloro-4-(trifluoromethyl)phenyl)-2,2,2-trifluoroethyl)-6-cyclopropyl-2-methyl-2,6-dihydro-7*H-*pyrazolo[3,4-*c*]pyridin-7-one (**5-P25**) as an off white solid (47 mg, 10%). **Physical State:** off white solid. The racemic compound **5-P25** (47 mg) was purified from preparative-HPLC-SFC to afford Enantiomer-1 (**5-49**) (20 mg) and Enantiomer-2 (**5-48**) (20 mg).

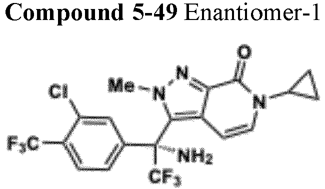

### (R)-3-(1-amino-1-(3-chloro-4-(trifluoromethyl)phenyl)-2,2,2-trifluoroethyl)-6-cyclopropyl-2-methyl-2,6-dihydro-7H-pyrazolo[3,4-c]pyridin-7-one (5-49)

**¹H NMR (400 MHz, DMSO-*d₆*):** *δ* 7.91-7.89 (m, 2H), 7.29 (d, *J =* 8.3 Hz, 1H), 7.05 (d, *J =* 7.8 Hz, 1H), 6.15 (d, *J=* 7.6 Hz, 1H), 3.80-3.70 (m, 5H), 3.28-3.24 (m, 1H), 1.00-0.97 (m, 2H), 0.83-0.79 (m, 2H) ppm. **LC-MS (ESI, m/z):** calcd for [M+H]⁺ 465.1; found: 465.1.

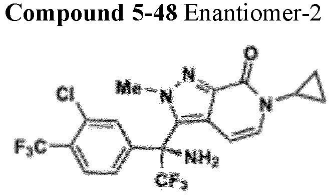

### (S)-3-(1-amino-1-(3-chloro-4-(trifluoromethyl)phenyl)-2,2,2-trifluoroethyl)-6-cyclopropyl-2-methyl-2,6-dihydro-7H-pyrazolo[3,4-c]pyridin-7-one (5-48)

**¹H NMR (400 MHz, DMSO-*d₆*):** *δ* 7.91-7.89 (m, 2H), 7.29 (d, *J =* 8.3 Hz, 1H), 7.05 (d, *J =* 7.8 Hz, 1H), 6.15 (d, *J =* 7.6 Hz, 1H), 3.80-3.70 (m, 5H), 3.28-3.24 (m, 1H), 1.00-0.97 (m, 2H), 0.83-0.79 (m, 2H) ppm. **LC-MS (ESI, m/z):** calcd for [M+H]⁺ 465.1; found: 465.1.

### Synthesis of intermediate compounds 5-50 and 5-51

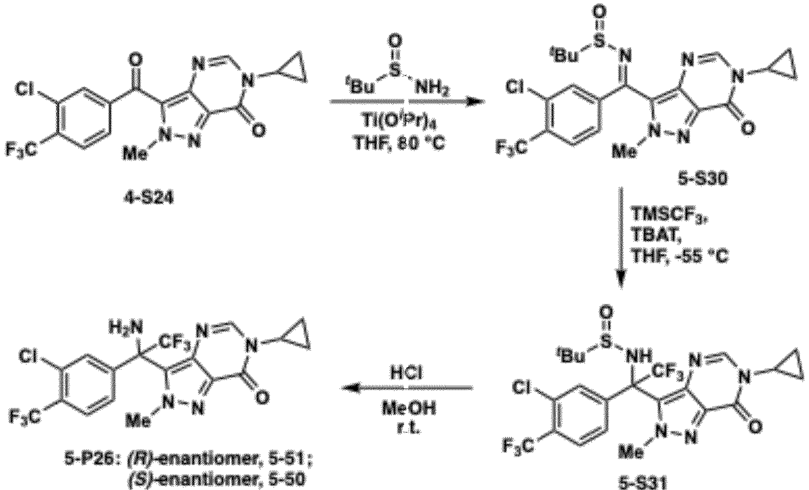

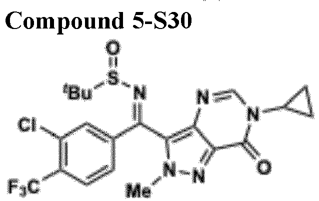

### N-((3-chloro-4-(trifluoromethyl)phenyl)(6-cyclopropyl-2-methyl-7-oxo-6,7-dihydro-2H-pyrazolo[4,3-d]pyrimidin-3-yl)methylene)-2-methylpropane-2-sulfinamide (5-S30):

To a stirred solution of 3-(3-chloro-4-(trifluoromethyl)benzoyl)-6-cyclopropyl-2-methyl-2,6-dihydro-7*H*-pyrazolo[4,3-*d*]pyrimidin-7-one (**4-S24**) (410 mg, 1.03 mmol) and 2-methylpropane-2-sulfinamide (250 mg, 2.07 mmol) in anhydrous THF (10 mL), Ti(OiPr)₄ (2.2 mL, 7.23 mmol) was added and the reaction mixture was stirred at 80 °C for 5 h. After completion, the reaction mixture was quenched with aqueous saturated NH₄Cl solution, extracted with ethyl acetate, washed with water, brine, dried over anhydrous Na₂SO₄ and concentrated. The crude product was purified by CombiFlash chromatography (60% ethyl acetate in hexane) to afford *N*-((3-chloro-4-(trifluoromethyl)phenyl)(6-cyclopropyl-2-methyl-7-oxo-6,7-dihydro-2*H*-pyrazolo[4,3-*d*]pyrimidin-3-yl)methylene)-2-methylpropane-2-sulfinamide (**5-S30**) as a yellow solid (450 mg, 87%). **LC-MS (ESI, m/z):** calcd for [M+H]⁺ 500.1; found: 500.2.

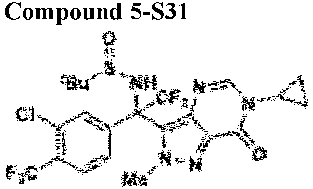

### N-(1-(3-chloro-4-(trifluoromethyl)phenyl)-1-(6-cyclopropyl-2-methyl-7-oxo-6,7-dihydro-2H-pyrazolo[4,3-d]pyrimidin-3-yl)-2,2,2-trifluoroethyl)-2-methylpropane-2-sulfinamide (5-S31):

To a stirred solution of *N*-((3-chloro-4-(trifluoromethyl)phenyl)(6-cyclopropyl-2-methyl-7-oxo-6,7-dihydro-2*H*-pyrazolo[4,3-*d*]pyrimidin-3-yl)methylene)-2-methylpropane-2-sulfinamide (**5-S30**) (350 mg, 0.7 mmol) and TBAT (416 mg, 0.77 mmol) in anhydrous THF (5 mL), TMSCF₃ (0.12 mL, 0.84 mmol) in anhydrous THF (2 mL) was added at -55 °C and the reaction mixture was stirred at that temperature for 1 h (TLC-monitoring). After completion, the reaction mixture was quenched with aqueous saturated NH₄Cl solution, extracted with ethyl acetate, washed with brine, dried over anhydrous Na₂SO₄ and concentrated. The crude *N*-(1-(3-chloro-4-(trifluoromethyl)phenyl)-1-(6-cyclopropyl-2-methyl-7-oxo-6,7-dihydro-2*H-*pyrazolo[4,3-*d*]pyrimidin-3-yl)-2,2,2-trifluoroethyl)-2-methylpropane-2-sulfinamide (**5-S31**) was directly taken for the next step (395 mg).**LC-MS (ESI, m/z):** calcd for [M+H]⁺ 570.1; found: 570.5.

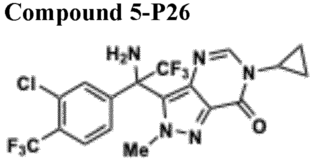

### 3-(1-amino-1-(3-chloro-4-(trifluoromethyl)phenyl)-2,2,2-trifluoroethyl)-6-cyclopropyl-2-methyl-2,6-dihydro-7H-pyrazolo[4,3-d]pyrimidin-7-one (5-P26):

A solution of *N*-(1-(3-chloro-4-(trifluoromethyl)phenyl)-1-(6-cyclopropyl-2-methyl-7-oxo-6,7-dihydro-2*H*-pyrazolo[4,3-*d*]pyrimidin-3-yl)-2,2,2-trifluoroethyl)-2-methylpropane-2-sulfinamide (**5-S31**) (500 mg, 0.877 mmol) in methanolic HCl (4N) (7 mL) was stirred at room temperature for 1 h. After completion, the reaction mixture was concentrated, quenched with aqueous NaHCO₃ solution, extracted with ethyl acetate, washed with brine, dried over anhydrous Na₂SO₄ and concentrated. The crude product was purified by CombiFlash chromatography (60% ethyl acetate in hexane) to afford 3-(1-amino-1-(3-chloro-4-(trifluoromethyl)phenyl)-2,2,2-trifluoroethyl)-6-cyclopropyl-2-methyl-2,6-dihydro-7*H-*pyrazolo[4,3-*d*]pyrimidin-7-one (**5-P26**) as an off white solid (170 mg, 42%). **Physical State:** off white solid. The racemic compound **5-P26** (170 mg) was purified from preparative-HPLC-SFC to afford Enantiomer-1 (**5-51**) (60 mg) and Enantiomer-2 (**5-50**) (60 mg).

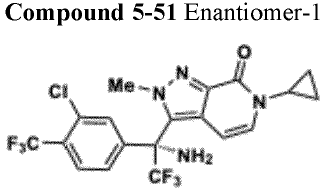

### (R)-3-(1-amino-1-(3-chloro-4-(trifluoromethyl)phenyl)-2,2,2-trifluoroethyl)-6-cyclopropyl-2-methyl-2,6-dihydro-7H-pyrazolo[4,3-d]pyrimidin-7-one (5-51)

**¹H NMR (400 MHz, DMSO-*d*₆):** *δ* 8.06 (s, 1H), 7.88-7.84 (m, 2H), 7.34 (d, *J =* 8.2 Hz, 1H), 3.71 (s, 2H), 3.66 (s, 3H), 3.19-3.13 (m, 1H), 1.05-1.00 (m, 2H), 0.95-0.90 (m, 2H) ppm. **LC-MS (ESI, m/z):** calcd for [M+H]⁺ 466.1; found: 466.2. **[α]²⁵_{D}** = +46.537° (c = 0.2600 g/100 cm³, CH₃OH).

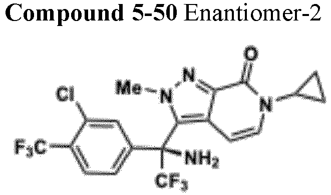

***(S)-3-(1-amino-1-(3-chloro-4-(trifluoromethyl)phenyl)-2,2,2-trifluoroethyl)-6-cyclopropyl-2-methyl-2,6-dihydro-7H-pyrazolo[4,3-d]pyrimidin-7-one* (5-50) ¹H NMR (400 MHz, DMSO-*d*₆):** *δ* 8.06 (s, 1H), 7.88-7.84 (m, 2H), 7.34 (d, *J =* 8.2 Hz, 1H), 3.71 (s, 2H), 3.66 (s, 3H), 3.19-3.13 (m, 1H), 1.05-1.00 (m, 2H), 0.95-0.90 (m, 2H) ppm. **LC-MS (ESI, m/z):** calcd for [M+H]⁺466.1; found: 466.2. **[α]²⁵_{D}** = -46.554° (c = 0.2685 g/100 cm³, CH₃OH).

### Synthesis of compounds 5-52 and 5-53

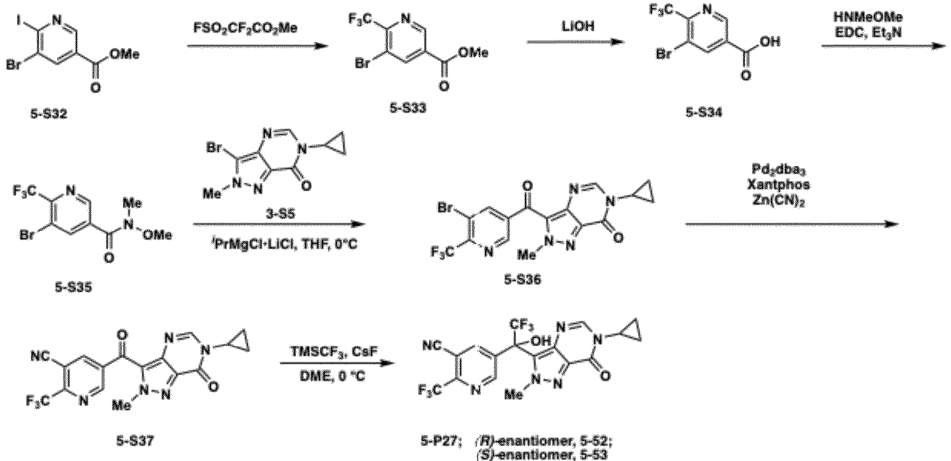

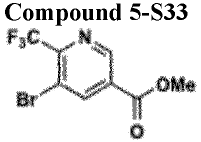

### methyl 5-bromo-6-(trifluoromethyl)nicotinate (5-S33):

A stirred solution of methyl 5-bromo-6-iodonicotinate (16.5 g, 48.26 mmol) in anhydrous DMF (50 mL), was degassed with argon for 15 minutes. After that CuI (11.95 gm, 62.73 mmol) followed by HMPA (41.98 mL, 241.28 mmol) and methyl 2,2-difluoro-2-(fluorosulfonyl)acetate (30.72 mL, 241.28 mmol) were added. The reaction mixture was then stirred at 80 °C for 16 h. After completion, the reaction mixture was concentrated, quenched with aqueous saturated NH₄Cl solution, extracted with ethyl acetate, washed with water, brine, dried over anhydrous Na₂SO₄ and concentrated. The crude product was purified by CombiFlash chromatography (5% ethyl acetate in hexane) to afford methyl 5-bromo-6-(trifluoromethyl)nicotinate (**5-S33**) as a white solid (12 g, impure). **LC-MS (ESI, m/z):** calcd for [M+H]⁺285.0; found: 284.2.

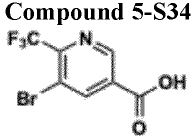

### 5-bromo-6-(trifluoromethyl)nicotinic acid (5-S34):

To a stirred solution of methyl 5-bromo-6-(trifluoromethyl)nicotinate (**5-S34**) (12 g, 42.25 mmol) in THF-Water (3:1) (50 mL), LiOH•H₂O (3.55 g, 84.5 mmol) was added and the reaction mixture was stirred at room temperature for 2 h. After completion, the reaction mixture was concentrated, neutralized with 5% aqueous H₃PO₄ solution, extracted with ethyl acetate, washed with brine, dried over anhydrous Na₂SO₄ and concentrated. The crude 5-bromo-6-(trifluoromethyl)nicotinic acid (**5-S34**) was directly taken for the next step (11.4 g, impure). **LC-MS (ESI, m/z):** calcd for [M-H]⁻267.9; found: 268.0.

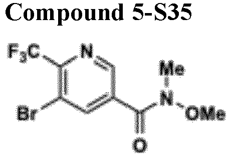

### 5-bromo-N-methoxy-N-methyl-6-(trifluoromethyl)nicotinamide (5-S35):

To a stirred solution of 5-bromo-6-(trifluoromethyl)nicotinic acid (**5-S34**) (11.4 g, 42.22 mmol) in DCM (50 mL), Et₃N (29.4 mL, 211.11 mmol) followed by EDC•HCl (16.188 g, 84.44 mmol) and HOBT (11.41 g, 84.44 mmol) were added. The mixture was stirred at room temperature for 5 minutes and then Weinreb salt (4.942 g, 50.67 mmol) was added and stirred at room temperature for 16 h. After completion, the reaction mixture was extracted with DCM, washed with aqueous NaHCO₃ solution, brine, dried over anhydrous Na₂SO₄ and concentrated. The crude product was purified by CombiFlash chromatography (20% ethyl acetate in hexane) to afford 5-bromo-N-methoxy-*N*-methyl-6-(trifluoromethyl)nicotinamide **(5-S35)** (9.5 g, impure) as a brown oil. The impure 5-bromo-*N*-methoxy-*N*-methyl-6-(trifluoromethyl)nicotinamide (**5-S35**) was further purified by Preparative-HPLC (RP) to afford pure 5-bromo-N-methoxy-*N-*methyl-6-(trifluoromethyl)nicotinamide **(5-S35)** as a colourless oil (2.7 g, 20%). **¹H NMR (400 MHz, CDCl₃**) *δ* 8.92 (s, 1H), 8.38 (s, 1H), 3.58 (s, 3H), 3.40 (s, 3H) ppm.**LC-MS (ESI, m/z):** calcd for [M+H]⁺ 313.0; found: 313.0.

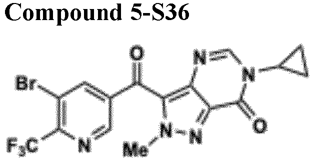

### 3-(5-bromo-6-(trifluoromethyl)nicotinoyl)-6-cyclopropyl-2-methyl-2,6-dihydro-7H-pyrazolo[4,3-d]pyrimidin-7-one (5-S36):

To a stirred solution of 3-bromo-6-cyclopropyl-2-methyl-2,6-dihydro-7*H*-pyrazolo[4,3-*d*]pyrimidin-7-one (**3-S5**) (300 mg, 1.11 mmol) in anhydrous THF (5 mL), iPrMgCl•LiCl (1.3 M in THF) (1.12 mL, 1.44 mmol) was added dropwise at 0 °C and stirred for 15 minutes. After that 5-bromo-*N*-methoxy-*N*-methyl-6-(trifluoromethyl)nicotinamide (**5-S35**) (349 mg, 1.11 mmol) in anhydrous THF (2 mL) was added and stirred at 0 °C for 30 minutes. After completion, the reaction mixture was quenched with aqueous saturated NH₄Cl solution, extracted with ethyl acetate, washed with brine, dried over anhydrous Na₂SO₄ and concentrated. The crude product was purified by CombiFlash chromatography (30% ethyl acetate in hexane) to afford 3-(5-bromo-6-(trifluoromethyl)nicotinoyl)-6-cyclopropyl-2-methyl-2,6-dihydro-7*H-*pyrazolo[4,3-*d*]pyrimidin-7-one (**5-S36**) as a yellow gum (90 mg, 18%). **¹H NMR (400 MHz, CDCl₃**) *δ* 8.99 (s, 1H), 8.51 (s, 1H), 7.91 (s, 1H), 4.45 (s, 3H), 3.25-3.20 (m, 1H), 1.24-1.15 (m, 2H), 0.92-0.85 (m, 2H) ppm. **LC-MS (ESI, m/z):** calcd for [M+H]⁺ 442.0; found: 442.2.

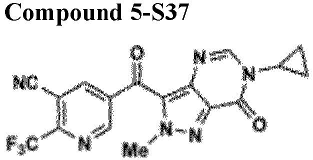

### 5-(6-cyclopropyl-2-methyl-7-oxo-6,7-dihydro-2H-pyrazolo[4,3-d]pyrimidine-3-carbonyl)-2-(trifluoromethyl)nicotinonitrile (5-S37):

In a reaction tube, a stirred solution of 3-(5-bromo-6-(trifluoromethyl)nicotinoyl)-6-cyclopropyl-2-methyl-2,6-dihydro-7*H*-pyrazolo[4,3-*d*]pyrimidin-7-one (**5-S36**) (106 mg, 0.24 mmol) in DMF (5 mL), was degassed with argon for 15 minutes and then Pd₂(dba)₃ (22 mg, 0.024 mmol) followed by Zn(CN)₂ (56 mg, 0.48 mmol) and xantphos (28 mg, 0.05 mmol) were added. The tube was sealed, and the reaction mixture was stirred at 140 °C for 10 h. After completion, the reaction mixture was diluted with cold water, extracted with ethyl acetate, washed with brine, dried over anhydrous Na₂SO₄ and concentrated (100 mg, crude). The crude LCMS indicated that 5-(6-cyclopropyl-2-methyl-7-oxo-6,7-dihydro-2*H*-pyrazolo[4,3-*d*]pyrimidine-3-carbonyl)-2-(trifluoromethyl)nicotinonitrile (**5-S37**) along with major 5-((6-cyclopropyl-2-methyl-7-oxo-6,7-dihydro-2*H*-pyrazolo[4,3-*d*]pyrimidin-3-yl)(hydroxy)methyl)-2-(trifluoromethyl)nicotinonitrile was formed. To a stirred solution of the crude product mixture (100 mg, 0.26 mmol) in DCM (5 mL), MnO₂ (223 mg, 2.56 mmol) was added and the reaction mixture was stirred at room temperature for 16 h. After completion, the reaction mixture was filtered through Celite bed and the filtrate was concentrated. The crude product was purified by CombiFlash chromatography (30% ethyl acetate in hexane) to afford pure 5-(6-cyclopropyl-2-methyl-7-oxo-6,7-dihydro-2*H*-pyrazolo[4,3-*d*]pyrimidine-3-carbonyl)-2-(trifluoromethyl)nicotinonitrile (**5-S37**) as a pale yellow solid (80 mg, 80%). **¹H NMR (400 MHz, CDCl₃)** *δ* 9.25 (s, 1H), 8.63 (s, 1H), 7.91 (s, 1H), 4.48 (s, 3H), 3.25-3.20 (m, 1H), 1.27-1.21 (m, 2H), 0.93-0.90 (m, 2H) ppm. **LC-MS (ESI, m/z):** calcd for [M+H]⁺ 389.1; found: 389.2.

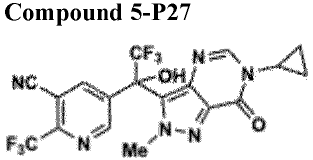

### 5-(1-(6-cyclopropyl-2-methyl-7-oxo-6,7-dihydro-2H-pyrazolo[4,3-d]pyrimidin-3-yl)-2,2,2-trifluoro-1-hydroxyethyl)-2-(trifluoromethyl)nicotinonitrile (5-P27):

To a stirred solution of 5-(6-cyclopropyl-2-methyl-7-oxo-6,7-dihydro-2*H*-pyrazolo[4,3-*d*]pyrimidine-3-carbonyl)-2-(trifluoromethyl)nicotinonitrile (**5-S37**) (80 mg, 0.21 mmol) in DME (3 mL), CsF (16 mg, 0.1 mmol) followed by TMSCF₃ (44 mg, 0.31 mmol) was added at 0 °C and the reaction mixture was stirred at room temperature for 2 h. After completion, the reaction mixture was quenched with aqueous saturated NH₄Cl solution, extracted with ethyl acetate, washed with brine, dried over anhydrous Na₂SO₄ and concentrated. The crude product was purified by CombiFlash chromatography (40% ethyl acetate in hexane) to afford 5-(1-(6-cyclopropyl-2-methyl-7-oxo-6,7-dihydro-2H-pyrazolo[4,3-*d*]pyrimidin-3-yl)-2,2,2-trifluoro-1-hydroxyethyl)-2-(trifluoromethyl)nicotinonitrile (**5-P27**) as an off white solid (60 mg, 64%). **Physical State**: off white solid. The racemic compound **5-P27** (60 mg) was purified from preparative-HPLC-SFC to afford Enantiomer-1 (**5-53**) (20 mg) and Enantiomer-2 (**5-52**) (60 mg).

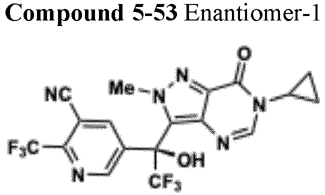

***(S)-5-(1-(6-cyclopropyl-2-methyl-7-oxo-6,7-dihydro-2H-pyrazolo[4,3-d]pyrimidin-3-yl)-2,2,2-trifluoro-1-hydroxyethyl)-2-(trifluoromethyl)nicotinonitrile* (5-53) ¹H NMR (400 MHz, DMSO-*d*₆):** *δ* 9.05 (s, 1H), 8.79 (s, 1H), 8.61 (s, 1H), 8.04 (s, 1H), 3.87 (s, 3H), 3.20-3.15 (m, 1H), 1.05-1.00 (m, 2H), 0.90-0.85 (m, 2H) ppm. **LC-MS (ESI, m/z)**: calcd for [M-H]⁻ 457.1; found: 457.2.

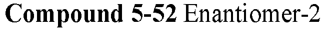

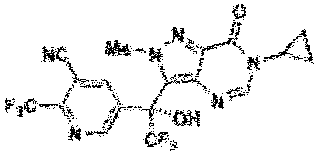

***(R)-5-(1-(6-cyclopropyl-2-methyl-7-oxo-6,7-dihydro-2H-pyrazolo[4,3-d]pyrimidin-3-yl)-2,2,2-trifluoro-1-hydroxyethyl)-2-(trifluoromethyl)nicotinonitrile* (5-52) ¹H NMR (400 MHz, DMSO-*d*₆):** *δ* 8.06 (s, 1H), 7.88-7.84 (m, 2H), 7.34 (d, *J =* 8.2 Hz, 1H), 3.71 (s, 2H), 3.66 (s, 3H), 3.19-3.13 (m, 1H), 1.05-1.00 (m, 2H), 0.95-0.90 (m, 2H) ppm. **¹H NMR (400 MHz, DMSO-*d*₆):** *δ* 9.05 (s, 1H), 8.79 (s, 1H), 8.61 (s, 1H), 8.04 (s, 1H), 3.87 (s, 3H), 3.20-3.15 (m, 1H), 1.05-1.00 (m, 2H), 0.90-0.85 (m, 2H) ppm. LC-MS **(ESI, m/z)**: calcd for [M-H]⁻457.1; found: 457.2.

### Synthesis of compounds 5-54 and 5-55

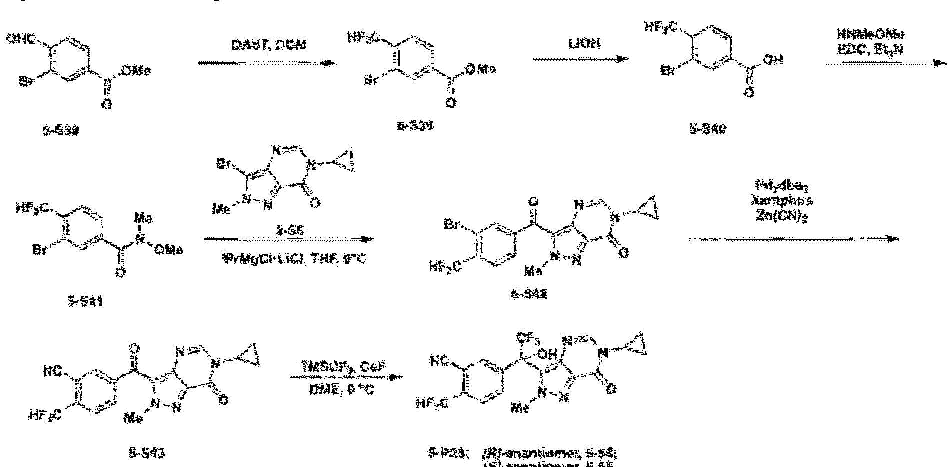

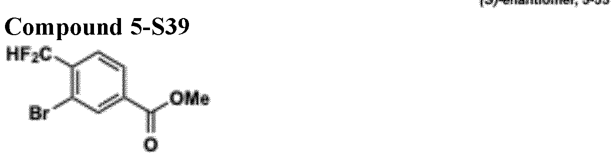

### methyl 3-bromo-4-(difluoromethyl)benzoate (5-S39):

To a stirred solution of methyl 3-bromo-4-formylbenzoate (**5-S38**) (50 mg, 0.21 mmol) in anhydrous DCM (2 mL), was added DAST (41 uL, 0.31 mmol) at 0 °C and the reaction mixture was stirred at room temperature for overnight. After completion, the reaction mixture was quenched with ice cold aqueous NaHCO₃ solution, extracted with dichloromethane, washed with brine, dried over anhydrous Na₂SO₄ and concentrated. The crude product was purified by CombiFlash chromatography (5% ethyl acetate in hexane) to afford methyl 3-bromo-4-(difluoromethyl)benzoate (**5-S39**) as a colourless oil (50 mg, 92%). **¹H NMR (400 MHz, CDCl₃**): *δ* 8.27 (s, 1H), 8.06 (d, *J* = 8.0 Hz, 1H), 7.73 (d, *J* = 8.1 Hz, 1H), 6.92 (t, *J* = 54.5 Hz, 1H), 3.94 (s, 3H) ppm.

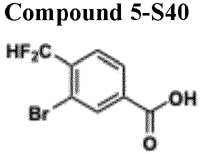

### 3-bromo-4-(difluoromethyl)benzoic acid (5-S40):

To a stirred solution of methyl 3-bromo-4-(difluoromethyl)benzoate (**5-S39**) (600 mg, 2.26 mmol) in THF-Water (5 mL), LiOH•H₂O (190 mg, 4.53 mmol) was added and the reaction mixture was stirred at room temperature for 2 h. After completion, the reaction mixture was concentrated, neutralized with 6N HCl, extracted with ethyl acetate, washed with brine, dried over anhydrous Na₂SO₄ and concentrated. The crude 3-bromo-4-(difluoromethyl)benzoic acid (**5-S40**) was directly taken for the next step (560 mg, crude). **¹H NMR (400 MHz, DMSO-*d*₆):** *δ* 13.62 (s, 1H), 8.18 (s, 1H), 8.06 (d, *J* = 8.0 Hz, 1H), 7.81 (d, *J* = 8.1 Hz, 1H), 7.21 (t, *J* = 53.9 Hz, 1H) ppm. **LC-MS (ESI, m/z):** calcd for [M-H]⁻ 248.9; found: 249.1.

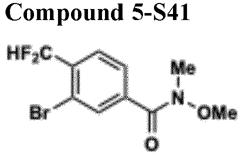

### 3-bromo-4-(difluoromethyl)-N-methoxy-N-methylbenzamide (5-S41):

To a stirred solution of 3-bromo-4-(difluoromethyl)benzoic acid (**5-S40**) (560 mg, 2.231 mmol) in DCM (10 mL), Et₃N (1.56 mL, 11.154 mmol) followed by EDC•HCl (855 mg, 4.462 mmol) and HOBT (603 mg, 4.462 mmol) were added. The mixture was stirred at room temperature for 5 minutes and then Weinreb salt (261 mg, 2.677 mmol) was added and stirred at room temperature for overnight. After completion, the reaction mixture was extracted with DCM, washed with aqueous NaHCO₃ solution, brine, dried over anhydrous Na₂SO₄ and concentrated. The crude product was purified by Combiflash chromatography (20% ethyl acetate in hexane) to afford 3-bromo-4-(difluoromethyl)-*N*-methoxy-*N*-methylbenzamide (**5-S41**) as an off white solid (520 mg, 79%). **¹H NMR (400 MHz, CDCl₃**) *δ* 7.92 (s, 1H), 7.72 (d, *J =* 8.1 Hz, 1H), 7.69 (d, *J =* 8.1 Hz, 1H), 6.91 (t, *J =* 54.6 Hz, 1H), 3.54 (s, 3H), 3.37 (s, 3H) ppm. **LC-MS** (**ESI, m/z**): calcd for [M+H]⁺ 294.0; found: 294.1.

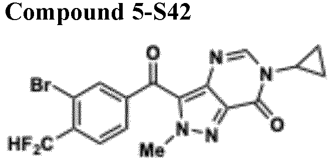

### 3-(3-bromo-4-(difluoromethyl)benzoyl)-6-cyclopropyl-2-methyl-2,6-dihydro-7H-pyrazolo[4,3-d]pyrimidin-7-one (5-S42):

To a stirred solution of 3-bromo-6-cyclopropyl-2-methyl-2,6-dihydro-7*H*-pyrazolo[4,3-*d*]pyrimidin-7-one (**3-S5**) (400 mg, 1.49 mmol) in anhydrous THF (7 mL), iPrMgCl•LiCl (1.3M in THF) (1.5 mL, 1.94 mmol) was added dropwise at 0 °C and stirred for 15 minutes. After that 3-bromo-4-(difluoromethyl)-*N*-methoxy-*N*-methylbenzamide (**5-S41**) (437 mg, 1.49 mmol) in anhydrous THF (2 mL) was added and stirred at 0 °C for 30 minutes. After completion, the reaction mixture was quenched with aqueous saturated NH₄Cl solution, extracted with ethyl acetate, washed with brine, dried over anhydrous Na₂SO₄ and concentrated. The crude product was purified by CombiFlash chromatography (30% ethyl acetate in hexane) to afford 3-(3-bromo-4-(difluoromethyl)benzoyl)-6-cyclopropyl-2-methyl-2,6-dihydro-7*H-*pyrazolo[4,3-*d*]pyrimidin-7-one (**5-S42**) as a yellow gum (185 mg, 29%). **¹H NMR (400 MHz, CDCl₃**) *δ* 8.16 (s, 1H), 7.95 (d, *J* = 8.0 Hz, 1H), 7.91 (s, 1H), 7.80 (d, *J* = 8.0 Hz, 1H), 6.97 (t, *J=* 54.4 Hz, 1H), 4.42 (s, 3H), 3.27-3.22 (m, 1H), 1.27-1.22 (m, 2H), 0.96-0.90 (m, 2H) ppm. **LC-MS (ESI, m/z)**: calcd for [M+H]⁺ 423.0; found: 423.1.

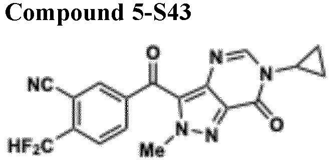

### 5-(6-cyclopropyl-2-methyl-7-oxo-6,7-dihydro-2H-pyrazolo[4,3-d]pyrimidine-3-carbonyl)-2-(difluoromethyl)benzonitrile (5-S43):

In a reaction tube, a stirred solution of 3-(3-bromo-4-(difluoromethyl)benzoyl)-6-cyclopropyl-2-methyl-2,6-dihydro-7*H*-pyrazolo[4,3-*d*]pyrimidin-7-one (**5-S42**) (153 mg, 0.36 mmol) in DMF (5 mL), was degassed with argon for 15 minutes and then Pd₂(dba)₃ (33 mg, 0.04 mmol) followed by Zn(CN)₂ (85 mg, 0.72 mmol) and xantphos (42 mg, 0.07 mmol) were added. The tube was sealed, and the reaction mixture was stirred at 140 °C for 10 h. After completion, the reaction mixture was diluted with cold water, extracted with ethyl acetate, washed with brine, dried over anhydrous Na₂SO₄ and concentrated (150 mg, crude). LCMS indicated that desire product 5-(6-cyclopropyl-2-methyl-7-oxo-6,7-dihydro-2H-pyrazolo[4,3-*d*]pyrimidine-3-carbonyl)-2-(difluoromethyl)benzonitrile (**5-S43**) along with corresponding reduced hydroxy product 5-(6-cyclopropyl-2-methyl-7-oxo-6,7-dihydro-2*H*-pyrazolo[4,3-*d*]pyrimidine-3-carbonyl)-2-(difluoromethyl)benzonitrile was formed. To a stirred solution of the crude mixture (150 mg, 0.404 mmol) in DCM (5 mL), MnO₂ (351 mg, 4.039 mmol) was added and the reaction mixture was stirred at room temperature for overnight. After completion, the reaction mixture was filtered through celite bed, washed with dichloromethane and the filtrate was concentrated. The crude product was purified by CombiFlash chromatography (30% ethyl acetate in hexane) to afford 5-(6-cyclopropyl-2-methyl-7-oxo-6,7-dihydro-2*H*-pyrazolo[4,3-*d*]pyrimidine-3-carbonyl)-2-(difluoromethyl)benzonitrile (**5-S43**) as an off white solid (95 mg, 64%). **LC-MS (ESI, m/z)**: calcd for [M+H]⁺ 370.1; found: 370.2.

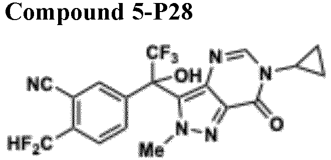

### 5-(1-(6-cyclopropyl-2-methyl-7-oxo-6,7-dihydro-2H-pyrazolo[4,3-d]pyrimidin-3-yl)-2,2,2-trifluoro-1-hydroxyethyl)-2-(difluoromethyl)benzonitrile (5-P28):

To a stirred solution of 5-(6-cyclopropyl-2-methyl-7-oxo-6,7-dihydro-2*H*-pyrazolo[4,3-*d*]pyrimidine-3-carbonyl)-2-(difluoromethyl)benzonitrile (**6**) (95 mg, 0.26 mmol) in DME (3 mL), CsF (20 mg, 0.13 mmol) followed by TMSCF₃ (55 mg, 0.39 mmol) was added at 0 °C and the reaction mixture was stirred at room temperature for 2 h. After completion, the reaction mixture was quenched with aqueous saturated NH₄Cl solution, extracted with ethyl acetate, washed with brine, dried over anhydrous Na₂SO₄ and concentrated. The crude product was purified by Combiflash chromatography (40% ethyl acetate in hexane) to afford 5-(1-(6-cyclopropyl-2-methyl-7-oxo-6,7-dihydro-2*H*-pyrazolo[4,3-*d*]pyrimidin-3-yl)-2,2,2-trifluoro-1-hydroxyethyl)-2-(difluoromethyl)benzonitrile (**5-P28**) as an off white solid (40 mg, 35%). **Physical State**: off white solid. The racemic compound **5-P28** (40 mg) was purified from preparative-HPLC-SFC to afford Enantiomer-1 (**5-55**) (10 mg) and Enantiomer-2 (**5-54**) (10 mg).

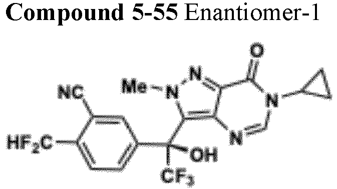

### (S)-5-(1-(6-cyclopropyl-2-methyl-7-oxo-6,7-dihydro-2H-pyrazolo[4,3-d]pyrimidin-3-yl)-2,2,2-trifluoro-1-hydroxyethyl)-2-(difluoromethyl)benzonitrile (5-55)

**¹H NMR (400 MHz, DMSO-*d*₆):** *δ* 8.49 (s, 1H), 8.09 (s, 1H), 7.99 (s, 1H), 7.89 (d, *J =* 8.3 Hz, 1H), 7.80 (d, *J* = 8.3 Hz, 1H), 7.31 (t, *J =* 54.1 Hz, 1H), 3.70 (s, 3H), 3.20-3.17 (m, 1H), 1.04-1.01 (m, 2H), 0.94-0.91 (m, 2H) ppm. **LC-MS (ESI, m/z):** calcd for [M+H]⁺ 440.1; found: 440.3.

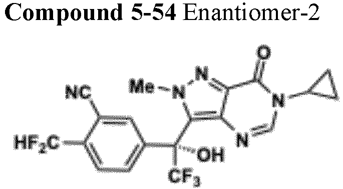

***(R)-5-(1-(6-cyclopropyl-2-methyl-7-oxo-6,7-dihydro-2H-pyrazolo[4,3-d]pyrimidin-3-yl)-2,2,2-trifluoro-1-hydroxyethyl)-2-(difluoromethyl)benzonitrile* (5-54) ¹H NMR (400 MHz, DMSO-*d*₆):** *δ* 8.49 (s, 1H), 8.09 (s, 1H), 7.99 (s, 1H), 7.89 (d, *J* = 8.3 Hz, 1H), 7.80 (d, *J* = 8.3 Hz, 1H), 7.31 (t, *J* = 54.1 Hz, 1H), 3.70 (s, 3H), 3.20-3.17 (m, 1H), 1.04-1.01 (m, 2H), 0.94-0.91 (m, 2H) ppm. **LC-MS (ESI, m/z):** calcd for [M+H]⁺ 440.1; found: 440.2.

### Synthesis of compounds 5-56 and 5-57

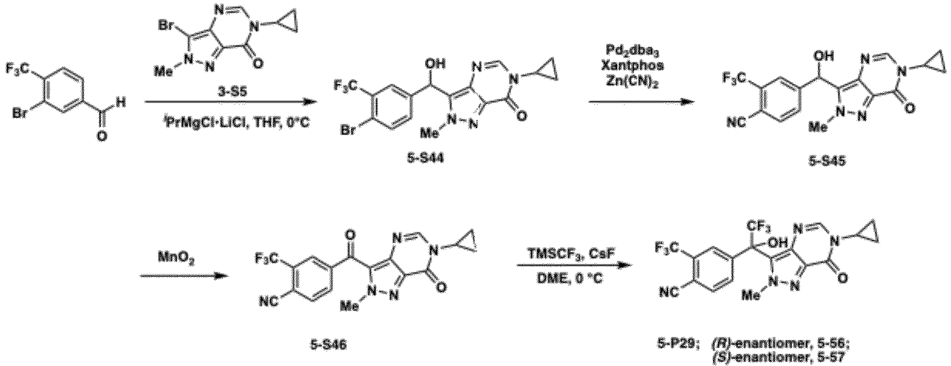

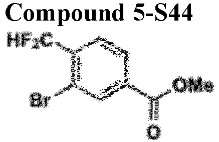

### 3-((4-bromo-3-(trifluoromethyl)phenyl)(hydroxy)methyl)-6-cyclopropyl-2-methyl-2,6-dihydro-7H-pyrazolo[4,3-d]pyrimidin-7-one (5-S44):

To a stirred solution of 3-bromo-6-cyclopropyl-2-methyl-2,6-dihydro-7*H*-pyrazolo[4,3-*d*]pyrimidin-7-one (**3-S5**) (600 mg, 2.23 mmol) in anhydrous THF (7 mL), iPrMgCl•LiCl (1.3M in THF) (2.23 mL, 2.9 mmol) was added dropwise at 0 °C and stirred for 15 minutes. After that 4-bromo-3-(trifluoromethyl)benzaldehyde (564 mg, 2.23 mmol) in anhydrous THF (3 mL) was added and stirred at 0 °C for 30 minutes. After completion, the reaction mixture was quenched with aqueous saturated NH₄Cl solution, extracted with ethyl acetate, washed with brine, dried over anhydrous Na₂SO₄ and concentrated. The crude product was purified by CombiFlash chromatography (70% ethyl acetate in hexane) to afford 3-((4-bromo-3-(trifluoromethyl)phenyl)(hydroxy)methyl)-6-cyclopropyl-2-methyl-2,6-dihydro-7*H-*pyrazolo[4,3-*d*]pyrimidin-7-one (**5-S44**) as a white solid (580 mg, 59%). **¹H NMR (400 MHz, DMSO-*d*₆):** *δ* 7.97 (s, 1H), 7.89 (s, 1H), 7.85 (d, *J =* 8.3 Hz, 1H), 7.48 (d, *J =* 7.9 Hz, 1H), 6.76 (d, *J* = 4.2 Hz, 1H), 6.32 (d, *J* = 3.5 Hz, 1H), 3.97 (s, 3H), 3.20-3.15 (m, 1H), 1.05-1.00 (m, 2H), 0.90-0.85 (m, 2H) ppm. **LC-MS (ESI,** m/z): calcd for [M+H]⁺ 443.03; found: 443.24.

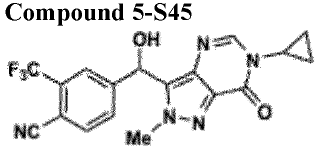

### 4-((6-cyclopropyl-2-methyl-7-oxo-6,7-dihydro-2H-pyrazolo[4,3-d]pyrimidin-3-yl)(hydroxy)methyl)-2-(trifluoromethyl)benzonitrile (5-S45):

In a reaction tube, a stirred solution of 3-((4-bromo-3-(trifluoromethyl)phenyl)(hydroxy)methyl)-6-cyclopropyl-2-methyl-2,6-dihydro-7*H-*pyrazolo[4,3-*d*]pyrimidin-7-one (**5-S44**) (300 mg, 0.68 mmol) in DMF (5 mL), was degassed with argon for 15 minutes and then Pd₂(dba)₃ (62 mg, 0.07 mmol) followed by Zn(CN)₂ (159 mg, 1.35 mmol) and Xantphos (78 mg, 0.14 mmol) were added. The tube was sealed, and the reaction mixture was stirred at 140 °C for 10 h. After completion, the reaction mixture was diluted with cold water, extracted with ethyl acetate, washed with brine, dried over anhydrous Na₂SO₄ and concentrated. The crude product was purified by CombiFlash chromatography (70% ethyl acetate in hexane) to afford 4-((6-cyclopropyl-2-methyl-7-oxo-6,7-dihydro-2*H-*pyrazolo[4,3-*d*]pyrimidin-3-yl)(hydroxy)methyl)-2-(trifluoromethyl)benzonitrile (**5-S45**) as an off white solid (190 mg, 72%). **¹H NMR (400 MHz, DMSO-*d₆*):** *δ* 8.15 (d, *J =* 8.0 Hz, 1H), 8.04 (s, 1H), 7.93 (s, 1H), 7.81 (d, *J* = 8.0 Hz, 1H), 6.89 (d, *J* = 4.1 Hz, 1H), 6.42 (d, *J* = 4.1 Hz, 1H), 4.01 (s, 3H), 3.15-3.10 (m, 1H), 1.01-0.99 (m, 2H), 0.90-0.85 (m, 2H) ppm. **LC-MS (ESI, m/z)**: calcd for [M+H]⁺ 390.1; found: 390.3.

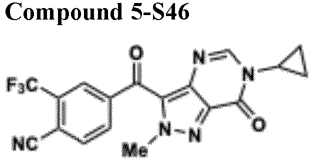

### 4-(6-cyclopropyl-2-methyl-7-oxo-6,7-dihydro-2H-pyrazolo[4,3-d]pyrimidine-3-carbonyl)-2-(trifluoromethyl)benzonitrile (5-S46):

To a stirred solution of 4-((6-cyclopropyl-2-methyl-7-oxo-6,7-dihydro-2*H*-pyrazolo[4,3-*d*]pyrimidin-3-yl)(hydroxy)methyl)-2-(trifluoromethyl)benzonitrile (**5-S45**) (350 mg, 0.9 mmol) in DCM (10 mL), MnO₂ (782 mg, 8.99 mmol) was added and the reaction mixture was stirred at room temperature for 4 h. After completion, the reaction mixture was filtered through Celite bed and the filtrate was concentrated. The crude 4-(6-cyclopropyl-2-methyl-7-oxo-6,7-dihydro-2*H*-pyrazolo[4,3-*d*]pyrimidine-3-carbonyl)-2-(trifluoromethyl)benzonitrile (**5-S46**) was taken directly for the next step (350 mg, crude).**¹H NMR (400 MHz, DMSO-d*₆***) *δ* 8.37 (d, *J* = 7.9 Hz, 1H), 8.33 (s, 1H), 8.27 (d, *J =* 8.0 Hz, 1H), 8.07 (s, 1H), 4.34 (s, 3H), 3.19-3.16 (m, 1H), 1.05-1.00 (m, 2H), 0.90-0.85 (m, 2H) ppm. **LC-MS (ESI, m/z):** calcd for [M+H]⁺ 388.10; found: 388.2.

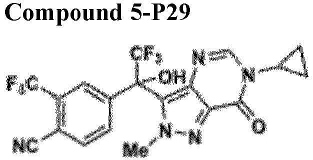

### 4-(1-(6-cyclopropyl-2-methyl-7-oxo-6,7-dihydro-2H-pyrazolo[4,3-d]pyrimidin-3-yl)-2,2,2-trifluoro-1-hydroxyethyl)-2-(trifluoromethyl)benzonitrile (5-P29):

To a stirred solution of 4-(6-cyclopropyl-2-methyl-7-oxo-6,7-dihydro-2*H*-pyrazolo[4,3-*d*]pyrimidine-3-carbonyl)-2-(trifluoromethyl)benzonitrile (**5-S46**) (200 mg, 0.52 mmol) in DME (5 mL), CsF (39 mg, 0.26 mmol) followed by TMSCF₃ (84 uL, 0.57 mmol) was added at 0 °C and the mixture was stirred at room temperature for 2 h. After completion, the reaction mixture was quenched with aqueous saturated NH₄Cl solution, extracted with ethyl acetate, washed with brine, dried over anhydrous Na₂SO₄ and concentrated. The crude product was purified by CombiFlash chromatography (70% ethyl acetate in hexane) to afford 4-(1-(6-cyclopropyl-2-methyl-7-oxo-6,7-dihydro-2H-pyrazolo[4,3-*d*]pyrimidin-3-yl)-2,2,2-trifluoro-1-hydroxyethyl)-2-(trifluoromethyl)benzonitrile (**5-P29**) as an off white solid (60 mg, 25%).

**Physical State**: off white solid.

The racemic compound **5-P29** was purified from Prep-HPLC-SFC to afford Enantiomer-1 (**5-57**) (15 mg) (RT 6.425 min) and Enantiomer-2 (**5-56**) (15 mg) (RT 7.314 min).

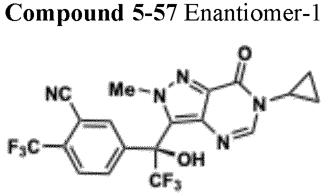

### (S)-5-(1-(6-cyclopropyl-2-methyl-7-oxo-6,7-dihydro-2H-pyrazolo[4,3-d]pyrimidin-3-yl)-2,2,2-trifluoro-1-hydroxyethyl)-2-(trifluoromethyl)benzonitrile (5-57)

**¹H NMR (400 MHz, DMSO-*d*₆):** *δ* 8.66 (s, 1H), 8.23 (d, *J =* 8.1 Hz, 1H), 8.08 (s, 1H), 8.01 (s, 1H), 7.72 (d, *J=* 8.1 Hz, 1H), 3.72 (s, 3H), 3.20-3.10 (m, 1H), 1.03-1.00 (m, 2H), 0.95-0.90 (m, 2H) ppm. **LC-MS (ESI, m/z):** calcd for [M+H]⁺ 458.1; found: 458.2.

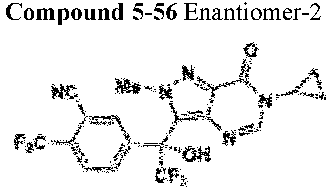

### (R)-5-(1-(6-cyclopropyl-2-methyl-7-oxo-6,7-dihydro-2H-pyrazolo[4,3-d]pyrimidin-3-yl)-2,2,2-trifluoro-1-hydroxyethyl)-2-(trifluoromethyl)benzonitrile (5-56)

**¹H NMR (400 MHz, DMSO-*d*₆):** *δ* 8.67 (s, 1H), 8.23 (d, *J* = 7.9 Hz, 1H), 8.08 (s, 1H), 8.01 (s, 1H), 7.72 (d, *J* = 8.0 Hz, 1H), 3.72 (s, 3H), 3.20-3.10 (m, 1H), 1.03-0.98 (m, 2H), 0.95-0.90 (m, 2H) ppm. **LC-MS (ESI, m/z):** calcd for [M+H]⁺ 458.1; found: 458.2.

### Example 2: Activity of the compounds according to the invention

The activity of the compounds of the invention was tested as described below. The data are presented below under **Table 1 below.**

**Table 1**

| **Patent ID** | **P. falciparum DHODH (IC₅₀) [µM]** | **P. falciparum 3D7 (EC₅₀) [µM]** | **P. vivax DHODH (IC₅₀) [µM]** | **Human DHODH IC₅₀ [µM]** |
|---|---|---|---|---|
| 1-1 | 0.046 | 0.016 | 0.040 | >30 |
| 1-3 | 0.017 | 0.0013 | 0.017 | >30 |
| 1-5 | 0.017 | 0.00075 | 0.021 | 0.91 |
| 1-7 | 0.06 | 0.0065 | 0.026 | 14 |
| 1-9 | 0.016 | 0.00092 | 0.0069 | 14 |
| 1-10 | 0.025 | 0.0014 | 0.0097 | >30 |
| 1-13 | 0.007 | 0.00049 | 0.011 | 0.67 |
| 1-14 | 0.014 | 0.00064 | 0.018 | 4.2 |
| 1-16 | 0.016 | 0.0032 | 0.023 | >30 |
| 1-18 | 0.012 | 0.009 | 0.011 | 22 |
| 1-20 | 0.013 | 0.0005 | 0.019 | 1.4 |
| 1-22 | 0.033 | 0.0027 | 0.048 | 5.3 |
| 1-24 | 0.024 | 0.0012 | 0.033 | 6.2 |
| 1-26 | 0.046 | 0.0067 | 0.048 | >30 |
| 1-28 | 0.026 | 0.0046 | 0.011 | >100 |
| 1-30 | 0.018 | 0.007 | 0.0079 | 34 |
| 1-32 | 0.039 | 0.0024 | 0.0080 | >100 |
| 1-34 | 0.021 | 0.0003 | 0.0041 | 3.9 |
| 1-36 | 0.045 | 0.0014 | 0.0092 | >100 |
| 1-38 | 0.017 | 0.0015 | 0.0043 | 88 |
| 1-40 | 0.017 | 0.00042 | 0.0052 | 80 |
| 1-42 | 0.013 | 0.00049 | 0.0079 | 41 |
| 1-44 | 0.063 | 0.0072 | 0.029 | >100 |
| 1-46 | 0.01 | 0.00020 | 0.0058 | 41 |
| 1-48 | 0.081 | 0.018 | 0.044 | >100 |
| 1-50 | 0.054 | 0.0015 | 0.027 | 46 |
| 2-1 | 0.029 | 0.012 | 0.01 | 2.3 |
| 2-3 | 0.029 | 0.0099 | 0.011 | 2.7 |
| 2-5 | 0.019 | 0.0013 | 0.019 | 4.8 |
| 2-7 | 0.023 | 0.0042 | 0.017 | >30 |
| 2-10 | 0.039 | 0.015 | 0.021 | >30 |
| 2-11 | 0.0096 | 0.0019 | 0.0099 | >30 |
| 2-14 | 0.018 | 0.0027 | 0.019 | >30 |
| 2-15 | 0.0062 | 0.00032 | 0.0098 | 1.7 |
| 2-17 | 0.0084 | 0.0017 | 0.0095 | 2.5 |
| 2-19 | 0.0069 | 0.00035 | 0.0029 | 11 |
| 3-1 | 0.021 | 0.0022 | 0.0079 | >30 |
| 3-3 | 0.022 | 0.004 | 0.0083 | >30 |
| 3-5 | 0.016 | 0.00094 | 0.017 | 6.8 |
| 3-7 | 0.027 | 0.0016 | 0.055 | 14 |
| 3-9 | 0.048 | 0.012 | 0.059 | >30 |
| 3-11 | 0.032 | 0.00052 | 0.0094 | >100 |
| 4-1 | 0.021 | 0.0026 | 0.013 | >30 |
| 4-4 | 0.012 | 0.00061 | 0.0090 | 15 |
| 4-5 | 0.016 | 0.01 | 0.007 | 1.3 |
| 4-8 | 0.0093 | 0.0011 | 0.0062 | 1 |
| 4-9 | 0.044 | 0.014 | nd | 21 |
| 4-11 | 0.042 | 0.0026 | 0.041 | 13 |
| 4-14 | 0.083 | 0.016 | 0.039 | >100 |
| 4-16 | 0.017 | 0.0074 | 0.0078 | 16 |
| 4-18 | 0.012 | 0.0034 | 0.0062 | 4.7 |
| 4-20 | 0.0082 | 0.00044 | 0.0083 | 20 |
| 4-22 | 0.023 | 0.0041 | 0.020 | 4.6 |
| 4-24 | 0.011 | 0.0021 | 0.0087 | 3.2 |
| 4-26 | 0.0095 | 0.00091 | 0.0052 | 1.4 |
| 4-27 | 0.047 | 0.0065 | 0.040 | >30 |
| 5-1 | 0.021 | 0.0038 | 0.020 | >30 |
| 5-3 | 0.018 | 0.0017 | 0.028 | >30 |
| 5-5 | 0.014 | 0.00051 | 0.019 | >30 |
| 5-7 | 0.013 | 0.00017 | 0.0091 | 4.7 |
| 5-9 | 0.014 | 0.00052 | 0.0098 | 9.2 |
| 5-11 | 0.032 | 0.0019 | 0.025 | >30 |
| 5-13 | 0.022 | 0.0017 | 0.019 | >30 |
| 5-15 | 0.0086 | 0.00029 | 0.0093 | 24 |
| 5-17 | 0.0046 | 0.000046 | 0.0061 | 5.6 |
| 5-19 | 0.012 | 0.00035 | 0.020 | 17 |
| 5-21 | 0.031 | 0.0021 | 0.029 | >30 |
| 5-22 | 0.011 | 0.0061 | 0.0077 | 22 |
| 5-26 | 0.017 | 0.00039 | 0.025 | >30 |
| 5-32 | 0.005 | 0.00025 | 0.0085 | 14 |
| 5-40 | 0.018 | 0.0031 | 0.022 | >30 |
| 5-45 | 0.0082 | 0.00085 | 0.0085 | 30 |
| 5-49 | 0.0039 | 0.00064 | 0.0042 | 9 |
| 5-51 | 0.0043 | 0.00071 | 0.004 | 30 |
| 5-52 | 0.062 | 0.011 | 0.046 | >30 |
| 5-54 | 0.044 | 0.003 | 0.0035 | >30 |
| 5-56 | 0.018 | 0.00014 | nd | nd |

| | | | | |
|---|---|---|---|---|
| ***nd, not determined.*** | | | | |

### DHODH Protein Expression and Purification

For enzyme kinetic analysis recombinant expression of the N-terminally truncated soluble domains of DHODH from various *Plasmodium* (*Pf*DHODH and *Pv*DHODH) and mammalian species (human, rat, mouse and dog) was performed in *E. coli* phage-resistant BL21-DE3 cells in the presence of the appropriate antibiotics as previously described from previously described expression plasmids (Coteron et al., 2011, J Med Chem, 54 (15), 5540-5561. DOI: 10.1021/jm200592f*;* Phillips et al., 2015, Sci Transl Med, 7 (296), 296ra111. DOI: 10.1126/scitranslmed.aaa6645*;* Deng et al., 2014, J Med Chem, 57 (12), 5381-5394. DOI: 10.1021/jm500481t*;* Baldwin et al., 2002, J Biol Chem, 277 (44), 41827-41834. DOI: 10.1074/jbc.M206854200).

Constructs used were as follows: N-terminal His₆-tagged *Pf*DHODH residues 158-569 (*Pf* start ¹⁵⁸FESYDP) in pRSETb; C-terminal His₆-tagged *Pv*DHODH residues 158-572 in pET-22b (N-terminal start ¹⁵⁸MYFESYDP); C-terminal His₆-tagged *Hs*DHODH residues 29-395 in pET-22b (start ²⁹MATGDE). Proteins were expressed and purified on a Pharmacia FPLC using HisTrap HP column chromatography followed by gel filtration chromatography on a Pharmacia HiLoad 16/600 Superdex 200 column, and protein concentration determined based on OD280 using the previously described extinction coefficients (Kokkonda et al., 2020, J Med Chem, 63 (9), 4929-4956. DOI: 10.1021/acs.jmedchem.0c00311*).* Purified proteins were >95% pure based on SDS-PAGE analysis. Purified protein was concentrated to 20 - 24 mg/ml in buffer (100 mM HEPES, pH 8.0, 300 mM NaCl, 15% Glycerol, 0.05% Triton X-100 reduced, 1 mM DTT), snap frozen in liquid nitrogen and stored in -80 freezer.

### Kinetic Analysis of DHODH inhibition. DHODH activity.

DHODH activity was monitored in a steady-state assay using the 2,6-dichloroindophenol (DCIP) method at 25°C in assay buffer (100 mM HEPES, pH 8.0, 150 mM NaCl, 1% glycerol, 0.1% Triton X-100 reduced) plus substrates 20 µM CoQD, 200 µM DHO, 120 µM DCIP, and 5 nM enzyme as previously described (Malmquist et al, 2008, Biochemistry, 47 (8), 2466-2475. DOI: 10.1021/bi702218c*).*

Compound stocks were prepared at 10-100 mM in DMSO, and diluted working stocks in DMSO were generated as appropriate. Compounds were plated from DMSO stocks in 96 well plate format using a Tecan D300e digital dispenser to generate a 3-fold dilution series over a concentration range of 0.0003 -100 µM (or 30 µM), yielding a final DMSO concentration of 1% in the assay. The top concentration used in the dose response was determined by the initial DMSO stock concentrations:100 µM for 100 mM stocks, and 30 µM for 10 mM stocks. Triplicate rate data were collected at each inhibitor concentration. Inhibitory IC₅₀ values were determined by fitting the data to log (inhibitor) vs response equation Y = bottom + (top - bottom)/(1 + 10A ((X - Log IC50))) in GraphPad Prism. DSM265 ² was plated as an assay control in all studies.

**Asexual blood-stage EC₅₀ assays**. *P. falciparum* growth inhibition assays were conducted in 384 well plate format using the SYBR green method (Smilkstein et al., 2004, Antimicrob Agents Chemother, 48 (5), 1803-1806. DOI: 10.1128/aac.48.5.1803-1806.2004*;* Singh et al., 2011, Exp Parasitol, 127 (1), 318-321. DOI: 10.1016/j.exppara.2010.08.007 From NLMMedline*).* Compound stocks prepared in DMSO were plated using a Tecan D300e automated low volume liquid handler. Ring-stage 3D7 or Dd2 parasites (60 µL cells) (2% hematocrit, 0.5% parasitemia, final DMSO concentration 0.5%) were added to plates, incubated with compounds over a range of concentrations in technical triplicate for 72 h at 37 °C, 5% CO₂, and then frozen (-80°C) overnight before analysis. Sigma-SYBR^{®} Green I nucleic acid gel stock (Sigma) diluted 1/5000 fold in buffer (final 20 mM Tris-HCl pH 7.5, 5 mM EDTA, 0.008% w/vol saponin, 0.2% vol/vl Triton X-100) was added to cells (30 uL Sybr green), mixed, and stored at RT for 4 h in the dark before reading on a BioTek Synergy H1 Hybrid plate reader to follow fluorescence (485 nm excitation, 535 or 528 nm emission). Data were analyzed by non-linear regression using GraphPad Prism to determine the EC₅₀ by fitting to the built-in equation (log(inhibitor) vs response-variable slope with four parameters).

**Cytotoxicity assays in human HepG2 cells.** Cytotoxicity assays were conducted versus HepG2 cells (ATCC HB-8065) as described (Lawong et al., 2021, J Med Chem, 64 (5), 2739-2761. DOI: 10.1021/acs.jmedchem.0c02022*;* Imlay et al., 2023, ACS Infect Dis, 9 (3), 527-539. DOI: 10.1021/acsinfecdis.2c00527 *From NLM Medline).*

Similar to parasite assays, compounds (30 nL) were dispensed into 384-well plates using a Labcyte-Echo 555 acoustic dispenser. HepG2 cells (60 µL, 600 cells per well) suspended in EMEM medium (Sigma) supplemented with 5% fetal bovine serum (heat inactivated, Gibco) and 2 mM glutamine (Gibco) were then added. CD437 (Sigma) and methotrexate were used as positive controls. Plates were incubated at 37 °C, 5% CO₂. After 72 hours, viability was assessed using the CellTiter Glo assay (Promega), which measures intracellular ATP.

Altogether, those data support that the compounds of the invention have anti-Plasmodium activity that is equivalent to < 50 nM while showing improved stability and species selectivity, namely equivalent activity on the two most important human Plasmodium species, *P. falciparum* and *P. vivax.*

## Claims

1. A compound according to Formula (I): 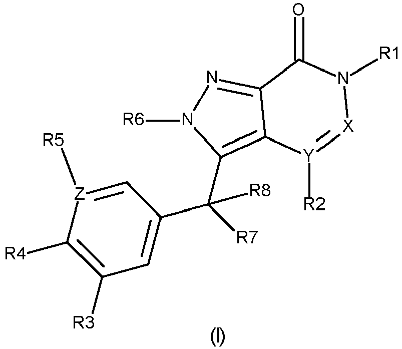 wherein **R1** is selected from an optionally substituted C₁-C₄ alkyl and optionally substituted C₃-C₆ cycloalkyl; **X** is selected from N or CR9; **Y-R2** is CR2 or N; **R2** is selected from H, halogen, an optionally substituted C₁-C₄ alkyl and optionally substituted C₃-C₆ cycloalkyl; **R3, R4** and **R5** are independently selected from Cl, F, CF₃, CN and H; **Z-R5** is selected from nitrogen and **CR5; R6** is selected from an optionally substituted C₁-C₄ alkyl and optionally substituted C₃-C₆ cycloalkyl; **R7** is selected from H, an optionally substituted C₁-C₄ alkyl and an optionally substituted C₃-C₆ cycloalkyl; **R8** is selected from OH and NH₂; **R9** is selected from H and optionally substituted C₁-C₄ alkyl; as well as pharmaceutically acceptable salts, tautomers, polymorphs, racemic mixtures, optically active forms thereof, wherein the term "substituted" refers to groups substituted with from 1 to 5 substituents selected from the group consisting of "C₁-C₆ alkyl," "C₃-C₈-cycloalkyl," "heterocycloalkyl," "C₁-C₆ alkyl C₃-C₈-cycloalkyl," "C₁-C₆ alkyl heterocycloalkyl," "amino," "ammonium," "aryl," "heteroaryl," "alkoxy," "halogen," trihalomethyl, cyano, and hydroxy.

2. A compound according to claim 1, wherein R1 is optionally substituted C₁-C₄ alkyl.

3. A compound according to claim 1, wherein R1 is optionally substituted C₃-C₆ cycloalkyl.

4. A compound according to any one of claims 1 to 3, wherein Y-R2 is N and X is CR9.

5. A compound according to any one of claims 1 to 3, wherein X is N and Y-R2 is CR2.

6. A compound according to any one of the preceding claims, wherein ZR5 is N.

7. A compound according to any one of claims 1 to 5, wherein ZR5 is CR5.

8. A compound according to any one of the preceding claims, wherein R4 is CF₃.

9. A compound according to any one of the preceding claims, wherein R5 is H.

10. A compound according to any one of the preceding claims, wherein R7 is an optionally substituted C₁-C₄ alkyl.

11. A compound according to any one of claims 1 to 9, wherein R7 is an optionally substituted C₃-C₆ cycloalkyl.

12. A compound according to any one of the preceding claims, wherein R8 is OH.

13. A compound according to any one of claims 1 to 11, wherein R8 is NH₂.

14. A compound according to any one of claims 1 to 13 selected from the following group:
(R)-3-(1-(5-chloro-6-(trifluoromethyl)pyridin-3-yl)-1-hydroxy-2-methylpropyl)-6-cyclopropyl-2-methyl-2,6-dihydro-7H-pyrazolo[3,4-c]pyridin-7-one;
(R)-5-(cyclopropyl(6-cyclopropyl-2-methyl-7-oxo-6,7-dihydro-2H-pyrazolo[3,4-c]pyridin-3-yl)(hydroxy)methyl)-2-(trifluoromethyl)benzonitrile;
(R)-5-(1-(6-cyclopropyl-2-methyl-7-oxo-6,7-dihydro-2H-pyrazolo[3,4-c]pyridin-3-yl)-1-hydroxypropyl)-2-(trifluoromethyl)benzonitrile;
3-(1-(5-chloro-6-(trifluoromethyl)pyridin-3-yl)-1-hydroxypropyl)-6-cyclopropyl-2,5-dimethyl-2,6-dihydro-7H-pyrazolo[3,4-c]pyridin-7-one;
(R)-3-(1-(3-chloro-4-fluorophenyl)-1-hydroxyethyl)-6-cyclopropyl-2-methyl-2,6-dihydro-7H-pyrazolo[3,4-c]pyridin-7-one;
(R)-3-(1-(5-chloro-6-(trifluoromethyl)pyridin-3-yl)-1-hydroxyethyl)-6-cyclopropyl-2-methyl-2,6-dihydro-7H-pyrazolo[3,4-c]pyridin-7-one;
(R)-3-(1-(3-chloro-4-fluorophenyl)-1-hydroxypropyl)-6-cyclopropyl-2-methyl-2,6-dihydro-7H-pyrazolo[3,4-c]pyridin-7-one;
(R)-3-(1-(5-chloro-6-(trifluoromethyl)pyridin-3-yl)-1-hydroxypropyl)-6-cyclopropyl-2-methyl-2,6-dihydro-7H-pyrazolo[3,4-c]pyridin-7-one;
(R)-3-((5-chloro-6-(trifluoromethyl)pyridin-3-yl)(cyclopropyl)(hydroxy)methyl)-6-cyclopropyl-2-methyl-2,6-dihydro-7H-pyrazolo[3,4-c]pyridin-7-one;
(R)-3-((3-chloro-4-fluorophenyl)(cyclopropyl)(hydroxy)methyl)-6-cyclopropyl-2-methyl-2,6-dihydro-7H-pyrazolo[3,4-c]pyridin-7-one;
(R)-3-(1-(4-chloro-3,5-difluorophenyl)-1-hydroxypropyl)-6-cyclopropyl-2-methyl-2,6-dihydro-7H-pyrazolo[3,4-c]pyridin-7-one;
(R)-6-cyclopropyl-3-(1-hydroxy-1-(6-(trifluoromethyl)pyridin-3-yl)propyl)-2-methyl-2,6-dihydro-7H-pyrazolo[3,4-c]pyridin-7-one;
(R)-6-ethyl-4-fluoro-3-(1-hydroxy-1-(6-(trifluoromethyl)pyridin-3-yl)propyl)-2-methyl-2,6-dihydro-7H-pyrazolo[3,4-c]pyridin-7-one;
(R)-6-cyclopropyl-3-(cyclopropyl(hydroxy)(6-(trifluoromethyl)pyridin-3-yl)methyl)-2-methyl-2,6-dihydro-7H-pyrazolo[3,4-c]pyridin-7-one;
(R)-3-((5-chloro-6-(trifluoromethyl)pyridin-3-yl)(hydroxy)methyl)-4-cyclopropyl-6-ethyl-2-methyl-2,6-dihydro-7H-pyrazolo[3,4-c]pyridin-7-one;
(R)-3-((4-chloro-3-fluorophenyl)(hydroxy)methyl)-6-ethyl-2,4-dimethyl-2,6-dihydro-7H-pyrazolo[3,4-c]pyridin-7-one;
(R)-3-((5-chloro-6-(trifluoromethyl)pyridin-3-yl)(cyclopropyl)(hydroxy)methyl)-6-ethyl-2-methyl-2,6-dihydro-7H-pyrazolo[3,4-c]pyridin-7-one;
(R)-3-(1-(3-chloro-4-fluorophenyl)-1-hydroxypropyl)-6-ethyl-2-methyl-2,6-dihydro-7H-pyrazolo[3,4-c]pyridin-7-one;
(R)-3-(1-(5-chloro-6-(trifluoromethyl)pyridin-3-yl)-1-hydroxyethyl)-6-ethyl-2-methyl-2,6-dihydro-7H-pyrazolo[3,4-c]pyridin-7-one;
(R)-3-((3-chloro-4-fluorophenyl)(cyclopropyl)(hydroxy)methyl)-6-ethyl-2-methyl-2,6-dihydro-7H-pyrazolo[3,4-c]pyridin-7-one;
(R)-3-(1-(3-chloro-4-fluorophenyl)-1-hydroxyethyl)-6-ethyl-2-methyl-2,6-dihydro-7H-pyrazolo[3,4-c]pyridin-7-one;
(R)-3-(1-(5-chloro-6-(trifluoromethyl)pyridin-3-yl)-1-hydroxypropyl)-6-ethyl-2-methyl-2,6-dihydro-7H-pyrazolo[3,4-c]pyridin-7-one;
(R)-3-(cyclopropyl(hydroxy)(6-(trifluoromethyl)pyridin-3-yl)methyl)-6-ethyl-2-methyl-2,6-dihydro-7H-pyrazolo[3,4-c]pyridin-7-one;
(R)-3-(1-(3-chloro-4-(trifluoromethyl)phenyl)-1-hydroxyethyl)-6-ethyl-2-methyl-2,6-dihydro-7H-pyrazolo[3,4-c]pyridin-7-one;
(R)-6-ethyl-3-(1-hydroxy-2-methyl-1-(6-(trifluoromethyl)pyridin-3-yl)propyl)-2-methyl-2,6-dihydro-7H-pyrazolo[3,4-c]pyridin-7-one;
(R)-6-ethyl-3-(1-hydroxy-1-(6-(trifluoromethyl)pyridin-3-yl)propyl)-2-methyl-2,6-dihydro-7H-pyrazolo[3,4-c]pyridin-7-one;
(R)-3-(1-(5-chloro-6-(trifluoromethyl)pyridin-3-yl)-1-hydroxypropyl)-2,6-dicyclopropyl-2,6-dihydro-7H-pyrazolo[3,4-d]pyridazin-7-one;
(R)-3-(1-(5-chloro-6-(trifluoromethyl)pyridin-3-yl)-1-hydroxypropyl)-6-cyclopropyl-2-ethyl-2,6-dihydro-7H-pyrazolo[3,4-d]pyridazin-7-one;
(R)-5-(1-(6-cyclopropyl-2-methyl-7-oxo-6,7-dihydro-2H-pyrazolo[3,4-d]pyridazin-3-yl)-1-hydroxypropyl)-2-(trifluoromethyl)benzonitrile;
(R)-5-(cyclopropyl(6-cyclopropyl-2-methyl-7-oxo-6,7-dihydro-2H-pyrazolo[3,4-d]pyridazin-3-yl)(hydroxy)methyl)-2-(trifluoromethyl)benzonitrile;
(R)-3-((5-chloro-6-(trifluoromethyl)pyridin-3-yl)(cyclopropyl)(hydroxy)methyl)-6-cyclopropyl-2-methyl-2,6-dihydro-7H-pyrazolo[3,4-d]pyridazin-7-one;
(R)-3-((3-chloro-4-(trifluoromethyl)phenyl)(cyclopropyl)(hydroxy)methyl)-6-cyclopropyl-2-methyl-2,6-dihydro-7H-pyrazolo[3,4-d]pyridazin-7-one;
(R)-3-(1-(5-chloro-6-(trifluoromethyl)pyridin-3-yl)-1-hydroxypropyl)-6-cyclopropyl-2-methyl-2,6-dihydro-7H-pyrazolo[3,4-d]pyridazin-7-one;
(R)-3-(1-(3-chloro-4-(trifluoromethyl)phenyl)-1-hydroxypropyl)-6-cyclopropyl-2-methyl-2,6-dihydro-7H-pyrazolo[3,4-d]pyridazin-7-one;
(R)-3-(1-(3-chloro-4-fluorophenyl)-1-hydroxypropyl)-6-cyclopropyl-2-methyl-2,6-dihydro-7H-pyrazolo[3,4-d]pyridazin-7-one;
(R)-3-(1-(3-chloro-4-(trifluoromethyl)phenyl)-1-hydroxypropyl)-6-ethyl-2-methyl-2,6-dihydro-7H-pyrazolo[3,4-d]pyridazin-7-one;
(R)-3-(1-(5-chloro-6-(trifluoromethyl)pyridin-3-yl)-1-hydroxyethyl)-6-cyclopropyl-2-methyl-2,6-dihydro-7H-pyrazolo[4,3-d]pyrimidin-7-one;
(R)-3-((5-chloro-6-(trifluoromethyl)pyridin-3-yl)(cyclopropyl)(hydroxy)methyl)-6-cyclopropyl-2-methyl-2,6-dihydro-7H-pyrazolo[4,3-d]pyrimidin-7-one;
(R)-3-(1-(5-chloro-6-(trifluoromethyl)pyridin-3-yl)-1-hydroxypropyl)-6-cyclopropyl-2-methyl-2,6-dihydro-7H-pyrazolo[4,3-d]pyrimidin-7-one;
(R)-6-cyclopropyl-3-(1-hydroxy-1-(6-(trifluoromethyl)pyridin-3-yl)propyl)-2-methyl-2,6-dihydro-7H-pyrazolo[4,3-d]pyrimidin-7-one;
(R)-6-cyclopropyl-3-(cyclopropyl(hydroxy)(6-(trifluoromethyl)pyridin-3-yl)methyl)-2-methyl-2,6-dihydro-7H-pyrazolo[4,3-d]pyrimidin-7-one;
(R)-5-(1-(6-ethyl-2-methyl-7-oxo-6,7-dihydro-2H-pyrazolo[4,3-d]pyrimidin-3-yl)-1-hydroxyethyl)-2-(trifluoromethyl)benzonitrile;
(R)-3-(1-amino-1-(5-chloro-6-(trifluoromethyl)pyridin-3-yl)-2,2,2-trifluoroethyl)-6-cyclopropyl-2-methyl-2,6-dihydro-7H-pyrazolo[3,4-c]pyridin-7-one;
(R)-3-(1-amino-1-(3-chloro-4-(trifluoromethyl)phenyl)ethyl)-6-cyclopropyl-2-methyl-2,6-dihydro-7H-pyrazolo[3,4-c]pyridin-7-one;
(R)-3-(1-amino-1-(3-fluoro-4-(trifluoromethyl)phenyl)propyl)-6-cyclopropyl-2-methyl-2,6-dihydro-7H-pyrazolo[3,4-c]pyridin-7-one;
(R)-3-(1-amino-1-(3-chloro-4-(trifluoromethyl)phenyl)propyl)-6-cyclopropyl-2-methyl-2,6-dihydro-7H-pyrazolo[3,4-c]pyridin-7-one;
(R)-3-(amino(5-chloro-6-(trifluoromethyl)pyridin-3-yl)(cyclopropyl)methyl)-6-cyclopropyl-2-methyl-2,6-dihydro-7H-pyrazolo[3,4-c]pyridin-7-one;
(R)-3-(1-amino-1-(5-chloro-6-(trifluoromethyl)pyridin-3-yl)propyl)-6-cyclopropyl-2-methyl-2,6-dihydro-7H-pyrazolo[3,4-c]pyridin-7-one;
(R)-5-(amino(6-ethyl-2,4-dimethyl-7-oxo-6,7-dihydro-2H-pyrazolo[3,4-c]pyridin-3-yl) methyl)-2-(trifluoromethyl)benzonitrile;
(R)-3-(amino(3-chloro-4-(trifluoromethyl)phenyl)(cyclopropyl)methyl)-6-cyclopropyl-2-methyl-2,6-dihydro-7H-pyrazolo[3,4-d]pyridazin-7-one;
(R)-3-(1-amino-1-(3-chloro-4-(trifluoromethyl)phenyl)propyl)-6-cyclopropyl-2-methyl-2,6-dihydro-7H-pyrazolo[3,4-d]pyridazin-7-one;
(R)-3-(1-amino-1-(3-chloro-4-(trifluoromethyl)phenyl)ethyl)-6-cyclopropyl-2-methyl-2,6-dihydro-7H-pyrazolo[4,3-d]pyrimidin-7-one;
(R)-3-(1-amino-1-(3-chloro-4-fluorophenyl)propyl)-6-cyclopropyl-2-methyl-2,6-dihydro-7H-pyrazolo[4,3-d]pyrimidin-7-one;
(R)-3-(amino(3-chloro-4-(trifluoromethyl)phenyl)(cyclopropyl)methyl)-6-cyclopropyl-2-methyl-2,6-dihydro-7H-pyrazolo[4,3-d]pyrimidin-7-one;
(R)-3-(1-amino-1-(3-chloro-4-(trifluoromethyl)phenyl)propyl)-6-cyclopropyl-2-methyl-2,6-dihydro-7H-pyrazolo[4,3-d]pyrimidin-7-one;
(R)-3-(1-amino-1-(5-chloro-6-(trifluoromethyl)pyridin-3-yl)propyl)-6-cyclopropyl-2-methyl-2,6-dihydro-7H-pyrazolo[4,3-d]pyrimidin-7-one;
(R)-3-(1-(5-chloro-6-(trifluoromethyl)pyridin-3-yl)-2,2,2-trifluoro-1-hydroxyethyl)-6-cyclopropyl-2-methyl-2,6-dihydro-7H-pyrazolo[3,4-d]pyridazin-7-one;
(R)-3-(1-(5-chloro-6-(trifluoromethyl)pyridin-3-yl)-2,2,2-trifluoro-1-hydroxyethyl)-6-cyclopropyl-2-methyl-2,6-dihydro-7H-pyrazolo[4,3-d]pyrimidin-7-one;
(R)-3-(1-(5-chloro-6-(trifluoromethyl)pyridin-3-yl)-2,2,2-trifluoro-1-hydroxyethyl)-6-cyclopropyl-2-methyl-2,6-dihydro-7H-pyrazolo[3,4-c]pyridin-7-one;
(R)-3-(1-(3-chloro-4-(trifluoromethyl)phenyl)-2,2,2-trifluoro-1-hydroxyethyl)-6-cyclopropyl-2-methyl-2,6-dihydro-7H-pyrazolo[3,4-c]pyridin-7-one;
(R)-3-(1-(3-chloro-4-fluorophenyl)-2,2,2-trifluoro-1-hydroxyethyl)-6-cyclopropyl-2-methyl-2,6-dihydro-7H-pyrazolo[3,4-c]pyridin-7-one;
(R)-6-cyclopropyl-2-methyl-3-(2,2,2-trifluoro-1-hydroxy-1-(6-(trifluoromethyl)pyridin-3-yl) ethyl)-2,6-dihydro-7H-pyrazolo[3,4-c]pyridin-7-one;
(R)-5-(1-(6-cyclopropyl-2-methyl-7-oxo-6,7-dihydro-2H-pyrazolo[3,4-d]pyridazin-3-yl)-2,2,2-trifluoro-1-hydroxyethyl)-2-(trifluoromethyl)benzonitrile;
(R)-3-(1-(3-chloro-4-(trifluoromethyl)phenyl)-2,2,2-trifluoro-1-hydroxyethyl)-6-cyclopropyl-2-methyl-2,6-dihydro-7H-pyrazolo[3,4-d]pyridazin-7-one;
(R)-3-(1-(3-chloro-4-(trifluoromethyl)phenyl)-2,2,2-trifluoro-1-hydroxyethyl)-6-cyclopropyl-2-methyl-2,6-dihydro-7H-pyrazolo[4,3-d]pyrimidin-7-one;
(R)-5-(1-(6-cyclopropyl-2-methyl-7-oxo-6,7-dihydro-2H-pyrazolo[4,3-d]pyrimidin-3-yl)-2,2,2-trifluoro-1-hydroxyethyl)-2-(trifluoromethyl)benzonitrile;
(R)-6-cyclopropyl-2-methyl-3-(2,2,2-trifluoro-1-hydroxy-1-(6-(trifluoromethyl)pyridin-3-yl)ethyl)-2,6-dihydro-7H-pyrazolo[4,3-d]pyrimidin-7-one;
(R)-3-(1-(5-chloro-6-(trifluoromethyl)pyridin-3-yl)-2,2-difluoro-1-hydroxyethyl)-6-cyclopropyl-2-methyl-2,6-dihydro-7H-pyrazolo[4,3-d]pyrimidin-7-one;
(R)-3-(1-(3-chloro-4-(trifluoromethyl)phenyl)-2,2,2-trifluoro-1-hydroxyethyl)-2-methyl-6-(2,2,2-trifluoroethyl)-2,6-dihydro-7H-pyrazolo[4,3-d]pyrimidin-7-one;
(R)-3-(1-(3-chloro-4-(trifluoromethyl)phenyl)-2,2,2-trifluoro-1-hydroxyethyl)-6-isopropyl-2-methyl-2,6-dihydro-7H-pyrazolo[4,3-d]pyrimidin-7-one;
(R)-5-(1-amino-1-(6-cyclopropyl-2-methyl-7-oxo-6,7-dihydro-2H-pyrazolo[4,3-d]pyrimidin-3-yl)-2,2,2-trifluoroethyl)-2-(trifluoromethyl)benzonitrile;
(R)-5-(1-amino-1-(6-cyclopropyl-2-methyl-7-oxo-6,7-dihydro-2H-pyrazolo[3,4-c]pyridin-3-yl)-2,2,2-trifluoroethyl)-2-(trifluoromethyl)benzonitrile;
(R)-3-(1-amino-1-(3-chloro-4-(trifluoromethyl)phenyl)-2,2,2-trifluoroethyl)-6-cyclopropyl-2-methyl-2,6-dihydro-7H-pyrazolo[3,4-c]pyridin-7-one;
(R)-3-(1-amino-1-(3-chloro-4-(trifluoromethyl)phenyl)-2,2,2-trifluoroethyl)-6-cyclopropyl-2-methyl-2,6-dihydro-7H-pyrazolo[4,3-d]pyrimidin-7-one;
(R)-5-(1-(6-cyclopropyl-2-methyl-7-oxo-6,7-dihydro-2H-pyrazolo[4,3-d]pyrimidin-3-yl)-2,2,2-trifluoro-1-hydroxyethyl)-2-(trifluoromethyl)nicotinonitrile;
(R)-5-(1-(6-cyclopropyl-2-methyl-7-oxo-6,7-dihydro-2H-pyrazolo[4,3-d]pyrimidin-3-yl)-2,2,2-trifluoro-1-hydroxyethyl)-2-(difluoromethyl)benzonitrile, and
(R)-5-(1-(6-cyclopropyl-2-methyl-7-oxo-6,7-dihydro-2H-pyrazolo[4,3-d]pyrimidin-3-yl)-2,2,2-trifluoro-1-hydroxyethyl)-2-(trifluoromethyl)benzonitrile
as well as pharmaceutically acceptable salts, hydrates, solvates, tautomers, polymorphs, racemic mixtures, optically active forms thereof.

15. A compound according to any one of claims 1 to 14 for use as a medicament.

16. A compound according to any one of claims 1 to 14 for use as in the prevention or treatment of malaria.

17. A pharmaceutical formulation containing at least one compound according to any one of claims 1 to 14 and a pharmaceutically acceptable carrier, diluent or excipient thereof.

18. A pharmaceutical formulation according to claim 17, further comprising at least one co-agent useful in the treatment and/or prevention of malaria selected from artemisinin and its derivatives such as artemether, artesunate, dihydroartemisinin, chloroquine, hydroxychloroquine, quinine, mefloquine, amodiaquine, atovaquone/proguanil, doxycycline, clindamycin, halofantrine, lumefantrine, pyronaridine, pyrimethamine-sulfadoxine, ferroquine, tafenoquine, piperaquine and primaquine.

19. A pharmaceutical formulation according to claim 17 or 18, further comprising at least one co-agent useful in the treatment and/or prevention of malaria selected from Spiro[3H-indole-3,1'-[1H]pyrido[3,4-b]indol]-2(1H)-one, 5,7'-dichloro-6'-fluoro-2',3',4',9'-tetrahydro-3'-methyl-,(1'R,3'S)- (CAS Registry Number: 1193314-23-6), 2-(1,1-difluoroethyl)-5-methyl-N-[4-(pentafluoro-λ⁶-sulfanyl)phenyl]-[1,2,4]triazolo[1,5-a] pyrimidin-7-amine (CAS Registry Number: 1282041-94-4), [3,3'-Bipyridin]-2-amine, 5-[4-(methylsulfonyl)phenyl]-6'-(trifluoromethyl)- (CAS Registry Number: 1314883-11-8), Ethanone, 2-amino-1-[2-(4-fluorophenyl)-3-[(4-fluorophenyl)amino]-5,6-dihydroimidazo [1,2-a]pyrazin-7(8H)-yl]- (CAS Registry Number 1261109-90-3).

20. A method for the preparation of a compound according Formula (I) or a pharmaceutically acceptable salt thereof, said method comprising: 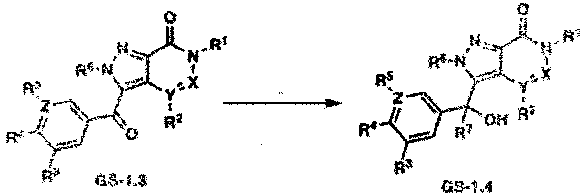 transforming a compound according to Formula GS-1.3 into a compound of G-S1.4 as described under Scheme 6 in presence of an alkyl Grignard or alkyl lithium, wherein R1, R2, R3, R4, R5, R6, R7, X, Y and Z are as defined in claim 1.

21. A method for the preparation of a compound according to Formula (I) comprising a step of transforming a compound according to Formula GS-1.3 into a compound of GS-1.9 after acidic hydrolyzation in presence of trifluoromethyltrimethylsilane: 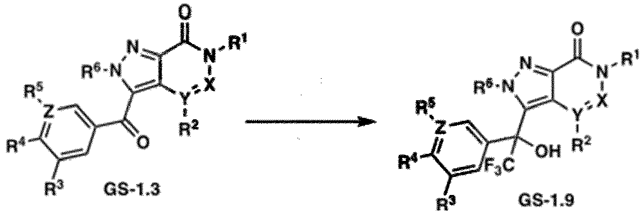 wherein R1, R2, R3, R4, R5, R6, R7, X, Y and Z are as defined in claim 1.

22. A method for the preparation of a compound according to Formula (I) comprising a step of transforming a compound according to Formula GS-1.13 into a compound of GS-1.14 after acidic hydrolyzation: 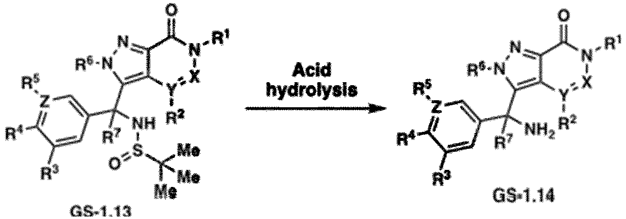 wherein R1, R2, R3, R4, R5, R6, R7, X, Y and Z are as defined in claim 1.

23. A method for the preparation of a compound according to Formula (I) comprising a step of transforming a compound according to Formula GS-1.17 into a compound of GS-1.18 after acidic hydrolyzation: 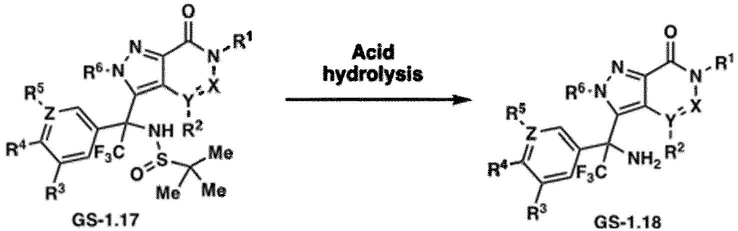 wherein R1, R2, R3, R4, R5, R6, R7, X, Y and Z are as defined in claim 1.

24. An intermediate of a formula selected from Formulae GS-1.3, andGS-1.17. 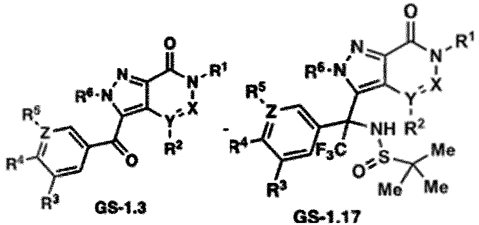 wherein R1, R2, R3, R4, R5, R6, X, Y and Z are as defined in claim 1.

## Patentansprüche

1. Verbindung nach der Formel (I): 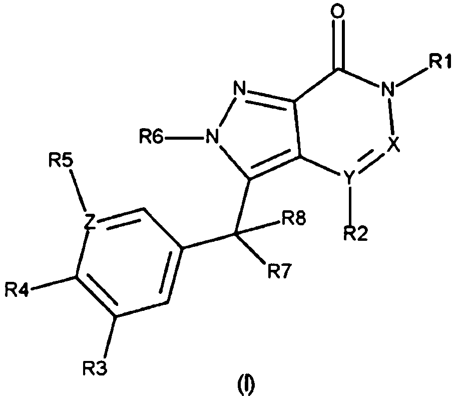 wobei **R1** ausgewählt ist aus einem wahlweise substituierten C₁-C₄-Alkyl und wahlweise substituiertem C₃-C₆-Cycloalkyl; **X** ausgewählt ist aus N oder CR9; **Y-R2** CR2 oder N ist; **R2** ausgewählt ist aus H, Halogen, einem wahlweise substituierten C₁-C₄-Alkyl und wahlweise substituiertem C₃-C₆-Cycloalkyl; **R3, R4** und **R5** unabhängig ausgewählt sind aus Cl, F, CF₃, CN und H; **Z-R5** ausgewählt ist aus Stickstoff und **CR5; R6** ausgewählt ist aus einem wahlweise substituierten C₁-C₄-Alkyl und wahlweise substituiertem C₃-C₆-Cycloalkyl; **R7** ausgewählt ist aus H, einem wahlweise substituierten C₁-C₄-Alkyl und einem wahlweise substituiertem C₃-C₆-Cycloalkyl; **R8** ausgewählt ist aus OH und NH₂; **R9** ausgewählt ist aus H und wahlweise substituiertem C₁-C₄-Alkyl; sowie pharmazeutisch annehmbaren Salzen, Tautomeren, Polymorphen, racemischen Gemischen, wahlweise aktiven Formen davon, wobei der Begriff "substituiert" Gruppen bezeichnet, die mit von 1 und 5 Substituenten substituiert sind, die ausgewählt sind aus der Gruppe, die besteht aus "C₁-C₆-Alkyl", "C₃-C₈-Cycloalkyl", "Heterocycloalkyl", "C₁-C₆-Alkyl-C₃-C₈-Cycloalkyl", "C₁-C₆-Alkyl-Heterocycloalkyl", "Amino", "Ammonium", "Aryl", "Heteroaryl", "Alkoxy", "Halogen", Trihalomethyl, Cyano und Hydroxy.

2. Verbindung nach Anspruch 1, wobei R1 wahlfrei substituiertes C₁-C₄-Alkyl ist.

3. Verbindung nach Anspruch 1, wobei R1 wahlfrei substituiertes C₃-C₆-Cycloalkyl ist.

4. Verbindung nach einem der Ansprüche 1 bis 3, wobei Y-R2 N ist und X CR9 ist.

5. Verbindung nach einem der Ansprüche 1 bis 3, wobei X N ist und Y-R2 CR2 ist.

6. Verbindung nach einem der vorhergehenden Ansprüche, wobei ZR5 N ist.

7. Verbindung nach einem der Ansprüche 1 bis 5, wobei ZR5 CR5 ist.

8. Verbindung nach einem der vorhergehenden Ansprüche, wobei R4 CF₃ ist.

9. Verbindung nach einem der vorhergehenden Ansprüche, wobei R5 H ist.

10. Verbindung nach einem der vorhergehenden Ansprüche, wobei R7 ein wahlweise substituiertes C₁-C₄-Alkyl ist.

11. Verbindung nach einem der Ansprüche 1 bis 9, wobei R7 ein wahlweise substituiertes C₃-C₆-Cycloalkyl ist.

12. Verbindung nach einem der vorhergehenden Ansprüche, wobei R8 OH ist.

13. Verbindung nach einem der Ansprüche 1 bis 11, wobei R8 NH₂ ist.

14. Verbindung nach einem der Ansprüche 1 bis 13, die ausgewählt ist aus der folgenden Gruppe:
(R)-3-(1-(5-Chlor-6-(trifluormethyl)pyridin-3-yl)-1-hydroxy-2-methylpropyl)-6-cyclopropyl-2-methyl-2,6-dihydro-7H-pyrazolo[3,4-c]pyridin-7-on;
(R)-5-(Cyclopropyl(6-cyclopropyl-2-methyl-7-oxo-6,7-dihydro-2H-pyrazolo[3,4-c]pyridin-3-yl) (hydroxy)methyl)-2-(trifluormethyl)benzonitril;
(R)-5-(1-(6-Cyclopropyl-2-methyl-7-oxo-6,7-dihydro-2H-pyrazolo[3,4-c]pyridin-3-yl)-1-hydroxypropyl)-2-(trifluormethyl)benzonitril;
3-(1-(5-Chlor-6-(trifluormethyl)pyridin-3-yl)-1-hydroxypropyl)-6-cyclopropyl-2,5-dimethyl-2,6-dihydro-7H-pyrazolo[3,4-c]pyridin-7-on;
(R)-3-(1-(3-Chlor-4-fluorophenyl)-1-hydroxyethyl)-6-cyclopropyl-2-methyl-2,6-dihydro-7H-pyrazolo[3,4-c]pyridin-7-on;
(R)-3-(1-(5-Chlor-6-(trifluormethyl)pyridin-3-yl)-1-hydroxyethyl)-6-cyclopropyl-2-methyl-2,6-dihydro-7H-pyrazolo[3,4-c]pyridin-7-on;
(R)-3-(1-(3-Chlor-4-fluorphenyl)-1-hydroxypropyl)-6-cyclopropyl-2-methyl-2,6-dihydro-7H-pyrazolo[3,4-c]pyridin-7-on;
(R)-3-(1-(5-Chlor-6-(trifluormethyl)pyridin-3-yl)-1-hydroxypropyl)-6-cyclopropyl-2-methyl-2,6-dihydro-7H-pyrazolo[3,4-c]pyridin-7-on;
(R)-3-((5-Chlor-6-(trifluormethyl)pyridin-3-yl)(cyclopropyl) (hydroxy)methyl)-6-cyclopropyl-2-methyl-2,6-dihydro-7H-pyrazolo[3,4-c]pyridin-7-on;
(R)-3-((3-Chlor-4-fluorphenyl) (cyclopropyl) (hydroxy)methyl)-6-cyclopropyl-2-methyl-2,6-dihydro-7H-pyrazolo[3,4-c]pyridin-7-on;
(R)-3-(1-(4-Chlor-3,5-difluorphenyl)-1-hydroxypropyl)-6-cyclopropyl-2-methyl-2,6-dihydro-7H-pyrazolo[3,4-c]pyridin-7-on;
(R)-6-Cyclopropyl-3-(1-hydroxy-1-(6-(trifluormethyl)pyridin-3-yl)propyl)-2-methyl-2,6-dihydro-7H-pyrazolo[3,4-c]pyridin-7-on;
(R)-6-Ethyl-4-fluor-3-(1-hydroxy-1-(6-(trifluormethyl)pyridin-3-yl)propyl)-2-methyl-2,6-dihydro-7H-pyrazolo[3,4-c]pyridin-7-on;
(R)-6-Cyclopropyl-3-(cyclopropyl(hydroxy)(6-(trifluormethyl)pyridin-3-yl)methyl)-2-methyl-2,6-dihydro-7H-pyrazolo[3,4-c]pyridin-7-on;
(R)-3-((5-Chlor-6-(trifluormethyl)pyridin-3-yl)(hydroxy)methyl)-4-cyclopropyl-6-ethyl-2-methyl-2,6-dihydro-7H-pyrazolo[3,4-c]pyridin-7-on;
(R)-3-((4-Chlor-3-fluorphenyl)(hydroxy)methyl)-6-ethyl-2,4-dimethyl-2,6-dihydro-7H-pyrazolo[3,4-c]pyridin-7-on;
(R)-3-((5-Chlor-6-(trifluormethyl)pyridin-3-yl)(cyclopropyl) (hydroxy)methyl)-6-ethyl-2-methyl-2,6-dihydro-7H-pyrazolo[3,4-c]pyridin-7-on;
(R)-3-(1-(3-Chlor-4-fluorphenyl)-1-hydroxypropyl)-6-ethyl-2-methyl-2,6-dihydro-7H-pyrazolo[3,4-c]pyridin-7-on;
(R)-3-(1-(5-Chlor-6-(trifluormethyl)pyridin-3-yl)-1-hydroxyethyl)-6-ethyl-2-methyl-2,6-dihydro-7H-pyrazolo[3,4-c]pyridin-7-on;
(R)-3-((3-Chlor-4-fluorphenyl) (cyclopropyl) (hydroxy)methyl)-6-ethyl-2-methyl-2,6-dihydro-7H-pyrazolo[3,4-c]pyridin-7-on;
(R)-3-(1-(3-Chlor-4-fluorphenyl)-1-hydroxyethyl)-6-ethyl-2-methyl-2,6-dihydro-7H-pyrazolo[3,4-c]pyridin-7-on;
(R)-3-(1-(5-Chlor-6-(trifluormethyl)pyridin-3-yl)-1-hydroxypropyl)-6-ethyl-2-methyl-2,6-dihydro-7H-pyrazolo[3,4-c]pyridin-7-on;
(R)-3-(Cyclopropyl(hydroxy) (6-(trifluormethyl)pyridin-3-yl)methyl)-6-ethyl-2-methyl-2,6-dihydro-7H-pyrazolo[3,4-c]pyridin-7-on;
(R)-3-(1-(3-Chlor-4-(trifluormethyl)phenyl)-1-hydroxyethyl)-6-ethyl-2-methyl-2,6-dihydro-7H-pyrazolo[3,4-c]pyridin-7-on;
(R)-6-Ethyl-3-(1-hydroxy-2-methyl-1-(6-(trifluormethyl)pyridin-3-yl)propyl)-2-methyl-2,6-dihydro-7H-pyrazolo[3,4-c]pyridin-7-on;
(R)-6-Ethyl-3-(1-hydroxy-1-(6-(trifluormethyl)pyridin-3-yl)propyl)-2-methyl-2,6-dihydro-7H-pyrazolo[3,4-c]pyridin-7-on;
(R)-3-(1-(5-Chlor-6-(trifluormethyl)pyridin-3-yl)-1-hydroxypropyl)-2,6-dicyclopropyl-2,6-dihydro-7H-pyrazolo[3,4-d]pyridazin-7-on;
(R)-3-(1-(5-Chlor-6-(trifluormethyl)pyridin-3-yl)-1-hydroxypropyl)-6-cyclopropyl-2-ethyl-2,6-dihydro-7H-pyrazolo[3,4-d]pyridazin-7-on;
(R)-5-(1-(6-Cyclopropyl-2-methyl-7-oxo-6,7-dihydro-2H-pyrazolo[3,4-d]pyridazin-3-yl)-1-hydroxypropyl)-2-(trifluormethyl)benzonitril;
(R)-5-(Cyclopropyl(6-cyclopropyl-2-methyl-7-oxo-6,7-dihydro-2H-pyrazolo[3,4-d]pyridazin-3-yl)(hydroxy)methyl)-2-(trifluormethyl)benzonitril;
(R)-3-((5-Chlor-6-(trifluormethyl)pyridin-3-yl)(cyclopropyl) (hydroxy)methyl)-6-cyclopropyl-2-methyl-2,6-dihydro-7H-pyrazolo[3,4-d]pyridazin-7-on;
(R)-3-((3-Chlor-4-(trifluormethyl)phenyl)(cyclopropyl)(hydroxy)methyl)-6-cyclopropyl-2-methyl-2,6-dihydro-7H-pyrazolo[3,4-d]pyridazin-7-on;
(R)-3-(1-(5-Chlor-6-(trifluormethyl)pyridin-3-yl)-1-hydroxypropyl)-6-cyclopropyl-2-methyl-2,6-dihydro-7H-pyrazolo[3,4-d]pyridazin-7-on;
(R)-3-(1-(3-Chlor-4-(trifluormethyl)phenyl)-1-hydroxypropyl)-6-cyclopropyl-2-methyl-2,6-dihydro-7H-pyrazolo[3,4-d]pyridazin-7-on;
(R)-3-(1-(3-Chlor-4-fluorphenyl)-1-hydroxypropyl)-6-cyclopropyl-2-methyl-2,6-dihydro-7H-pyrazolo[3,4-d]pyridazin-7-on;
(R)-3-(1-(3-Chlor-4-(trifluormethyl)phenyl)-1-hydroxypropyl)-6-ethyl-2-methyl-2,6-dihydro-7H-pyrazolo[3,4-d]pyridazin-7-on;
(R)-3-(1-(5-Chlor-6-(trifluormethyl)pyridin-3-yl)-1-hydroxyethyl)-6-cyclopropyl-2-methyl-2,6-dihydro-7H-pyrazolo[4,3-d]pyrimidin-7-on;
(R)-3-((5-Chlor-6-(trifluormethyl)pyridin-3-yl)(cyclopropyl) (hydroxy)methyl)-6-cyclopropyl-2-methyl-2,6-dihydro-7H-pyrazolo[4,3-d]pyrimidin-7-on;
(R)-3-(1-(5-Chlor-6-(trifluormethyl)pyridin-3-yl)-1-hydroxypropyl)-6-cyclopropyl-2-methyl-2,6-dihydro-7H-pyrazolo[4,3-d]pyrimidin-7-on;
(R)-6-Cyclopropyl-3-(1-hydroxy-1-(6-(trifluormethyl)pyridin-3-yl)propyl)-2-methyl-2,6-dihydro-7H-pyrazolo[4,3-d]pyrimidin-7-on;
(R)-6-Cyclopropyl-3-(cyclopropyl(hydroxy)(6-(trifluormethyl)pyridin-3-yl)methyl)-2-methyl-2,6-dihydro-7H-pyrazolo[4,3-d]pyrimidin-7-on;
(R)-5-(1-(6-Ethyl-2-methyl-7-oxo-6,7-dihydro-2H-pyrazolo[4,3-d]pyrimidin-3-yl)-1-hydroxyethyl)-2-(trifluormethyl)benzonitril;
(R)-3-(1-Amino-1-(5-chlor-6-(trifluormethyl)pyridin-3-yl)-2,2,2-trifluorethyl)-6-cyclopropyl-2-methyl-2,6-dihydro-7H-pyrazolo[3,4-c]pyridin-7-on;
(R)-3-(1-Amino-1-(3-chlor-4-(trifluormethyl)phenyl)ethyl)-6-cyclopropyl-2-methyl-2,6-dihydro-7H-pyrazolo[3,4-c]pyridin-7-on;
(R)-3-(1-Amino-1-(3-fluor-4-(trifluormethyl)phenyl)propyl)-6-cyclopropyl-2-methyl-2,6-dihydro-7H-pyrazolo[3,4-c]pyridin-7-on;
(R)-3-(1-Amino-1-(3-chlor-4-(trifluormethyl)phenyl)propyl)-6-cyclopropyl-2-methyl-2,6-dihydro-7H-pyrazolo[3,4-c]pyridin-7-on;
(R)-3-(Amino(5-chlor-6-(trifluormethyl)pyridin-3-yl)(cyclopropyl)methyl)-6-cyclopropyl-2-methyl-2,6-dihydro-7H-pyrazolo[3,4-c]pyridin-7-on;
(R)-3-(1-Amino-1-(5-chlor-6-(trifluormethyl)pyridin-3-yl)propyl)-6-cyclopropyl-2-methyl-2,6-dihydro-7H-pyrazolo[3,4-c]pyridin-7-on;
(R)-5-(Amino(6-ethyl-2,4-dimethyl-7-oxo-6,7-dihydro-2H-pyrazolo[3,4-c]pyridin-3-yl)methyl)-2-(trifluormethyl)benzonitril;
(R)-3-(Amino(3-chlor-4-(trifluormethyl)phenyl)(cyclopropyl)methyl)-6-cyclopropyl-2-methyl-2,6-dihydro-7H-pyrazolo[3,4-d]pyridazin-7-on;
(R)-3-(1-Amino-1-(3-chlor-4-(trifluormethyl)phenyl)propyl)-6-cyclopropyl-2-methyl-2,6-dihydro-7H-pyrazolo[3,4-d]pyridazin-7-on;
(R)-3-(1-Amino-1-(3-chlor-4-(trifluormethyl)phenyl)ethyl)-6-cyclopropyl-2-methyl-2,6-dihydro-7H-pyrazolo[4,3-d]pyrimidin-7-on;
(R)-3-(1-Amino-1-(3-chlor-4-fluorphenyl)propyl)-6-cyclopropyl-2-methyl-2,6-dihydro-7H-pyrazolo[4,3-d]pyrimidin-7-on;
(R)-3-(Amino(3-chlor-4-(trifluormethyl)phenyl)(cyclopropyl)methyl)-6-cyclopropyl-2-methyl-2,6-dihydro-7H-pyrazolo[4,3-d]pyrimidin-7-on;
(R)-3-(1-Amino-1-(3-chlor-4-(trifluormethyl)phenyl)propyl)-6-cyclopropyl-2-methyl-2,6-dihydro-7H-pyrazolo[4,3-d]pyrimidin-7-on;
(R)-3-(1-Amino-1-(5-chlor-6-(trifluormethyl)pyridin-3-yl)propyl)-6-cyclopropyl-2-methyl-2,6-dihydro-7H-pyrazolo[4,3-d]pyrimidin-7-on;
(R)-3-(1-(5-Chlor-6-(trifluormethyl)pyridin-3-yl)-2,2,2-trifluor-1-hydroxyethyl)-6-cyclopropyl-2-methyl-2,6-dihydro-7H-pyrazolo[3,4-d]pyridazin-7-on;
(R)-3-(1-(5-Chlor-6-(trifluormethyl)pyridin-3-yl)-2,2,2-trifluor-1-hydroxyethyl)-6-cyclopropyl-2-methyl-2,6-dihydro-7H-pyrazolo[4,3-d]pyrimidin-7-on;
(R)-3-(1-(5-Chlor-6-(trifluormethyl)pyridin-3-yl)-2,2,2-trifluor-1-hydroxyethyl)-6-cyclopropyl-2-methyl-2,6-dihydro-7H-pyrazolo[3,4-c]pyridin-7-on;
(R)-3-(1-(3-Chlor-4-(trifluormethyl)phenyl)-2,2,2-trifluor-1-hydroxyethyl)-6-cyclopropyl-2-methyl-2,6-dihydro-7H-pyrazolo[3,4-c]pyridin-7-on;
(R)-3-(1-(3-Chlor-4-fluorphenyl)-2,2,2-trifluor-1-hydroxyethyl)-6-cyclopropyl-2-methyl-2,6-dihydro-7H-pyrazolo[3,4-c]pyridin-7-on;
(R)-6-Cyclopropyl-2-methyl-3-(2,2,2-trifluor-1-hydroxy-1-(6-(trifluormethyl)pyridin-3-yl)ethyl)-2,6-dihydro-7H-pyrazolo[3,4-c]pyridin-7-on;
(R)-5-(1-(6-Cyclopropyl-2-methyl-7-oxo-6,7-dihydro-2H-pyrazolo[3,4-d]pyridazin-3-yl)-2,2,2-trifluor-1-hydroxyethyl)-2-(trifluormethyl)benzonitril;
(R)-3-(1-(3-Chlor-4-(trifluormethyl)phenyl)-2,2,2-trifluor-1-hydroxyethyl)-6-cyclopropyl-2-methyl-2,6-dihydro-7H-pyrazolo[3,4-d]pyridazin-7-on;
(R)-3-(1-(3-Chlor-4-(trifluormethyl)phenyl)-2,2,2-trifluor-1-hydroxyethyl)-6-cyclopropyl-2-methyl-2,6-dihydro-7H-pyrazolo[4,3-d]pyrimidin-7-on;
(R)-5-(1-(6-Cyclopropyl-2-methyl-7-oxo-6,7-dihydro-2H-pyrazolo[4,3-d]pyrimidin-3-yl)-2,2,2-trifluor-1-hydroxyethyl)-2-(trifluormethyl)benzonitril;
(R)-6-Cyclopropyl-2-methyl-3-(2,2,2-trifluor-1-hydroxy-1-(6-(trifluormethyl)pyridin-3-yl)ethyl)-2,6-dihydro-7H-pyrazolo[4,3-d]pyrimidin-7-on;
(R)-3-(1-(5-Chlor-6-(trifluormethyl)pyridin-3-yl)-2,2-difluor-1-hydroxyethyl)-6-cyclopropyl-2-methyl-2,6-dihydro-7H-pyrazolo[4,3-d]pyrimidin-7-on;
(R)-3-(1-(3-Chlor-4-(trifluormethyl)phenyl)-2,2,2-trifluor-1-hydroxyethyl)-2-methyl-6-(2,2,2-trifluorethyl)-2,6-dihydro-7H-pyrazolo[4,3-d]pyrimidin-7-on;
(R)-3-(1-(3-Chlor-4-(trifluormethyl)phenyl)-2,2,2-trifluor-1-hydroxyethyl)-6-isopropyl-2-methyl-2,6-dihydro-7H-pyrazolo[4,3-d]pyrimidin-7-on;
(R)-5-(1-Amino-1-(6-cyclopropyl-2-methyl-7-oxo-6,7-dihydro-2H-pyrazolo[4,3-d]pyrimidin-3-yl)-2,2,2-trifluorethyl)-2-(trifluormethyl)benzonitril;
(R)-5-(1-Amino-1-(6-cyclopropyl-2-methyl-7-oxo-6,7-dihydro-2H-pyrazolo[3,4-c]pyridin-3-yl)-2,2,2-trifluorethyl)-2-(trifluormethyl)benzonitril;
(R)-3-(1-Amino-1-(3-chlor-4-(trifluormethyl)phenyl)-2,2,2-trifluorethyl)-6-cyclopropyl-2-methyl-2,6-dihydro-7H-pyrazolo[3,4-c]pyridin-7-on;
(R)-3-(1-Amino-1-(3-chlor-4-(trifluormethyl)phenyl)-2,2,2-trifluorethyl)-6-cyclopropyl-2-methyl-2,6-dihydro-7H-pyrazolo[4,3-d]pyrimidin-7-on;
(R)-5-(1-(6-Cyclopropyl-2-methyl-7-oxo-6,7-dihydro-2H-pyrazolo[4,3-d]pyrimidin-3-yl)-2,2,2-trifluor-1-hydroxyethyl)-2-(trifluormethyl)nicotinonitril;
(R)-5-(1-(6-Cyclopropyl-2-methyl-7-oxo-6,7-dihydro-2H-pyrazolo[4,3-d]pyrimidin-3-yl)-2,2,2-trifluor-1-hydroxyethyl)-2-(difluormethyl)benzonitril, und
(R)-5-(1-(6-Cyclopropyl-2-methyl-7-oxo-6,7-dihydro-2H-pyrazolo[4,3-d]pyrimidin-3-yl)-2,2,2-trifluor-1-hydroxyethyl)-2-(trifluormethyl)benzonitril,
sowie pharmazeutisch annehmbaren Salzen, Hydraten, Solvaten, Tautomeren, Polymorphen, racemischen Gemischen, wahlweise aktiven Formen davon.

15. Verbindung nach einem der Ansprüche 1 bis 14 zur Verwendung als ein Arzneimittel.

16. Verbindung nach einem der Ansprüche 1 bis 14 zur Verwendung bei der Prävention oder Behandlung von Malaria.

17. Pharmazeutische Formulierung, die mindestens eine Verbindung nach einem der Ansprüche 1 bis 14 und ein/en pharmazeutisch annehmbaren/annehmbares Träger, Verdünnungsmittel oder Hilfsstoff davon umfasst.

18. Pharmazeutische Formulierung nach Anspruch 17, ferner umfassend mindestens ein Co-Agens, das bei der Behandlung und/oder Prävention von Malaria nützlich ist und ausgewählt ist aus Artemisinin und seinen Derivaten, wie Artemether, Artesunat, Dihydroartemisinin, Chloroquin, Hydroxychloroquin, Chinin, Mefloquin, Amodiaquin, Atovaquon/Proguanil, Doxycyclin, Clindamycin, Halofantrin, Lumefantrin, Pyronaridin, Pyrimethamin-Sulfadoxin, Ferroquin, Tafenoquin, Piperaquin und Primaquin.

19. Pharmazeutische Zusammensetzung nach Anspruch 17 oder 18, ferner umfassend mindestens ein Co-Agens, das bei der Behandlung und/oder Prävention von Malaria nützlich ist und ausgewählt ist aus Spiro[3H-indol-3,1'-[1H]pyrido[3,4-b]indol]-2(1H)-on, 5,7'-Dichlor-6'-fluor-2',3',4',9'-tetrahydro-3'-methyl-, (1'R,3'S)- (CAS-Registrierungsnummer: 1193314-23-6), 2-(1,1-Difluorethyl)-5-methyl-N-[4-(pentafluor-λ⁶-sulfanyl)phenyl]-[1,2,4]triazolo[1,5-a]pyrimidin-7-amin (CAS-Registrierungsnummer: 1282041-94-4), [3,3'-Bipyridin]-2-amin, 5-[4-(Methylsulfonyl)phenyl]-6'-(trifluormethyl)- (CAS-Registrierungsnummer: 1314883-11-8), Ethanon, 2-Amino-1-[2-(4-fluorphenyl)-3-[(4-fluorphenyl)amino]-5,6-dihydroimidazo[1,2-a]pyrazin-7(8H)-yl (CAS-Registrierungsnummer 1261109-90-3).

20. Verfahren zur Herstellung einer Verbindung nach der Formel (I) oder eines pharmazeutisch annehmbaren Salzes davon, wobei das Verfahren umfasst: 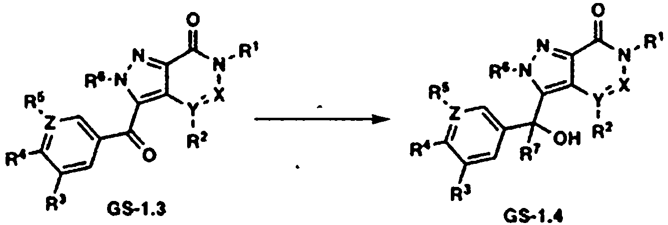 Umwandeln einer Verbindung nach der Formel GS-1.3 in eine Verbindung von G-S1.4 wie unter Schema 6 beschrieben bei Vorhandensein von Alkyl-Grignard oder Alkyllithium, wobei R1, R2, R3, R4, R5, R6, R7, X, Y und Z sind wie in Anspruch 1 definiert.

21. Verfahren zur Herstellung einer Verbindung nach der Formel (I), das einen Schritt des Umwandelns einer Verbindung nach der Formel GS-1.3 in eine Verbindung von GS-1.9 nach saurer Hydrolyse bei Vorhandensein von Trifluormethyltrimethylsilan umfasst: 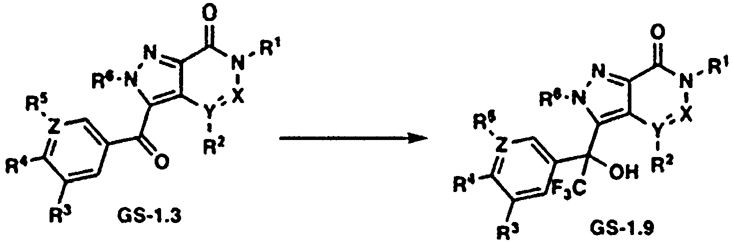 wobei R1, R2, R3, R4, R5, R6, R7, X, Y und Z sind wie in Anspruch 1 definiert.

22. Verfahren zur Herstellung einer Verbindung nach der Formel (I), das einen Schritt des Umwandelns einer Verbindung nach der Formel GS-1.13 in eine Verbindung von GS-1.14 nach saurer Hydrolyse umfasst: 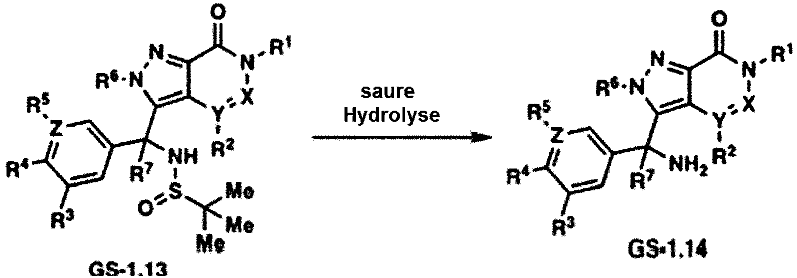 wobei R1, R2, R3, R4, R5, R6, R7, X, Y und Z sind wie in Anspruch 1 definiert.

23. Verfahren zur Herstellung einer Verbindung nach der Formel (I), das einen Schritt des Umwandelns einer Verbindung nach der Formel GS-1.17 in eine Verbindung von GS-1.18 nach saurer Hydrolyse umfasst: 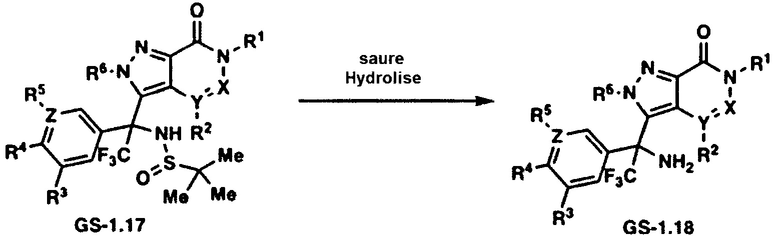 wobei R1, R2, R3, R4, R5, R6, R7, X, Y und Z sind wie in Anspruch 1 definiert.

24. Zwischenprodukt mit einer Formel, die ausgewählt ist aus den Formeln GS-1.3 und GS-1.17. 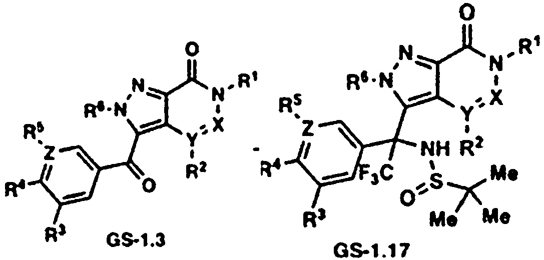 wobei R1, R2, R3, R4, R5, R6, R7, X, Y und Z sind wie in Anspruch 1 definiert.

## Revendications

1. Composé selon la formule (I) : 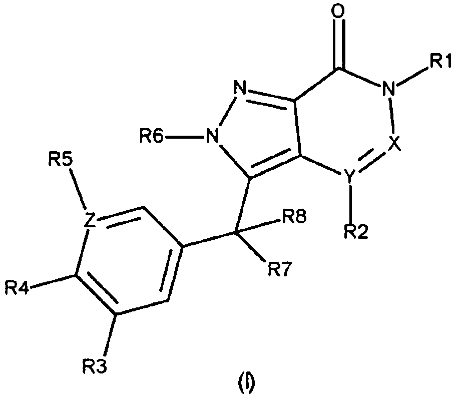 dans laquelle R1 est choisi parmi un alkyle en C₁-C₄ éventuellement substitué et un cycloalkyle en C₃-C₆ éventuellement substitué ; X est choisi parmi N ou CR9 ; Y-R2 est CR2 ou N ; R2 est choisi parmi H, halogène, un alkyle en C₁-C₄ éventuellement substitué et un cycloalkyle en C₃-C₆ éventuellement substitué ; R3, R4 et R5 sont choisis indépendamment parmi Cl, F, CF₃, CN et H ; Z-R5 est choisi parmi azote et CR5 ; R6 est choisi parmi un alkyle en C₁-C₄ éventuellement substitué et un cycloalkyle en C₃-C₆ éventuellement substitué ; R7 est choisi parmi H, un alkyle en C₁-C₄ éventuellement substitué et un cycloalkyle en C₃-C₆ éventuellement substitué ; R8 est choisi parmi OH et NH₂ ; R9 est choisi parmi H et un alkyle en C₁-C₄ éventuellement substitué ; ainsi que ses sels, tautomères, polymorphes, mélanges racémiques, et formes optiquement actives pharmaceutiquement acceptables, où le terme « substitué » désigne des groupes substitués par 1 à 5 substituants choisis dans le groupe consistant en « alkyle en C₁-C₆ », « cycloalkyle en C₃-C₈ », « hétérocycloalkyle », « alkyle en C₁-C₆-cycloalkyle en C₃-C₈ », « alkyle en C₁-C₆-hétérocycloalkyle », « amino », « ammonium », « aryle », « hétéroaryle », « alcoxy », « halogène », trihalogénométhyle, cyano et hydroxy.

2. Composé selon la revendication 1, dans lequel R1 est un alkyle en C₁-C₄ éventuellement substitué.

3. Composé selon la revendication 1, dans lequel R1 est un cycloalkyle en C₃-C₆ éventuellement substitué.

4. Composé selon l'une quelconque des revendications 1 à 3, dans lequel Y-R2 est N et X est CR9.

5. Composé selon l'une quelconque des revendications 1 à 3, dans lequel X est N et Y-R2 est CR2.

6. Composé selon l'une quelconque des revendications précédentes, dans lequel ZR5 est N.

7. Composé selon l'une quelconque des revendications 1 à 5, dans lequel ZR5 est CR5.

8. Composé selon l'une quelconque des revendications précédentes, dans lequel R4 est CF₃.

9. Composé selon l'une quelconque des revendications précédentes, dans lequel R5 est H.

10. Composé selon l'une quelconque des revendications précédentes, dans lequel R7 est un alkyle en C₁-C₄ éventuellement substitué.

11. Composé selon l'une quelconque des revendications 1 à 9, dans lequel R7 est un cycloalkyle en C₃-C₆ éventuellement substitué.

12. Composé selon l'une quelconque des revendications précédentes, dans lequel R8 est OH.

13. Composé selon l'une quelconque des revendications 1 à 11, dans lequel R8 est NH₂.

14. Composé selon l'une quelconque des revendications 1 à 13 choisi dans le groupe suivant :
(R)-3-(1-(5-chloro-6-(trifluorométhyl)pyridin-3-yl)-1-hydroxy-2-méthylpropyl)-6-cyclopropyl-2-méthyl-2,6-dihydro-7H-pyrazolo[3,4-c]pyridin-7-one ;
(R)-5-(cyclopropyl(6-cyclopropyl-2-méthyl-7-oxo-6,7-dihydro-2H-pyrazolo[3,4-c]pyridin-3-yl) (hydroxy)méthyl)-2-(trifluorométhyl)benzonitrile ;
(R)-5-(1-(6-cyclopropyl-2-méthyl-7-oxo-6,7-dihydro-2H-pyrazolo[3,4-c]pyridin-3-yl)-1-hydroxypropyl)-2-(trifluorométhyl)benzonitrile ;
3-(1-(5-chloro-6-(trifluorométhyl)pyridin-3-yl)-1-hydroxypropyl)-6-cyclopropyl-2,5-diméthyl-2,6-dihydro-7H-pyrazolo[3,4-c]pyridin-7-one ;
(R)-3-(1-(3-chloro-4-fluorophényl)-1-hydroxyéthyl)-6-cyclopropyl-2-méthyl-2,6-dihydro-7H-pyrazolo[3,4-c]pyridin-7-one ;
(R)-3-(1-(5-chloro-6-(trifluorométhyl)pyridin-3-yl)-1-hydroxyéthyl)-6-cyclopropyl-2-méthyl-2,6-dihydro-7H-pyrazolo[3,4-c]pyridin-7-one ;
(R)-3-(1-(3-chloro-4-fluorophényl)-1-hydroxypropyl)-6-cyclopropyl-2-méthyl-2,6-dihydro-7H-pyrazolo[3,4-c]pyridin-7-one ;
(R)-3-(1-(5-chloro-6-(trifluorométhyl)pyridin-3-yl)-1-hydroxypropyl)-6-cyclopropyl-2-méthyl-2,6-dihydro-7H-pyrazolo[3,4-c]pyridin-7-one ;
(R)-3-((5-chloro-6-(trifluorométhyl)pyridin-3-yl)(cyclopropyl) (hydroxy)méthyl)-6-cyclopropyl-2-méthyl-2,6-dihydro-7H-pyrazolo[3,4-c]pyridin-7-one ;
(R)-3-((3-chloro-4-fluorophényl)(cyclopropyl)(hydroxy)méthyl)-6-cyclopropyl-2-méthyl-2,6-dihydro-7H-pyrazolo[3,4-c]pyridin-7-one ;
(R)-3-(1-(4-chloro-3,5-difluorophényl)-1-hydroxypropyl)-6-cyclopropyl-2-méthyl-2,6-dihydro-7H-pyrazolo[3,4-c]pyridin-7-one ;
(R)-6-cyclopropyl-3-(1-hydroxy-1-(6-(trifluorométhyl)pyridin-3-yl)propyl)-2-méthyl-2,6-dihydro-7H-pyrazolo[3,4-c]pyridin-7-one ;
(R)-6-éthyl-4-fluoro-3-(1-hydroxy-1-(6-(trifluorométhyl)pyridin-3-yl)propyl)-2-méthyl-2,6-dihydro-7H-pyrazolo[3,4-c]pyridin-7-one ;
(R)-6-cyclopropyl-3-(cyclopropyl(hydroxy)(6-(trifluorométhyl)pyridin-3-yl)méthyl)-2-méthyl-2,6-dihydro-7H-pyrazolo[3,4-c]pyridin-7-one ;
(R)-3-((5-chloro-6-(trifluorométhyl)pyridin-3-yl)(hydroxy)méthyl)-4-cyclopropyl-6-éthyl-2-méthyl-2,6-dihydro-7H-pyrazolo[3,4-c]pyridin-7-one ;
(R)-3-((4-chloro-3-fluorophényl)(hydroxy)méthyl)-6-éthyl-2,4-diméthyl-2,6-dihydro-7H-pyrazolo[3,4-c]pyridin-7-one ;
(R)-3-((5-chloro-6-(trifluorométhyl)pyridin-3-yl)(cyclopropyl)(hydroxy)méthyl)-6-éthyl-2-méthyl-2,6-dihydro-7H-pyrazolo[3,4-c]pyridin-7-one ;
(R)-3-(1-(3-chloro-4-fluorophényl)-1-hydroxypropyl)-6-éthyl-2-méthyl-2,6-dihydro-7H-pyrazolo[3,4-c]pyridin-7-one ;
(R)-3-(1-(5-chloro-6-(trifluorométhyl)pyridin-3-yl)-1-hydroxyéthyl)-6-éthyl-2-méthyl-2,6-dihydro-7H-pyrazolo[3,4-c]pyridin-7-one ;
(R)-3-((3-chloro-4-fluorophényl)(cyclopropyl) (hydroxy)méthyl)-6-éthyl-2-méthyl-2,6-dihydro-7H-pyrazolo[3,4-c]pyridin-7-one ;
(R)-3-(1-(3-chloro-4-fluorophényl)-1-hydroxyéthyl)-6-éthyl-2-méthyl-2,6-dihydro-7H-pyrazolo[3,4-c]pyridin-7-one ;
(R)-3-(1-(5-chloro-6-(trifluorométhyl)pyridin-3-yl)-1-hydroxypropyl)-6-éthyl-2-méthyl-2,6-dihydro-7H-pyrazolo[3,4-c]pyridin-7-one ;
(R)-3-(cyclopropyl(hydroxy)(6-(trifluorométhyl)pyridin-3-yl)méthyl)-6-éthyl-2-méthyl-2,6-dihydro-7H-pyrazolo[3,4-c]pyridin-7-one ;
(R)-3-(1-(3-chloro-4-(trifluorométhyl)phényl)-1-hydroxyéthyl)-6-éthyl-2-méthyl-2,6-dihydro-7H-pyrazolo[3,4-c]pyridin-7-one ;
(R)-6-éthyl-3-(1-hydroxy-2-méthyl-1-(6-(trifluorométhyl)pyridin-3-yl)propyl)-2-méthyl-2,6-dihydro-7H-pyrazolo[3,4-c]pyridin-7-one ;
(R)-6-éthyl-3-(1-hydroxy-1-(6-(trifluorométhyl)pyridin-3-yl)propyl)-2-méthyl-2,6-dihydro-7H-pyrazolo[3,4-c]pyridin-7-one ;
(R)-3-(1-(5-chloro-6-(trifluorométhyl)pyridin-3-yl)-1-hydroxypropyl)-2,6-dicyclopropyl-2,6-dihydro-7H-pyrazolo[3,4-d]pyridazin-7-one ;
(R)-3-(1-(5-chloro-6-(trifluorométhyl)pyridin-3-yl)-1-hydroxypropyl)-6-cyclopropyl-2-éthyl-2,6-dihydro-7H-pyrazolo[3,4-d]pyridazin-7-one ;
(R)-5-(1-(6-cyclopropyl-2-méthyl-7-oxo-6,7-dihydro-2H-pyrazolo[3,4-d]pyridazin-3-yl)-1-hydroxypropyl)-2-(trifluorométhyl)benzonitrile ;
(R)-5-(cyclopropyl(6-cyclopropyl-2-méthyl-7-oxo-6,7-dihydro-2H-pyrazolo[3,4-d]pyridazin-3-yl)(hydroxy)méthyl)-2-(trifluorométhyl)benzonitrile ;
(R)-3-((5-chloro-6-(trifluorométhyl)pyridin-3-yl)(cyclopropyl) (hydroxy)méthyl)-6-cyclopropyl-2-méthyl-2,6-dihydro-7H-pyrazolo[3,4-d]pyridazin-7-one ;
(R)-3-((3-chloro-4-(trifluorométhyl)phényl)(cyclopropyl)(hydroxy)méthyl)-6-cyclopropyl-2-méthyl-2,6-dihydro-7H-pyrazolo[3,4-d]pyridazin-7-one ;
(R)-3-(1-(5-chloro-6-(trifluorométhyl)pyridin-3-yl)-1-hydroxypropyl)-6-cyclopropyl-2-méthyl-2,6-dihydro-7H-pyrazolo[3,4-d]pyridazin-7-one ;
(R)-3-(1-(3-chloro-4-(trifluorométhyl)phényl)-1-hydroxypropyl)-6-cyclopropyl-2-méthyl-2,6-dihydro-7H-pyrazolo[3,4-d]pyridazin-7-one ;
(R)-3-(1-(3-chloro-4-fluorophényl)-1-hydroxypropyl)-6-cyclopropyl-2-méthyl-2,6-dihydro-7H-pyrazolo[3,4-d]pyridazin-7-one ;
(R)-3-(1-(3-chloro-4-(trifluorométhyl)phényl)-1-hydroxypropyl)-6-éthyl-2-méthyl-2,6-dihydro-7H-pyrazolo[3,4-d]pyridazin-7-one ;
(R)-3-(1-(5-chloro-6-(trifluorométhyl)pyridin-3-yl)-1-hydroxyéthyl)-6-cyclopropyl-2-méthyl-2,6-dihydro-7H-pyrazolo[4,3-d]pyrimidin-7-one ;
(R)-3-((5-chloro-6-(trifluorométhyl)pyridin-3-yl)(cyclopropyl) (hydroxy)méthyl)-6-cyclopropyl-2-méthyl-2,6-dihydro-7H-pyrazolo[4,3-d]pyrimidin-7-one ;
(R)-3-(1-(5-chloro-6-(trifluorométhyl)pyridin-3-yl)-1-hydroxypropyl)-6-cyclopropyl-2-méthyl-2,6-dihydro-7H-pyrazolo[4,3-d]pyrimidin-7-one ;
(R)-6-cyclopropyl-3-(1-hydroxy-1-(6-(trifluorométhyl)pyridin-3-yl)propyl)-2-méthyl-2,6-dihydro-7H-pyrazolo[4,3-d]pyrimidin-7-one ;
(R)-6-cyclopropyl-3-(cyclopropyl(hydroxy)(6-(trifluorométhyl)pyridin-3-yl)méthyl)-2-méthyl-2,6-dihydro-7H-pyrazolo[4,3-d]pyrimidin-7-one ;
(R)-5-(1-(6-éthyl-2-méthyl-7-oxo-6,7-dihydro-2H-pyrazolo[4,3-d]pyrimidin-3-yl)-1-hydroxyéthyl)-2-(trifluorométhyl)benzonitrile ;
(R)-3-(1-amino-1-(5-chloro-6-(trifluorométhyl)pyridin-3-yl)-2,2,2-trifluoroéthyl)-6-cyclopropyl-2-méthyl-2,6-dihydro-7H-pyrazolo[3,4-c]pyridin-7-one ;
(R)-3-(1-amino-1-(3-chloro-4-(trifluorométhyl)phényl)éthyl)-6-cyclopropyl-2-méthyl-2,6-dihydro-7H-pyrazolo[3,4-c]pyridin-7-one ;
(R)-3-(1-amino-1-(3-fluoro-4-(trifluorométhyl)phényl)propyl)-6-cyclopropyl-2-méthyl-2,6-dihydro-7H-pyrazolo[3,4-c]pyridin-7-one ;
(R)-3-(1-amino-1-(3-chloro-4-(trifluorométhyl)phényl)propyl)-6-cyclopropyl-2-méthyl-2,6-dihydro-7H-pyrazolo[3,4-c]pyridin-7-one ;
(R)-3-(amino(5-chloro-6-(trifluorométhyl)pyridin-3-yl)(cyclopropyl)méthyl)-6-cyclopropyl-2-méthyl-2,6-dihydro-7H-pyrazolo[3,4-c]pyridin-7-one ;
(R)-3-(1-amino-1-(5-chloro-6-(trifluorométhyl)pyridin-3-yl)propyl)-6-cyclopropyl-2-méthyl-2,6-dihydro-7H-pyrazolo[3,4-c]pyridin-7-one ;
(R)-5-(amino(6-éthyl-2,4-diméthyl-7-oxo-6,7-dihydro-2H-pyrazolo[3,4-c]pyridin-3-yl)méthyl)-2-(trifluorométhyl)benzonitrile ;
(R)-3-(amino(3-chloro-4-(trifluorométhyl)phényl)(cyclopropyl)méthyl)-6-cyclopropyl-2-méthyl-2,6-dihydro-7H-pyrazolo[3,4-d]pyridazin-7-one ;
(R)-3-(1-amino-1-(3-chloro-4-(trifluorométhyl)phényl)propyl)-6-cyclopropyl-2-méthyl-2,6-dihydro-7H-pyrazolo[3,4-d]pyridazin-7-one ;
(R)-3-(1-amino-1-(3-chloro-4-(trifluorométhyl)phényl)éthyl)-6-cyclopropyl-2-méthyl-2,6-dihydro-7H-pyrazolo[4,3-d]pyrimidin-7-one ;
(R)-3-(1-amino-1-(3-chloro-4-fluorophényl)propyl)-6-cyclopropyl-2-méthyl-2,6-dihydro-7H-pyrazolo[4,3-d]pyrimidin-7-one ;
(R)-3-(amino(3-chloro-4-(trifluorométhyl)phényl)(cyclopropyl)méthyl)-6-cyclopropyl-2-méthyl-2,6-dihydro-7H-pyrazolo[4,3-d]pyrimidin-7-one ;
(R)-3-(1-amino-1-(3-chloro-4-(trifluorométhyl)phényl)propyl)-6-cyclopropyl-2-méthyl-2,6-dihydro-7H-pyrazolo[4,3-d]pyrimidin-7-one ;
(R)-3-(1-amino-1-(5-chloro-6-(trifluorométhyl)pyridin-3-yl)propyl)-6-cyclopropyl-2-méthyl-2,6-dihydro-7H-pyrazolo[4,3-d]pyrimidin-7-one ;
(R)-3-(1-(5-chloro-6-(trifluorométhyl)pyridin-3-yl)-2,2,2-trifluoro-1-hydroxyéthyl)-6-cyclopropyl-2-méthyl-2,6-dihydro-7H-pyrazolo[3,4-d]pyridazin-7-one ;
(R)-3-(1-(5-chloro-6-(trifluorométhyl)pyridin-3-yl)-2,2,2-trifluoro-1-hydroxyéthyl)-6-cyclopropyl-2-méthyl-2,6-dihydro-7H-pyrazolo[4,3-d]pyrimidin-7-one ;
(R)-3-(1-(5-chloro-6-(trifluorométhyl)pyridin-3-yl)-2,2,2-trifluoro-1-hydroxyéthyl)-6-cyclopropyl-2-méthyl-2,6-dihydro-7H-pyrazolo[3,4-c]pyridin-7-one ;
(R)-3-(1-(3-chloro-4-(trifluorométhyl)phényl)-2,2,2-trifluoro-1-hydroxyéthyl)-6-cyclopropyl-2-méthyl-2,6-dihydro-7H-pyrazolo[3,4-c]pyridin-7-one ;
(R)-3-(1-(3-chloro-4-fluorophényl)-2,2,2-trifluoro-1-hydroxyéthyl)-6-cyclopropyl-2-méthyl-2,6-dihydro-7H-pyrazolo[3,4-c]pyridin-7-one ;
(R)-6-cyclopropyl-2-méthyl-3-(2,2,2-trifluoro-1-hydroxy-1-(6-(trifluorométhyl)pyridin-3-yl)éthyl)-2,6-dihydro-7H-pyrazolo[3,4-c]pyridin-7-one ;
(R)-5-(1-(6-cyclopropyl-2-méthyl-7-oxo-6,7-dihydro-2H-pyrazolo[3,4-d]pyridazin-3-yl)-2,2,2-trifluoro-1-hydroxyéthyl)-2-(trifluorométhyl)benzonitrile ;
(R)-3-(1-(3-chloro-4-(trifluorométhyl)phényl)-2,2,2-trifluoro-1-hydroxyéthyl)-6-cyclopropyl-2-méthyl-2,6-dihydro-7H-pyrazolo[3,4-d]pyridazin-7-one ;
(R)-3-(1-(3-chloro-4-(trifluorométhyl)phényl)-2,2,2-trifluoro-1-hydroxyéthyl)-6-cyclopropyl-2-méthyl-2,6-dihydro-7H-pyrazolo[4,3-d]pyrimidin-7-one ;
(R)-5-(1-(6-cyclopropyl-2-méthyl-7-oxo-6,7-dihydro-2H-pyrazolo[4,3-d]pyrimidin-3-yl)-2,2,2-trifluoro-1-hydroxyéthyl)-2-(trifluorométhyl)benzonitrile ;
(R)-6-cyclopropyl-2-méthyl-3-(2,2,2-trifluoro-1-hydroxy-1-(6-(trifluorométhyl)pyridin-3-yl)éthyl)-2,6-dihydro-7H-pyrazolo[4,3-d]pyrimidin-7-one ;
(R)-3-(1-(5-chloro-6-(trifluorométhyl)pyridin-3-yl)-2,2-difluoro-1-hydroxyéthyl)-6-cyclopropyl-2-méthyl-2,6-dihydro-7H-pyrazolo[4,3-d]pyrimidin-7-one ;
(R)-3-(1-(3-chloro-4-(trifluorométhyl)phényl)-2,2,2-trifluoro-1-hydroxyéthyl)-2-méthyl-6-(2,2,2-trifluoroéthyl)-2,6-dihydro-7H-pyrazolo[4,3-d]pyrimidin-7-one ;
(R)-3-(1-(3-chloro-4-(trifluorométhyl)phényl)-2,2,2-trifluoro-1-hydroxyéthyl)-6-isopropyl-2-méthyl-2,6-dihydro-7H-pyrazolo[4,3-d]pyrimidin-7-one ;
(R)-5-(1-amino-1-(6-cyclopropyl-2-méthyl-7-oxo-6,7-dihydro-2H-pyrazolo[4,3-d]pyrimidin-3-yl)-2,2,2-trifluoroéthyl)-2-(trifluorométhyl)benzonitrile ;
(R)-5-(1-amino-1-(6-cyclopropyl-2-méthyl-7-oxo-6,7-dihydro-2H-pyrazolo[3,4-c]pyridin-3-yl)-2,2,2-trifluoroéthyl)-2-(trifluorométhyl)benzonitrile ;
(R)-3-(1-amino-1-(3-chloro-4-(trifluorométhyl)phényl)-2,2,2-trifluoroéthyl)-6-cyclopropyl-2-méthyl-2,6-dihydro-7H-pyrazolo[3,4-c]pyridin-7-one ;
(R)-3-(1-amino-1-(3-chloro-4-(trifluorométhyl)phényl)-2,2,2-trifluoroéthyl)-6-cyclopropyl-2-méthyl-2,6-dihydro-7H-pyrazolo[4,3-d]pyrimidin-7-one ;
(R)-5-(1-(6-cyclopropyl-2-méthyl-7-oxo-6,7-dihydro-2H-pyrazolo[4,3-d]pyrimidin-3-yl)-2,2,2-trifluoro-1-hydroxyéthyl)-2-(trifluorométhyl)nicotinonitrile ;
(R)-5-(1-(6-cyclopropyl-2-méthyl-7-oxo-6,7-dihydro-2H-pyrazolo[4,3-d]pyrimidin-3-yl)-2,2,2-trifluoro-1-hydroxyéthyl)-2-(difluorométhyl)benzonitrile et
(R)-5-(1-(6-cyclopropyl-2-méthyl-7-oxo-6,7-dihydro-2H-pyrazolo[4,3-d]pyrimidin-3-yl)-2,2,2-trifluoro-1-hydroxyéthyl)-2-(trifluorométhyl)benzonitrile
ainsi que leurs sels, hydrates, solvates, tautomères, polymorphes, mélanges racémiques, formes optiquement actives pharmaceutiquement acceptables.

15. Composé selon l'une quelconque des revendications 1 à 14 pour l'utilisation en tant que médicament.

16. Composé selon l'une quelconque des revendications 1 à 14 pour l'utilisation dans la prévention ou le traitement du paludisme.

17. Formulation pharmaceutique contenant au moins un composé selon l'une quelconque des revendications 1 à 14 et un véhicule, diluant ou excipient de celui-ci pharmaceutiquement acceptable.

18. Formulation pharmaceutique selon la revendication 17, comprenant en outre au moins un co-agent utile dans le traitement et/ou la prévention du paludisme choisi parmi l'artémisinine et ses dérivés tels que l'artéméther, l'artésunate, la dihydroartémisine, la chloroquine, l'hydroxychloroquine, la quinine, la méfloquine, l'amodiaquine, l'atovaquone/le proguanil, la doxycycline, la clindamycine, l'halofantrine, la luméfantrine, la pyronaridine, la pyriméthamine-sulfadoxine, la ferroquine, la tafénoquine, la pipéraquine et la primaquine.

19. Formulation pharmaceutique selon la revendication 17 ou 18, comprenant en outre au moins un co-agent utile dans le traitement et/ou la prévention du paludisme choisi parmi la spiro[3H-indole-3,1'-[1H]pyrido[3,4-b]indol]-2(1H)-one, la 5,7'-dichloro-6'-fluoro-2',3',4',9'-tétrahydro-3'-méthyl-(1'R,3'S)-(numéro d'enregistrement CAS : 1193314-23-6), la 2-(1,1-difluoroéthyl)-5-méthyl-N-[4-pentafluoro-λ⁶-sulfanyl)phényl]-[1,2,4]triazolo[1,5-a]pyrimidin-7-amine (numéro d'enregistrement CAS : 1282041-94-4), la [3,3'-bipyridine]-2-amine, la 5-[4-(méthylsulfonyl)phényl]-6'-(trifluorométhyl)-(numéro d'enregistrement CAS : 1314883-11-8), l'éthanone, la 2-amino-1-[2-(4-fluorophényl)-3-[(4-fluorophényl)amino]-5,6-dihydroimidazo-[1,2-a]pyrazin-7(8H)-yl]-(numéro d'enregistrement CAS : 1261109-90-3).

20. Méthode de préparation d'un composé selon la formule (I) ou d'un sel pharmaceutiquement acceptable de celui-ci, ladite méthode comprenant : 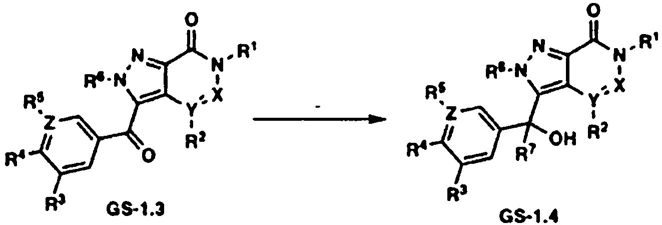 la transformation d'un composé selon la formule GS-1.3 en un composé de G-S1.4 comme décrit sous le schéma 6 en présence d'un alkyle de Grignard ou d'un alkyllithium, où R1, R2, R3, R4, R5, R6, R7, X, Y et Z sont tels que définis selon la revendication 1.

21. Méthode de préparation d'un composé selon la formule (I) comprenant une étape de transformation d'un composé selon la formule GS-1.3 en un composé de GS-1.9 après hydrolysation acide en présence de trifluorométhyltriméthylsilane : 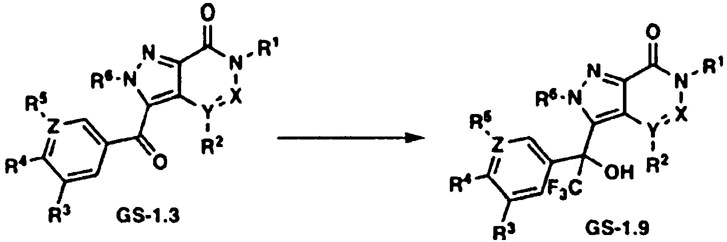 où R1, R2, R3, R4, R5, R6, R7, X, Y et Z sont tels que définis selon la revendication 1.

22. Méthode de préparation d'un composé selon la formule (I) comprenant une étape de transformation d'un composé selon la formule GS-1.13 en un composé de GS-1.14 après hydrolysation acide : 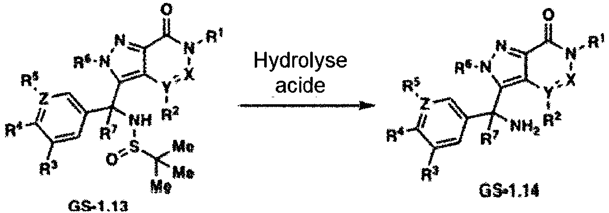 où R1, R2, R3, R4, R5, R6, R7, X, Y et Z sont tels que définis selon la revendication 1.

23. Méthode de préparation d'un composé selon la formule (I) comprenant une étape de transformation d'un composé selon la formule GS-1.17 en un composé de GS-1.18 après hydrolysation acide : 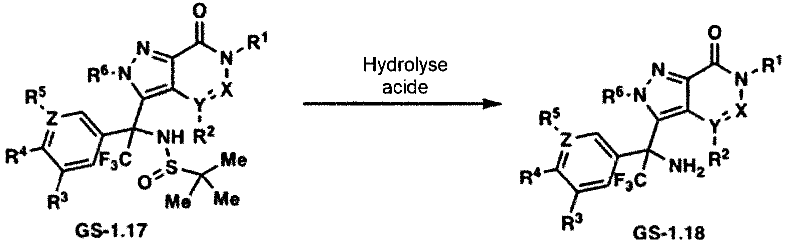 où R1, R2, R3, R4, R5, R6, R7, X, Y et Z sont tels que définis selon la revendication 1.

24. Intermédiaire d'une formule choisie parmi les formules GS-1.3, et GS-1.17. 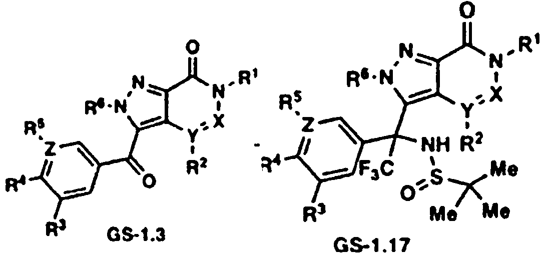 où R1, R2, R3, R4, R5, R6, X, Y et Z sont tels que définis selon la revendication 1.
